(19) Europäisches Patentamt
European Patent Office
Office européen des brevets

(11) EP 4 513 509 A2

(12) # EUROPEAN PATENT APPLICATION

(43) Date of publication:
**26.02.2025 Bulletin 2025/09**

(21) Application number: **24213821.2**

(22) Date of filing: **16.07.2021**

(51) International Patent Classification (IPC):
***G16H 50/20*** *(2018.01)*

(52) Cooperative Patent Classification (CPC):
**G16B 40/20; A61K 39/0011; G16B 15/30;**
G16H 50/20

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **17.07.2020 US 202063053307 P**

(62) Document number(s) of the earlier application(s) in accordance with Art. 76 EPC:
**21752405.7 / 4 182 924**

(71) Applicant: **Genentech Inc.**
**South San Francisco, California 94080-4990 (US)**

(72) Inventors:
- **LIU, Kai**
  **San Francisco, California 94080-4990 (US)**
- **LOUNSBURY, Nicolas, Winston**
  **San Francisco, California 94080-4990 (US)**
- **THRIFT, William, John**
  **San Francisco, California 94080-4990 (US)**
- **BROADWELL, Adric, Quade**
  **San Francisco, California 94080-4990 (US)**
- **DELAMARRE, Lélia, Yvonne**
  **San Francisco, California 94080-4990 (US)**
- **JHUNJHUNWALA, Suchit, Sushil**
  **San Francisco, California 94080-4990 (US)**

(74) Representative: **Hamer, Thomas Daniel et al**
**Kilburn & Strode LLP**
**Lacon London**
**84 Theobalds Road**
**London WC1X 8NL (GB)**

Remarks:
This application was filed on 19.11.2024 as a divisional application to the application mentioned under INID code 62.

(54) **ATTENTION-BASED NEURAL NETWORK TO PREDICT PEPTIDE BINDING, PRESENTATION, AND IMMUNOGENICITY**

(57) Embodiments disclosed herein generally relate to using an attention-based machine learning model to generate an output that includes at least one of an interaction prediction for a target interaction, an interaction affinity prediction, or an immunogenicity prediction relating to a target interaction for a corresponding peptide-immunoprotein complex (IPC) combination. A target interaction may be between a peptide and an immunogenicity complex (IPC) such as, for example, a major histocompatibility complex (MHC), a T cell receptor (TCR), or both. A pharmaceutical composition may be identified, manufactured, and/or used that includes one or more peptides for which one or more target interactions are predicted to be more likely. Methods of treatment may be defined and/or used that include administration of such a pharmaceutical composition.

EP 4 513 509 A2

## Description

### CROSS-REFERENCE TO RELATED APPLICATION

The present application claims priority to U.S. Provisional Application No. 63/053,307, filed July 17, 2020, entitled "Attention-Based Neural Network to Predict Peptide Binding, Presentation, and Immunogenicity," and is related to International Patent Application No. _, Attorney Docket No. 59868.23WO01, filed even date hereof, entitled "Attention-Based Neural Network to Predict Peptide Binding, Presentation, and Immunogenicity," both of which are incorporated by reference herein in their entirety.

### FIELD

This present disclosure generally relates to using machine-learning models (e.g., that include an attention mechanism) to generate predictions relating to whether peptides (e.g., mutant peptide) of interest will experience a target interaction(s) with an immunoprotein complex (IPC) (e.g., be bound to an MHC molecule, presented by an MHC molecule, be bound to a TCR, etc.), the affinity associated with such a target interaction(s), and/or the ability of the peptides to trigger an immune response. This present disclosure further relates to compositions that include and methods of using certain mutant peptides (or associated precursors or sequences) selected based on such predictions for treatment.

### BACKGROUND

Neoantigen vaccines are a relatively new approach for providing individualized cancer treatment. Neoantigens are tumor-specific antigens that are derived from somatic mutations in tumors and are presented by a subject's cancer cells and antigen presenting cells.

Neoantigen vaccines can prime a subject's T cells to recognize and attack cancer cells expressing one or more particular tumor neoantigens. This approach generates a tumor-specific immune response that spares healthy cells while targeting tumor cells. However, there is high variability across subjects as to which neoantigens are both produced by the subject's tumor cells and presented by the subject's major histocompatibility complex (MHC) molecules. Thus, an individualized vaccine may be developed and used for a particular subject. The individualized vaccine may be engineered or selected based on a subject-specific tumor profile. The tumor profile can be defined by determining DNA and/or RNA sequences from a subject's tumor cell and using the sequences to identify antigens that are present in tumor cells but absent in normal cells.

In many cases, the vast majority of mutant sequences that are detected in tumor cells correspond to neoantigens that are not actually presented on the tumor cell surface. Such neoantigens would be poor candidates for an individualized vaccine. For example, a detected peptide sequence may identify amino acids in a mutant peptide that is produced intracellularly but fails to bind with and/or to be presented (at a cell's surface) by an MHC-I or MHC-II molecule. Alternatively, a mutant peptide capable of being presented by an MHC-I or MHC-II molecule may not be produced intracellularly. In either instance, the mutant peptide would fail to trigger an immunological response by, for example, a CD8+ cytotoxic T lymphocyte, in the case of the MHC-I molecule, or by a CD4+ helper T-cell, in the case of the MHC-II molecule.

Therefore, a sequence analysis for identifying neoantigen candidates for a vaccine that merely focuses on detecting mutant peptide sequences or predicting for which mutant peptide sequences a single biological interaction will occur (e.g., whether a peptide will bind to a molecule) may generate many false positives. This type of sequence analysis would be ineffective in developing individualized vaccines that are intended to prime immunological responses.

Thus, it may be desirable to predict which neoantigens are presented by a given subject's tumor cells and/or for which a vaccine including the neoantigen will trigger a strong immunological response.

### SUMMARY

In one or more embodiments, a method is provided. The method includes accessing a set of peptide sequences characterizing a set of peptides, each peptide sequence of the set of peptide sequences having been identified by processing a disease sample from a subject. The method includes accessing an immunoprotein complex (IPC) sequence identified for an immunoprotein complex (IPC) of the subject. The method includes processing a set of peptide representations that represents the set of peptide sequences using a first attention block in an initial attention subsystem of an attention-based machine-learning model and an immunoprotein complex (IPC) representation that represents the IPC sequence using a second attention block in the initial attention subsystem to generate an output, wherein the output includes at least one of an interaction prediction, an interaction affinity prediction, or an immunogenicity prediction for a corresponding peptide-IPC combination. The method includes generating a report based on the output.

In one or more embodiments, a vaccine comprises one or more peptides; a plurality of nucleic acids that encode the one or more peptides; or a plurality of cells expressing the one or more peptides. The one or more peptides are selected from among the set of peptides based on the report generated by part or all of one or more methods disclosed herein. The one or more peptides are an incomplete subset of the set of peptides.

In one or more embodiments, a method is provided for manufacturing a vaccine. The method includes producing a vaccine comprising: one or more peptides; a plurality of nucleic acids that encode the one or more peptides; or a plurality of cells expressing the one or more peptides. The one or more peptides are selected from among the set of peptides based on the report generated by part or all of one or more methods disclosed herein. The one or more peptides are an incomplete subset of the set of peptides.

In one or more embodiments, a pharmaceutical composition is provided that comprises one or more peptides selected from among the set of peptides based on the report generated by the part or all of one or more methods disclosed herein. The one or more peptides are an incomplete subset of the set of peptides.

In one or more embodiments, a pharmaceutical composition is provided that comprises a nucleic acid sequence that encodes one or more peptides having been selected from among the set of peptides based on the report generated by part or all of one or more methods disclosed herein. The one or more peptides are an incomplete subset of the set of peptides.

In one or more embodiments, an immunogenic peptide is provided that is identified based on the report generated by part or all of one or more methods disclosed herein.

In one or more embodiments, a nucleic acid sequence is provided that is identified based on the report generated by part or all of one or more methods disclosed herein.

In one or more embodiments, a method of treating a subject is provided. The method includes administering at least one of one or more peptides, one or more pharmaceutical compositions, or one or more nucleic acid sequences identified based on the report generated by part or all of one or more methods disclosed herein.

In one or more embodiments, a method is provided that includes processing a set of biological samples obtained from a subject to generate a set of peptide sequences characterizing a set of peptides. The method includes processing the set of biological samples obtained from the subject to generate an immunoprotein complex (IPC) sequence identified for an immunoprotein complex (IPC) of the subject. The method includes generating a set of peptide representations that represents the set of peptide sequences using a first attention block in an initial attention subsystem of an attention-based machine-learning model. The method includes generating an immunoprotein complex (IPC) representation that represents the IPC sequence using a second attention block in the initial attention subsystem. The method includes processing the set of peptide representations and the IPC representation to generate an output, wherein the output includes at least one of an interaction prediction, an interaction affinity prediction, or an immunogenicity prediction for a corresponding peptide-IPC combination, the corresponding peptide-IPC combination including a peptide of the set of peptides.

In one or more embodiments, a method is provided. The method includes receiving, at a user device, a request to design an individualized vaccine for a subject. The method includes transmitting, from the user device, a communication to a remote system, the communication including an identifier of the subject. The remote system is configured to: access a set of peptide sequences characterizing a set of peptides, each peptide sequence of the set of peptide sequences having been identified by processing a disease sample from a subject; access an immunoprotein complex (IPC) sequence identified for an immunoprotein complex (IPC) of the subject; and process a set of peptide representations that represents the set of peptide sequences using a first attention block in an initial attention subsystem of an attention-based machine-learning model and an immunoprotein complex (IPC) representation that represents the IPC sequence using a second attention block in the initial attention subsystem to generate an output. The output includes at least one of an interaction prediction, an interaction affinity prediction, or an immunogenicity prediction for a corresponding peptide-IPC combination. The remote system is configured to generate a report based on the output; and transmit the report to the user device. The method includes receiving, at the user device, the report.

In one or more embodiments, a method is provided for manufacturing a treatment for a subject. The method includes method comprising receiving a report from a computing device. The computing device is configured to access a set of peptide sequences characterizing a set of peptides, each peptide sequence of the set of peptide sequences having been identified by processing a disease sample from a subject; access an immunoprotein complex (IPC) sequence identified for an immunoprotein complex (IPC) of the subject; and process a set of peptide representations that represents the set of peptide sequences using a first attention block in an initial attention subsystem of an attention-based machine-learning model and an immunoprotein complex (IPC) representation that represents the IPC sequence using a second attention block in the initial attention subsystem to generate an output. The output includes at least one of an interaction prediction, an interaction affinity prediction, or an immunogenicity prediction for a corresponding peptide-IPC combination. The computing device is configured to generate the report based on the output. The method further includes generating a treatment manufacturing plan for manufacturing the treatment based on the report.

In one or more embodiments, a method is provided that includes inputting a plurality of variant-coding sequences characterizing a plurality of mutant peptides into an attention-based machine-learning model, each variant-coding

sequence of the plurality of variant-coding sequences having been identified by processing a disease sample from a subject. The method includes inputting an immunoprotein complex (IPC) sequence identified for an immunoprotein complex (IPC) of the subject into the attention-based machine-learning model. The attention-based machine-learning model is configured to process a plurality of variant representations that represents the plurality of variant-coding sequences using a first attention block in an initial attention subsystem of an attention-based machine-learning model and an immunoprotein complex (IPC) representation that represents the IPC sequence using a second attention block in the initial attention subsystem to generate an output. The output includes at least one of an interaction prediction, an interaction affinity prediction, or an immunogenicity prediction for a corresponding mutant peptide-IPC combination. The method includes receiving a report generated based on the output; and selecting, based on the report, a subset of the plurality of mutant peptides to use in a treatment for the subject.

In one or more embodiments, a method is provided that includes receiving a peptide sequence that characterizes a mutant peptide, the peptide sequence including a variant with respect to a corresponding reference sequence; receiving an MHC sequence identified for a major histocompatibility complex (MHC); processing the peptide sequence and the MHC sequence using different processing paths within an attention-based machine-learning model to generate an output, wherein the output provides information about an immunological activity relating to both the mutant peptide and the MHC; and generating a report based on the output.

In one or more embodiments, a method is provided that includes receiving a peptide sequence that characterizes a mutant peptide, the peptide sequence including a variant with respect to a corresponding reference sequence; receiving a TCR sequence identified for a T cell receptor (TCR); processing the peptide sequence and the TCR sequence using different processing paths within an attention-based machine-learning model to generate an output, wherein the output provides information about an immunological activity relating to both the mutant peptide and the TCR; and generating a report based on the output.

In some embodiments, a system is provided that includes one or more data processors and a non-transitory computer readable storage medium containing instructions which, when executed on the one or more data processors, cause the one or more data processors to perform part or all of one or more methods disclosed herein.

In some embodiments, a computer-program product is provided that is tangibly embodied in a non-transitory machine-readable storage medium and that includes instructions configured to cause one or more data processors to perform part or all of one or more methods disclosed herein.

Some embodiments of the present disclosure include a system including one or more data processors. In some embodiments, the system includes a non-transitory computer readable storage medium containing instructions which, when executed on the one or more data processors, cause the one or more data processors to perform part or all of one or more methods and/or part or all of one or more processes disclosed herein. Some embodiments of the present disclosure include a computer-program product tangibly embodied in a non-transitory machine-readable storage medium, including instructions configured to cause one or more data processors to perform part or all of one or more methods and/or part or all of one or more processes disclosed herein.

The terms and expressions which have been employed are used as terms of description and not of limitation, and there is no intention in the use of such terms and expressions of excluding any equivalents of the features shown and described or portions thereof, but it is recognized that various modifications are possible within the scope of the invention claimed. Thus, it should be understood that although the present invention as claimed has been specifically disclosed by embodiments and optional features, modification and variation of the concepts herein disclosed may be resorted to by those skilled in the art, and that such modifications and variations are considered to be within the scope of this invention as defined by the appended claims.

## BRIEF DESCRIPTION OF THE DRAWINGS

The present disclosure is described in conjunction with the appended figures:

Fig. 1 is a block diagram of a prediction system in accordance with various embodiments.
Fig. 2 is a flowchart of a process for generating predictions using a machine learning model in accordance with one or more embodiments.
Fig. 3 is a schematic diagram of one configuration for the machine learning model from Fig. 1 in accordance with one or more embodiments.
Fig. 4A is a schematic diagram of a machine learning model 400 in accordance with one or more embodiments.
Fig. 4B is a schematic diagram of a different configuration for machine learning model 400 in accordance with one or more embodiments.
Fig. 4C is a schematic diagram of a different configuration for machine learning model 400 in accordance with one or more embodiments.
Fig. 5 is a schematic diagram of attention block 500 in accordance with one or more embodiments.

Fig. 6 is a flowchart of a process for processing a sequence representation using an exemplary self-attention layer in accordance with one or more embodiments.

Fig. 7 is a schematic diagram illustrating process 600 described in Fig. 6 above in accordance with one or more embodiments.

Fig. 8 is a flowchart of a process for generating information about the immunological activity of various peptides.

Fig. 9 is a flowchart of a process for generating information about the immunological activity of various peptides.

Fig. 10 is a flowchart of a process for training a machine learning model and using the trained machine learning model to generate predictions relating to peptides and MHCs in accordance with one or more embodiments

Fig. 11 is an illustration that includes a table of training data in accordance with one or more embodiments.

Fig. 12 is an illustration of a neoantigen candidate and the corresponding potential neoepitope candidates in accordance with one or more embodiments.

Fig. 13 is a flowchart of a process for training a machine learning model and using the trained machine learning model to generate predictions relating to peptides and TCRs in accordance with one or more embodiments.

Figs. 14A, 14B, and 14 C are plots with exemplary precision-recall (PR) curves in accordance with one or more embodiments.

Fig. 15 is a plot comparing exemplary average precision values of elution-ligand outputs of Model A and the P-MHC-I Model for each allele in a test data set in accordance with one or more embodiments.

Figs. 16A and 16B are plots that compare the performance of the P-MHC-I Model on a human dataset with the performance of the P-MHC-I Model on a mouse dataset in accordance with one or more embodiments.

Figs. 17A and 17B are plots that compare the performance of the P-MHC-II Model with Model C on the presentation data in accordance with one or more embodiments.

Figs. 18A and 18B are plots that compare the performance of the P-MHC-II Model with Model C, respectively, on a holdout dataset in accordance with one or more embodiments.

Fig. 19 is a plot showing a per genotype comparison of average precision for the P-MHC-II Model with Model C on a test dataset in accordance with one or more embodiments.

Fig. 20 is a plot of receiver operating characteristic (ROC) curves that illustrates performance of the P-MHC-I Model (EL output), Model A (EL output), and Model B (BA output) with respect to CD 8 multimer assay data (first test immunogenicity dataset) in accordance with one or more embodiments.

Figs. 21A-D are plots that illustrate the performance of the P-MHC-I Model (El output), Model A (EL output), and Model B (BA output) with respect to ELISpot assays (first test immunogenicity dataset) in accordance with one or more embodiments.

Figs. 22A-D are plots that illustrate the performance of Model A (BA output), Model A (EL output), Model C (BA output), and the P-MHC-I Model (EL output), respectively in accordance with one or more embodiments.

Fig. 23 is an illustration of a plot comparing ROC curves for the Model A (EL output), Model B (BA output), and P-MHC-I Model (EL output) using TESLA multimer assay data in accordance with one or more embodiments.

In the appended figures, similar components and/or features can have the same reference label. Further, various components of the same type can be distinguished by following the reference label by a dash and a second label that distinguishes among the similar components. If only the first reference label is used in the specification, the description is applicable to any one of the similar components having the same first reference label irrespective of the second reference label.

## DETAILED DESCRIPTION

### I. Overview

Recognizing the importance of being able to predict which mutant peptides (e.g., neoantigens) to select as candidates for an individualized vaccine, the embodiments described herein provide methodologies and systems for making such predictions more accurately than various currently available methods and systems. The embodiments described herein use machine-learning methodologies and systems to improve prediction performance by, for example, without limitation, reducing the number of false positives generated when analyzing sequences that characterize mutant peptides to determine the viability of those mutant peptides as vaccine candidates.

For example, the embodiments described herein provide a machine-learning model and various methodologies of using the machine-learning model and/or the output generated by the machine-learning model to analyze sequences identified from a disease sample from a subject. To predict whether a mutant peptide detected in the disease sample interacts with a major histocompatibility complex (MHC) molecule (e.g., MHC-I, MHC-II), predict the extent to which the mutant peptide interacts with the MHC molecule, or both, the machine-learning model initially processes a representation of a sequence characterizing the mutant peptide separately from the processing of a representation of an MHC sequence

corresponding to the MHC molecule. The sequence characterizing the mutant peptide may be referred to as a variant-coding sequence. The MHC sequence may be comprised of at least a portion of the full sequence of the MHC molecule (e.g., the full sequence, a pseudosequence of the MHC molecule that is the portion that interacts with the peptide-binding pocket, some other portion that includes the pseudosequence, etc.).

The machine-learning model includes various subsystems of processing. The machine-learning model may include, for example, a representation subsystem, a representation attention subsystem, a composite subsystem, a composite attention subsystem, and an output subsystem. Each "subsystem" may be comprised of one or more blocks, with each block being comprised of one or more sub-blocks and/or layers. A sub-block may be comprised of any number of layers (or units).

The representation subsystem may be used to generate a peptide representation of a peptide sequence (which may include a variant-coding sequence) and an MHC representation of the MHC sequence. The representation attention subsystem is used to process the representation of the peptide sequence independently of or separately from (e.g., in parallel) the representation of the MHC sequence. These two parallel processing paths may be configured similarly or differently, but each includes at least one attention mechanism. Processing the representations of the peptide sequence and the MHC sequence via these parallel processing paths improves the predictive performance of the machine-learning model.

Further, the embodiments described herein recognize and take into account that training a model corresponding to a series of biological events may require significantly more data than training a model corresponding to a single biological event. Training a model for sequence analysis may be particularly complicated due to the sheer number of sequences potentially observable. Not only are there millions of potential neoantigens, but genes encoding the proteins for MHC class-I molecules, for example, are also highly polymorphic: there are nearly 20,000 alleles of class-I human MHC. Thus, the embodiments described herein provide methodologies and systems for training the machine-learning model that both reduce a complexity of the training and improve training performance. For example, the variant-coding sequences used for training may be selected and/or trimmed such that training is performed using variant-coding sequences having an amino acid length at or below a threshold amino acid length (e.g., 14 amino acids). Generating a training dataset that includes variant-coding sequences having a length equal to or shorter than the threshold amino acid length may reduce the overall complexity of training as well as improve training and/or prediction performance (e.g., reduce variation in performance metrics per epoch to thereby improve prediction performance).

Accordingly, the techniques disclosed herein include machine-learning-based approaches for generating predictions relating to the immunological activity associated with a peptide, such as a mutant peptide. A machine learning model is provided that generates an output comprising one or more predictions. The output may, for example, generate one or more interaction predictions, one or more interaction affinity predictions, one or more immunogenicity predictions, or a combination thereof. An interaction prediction may include a prediction relating to whether a peptide (e.g., a mutant peptide, including a given ordered set of amino acids as identified by a given variant-coding sequence) experiences one or more target interactions. A target interaction may be, for example, binding to an IPC (e.g., an MHC molecule, a TCR), being presented by an MHC molecule at a cell surface, or another type of target interaction. An interaction affinity prediction may include a prediction of the affinity for one or more target interactions. For example, an interaction affinity prediction may indicate a binding affinity with respect to a peptide-MHC binding. An interaction (e.g., binding) affinity may be determined based on the tendency, strength, and/or stability of the interaction (e.g., binding).

Further, the output may include or indicate an immunogenicity of a peptide. For example, the output may predict whether a peptide will trigger an immune response in a particular subject or group of subj ects. These predictions can be generated for each of multiple mutant peptides, and the predictions can be used to select one or more mutant peptides to be included in a vaccine and/or used in treatment. For example, without limitation, mutant peptides associated with high predicted binding affinity, a high probability of being presented at tumor cell surfaces, and/or high predicted immunogenicity may be selected for inclusion in a vaccine or use in a treatment.

The embodiments described herein provide methods and systems for using an attention-based machine learning model to generate predictions about the immunological activity relating to peptides and immunoprotein complexes (IPCs). An IPC may be an MHC or a TCR. A set of peptide sequences characterizing a set of peptides may be accessed, each peptide sequence of the set of peptide sequences having been identified by processing a disease sample from a subject. An immunoprotein complex (IPC) sequence may be identified for an immunoprotein complex (IPC) of the subject. A set of peptide representations that represents the set of peptide sequences are processed using a first attention block in an initial attention subsystem of an attention-based machine-learning model and an immunoprotein complex (IPC) representation that represents the IPC sequence using a second attention block in the initial attention subsystem to generate an output. The output includes at least one of an interaction prediction, an interaction affinity prediction, or an immunogenicity prediction for a corresponding peptide-IPC combination. A report is generated based on the output.

The description below provides exemplary implementations of these methods and systems and ways in which the report that is generated may be used to plan for, design, and/or manufacture a treatment.

## II. Predictions Relating to Immunological Activity Involving Mutant Peptides using Attention-Based Machine-Learning Modeling

### II.A. Overview

Referring now to the figures, Fig. 1 is a block diagram of a prediction system 100 in accordance with various embodiments. Prediction system 100 is used to generate predictions relating to the immunological activity of peptides and, in particular, mutant peptides. Prediction system 100 includes computing platform 102, data store 104, and display system 106. Computing platform 102 may take various forms. In one or more embodiments, computing platform 102 includes a single computer (or computer system) or multiple computers in communication with each other. In other examples, computing platform 102 takes the form of a cloud computing platform.

Data store 104 and display system 106 are each in communication with computing platform 102. In some examples, data store 104, display system 106, or both may be considered part of or otherwise integrated with computing platform 102. Thus, in some examples, computing platform 102, data store 104, and display system 106 may be separate components in communication with each other, but in other examples, some combination of these components may be integrated together. Communication between the different components may be implemented using any number of wired communications links, wireless communications links, optical communications links, or a combination thereof.

Prediction system 100 includes sequence analyzer 108, which may be implemented using hardware, software, firmware, or a combination thereof. In one or more embodiments, sequence analyzer 108 is implemented in computing platform 102. Sequence analyzer 108 receives sequence data 110 for processing. For example, sequence data 110 may be sent as input into sequence analyzer 108, retrieved from data store 104 or some other type of storage (e.g., cloud storage), accessed from cloud storage, or obtained in some other manner. In some cases, sequence data 110 may be retrieved from data store 104 in response to receiving user input entered by a user via an input device.

Sequence data 110 may be generated from the processing of set of samples 112. Set of samples 112 may take the form of one or more biological samples from one or more subjects (e.g., a diseased sample, a healthy sample, a combination thereof). Set of samples 112 may include a sample obtained from a tumor of a subject. The tumor may be a manifestation of, for example, lung cancer, melanoma, breast cancer, ovarian cancer, prostate cancer kidney cancer, gastric cancer, colon cancer, testicular cancer, head and neck cancer, pancreatic cancer, brain cancer, B-cell lymphoma, acute myelogenous leukemia, chronic myelogenous leukemia, chronic lymphocytic leukemia, T cell lymphocytic leukemia, non-small cell lung cancer, small-cell lung cancer, or a combination thereof.

A sample in set of samples 112 may include, for example, various immunoprotein complex (IPC) molecules and various peptides, or a combination thereof. When set of samples 112 includes a diseased sample, the peptides may include one or more mutant peptides (e.g., neoantigens). The IPC molecules may include, for example, various MHC molecules, various TCR molecules, or a combination thereof.

In one or more embodiments, set of samples 112 includes immunoprotein complex (IPC) 114 (e.g., MHC Class I molecule, MHC Class II molecule, TCR, etc.), and amino acid chain 116. Amino acid chain 116 may be a chain of amino acids that includes a peptide 118, an N-flank 120, and a C-flank 122. Peptide 118 may be defined as including or excluding the N-terminus between peptide 118 and N-flank 120 and as including or excluding the C-terminus between peptide 118 and C-flank 122. Peptide 118 is considered a mutant peptide when peptide 118 includes one or more variants (e.g., one or more sequence variations) when compared to a corresponding reference sequence. In some embodiments, set of samples 112 also includes immunoprotein complex 123 (e.g., MHC Class Imolecule, MHC Class II molecule, TCR, etc.).

Set of samples 112 may be processed to generate sequence data 110. In some embodiments, when multiple samples in set of samples 112 may be processed at different times. In some embodiments, prediction system 110 includes a sample analyzer that is used in the processing of set of samples 112 to generate sequence data 110. Sequence data 110 includes, for example, at least one immunoprotein complex (IPC) sequence 124 (e.g., one IPC sequence 124 corresponding to immunoprotein complex 114) and at least one peptide sequence 126 (e.g., one peptide sequence 126 corresponding to peptide 118). Sequence data 110 may also include at least one N-flank sequence 128 (e.g., one N-flank sequence 128 corresponding to N-flank 120), at least one C-flank sequence 130 (e.g., one C-flank sequence 130 corresponding to C-flank 122), or both that correspond to the respective peptide sequence 126.

When immunoprotein complex 114 takes the form of an MHC, IPC sequence 124 may be, for example, an MHC sequence that characterizes at least a portion of the MHC. When immunoprotein complex 114 takes the form of a TCR, IPC sequence 124 may be, for example, a TCR sequence that characterizes at least a portion of the TCR. In still other embodiments, IPC sequence 124 may include both a TCR sequence and an MHC sequence characterizing at least a portion of a TCR molecule and at least a portion of an MHC molecule that can present a peptide to the TCR molecule, respectively. In some embodiments, sequence data 110 may include IPC sequence 124 in the form of an MHC sequence characterizing at least a portion immunoprotein complex 114 in the form of an MHC, as well as a separate TCR sequence 131 characterizing at least a portion of a TCR (e.g., immunoprotein complex 123) in set of samples 112.

Peptide sequence 126 characterizes at least a portion of peptide 118. N-flank sequence 128 characterizes at

least a portion of N-flank 120. For example, because the number of amino acids (or amino acid residues) upstream from the N-terminus may be large, the corresponding sequence for N-flank 120 may be trimmed to generate N-flank sequence 128. C-flank sequence 130 characterizes at least a portion of C-flank 122. In some cases, when the number of amino acids (or amino acid residues) downstream from the C-terminus is large, the corresponding sequence for C-flank 122 may be trimmed to generate C-flank sequence 130.

Sequence analyzer 108 receives sequence data 110 as input for processing. Sequence analyzer 108 includes machine learning model 132 that processes sequence data 110. In some embodiments, sequence analyzer 108 is sent directly into machine learning model 132 for processing. In other embodiments, sequence analyzer 108 preprocesses sequence data 110 prior to sending sequence data 110 into machine learning model 132 for processing.

Machine learning model 132 may be implemented in any of a number of different ways. In one or more embodiments, machine learning model 132 takes the form of an attention-based machine learning model. Machine learning model 132 may be used in either a training mode or a prediction mode. In the training mode, machine learning model 132 is trained using training dataset 133. Examples of data that may form training dataset are described further below in Section II.E. Machine learning model 132 is trained such that it can be used in the prediction mode.

Machine learning model 132 processes IPC sequence 124 via an IPC processing path 134 and peptide sequence 126 via a peptide processing path 136. The separation of these two paths for IPC and peptide enables the improved predictive performance of machine learning model 132. In some embodiments, machine learning model 132 further processes, N-flank sequence 128 via an N-flank processing path 138, C-flank sequence 130 via a C-flank processing path 140, or both.

IPC processing path 134 may be comprised of one or more different paths. For example, in some cases, IPC processing path 134 takes the form of an MHC processing path for processing, for example, IPC sequence 124 in the form of an MHC sequence. In other cases, IPC processing path 134 includes a TCR processing path for processing, for example, IPC sequence 124 in the form of a TCR sequence. In still other cases, IPC processing path 134 includes a processing path for processing IPC sequence 124 that includes both an MHC sequence and a TCR sequence. In some embodiments, when IPC processing path 134 takes the form of an MHC processing path, machine learning model 132 also includes TCR processing path 142 for processing, for example, TCR sequence 131. Examples of implementations for these different processing paths are described in greater detail below.

Machine learning model 132 processes sequence data 110 to generate an output that is used to generate report 144. Report 144 may include the exact output of machine learning model 132, may include a transformed or filtered version of the output, or both. In some cases, sequence analyzer 108 may generate notifications, recommendations, alerts, or other information based on the output of machine learning model 132, with this additional information being included in report 144.

Report 144 may be an output that includes, for example, information about immunological activity of interest with respect to one or more peptides (e.g., one or more mutant peptides). For example, report 144 may include information about the immunological activity relating to peptide 118 and immunoprotein complex 114 (e.g., MHC), peptide and immunoprotein complex 123 (e.g., TCR), or both. Report 144 may include, for example, interaction information 146, immunogenicity information 148, or both. Interaction information 1346 may provide predictions about a selected set of interactions between peptide 118 and immunoprotein complex 114, between peptide 118 and immunoprotein complex 123, or both. Immunogenicity information 148 may provide predictions about the immunogenicity of peptide 118.

In one or more embodiments, report 144 may be displayed on graphical user interface 150 on display system 106. A user may view report 144 and/or interact with report 144 via graphical user interface 150 and use report 144 to make decisions about the treatment of the subject from which at least one of set of samples 112 was obtained (or collected).

In some embodiments, prediction system 100 sends report 144 to remote system 152 (e.g., wirelessly). Remote system 152 may be a cloud computing platform, cloud storage, another computer system, a user device (e.g., a smartphone, a tablet, a laptop, etc.) or some other type of platform. In some embodiments, remote system 152 may be a treatment manufacturing system (or machine) or a portion thereof.

Fig. 2 is a flowchart of a process for generating predictions using a machine learning model in accordance with one or more embodiments. Process 200 may be implemented using prediction system 100 described in Fig. 1. For example, process 200 may be implemented using sequence analyzer 108 and machine learning model 132 in Fig. 1.

Process 200 may include, for example, step 202. Step 202 includes training an attention-based machine learning model using a training data set that includes training peptide sequence data, training immunoprotein complex (IPC) data, and training immunological activity data.

Step 204 includes accessing a set of peptide sequences characterizing a set of peptides, each peptide sequence of the set of peptide sequences having been identified by processing a disease sample from a subject.

Step 206 includes accessing an immunoprotein complex (IPC) sequence identified for an immunoprotein complex (IPC) of the subject.

Step 208 includes processing a set of peptide representations that represents the set of peptide sequences using a first attention block in an initial attention subsystem of an attention-based machine-learning model and an immunoprotein complex (IPC) representation that represents the IPC sequence using a second attention block in the initial attention subsystem to generate an output, wherein the output includes at least one of an interaction prediction, an interaction affinity prediction, or an immunogenicity prediction for a corresponding peptide-IPC combination. The first attention block is independent of the second attention block.

Step 210 includes generating a report based on the output. The report may be used to facilitate the design and/or manufacture of a treatment and/or treatment plan. For example, the report may identify a subset of peptides of the set of peptides or provide an indication of which ones to select for the subset of peptides for use in creating a treatment for the subject. The treatment may be, for example, the subset of peptides, a precursor for each of the subset of peptides, or some other form.

## II.B. Exemplary Architecture of Machine-Learning Model

### II.B. 1. General Characteristics and Implementation Considerations

As described above, in various embodiments, the machine learning model of the embodiments described herein, *e.g.,* machine-learning model 132, may be an attention-based machine-learning model (e.g., that includes one or more attention layers). Machine-learning model 132 can implement, for example, one or more self-attention layers. Machine-learning model 132 can use a self-attention mechanism, a global attention mechanism, a soft attention mechanism, a local attention mechanism, and/or a hard attention mechanism.

In some instances, the attention-based machine-learning model can be configured to learn alignments (e.g., between the peptide sequence and the MHC sequence). The alignments may be learned and performed using an attention-based alignment score function such as, for example, a content-based function, an additive function, a location-based function, a dot-product function, and/or a scaled dot-product function. Machine-learning model 132 can include one or more encoders, one or more transformers, and/or one or more transformer encoders. In some embodiments, Machine-learning model 132 may use one or more characteristics (such as, for example, one or more encoders) as described in Vaswani, A, et al., "Attention is All You Need." 31st Conference on Neural Information Systems, http://papers.nips.cc/paper/7181-attention-is-all-you-need.pdf, 2017.

Machine-learning model 132 may include one or more encoders configured to, for example, transform an input (e.g., a sequence representation representing, for example, an amino acid sequence, a nucleic acid sequence, a codon sequence, etc.) into a higher dimensional space. An encoder may be a transformer encoder. The encoder may be configured to implement an attention-based technique and/or to include one or more attention layers (e.g., one or more self-attention layers).

In some embodiments, machine-learning model 132 may use or may omit a convolutional layer, long-short term memory unit, recurrent structure, and/or recurrent component. For example, in some instances, machine-learning model 132 does not include any convolutional layer, any recurrent structure, any long-short term memory (LSTM) unit, and/or any recurrent component. In some instances, machine-learning model 132 is not a recurrent machine-learning model, and/or does not include a recurrent neural network. In some instances, the machine-learning model includes a recurrent neural network and/or may use position encoding to provide temporal information across one or more sequences. In some instances, machine-learning model 132 is not a convolutional machine-learning model and/or does not include a convolutional neural network.

Machine-learning model 132 may include multiple subsystems (or subnetworks). Each of the multiple subsystems can include an encoder, a transformer encoder, one or more attention layers, and/or one or more self-attention layers. Machine-learning model 132 may include attention blocks with a first attention block used to process a peptide representation independently of a second attention block used to process an IPC representation of part or all of an IPC sequence (e.g., an MHC pseudosequence). The independence of these attention blocks can facilitate parallel processing when using the machine-learning model. Further, the independence may improve the performance (e.g., accuracy of predictions) of machine learning model 132.

Within machine learning model 132, attention-based mechanisms may be configured such that an output value at any given layer depends, not only on a corresponding input value, but also on one, more, or all other input values. Thus, machine-learning model 132, a loss function, and/or optimization function may be configured to optimize an output corresponding to a single position representing a degree to which a given MHC molecule (represented by a corresponding input) will bind to and/or present a given peptide (represented by another corresponding input) and/or trigger immunogenicity in response to the given peptide. In some instances, any of a plurality of outputs of transformer encoders may represent such an occurrence probability, and/or a model may be trained accordingly. In some instances, an endpoint (e.g., surplus endpoint), such as a Beginning-of-Sequence element, may represent (in response to training) a binding, presentation, and/or immunogenicity probability. Aggregated outputs may be, for example, fed to another layer and/or to another subsystem or attention block (e.g., that includes an attention layer and/or a self-attention layer and/or that is a transformer encoder and/or an encoder).

In some instances, one, two, or all dimensions of an output from the other layer and/or from the other subsystem or attention block is of a same size as the input fed to the other layer and/or other subsystem or attention block. In some instances, an input fed to this other layer and/or other subsystem or attention block has a length along one axis that is greater than or equal to a sum of a number of amino acids in the IPC sequence, a number of amino acids in the peptide sequence, and potentially a number of amino acids in one or more of the N-flank and the C-flank. In some instances, the length of the dimension for the input is one longer than the total number of amino acids. The length of the input along the one axis may exceed the summed count of amino acids when, for example, an additional feature vector (e.g., a Beginning-of-Sequence feature vector) is appended to the amino-acid-specific feature values. Another dimension of the input can include a number of features (e.g., defined via a hyperparameter). An output generated by the other layer and/or other subsystem or attention block may have a same size as that of the input.

A subset of values of the output generated by the other layer and/or other subnetwork may be further processed by another neural network (e.g., a fully connected feedforward network). The subset of values may include a 1-dimensional vector of values that may correspond to one set of feature values. The 1-dimensional vector may correspond to feature values associated with the Beginning-of-Sequence feature vector.

A neural network within machine learning model 132 can be configured to output one or more results. The one or more results can include, for example, a numeric result, a binary result, and/or a categorical result. Each of the one or more results can predict whether and/or an extent to which an IPC and a peptide undergo a reaction of a particular type (e.g., bind together). Machine-learning model 132 may include one or more activation layers to produce a result of a target time (e.g., to transform a real-number interim value into a binary and/or categorical output). Machine-learning model 132 can be trained to generate multiple types of predictions (e.g., interaction predictions, interaction affinity predictions, and/or immunogenicity predictions). In some instances, a prediction may be binary or categorical. Other predictions may be non-binary or non-categorical. For example, a prediction may be scalar.

Machine-learning model 132 may include and/or may be included within an ensemble model. The ensemble model may include multiple (e.g., identical) sub-models that may be trained using different portions of the training data set.

II.B.2. Exemplary Configurations for Machine Learning Model

Fig. 3 is a schematic diagram of one configuration for machine learning model 132 from Fig. 1 in accordance with one or more embodiments. Machine learning model 132 is described with continuing reference to Fig. 1. Machine learning model 132 has configuration 300. With configuration 300, machine learning model 132 includes representation subsystem 302, initial attention subsystem 304, composite subsystem 306, composite attention subsystem 308, and output subsystem 310. Each "subsystem" within machine learning model 132 may be comprised of one or more blocks, one or more sub-blocks, one or more layers, or a combination thereof. Each "block" within machine learning model 132 may be comprised of one or more sub-blocks, one or more layers, or a combination thereof. Each "sub-block" of machine learning model 132 may be comprised of one or more layers (or units).

Representation subsystem 302 receives sequence data 110 as input and generates representations for the various sequences in sequence data 110. A "representation" may include, for example, a set of elements (e.g., each element comprising one or more values), each element representing or identifying one or more amino acids or one or more nucleic acids in the parent sequence of the representation. For example, each amino acid in the parent sequence may be represented by a unique binary string and/or vector of values that is distinct from the binary string and/or vector representing another amino acid.

Initial attention subsystem 304 receives these representations as input, processes these representations, and generates transformed representations that are sent into composite subsystem 306. Initial attention subsystem 304 is comprised of various attention blocks, each of which includes at least one self-attention layer.

In one or more embodiments, representation subsystem 302 may process peptide sequence 126 to generate peptide representation 312, which is then processed by attention block 314 in initial attention subsystem 304 to generate transformed peptide representation 316. This processing may form at least a portion of peptide processing path 136 in Fig. 1. Further, representation subsystem 302 may process IPC sequence 124 to generate IPC representation 318, which is then processed by attention block 320 in initial attention subsystem 304 to generate transformed IPC representation 322. This processing may form at least a portion of IPC processing path 134 in Fig. 1. When IPC sequence 124 is an MHC sequence, IPC representation 318 is referred to as an MHC representation and transformed IPC representation 322 is referred to as a transformed MHC representation. When IPC sequence 124 is a TCR sequence, IPC representation 318 is referred to as a TCR representation and transformed IPC representation 322 is referred to as a transformed TCR representation.

In some embodiments, representation subsystem 302 may process N-flank sequence 128 to generate N-flank representation 324, which is then processed by attention block 326 in initial attention subsystem 304 to generate transformed N-flank representation 328. This processing may form at least a portion of N-flank processing path 138 in Fig. 1. In some embodiments, representation subsystem 302 may process C-flank sequence 130 to generate C-flank

representation 330, which is then processed by attention block 332 in initial attention subsystem 304 to generate transformed C-flank representation 334. This processing may form at least a portion of C-flank processing path 140 in Fig. 1.

When machine learning model 132 also includes TCR processing path 142, representation subsystem 302 may process TCR sequence 131 to generate TCR representation 336, which is then processed by attention block 338 in initial attention subsystem 304 to generate transformed TCR representation 340. This processing may form at least a portion of TCR processing path 142 in Fig. 1.

Composite subsystem 306 receives the transformed representations (e.g., transformed peptide representation 316, transformed IPC representation 322, transformed N-flank representation 328, transformed C-flank representation 334, transformed TCR representation 340, or combination thereof) that are output from initial attention subsystem 304 and performs one or more operations to generate composite representation 342. Composite representation 342 may be, for example, an aggregate of the transformed representations that are output from initial attention subsystem 304. In one or more embodiments, composite representation may include a concatenation layer that concatenates the transformed representations that are output from initial attention subsystem 304. In some embodiments, composite representation 342 includes one or more additional feature vectors (e.g., which may be added to a beginning or end of a transformed representation). An additional feature vector may have, for example, a length equal to a number of features corresponding to each individual amino acid represented in the respective parent sequence. An additional feature may include, for example, a Beginning-of-Sequence (BoS) element.

Composite representation 342 is sent as input into composite attention subsystem 308. Composite attention subsystem 308 includes one or more attention blocks for processing composite representation 342. For example, composite attention subsystem 308 may include attention block 344 (which may be referred to as a composite attention block) that receives and processes composite representation 342. The output of composite attention subsystem 308 is sent into output subsystem 310 for processing, which generates report 144 as described above in Fig. 1.

Figs. 4A-4C are schematic diagrams of different configurations for a machine learning model 400 in accordance with one or more embodiments.

Fig. 4A is a schematic diagram of a machine learning model 400 in accordance with one or more embodiments. Machine learning model 400 is one example of an implementation for machine learning model 132 in Figs. 1 and 3. Machine learning model 400 is an attention-based machine learning model. Machine learning model 400 includes representation subsystem 401, initial attention subsystem 403, composite subsystem 405, composite attention subsystem 407, and output subsystem 409, which are examples of implementations for representation subsystem 302, initial attention subsystem 304, composite subsystem 306, composite attention subsystem 308, and output subsystem 310, respectively, in Fig. 3.

Representation subsystem 401 includes peptide representation block 402 and IPC representation block 404. In some embodiments, representation subsystem 401 further includes N-flank representation block 406, C-flank representation block 408, or both. In some embodiments, when IPC representation block 404 corresponds to MHC and is used as an MHC representation block, representation subsystem 401 may also include TCR representation block 410. Each of these different representation blocks includes at least one embedding layer and may include, for example, a positional encoder.

An embedding layer may embed a sequence by, for example, transforming an initial non-numeric representation (e.g., a string of amino-acid identifiers) into a numeric representation to generate an embedded representation. The embedding can be performed using, for example, one-hot encoding, evolutionarily-motivated encodings such as BLOSUM, randomly or pseudorandomly initialized learned embeddings, or a combination thereof. The embedded representation may be positionally encoded to generate an encoded representation. The sequence representation produced by a representation block may be the encoded representation or an aggregation (e.g., concatenation or sum) of the encoded representation and the embedded representation.

In some cases, various attention mechanisms may be unable to detect potential information conveyed by an order of values in an input data set. Positional encoders may be used and added to the embedded representation, with the positional encoding using an encoding algorithm that is learned or fixed. For example, a fixed positional encoding may be defined using a sine and/or cosine function (e.g., having an intra-sequence position and/or a dimension as the independent variables). The positional encoding may have a same dimension as the encoded representation. The positional encodings may be summed with the embedded representation to produce a position-indicative embedded representation of the sequence that is fed into initial attention subsystem 403.

For example, peptide representation block 402 may include embedding layer 412 that embeds a peptide sequence (e.g., peptide sequence 126 in Fig. 1) to generate an embedded peptide representation, and positional encoder 414 that encodes, positionally, the embedded peptide representation to generate a peptide representation (e.g., peptide representation 312 in Fig. 3) that represents the peptide sequence. IPC representation block 404 may include embedding layer 416 that embeds an IPC sequence (e.g., IPC sequence 124 in Fig. 1) to generate an embedded IPC representation, and positional encoder 418 that encodes, positionally, the embedded IPC representation to generate an IPC representation (e.g., IPC representation 318 in Fig. 3) that represents the IPC sequence.

Further, N-flank representation block 406 may include embedding layer 420 that embeds an N-flank sequence (e.g., N-flank sequence 128 in Fig. 1) to generate an embedded N-flank representation, and positional encoder 422 that encodes, positionally, the embedded N-flank representation to generate an N-flank representation (e.g., N-flank representation 324 in Fig. 3) that represents the N-flank sequence. C-flank representation 408 may include embedding layer 424 that embeds a C-flank sequence (e.g., C-flank sequence 130 in Fig. 1) to generate an embedded C-flank representation, and positional encoder 426 that encodes, positionally, the embedded C-flank representation to generate a C-flank representation (e.g., C-flank representation 330 in Fig. 3) that represents the C-flank sequence.

Still further, TCR representation block 410 may include embedding layer 428 that embeds a TCR sequence (e.g., TCR sequence 131 in Fig. 1) to generate an embedded TCR representation, and positional encoder 430 that encodes, positionally, the embedded TCR representation to generate a TCR representation (e.g., TCR representation 336 in Fig. 3) that represents the TCR sequence.

Embedding the sequence can include, for example, transforming an initial non-numeric representation (e.g., that include a string of amino-acid identifiers) into a numeric representation. The embedding can include one-hot encoding, evolutionarily-motivated encodings such as BLOSUM, or randomly or pseudorandomly initialized learned embeddings. The representation can include a sum and/or aggregation of (e.g., concatenation of) the positional encoding of the sequence and embedded sequence.

The representations generated by representation subsystem 401 are sent as input into initial attention subsystem 403 for processing. Initial attention subsystem 403 may include various self-attention mechanism that determine, for each of one, more, or all positions in a representation, an attention weight for (e.g., indicating how much attention to pay to) a value of each of one or more other positions. Attention weights can then be used to generate a transformed value for the position.

Initial attention subsystem 401 includes attention block 432 and attention block 434. Initial attention subsystem 401 may also include, in some embodiments, attention block 436, attention block 438, attention block 440, or a combination thereof. Attention block 432 receives a peptide representation from peptide representation block 402 and processes the peptide representation using set of attention sub-blocks 442 to generate a transformed peptide representation (e.g., transformed peptide representation 316 in Fig. 3). One example of an implementation for an attention sub-block is described in greater detail in Fig. 6 below. Attention block 434 receives an IPC representation from IPC representation block 404 and processes the IPC representation using set of attention sub-blocks 444 to generate a transformed IPC representation (e.g., transformed IPC representation 322 in Fig. 3).

Further, when included, attention block 436 receives an N-flank representation from N-flank representation block 406 and processes the N-flank representation using set of attention sub-blocks 446 to generate a transformed N-flank representation (e.g., transformed N-flank representation 328 in Fig. 3). Attention block 438 receives a C-flank representation from C-flank representation block 408 and processes the C-flank representation using set of attention sub-blocks 448 to generate a transformed C-flank representation (e.g., transformed C-flank representation 334 in Fig. 3). Attention block 440 receives a TCR representation from TCR representation block 410 and processes the TCR representation using set of attention sub-blocks 450 to generate a transformed TCR representation (e.g., transformed TCR representation 340 in Fig. 3).

The transformed representations output from initial attention subsystem 403 are sent into composite subsystem 405 for processing. Composite subsystem 405 includes composite block 452. Composite block 452 may form a composite representation (e.g., composite representation 342 in Fig. 3) using the transformed representations output from initial attention subsystem 403. For example, composite block 452 may aggregate, concatenate, or otherwise combine the transformed representations to form an initial composite representation. In some cases, composite block 452 also adds one or more additional feature vectors (e.g., BoS vector) within the initial composite representation.

In some embodiments, composite subsystem 405 may also include positional encoder 454. Positional encoder 454 encodes, positionally, the initial composite representation to thereby generate a composite representation that is output to composite attention subsystem 407. When positional encoder 454 is not present within composite subsystem 405, the initial composite representation generated by composite block 452 may be the composite representation output to composite attention subsystem 407.

Composite attention subsystem 407 may include attention block 456 (which may also be referred to as a composite attention block). Attention block 456 includes set of attention sub-blocks 458. Attention block 456 receives the composite representation generated by composite subsystem 405 and processes the composite representation using set of attention sub-blocks 458 to generate a transformed composite representation. This transformed composite representation is then output to output subsystem 409 for processing.

A size of an output generated by composite attention subsystem 407 or an attention sub-block within composite attention subsystem 407 may be equal to a size of an input fed to composite attention subsystem 407 or the attention sub-block within composite attention subsystem 407. The size may be, for example, $m \times n$, where $m$ is equal to a total number of amino acids being considered by 1 (e.g., for the Beginning of Sequence representation), and $n$ is equal to a number of

features (a predetermined value). A single column (having n values) can be selected to further process. The single column may be a first column and/or column associated with the Beginning-of-Sequence representation. In instances where only a portion of the output to composite attention subsystem 407 or the attention sub-block within composite attention subsystem 407 are fed to output subsystem 409, training of machine-learning model 400 may result in learned parameter values that convey pertinent information about both the IPC sequence and the peptide-related sequence(s) and peptide-IPC interactions to be represented in the Beginning-of-Sequence representation. In other instances, an aggregated representation may be pooled after output from composite attention subsystem 407 to yield a single vector, which may then be fed into output subsystem 409.

Output subsystem 409 may include various blocks, sub-blocks, layers, or combination thereof for generating a final output. In one or more embodiments, output subsystem 409 includes dropout block 460, fully connected block 462, and output block 464. Dropout block 460 may include, for example, one or more dropout layers. Fully connected block 462 may include, for example, one or more fully connected layers. Output block 464 may include, for example, one or more layers for filtering, selecting, transforming, or otherwise generating output. For example, output block 464 may include at least one max layer 465 that is configured to select a subset of the input received at output block 464 based on, for example, selected thresholds or ranges.

In some cases, the transformed composite representation is received and processed by dropout block 460 to generate a first output that is received by fully connected block 462. Fully connected block 462 may receive and process this first output to generate a second output, at least a portion of which is received by output block 464. Output block 464 receives and processes its input to generate interaction output 466, immunogenicity output 468, or both.

In some embodiments, fully connected block 462 may be configured to generate one or more outputs having a dimensionality that is smaller than a dimensionality fed into fully connected block 462 (e.g., smaller than the predefined number of features). For example, an output of the fully connected block 462 may include a single value, two values, or three values - each corresponding to a prediction pertaining to a target interaction or immune response. Fully connected block 462 may include, for example, a single hidden layer, two hidden layers or three or more hidden layers. A number of nodes in an initial hidden layer may be larger than a number of nodes in a subsequent hidden layer. For example, a first hidden layer can include 256 nodes, while a second hidden layer can include 126 nodes. In various embodiments, each output from fully connected block 462 may include a real number score, which may, for example, be converted to a binary and/or categorical result (e.g., using a trained activation function) and/or converted into a scaled number. For example, the scaled number may include a probability on a scale from 0 to 1.

Interaction output 466 may include, for example, set of interaction predictions 470, set of interaction affinity predictions 472, or both with respect to one or more target interactions. An interaction prediction may include, for example, a prediction for a corresponding peptide-IPC (e.g., peptide-MHC, peptide-TCR) combination of whether the IPC (e.g., MHC, TCR) will bind to the peptide. An interaction prediction may include, for example, a prediction for a corresponding peptide-IPC (e.g., peptide-MHC) combination of whether the IPC (e.g., MHC) will present the peptide at a cell surface. Further, an interaction affinity prediction may include, for example, a prediction of an affinity for a target interaction for a corresponding peptide-IPC (e.g., peptide-MHC, peptide-TCR) combination. The target interaction may be, for example, the binding of the peptide and the IPC. The affinity for the target interaction, which may be, for example, a binding affinity, indicates a strength, tendency, and/or stability of the binding between the peptide and the IPC.

Immunogenicity output 466 comprises a set of immunogenicity predictions. An immunogenicity prediction may include, for example, a prediction of immunogenicity with respect to a corresponding peptide-IPC combination. For example, an immunogenicity prediction may indicate the ability of the peptide to provoke an immune response with respect to the particular IPC of interest (e.g., TCR or MHC and TCR complex).

In some cases, a first portion of the output from fully connected block 462 is sent into output block 464, while a second portion of the output from fully connected block 462 is in its final form and used as set of interaction affinity predictions 472.

In other embodiments, the transformed composite representation received at output subsystem 409 is received and processed by fully connected block 462, which processes the transformed composite representation to generate a first output that is sent into dropout block 460. The output of dropout block 460 or a portion thereof may then be sent output block 464 for processing.

In some embodiments, the output from output subsystem 409 may include multiple results that include, for each IPC (e.g., MHC) allele, a prediction as to whether and/or a probability that the peptide binds to the IPC allele. The allele-specific predictions may be output, or in some case, max layer 465 may be used to determine a maximum of the allele-specific predictions, and the maximum can be output.

In this manner, output subsystem 409 may be implemented in any of a number of different ways, with any number of different blocks, sub-blocks, and/or layers that enable the generation of interaction output 466, immunogenicity output 468, or both. The processing of peptide sequences separately from the processing of IPC sequences (e.g., MHC sequences, TCR sequences, combined MHC-TCR sequences, etc.) prior to composite subsystem 405 increases the predictive performance of machine learning model 400. For example, generating the transformed peptide representation

using peptide representation block 402 and attention block 432 along a path that is separate from the generation of the transformed IPC representation using IPC representation block 404 and attention block 434 (and, if applicable, separate from the generation of the transformed TCR representation using TCR representation block 410 and attention block 440) prior to generating the composite representation increases the accuracy of the output generated output subsystem 409. Further, such processing may enable efficient processing (e.g., using reduced computing resources, quicker processing, etc.) because multiple peptide-IPC (and peptide-TCR) combinations may be considered in a modular way.

In various embodiments, machine learning model 400 may facilitate automated determination as to which particular IPC allele is predicted to bind to and present a peptide. For example, if an MHC molecule includes 6 MHC alleles (as is the case for humans), 6 iterations of at least part of a neural-network processing may be performed (e.g., in parallel)-one for each allele. Each processing may use, as input, an MHC representation of an MHC sequence for the MHC allele and a peptide representation of at least a portion of the peptide's sequence. Each processing may generate an output corresponding to a prediction as to whether the peptide will bind to and/or be presented by the MHC allele. It may be inferred that the peptide associated with the highest prediction value (e.g., indicating a most likely binding and/or presentation prediction) across the alleles is the one to which the peptide would bind and the one that would present the peptide.

In some instances, for 6 MHC alleles, six composite representations may be created by running the 6 different MHC allele sequences through the same IPC representation block 404 and generating a composite representation for each allele-peptide combination. In some embodiments, each of the six composite representations may be aggregated (e.g., concatenated) together, along with a Beginning-of- Sequence token (vector) that has been embedded with the embedding layer. Each of six composite representations can then be fed through composite subsystem 407 as described above.

In some embodiments, the processed Beginning-of- Sequence token can be extracted and fed to fully connected block 462 to output directly to a final node of machine learning model 400. This BoS token may represent node presentation likelihood. In some cases, each fully connected sub-block within fully connected block 462 may have dropout applied and be followed by a batch normalization layer. In some embodiments, output block 464 is used for deconvolution such that ~6 paired peptide-MHC interactions will correspond to a single selected MHC allele by applying an activation function (e.g., via max layer 465 which may include a softmax function) on the ~6 presentation predictions. During training, the selected peptide-MHC interaction output can be normalized as a value between 0 and 1 and can be compared to a true presentation value using a loss function (e.g., binary loss function) to generate an error for tuning the model parameters.

In still other embodiments, one or more of the attention blocks or attention sub-blocks included in machine learning model 400 may be replaced with another type of network and/or processing unit to convert a representation of one or more sequences. The conversion may represent an extent to which various amino acids (at particular positions) are predicted to influence a binding affinity and/or presentation probability and/or an extent to which various particular combinations of amino acids (at particular positions), occurring over a single sequence or across sequences, are predicted to influence a binding affinity and/or presentation. For example, one or more attention sub-blocks may be replaced by one or more gated recurrent units.

Fig. 4B is a schematic diagram of a different configuration for machine learning model 400 in accordance with one or more embodiments. With the configuration depicted in Fig. 4B, representation subsystem 401 includes aggregate representation block 480. Aggregate representation block 480 receives an aggregate sequence such as, for example, an aggregate of a peptide sequence (e.g., peptide sequence 126 in Fig. 1) and an N-flank sequence (e.g., N-flank sequence 128 in Fig. 1) and/or a C-flank sequence (e.g., C-flank sequence 130 in Fig. 1).

Aggregate representation block 480 may include, for example, embedding layer 482 that processes the aggregate sequence to form an embedded aggregate representation that may be received by positional encoder 483, which positionally, encodes the embedded aggregate representation generate aggregate representation 484. Thus, aggregate representation 484 may include a peptide representation 485 of the parent peptide sequence and an N-flank representation 486 of the parent N-flank sequence and/or C-flank representation 487 of the parent C-flank sequence.

Aggregate representation 484 is output from aggregate representation block 480 and sent to attention block 488 in initial attention subsystem 403 for processing. Attention block 488 includes set of attention sub-blocks 489 that process aggregate representation 484 to generate a transformed aggregate representation that is sent to composite block 452 for processing.

In some embodiments, if the aggregate sequence sent into aggregate representation block 480 includes either the N-flank sequence or the C-flank sequence but not the other, then machine learning model 400 may also include the corresponding representation block (e.g., N-flank representation block 406 or C-flank representation block 408) and the corresponding attention block (e.g., attention block 436 or attention block 438, respectively) for the sequence not included in the aggregate sequence.

Fig. 4C is a schematic diagram of a different configuration for machine learning model 400 in accordance with one or more embodiments. With the configuration depicted in Fig. 4C, the peptide representation and the N-flank representation, and optionally, the C-flank representation, generated by representation subsystem 401 are sent into aggregate block

490. Aggregate block 490 may aggregate (e.g., concatenate) these representations to form an aggregate representation that is sent into attention block 492. Attention block 492 includes set of attention sub-blocks 494 that process the aggregate representation to generate a transformed aggregate representation that is sent to composite block 452 for processing.

As shown by Figs. 4A-4C, machine learning model 400 may be implemented in a number of different ways using any number of or combination of blocks, sub-blocks, and/or layers within the various subsystems. Thus, machine learning model 400 is modular and may be customizable for the given task.

Fig. 5 is a schematic diagram of attention block 500 in accordance with one or more embodiments. Attention block 500 may be one example of an implementation for an attention block in initial attention subsystem 304 in Fig. 3, composite attention subsystem 308 in Fig. 3, or initial attention subsystem 403 in Figs. 4A-C. Further, attention block 500 may be one example of an implementation for attention block 456 in Figs. 4A-4C.

Attention block 500 includes one or more attention sub-blocks. For example, attention block 500 may include attention sub-block 1 501 and, optionally, one or more other attention sub-blocks up to attention sub-block n 504. When multiple attention sub-blocks are present in attention block 500, these attention sub-blocks may be connected serially (e.g., daisy-chained together to produce a final output).

Attention sub-block 1 501 may be implemented in various ways. In one or more embodiments, attention sub-block 1 501 includes, for example, self-attention layer 506, add and normalization layer 508, feed forward layer 510, and add and normalization layer 512. With this configuration for attention sub-block 501, attention sub-block 1 501 may also be referred as a transformer encoder. Self-attention layer 506 may be implemented using, for example, a one-head attention unit or a multi-head attention unit. If present, the one or more other attention sub-blocks in attention block 500 up to attention sub-block n may be implemented in a manner similar to attention sub-block 1 501.

In an add and normalization layer, a transformed representation may be added to the position-indicative embedded representation of a sequence (via a residual connection), and the summed representation can be normalized. The normalized data can be fed to the corresponding feed forward layer 510 (e.g.., a fully connected feedforward network). The feedforward network can affect (for example), for each position, one, two, three, or more linear transformations and/or may include an activation (e.g., a ReLU activation) between each of the linear transformations. For example, the feedforward layer can be represented by:

$$FF(x) = \max(0, xW_1 + b_1)W_2 + b_2,$$

where x is an input to the layer, $W_1$ and $W_2$ are slopes of the linear transformations and $b_1$ and $b_2$ are intercepts of the linear transformation. A dimensionality of an output of a particular attention sub-block's feed forward layer may be the same as a dimensionality of an input to the attention sub-block's feed forward layer. Thus, in some instances, to preserve representations of various types of information, the input and output can be summed and normalized (e.g., via another residual connection through another add and normalization layer).

II.B.3. <u>Exemplary Mechanism for Self-Attention</u>

Fig. 6 is a flowchart of a process for processing a sequence representation using an exemplary self-attention layer in accordance with one or more embodiments. Process 600 may be used by, for example, one or more of the attention blocks present in machine learning model 132 in Figs. 1 and 3, one or more of the attention blocks present machine learning model 400 in Figs. 4A-4C, and/or attention block 500 in Fig. 5.

Step 602 includes receiving a sequence representation that includes a plurality of elements. The sequence representation represents an amino-acid sequence or a genetic nucleic-acid sequence, or a codon sequence within a genetic sequence. In one or more embodiments, each element of the plurality of elements in the sequence representation represents an amino acid (or amino acid residue), a nucleic acid, a codon, etc. Further, each element is associated with a unique position in the sequence.

The sequence representation may be, for example, a peptide representation, an IPC representation, an N-flank representation, a C-flank representation, an MHC representation, a TCR representation, an aggregate representation, or another type of representation. For example, the sequence representation may represent part or all of: a variant-coding sequence, part or all of a sequence that encodes a wild-type or mutant peptide, an epitope sequence (e.g., that includes a variant), a candidate neoepitope sequence, part or all of a neoantigen sequence, a sequence that begins or ends at a terminus of a peptide (e.g., an N-flank or C-flank), an MHC sequence (e.g., an MHC pseudosequence). The sequence representation may be, for example, generated using representation subsystem 302 in Fig. 3 or representation subsystem 401 in Figs. 4A-4C.

Step 304 includes determining a key vector, a value vector, and a query vector for each element in the sequence representation using a set of key weights, a set of value weights, and a set of query weights, respectively. If, for example, a sequence represented in the sequence representation includes, e.g., 20 amino acids, 20 key vectors, 20 value vectors,

and 20 query vectors may be generated. An element in the sequence representation may correspond to, for example, a row or column in a 2-dimensional sequence representation (e.g., where a first dimension represents different amino acids in a sequence and a second dimension represents, for example, different components characterizing individual amino acids).

In some embodiments, the set of key weights are in the form of a key weight matrix. The key weight matrix for a particular element may have a size equal to a length of the element by a length that the key vector is to be. For example, the element may have a length of 20 (e.g., each value corresponding to a binary indication as to whether the amino acid in the sequence is the same as a specific 1 of 21 amino acids), and if a length of a key vector is to be 5 (e.g., representing 5 components or features), the key weight matrix can have a size of [5, 21]. The key weight matrix can be learned during training (e.g., and randomly initialized at the start of training).

The value vector for an element may have the same size as the key vector for the element. The value vector can be determined using a set of value weights, which may be learned during training and which may be included within a value weight matrix. The value weight matrix for a given element can have a size of the key weight matrix and/or may have a size defined based on a length of that element and a length that the value vector is to be.

The query vector for an element may have a same size as the key vector and/or the value vector for the element. The query vector can be determined using a set of query weights, which may be learned during training and which may be included within a query weight matrix. The query weight matrix for an element can have a size of the key weight matrix and/or the value weight matrix and/or may have a size defined based on a length of the element and a length that the query vector is to be.

Step 606 includes generating, for each element in the sequence representation, a set of element-focused attention scores using the element's query vector (generated using the query weights and the sequence representation) and multiple elements' key vectors (generated using key weights and the sequence representation). For a given element, the set of element-focused attention scores can indicate how much weight to give the value vector of the given element. The multiple elements for which the key vectors are use in generating the set of element-focused attention scores for a selected element in the sequence representation may include some or all of the elements in the sequence representation (e.g., representations of some or all of the amino acids represented). The multiple elements can include the element of focus (e.g., a particular amino acid for which the set of element-focused attention scores is being determined).

The set of element-focused attention scores is generated by generating, for each element of the sequence representation, an attention score for each pairing of the element of focus (the first element) with the same or different element (the second element). The attention score for this pairing can be defined as a product of the first element's query vector and the second element's key vector.

In some instances, step 606 may include implementing an activation function and/or normalization. The normalization can be based on a dimensionality of the key vector (or of the query vector). For example, the normalization can be defined to be the square root of a length of a key vector. The activation function can include a softmax function. In some instances, the normalization is applied before the activation function.

Step 608 includes performing a transformation of the plurality of elements to form a plurality of modified elements, wherein the transformation is performed using the set of element-focused attention scores generated for each of the plurality of elements and the value vector determined for each of the plurality of elements. For example, if a sequence representation includes 11 elements (e.g., representing 11 amino acids), and if attention scores are determined for all pairwise combinations of the elements, a modified sequence representation comprising a plurality of modified elements is generated in which a modified element is defined using may be defined to be a weighted average of all elements' value vectors (using the attention scores for the weighting).

Step 610 includes generating an encoding of the sequence using the transformed sequence representation, the initial sequence representation, and a feedforward network. For example, the transformed sequence representation and initial sequence representation may be summed. This result may still include multiple elements (e.g., each updated via the transformation, summing, and normalization). The feedforward neural network can then process the summed representations (e.g., by performing one, two, or more linear transformations and/or implementing one or more activation functions). Summing the representations can reintroduce positional information that may be obscured in the transformed sequence representation (due to attending to other elements' values when generating a transformed value vector for a given element).

The feedforward neural network can be configured to separately process each of the updated multiple elements (e.g., using a same technique and/or same set of parameters). Thus, the input to the feedforward network can include a vector that corresponds to a single element, a single amino acid, and/or single sequence position. The feedforward network can be configured such that an output of the feedforward network is a same size as an input to the feedforward network. In some instances, instead of processing the transformed sequence representation and initial sequence representation using a feedforward network, a convolution (e.g., a 1-dimensional convolution) is instead employed to perform a localized transformation that operates identically across the positions/elements. A 1-dimensional convolutional may be used as another way to interpret the functioning of the feedforward neural network.

The technique illustrated in Fig. 6 pertains to single-head attention (where key vectors, value vectors and query vectors are used to calculate attention scores). Multi-head attention may alternatively be used. Each attention head in multi-head attention may be associated with its own set of key weights, its own set of value weights, and its own set of query weights. Each attention head in multi-head attention can then produce a distinct key vector, a distinct value vector and a distinct query vector. Each attention head in multi-head attention can use these distinct vectors to produce attention scores and transformed values for each element. Transformed values can be concatenated and projected.

It should be further be appreciated that, while Fig. 6 refers to calculation and use of various vectors, matrix representations may instead be used. Matrix representations may facilitate performing calculations across elements efficiently as opposed to iteratively calculating various vectors individually.

Fig. 7 is a schematic diagram illustrating process 600 described in Fig. 6 above in accordance with one or more embodiments. In Fig. 7, representation and attention process 700 receives sequence 702 as input. Sequence 702 may be, for example, an amino acid sequence.

In the illustrative example in Fig. 7, sequence 702 includes a plurality of amino acids 704 (4 amino acids: $x^1$-$x^4$). A sequence representation 706 comprising a plurality of elements $a^1$-$a^4$ is generated via embedding and, in some embodiments, positional encoding. Each element $a^i$ may ve, for example, a numeric vector. Sequence representation 706 may be one example of the sequence representation received in step 602 in Fig. 6.

Vectors 708 (e.g., a query vector *q'*, key vector $k^i$ and value vector $v^i$) can be generated for each element $a^i$. Vectors 708 may be examples of implementations for the vectors generated in step 604 in Fig. 6. The illustrated example corresponds to generating select element-focused attention scores 710, $\hat{a}_{1,i}$, with a focus on the first element, *al.* Element-focused attention scores 710 are an example of one set of element-focused attention scores generated for a particular element in step 606 in Fig. 6. Each of the element-focused attention scores $\hat{a}_{1,i}$ is defined to be a dot product of $q^1$ with $k^i$. The weighted sum of the value vectors $v^i$, with the weights being set to $\hat{a}_{1,i}$, are computed to perform a transformation that generated a modified element 712, $b^1$. Modified element 712 is one example of a modified element generated in step 608 in Fig. 6. Similar transformations may be performed for the other elements of sequence representation 706.

## II.C. Exemplary Methodologies using Machine Learning Model

Machine learning model 132 in Figs 1 and 3 and machine learning model 400 in Figs. 4A-4C may be used in various ways to generate predictions about the immunological activity (e.g., predicted binding, binding affinity, predicted presentation occurrence, immunogenicity, etc.) associated with various peptides, including mutant peptides (e.g., neoantigens).

Fig. 8 is a flowchart of a process for generating information about the immunological activity of various peptides. At least a portion of process 800 may be implemented using, for example, without limitation, prediction system 100 described in Fig. 1. For example, at least a portion of process 800 may be implemented using, for example, without limitation, machine learning model 132 from Figs. 1 and 3 or machine learning model 400 from Figs. 4A-4C.

Step 802 includes receiving a peptide sequence that characterizes a mutant peptide, the peptide sequence including a variant with respect to a corresponding reference sequence. The peptide sequence characterizes the mutant peptide by characterizing at least a portion of the mutant peptide. The mutant peptide may be, for example, a neoantigen. Step 802 may be performed by, for example, retrieving the peptide sequence from a data store (e.g., data store 104 in Fig. 1, a cloud storage, a server or server system, etc.). In some embodiments, the peptide sequence may be one of a plurality of peptide sequences that are processed through the machine learning model.

Step 804 includes receiving an immunoprotein complex (IPC) sequence identified for an immunoprotein complex (IPC). The IPC may be, for example, an MHC, a TCR, or an MHC-TCR complex. Thus, the IPC sequence may be an MHC sequence, a TCR sequence, or an MHC-TCR sequence. The IPC sequence characterizes the IPC by characterizing at least a portion of the IPC. Step 802 may be performed by, for example, retrieving the IPC sequence from a data store (e.g., data store 104 in Fig. 1, a cloud storage, a server or server system, etc.). In some embodiments, the IPS sequence may be one of a plurality of IPC sequences that are processed through the machine learning model.

Step 806 includes processing the peptide sequence and the IPC sequence using different processing paths within an attention-based machine-learning model to generate an output, wherein the output provides information about an immunological activity relating to both the mutant peptide and the IPC. Step 806 includes, for example, processing the peptide sequence through a corresponding representation block to generate a peptide representation that is processed through a corresponding attention block to generate a transformed peptide representation that represents the peptide sequence. This peptide processing path is separate from the IPC processing path in which the IPC sequence is processed through a corresponding representation block to generate an IPC representation (e.g., an MHC representation, a TCR representation, an MHC-TCR representation) that is processed through a corresponding attention block to generate a transformed IPC representation (e.g., a transformed MHC representation, a transformed TCR representation, a transformed MHC-TCR representation) that represents the IPC sequence.

In some embodiments, the peptide representation is part of an aggregate representation that also includes an N-flank representation for an N-flank sequence and/or a C-flank representation for a C-flank sequence. In such embodiments, the aggregate processing path (which would inherently include the peptide processing path) remains separate from the IPC processing path.

In various embodiments, in step 806, the transformed peptide representation and the transformed IPC representation are used to form a composite representation that is then further processed to generate the output. For example, the composite representation may be transformed using an attention block to generate a transformed composite representation that is then processed to generate the output. The output may include, for example, without limitation, a set of interaction predictions, a set of interaction affinity predictions, a set of immunogenicity predictions, or a combination thereof.

Step 808 includes generating a report based on the output. The report may include the output. In other embodiments, the report includes a transformed or filtered version of the output. In still other embodiments, the report includes a summary, synopsis, or visual representation of the output.

In some embodiments, process 800 further includes step 810. Step 810 includes performing a set of actions based on the report. The set of actions may include various actions relating to the design and/or manufacturing of a treatment based on the report.

Fig. 9 is a flowchart of a process for generating information about the immunological activity of various peptides. At least a portion of process 900 may be implemented using, for example, without limitation, prediction system 100 described in Fig. 1. For example, at least a portion of process 900 may be implemented using, for example, without limitation, machine learning model 132 from Figs. 1 and 3 or machine learning model 400 from Figs. 4A-4C.

Step 902 includes receiving sequence data that includes a plurality of peptide sequences and a plurality of IPC sequences.

Step 904 includes generating a plurality of peptide-IPC combinations using the peptide sequences and the IPC sequences. Each of the peptide-IPC combinations is a unique combination.

Step 906 includes inputting, for each peptide-IPC combination, the peptide sequence corresponding to the peptide-IPC combination into a peptide processing path of a machine learning model and the IPC sequence corresponding to the peptide-IPC combination into an IPC processing path of a machine learning model.

Step 908 includes processing, for each peptide-IPC combination, a peptide representation of the peptide sequence using a first attention block and processing an IPC representation of the IPC sequence using a second attention block to generate a transformed peptide representation and a transformed IPC representation, respectively.

Step 910 includes generating, for each peptide-IPC combination, a composite representation using the transformed peptide representation and the transformed IPC representation.

Step 912 includes processing, for each peptide-IPC combination, the composite representation using a third attention block to generate a transformed composite representation.

Step 914 includes generating an output based on the transformed composite representations. The output may provide an indication of which of the peptide sequences can may be used to generate a treatment. For example, the output may provide an indication of which peptide sequences (and thereby, a peptide that contains that peptide sequence) have a high likelihood of binding to an MHC, a high likelihood of being presented by an MHC, a high interaction affinity for the peptide-MHC binding, and/or a high likelihood of being immunogenic to thereby trigger an immune response.

II.C. 1. <u>Exemplary Methodology: Peptides and MHCs</u>

Fig. 10 is a flowchart of a process for training a machine learning model and using the trained machine learning model to generate predictions relating to peptides and MHCs in accordance with one or more embodiments. Process 1000 may be performed using prediction system 100 in Fig. 1. For example, process 1000 may be implemented using machine learning model 132 in Figs. 1 and 3 or machine learning model 400 in Figs. 4A-4C. In some instances, part or all of process 1000 may be performed at a remote computing system that is remote relative to a user device and/or laboratory. The remote computing system may be a cloud computing system.

Step 1002 includes accessing a training data set with training elements identifying training peptide sequence data, training MHC sequence data, and training immunological activity data. The training data set may be one example of an implementation for training data 133 in Fig. 1. The training immunological activity data may include, for example, interaction indications.

The training peptide sequence data may include, for example, one or more peptide sequences (which may include variant-coding sequences) for training. A peptide sequence can identify an ordered set of amino acids within a peptide (e.g., a neoantigen). The peptide sequence can identify amino acids within an epitope (e.g., that includes a variant and/or that includes or that is a neoepitope) of the peptide. In some embodiments, the peptide sequence is within an aggregate sequence that also include an N-flank sequence (e.g., characterizing a chain of amino acids at an N-terminus of the corresponding peptide) or a C-flank sequence (e.g., characterizing a chain of amino acids at an C-terminus of the corresponding peptide). Neither the N-flank nor the C-flank bind to an MHC molecule, though each may influence whether

it is presented by an MHC molecule.

The training MHC sequence data may include one or more MHC sequences for training. An MHC sequence may, for example, identify amino acids within part or all of an MHC molecule (e.g., an MHC-I molecule or an MHC-II molecule). The MHC sequence can include an MHC pseudosequence (e.g., that includes 34 amino acids). The MHC sequence can identify amino acids within, for example, 1, 2, 3, 4, 5 or 6 MHC alleles for MHC-I or 1,2,3,4,5,6,7,8,9,10,11,12 MHC allotypes for MHC-II. The MHC sequence can identify amino acids constituting part or all of an HLA molecule.

The training immunological activity data may include, for example, one or more interaction indications for one or more peptide-MHC combinations. For example, the training data set may include training elements, in which each training element includes a peptide sequence and an MHC sequence for training, as well as one or more interaction indications for the corresponding peptide-MHC combination. An interaction indication may indicate whether a target interaction (e.g., binding of peptide and MHC, presentation of peptide on cell surface by MHC) occurs between the peptide and MHC or an affinity for the target interaction.

The interaction indication may be, for example, a label. A negative interaction label may indicate that a peptide does not bind to and/or is not presented by an MHC molecule. A positive interaction label may indicate that a peptide binds to and/or is presented by an MHC molecule. Further, an interaction label may indicate a probability that the peptide binds to the MHC molecule, a probability that the MHC molecule presents the peptide at a cell surface, a binding affinity for the peptide-MHC combination, a strength of the binding between the peptide and the MHC molecule, a stability of the binding between the peptide and the MHC molecule, a tendency of the peptide to bind with the MHC, or another metric or characteristic associated with an interaction between the MHC and the peptide.

The training data set may have been generated via, for example, *in vitro* or *in vivo* experiments and/or based on medical records. The training data may have been generated based on one or more techniques disclosed in Section II.E below.

Accessing the training data set may include, for example, retrieving the training data set from a local or remote storage, loading the training data set, and/or requesting (and receiving) part or all of the training data set from one or more data stores (e.g., a cloud data storage, a server system, or some other data source).

In some instances, an initial training data set (e.g., which include variant-coding sequences) may include predominately negative data, in that a relatively small portion of the sequence combinations (e.g., peptide-MHC combinations) is found to be associated with an actual target interaction. The training data set may be designed to include negative training data elements. In some embodiments, a negative training data element may be defined to identify amino acids within a pseudo-randomly selected fragment of a protein of origin in the positive set (corresponding to observed presentation). For example, the negative training data element may be simulated based on the positive set. The fragment may be selected to have a length within a predefined range (e.g., between 8 and 14 amino acids for MHC-I and 8-30 amino acids for MHC-II, using a uniform probability). N-terminal and C-terminal flanking sequences may be retained within the negative training data element, potentially imposing a maximum length (e.g., of 10 amino acids). Any peptide fragment (e.g., at least a 9-mer) that overlapped with a positive peptide may be discarded from the negative training data.

In various embodiments, the negative training data elements are simulated based on the positive data elements. Further, the training data is selected such that a different set of negative training data elements is used per epoch of the training period. For example, for each epoch, a different "negative subset" of negative peptide sequences may be selected from the overall space of available negative peptide sequences identified based on the positive set of peptide sequences. The negative subset selected for each epoch may be unique in that no negative peptide sequence is repeated in any of the negative subsets for the total number of epochs. Thus, the training data used for each epoch of the training period includes the same positive set of peptide sequences but an entirely different set of negative peptide sequences. This technique, which may be referred to as "negative set switching" may provide overall robustness to the training and helps to ensure either a reduced number of false negatives (e.g., false negative indications/predictions) by the machine learning model or to ensure that no false negative is repeated more than once. Further, with this technique, the machine learning model may be trained on a total number of negative peptide sequences that is equal to the number of positive peptide sequences multiplied by the number of epochs in the training period.

Step 1004 includes training a machine learning model using the training data set. The machine learning model may be, for example, machine learning model 132 in Figs. 1 and 3 or the machine learning model may be, for example, machine learning model 400 in Figs. 4A-4C.

Machine learning model 132 may be trained using a static or dynamic learning rate. A dynamic learned rate can be produced using, for example, learning-rate annealing. Training may be performed using, for example, a classification loss function and/or a regression loss function. A loss function can be based on, for example, mean square error, median square error, mean absolute error, median absolute error, an entropy-based error, a cross entropy error, and/or a binary cross entropy error. Validation data (e.g., a separated subset of the training data set used to train the machine learning model 132 may be used to assess a performance of machine-learning model 132 as it is being trained. Training may be terminated if and/or when a target performance is obtained, and/or a maximum number of training iterations have been completed.

Step 1006 includes accessing a subject-specific set of variant-coding sequences corresponding to a set of mutant peptides. As described above, a variant-coding sequence is one example of a peptide sequence. The subject-specific set of variant-coding sequences can correspond to a set of mutant peptides, such that each of the subject-specific set of variant-coding sequences identifies amino acids within a corresponding mutant peptide of the set of mutant peptides and/or such that each of the subject-specific set of variant-coding sequences identifies one or more amino acids in a mutation. Each of the subject-specific set of variant-coding sequences can be associated with a particular subject (e.g., human subject). The particular subject may have been diagnosed with, may have. and/or may have experienced symptoms and/or received test results associated with a particular medical condition (e.g., cancer). For example, the subject-specific set of variant-coding sequences may have been identified by processing a sample from a tumor. The sample may be or may be included within, for example, set of samples 112 in Fig. 1.

The subject-specific set of variant-coding sequences may be identified using a technique disclosed herein (e.g., in Section II.D). For example, the subject-specific set of variant-coding sequencings may have been identified by performing a sequencing technique to identify peptides in a disease sample and comparing the identified peptides to those detected in a healthy sample or reference database to identify unique sequences. In some embodiments, if the unique sequences are nucleic-acid sequences, each unique nucleic-acid sequence may be transformed into an amino-acid sequence.

Each of the subject-specific set of variant-coding sequences can identify amino acids within a peptide (which may be amino acids within the neoepitope of a neoantigen). In some instances, each of one, more. or all the subject-specific set of variant-coding sequences may be part of a corresponding aggregate sequence that further includes a sequence at an N-flank of the peptide and/or a sequence at a C-flank of the peptide.

Accessing the subject-specific set of variant-coding sequences can include, for example, retrieving the subject-specific set of variant-coding sequences from a local or remote storage and/or requesting the subject-specific set of variant-coding sequences from another device. Accessing the subject-specific set of variant-coding sequences can include and/or can be performed in combination with determining the subject-specific set of variant-coding sequences.

The subject-specific set of variant-coding sequences may have been obtained be identifying peptide sequences within a disease sample of the subject and determining which of the peptide sequences are not represented within a reference, healthy-sample and/or wild-type sequence set. In instances in which a healthy sample is used for the comparison, the healthy sample may have been (but need not have been) collected from the subject.

Step 1008 includes accessing an MHC sequence corresponding to an MHC. The MHC sequence may include, for example, a pseudosequence of an MHC (e.g., MHC molecule) within the sample collected from a subject. In some instances, the MHC sequence and the subject-specific set of variant-coding sequences are identified from a same sample from the subject or from multiple samples from the subject (e.g., a disease sample and a healthy sample). In some instances, the MHC sequence and the subject-specific set of variant-coding sequences are identified from samples from the subject and one or more other subjects. Thus, in some cases, the MHC sequence may be subject-specific. The MHC sequence may be or may have been determined using, for example, a sequencing and/or mass-spectrometry technique.

Accessing the MHC sequence may include, for example, retrieving the MHC sequence from a local or remote storage and/or requesting the subject-specific MHC sequence from another device. Accessing the MHC sequence can include and/or performed in combination with determining the MHC sequence.

Step 1010 includes, for example, processing the set of subj ect-specific variant-coding sequences and the MHC sequence using the trained machine learning model to generate an output. Step 1010 may include processing each unique combination (e.g., variant-coding-MHC combination or peptide-MHC combination) of a subject-specific variant-coding sequence of the set of subject-specific variant-coding sequences and the MHC sequence to generate the output.

The output generated by the machine learning model may be include a same or similar type of data as included in the training immunological activity data used to train the machine-learning model. For each unique combination, the machine-learning model generates an output that includes at least one of a set of interaction predictions or a set of interaction affinity predictions.

An interaction prediction in the set of interaction predictions includes a prediction about whether a target interaction between a mutant peptide that includes the variant-coding sequence and an MHC that includes the MHC sequence will occurs. For example, the interaction prediction may include a binary or categorical prediction as whether a mutant peptide with an amino-acid structure as indicated by the subject-specific variant-coding sequence will be presented by and/or bind to an MHC molecule with an amino-acid structure as indicated by the MHC sequence. An interaction affinity prediction in the set of interaction affinity predictions includes a prediction about an affinity for the target interaction. This affinity may be defined based on, for example, the strength, tendency, and/or stability of the target interaction. For example, the interaction affinity prediction may include a predicted real-number binding affinity associated with a mutant peptide that includes amino acids identified within the subject-specific variant-coding sequence and an MHC molecule including amino acids as identified within the MHC sequence.

Step 1012 includes generating a report based on the output of the machine-learning model. The report may be implemented as, for example, report 144 in Figs. 1 and 3. The report may be or include the output. In some cases, the report

may be a transformed or filtered version of the output.

In one or more embodiments, the subject-specific set of variant-coding sequences is filtered, ranked, and/or otherwise processed based on the output to generate information for inclusion in the report. For example, the subject-specific set of variant-coding sequences may be filtered to exclude sequences for which a predicted interaction affinity (e.g., binding affinity) was below a predefined affinity threshold and/or for which it was predicted that the target interaction (e.g., presentation by or binding to the MHC molecule) would not or would be unlikely to occur. In some instances, a filtering is performed to identify a predetermined number and/or fraction of the subject-specific set of variant-coding sequences. For example, a filtering can be performed to identify 10, 20, 40, 60, 80, 100, 500 or 1,000 variant-coding sequences associated with relatively high predicted probabilities (e.g., relative to unselected variant-coding sequences in the subject-specific set of variant-coding sequences) as to whether the mutant peptide will bind to and/or be presented by an MHC molecule.

The report may identify one or more variant-coding sequences (e.g., that were not filtered out from the set) and/or one or more mutant peptides (e.g., associated with selected variant-coding sequences). A mutant peptide may be identified by, for example, its name, by its sequence, and/or by identifying both a corresponding wild-type sequence and a variant represented in a variant-coding sequence.

The report may, in some embodiments, identify one or more predictions associated with one or more variant-coding sequences or one or more mutant peptides. The report may include a name of the subject. The report may, for example, be presented locally (e.g., for display on a display system of a user device, sent as a notification on a user device, etc.) and/or transmitted to another device (e.g., sent to a cloud computing system, sent to a cloud storage, sent to a user device associated with a medical profession or laboratory professional, transmitted as an email, etc.).

Fig. 11 is an illustration that includes a table of training data in accordance with one or more embodiments. Table 1100 comprises training data 1102 (e.g., a training data set). Training data 1102 may be one example of a portion of training data 133 in Fig. 1. Training data 1102 may be one example of a portion of a training data set such as the training dataset described in step 1002 in Fig. 11.

Training data 1102 includes allele identifier 1106, training N-flank sequence 1108, training peptide sequence 1110, training C-flank sequence 1112, training MHC sequence 1114 (e.g., MHC pseudosequence), binding affinity 1116, and presentation indication 1118. Binding affinity 1116 indicates the detected (e.g., observed) binding affinity for the binding of the peptide characterized by training peptide sequence 1110 and the respective MHC characterized by training MHC sequence 1114. Presentation indication 1118 indicates whether the binding or presentation of the peptide by the MHC was detected (or observed).

Fig. 12 is an illustration of a neoantigen candidate and the corresponding potential neoepitope candidates in accordance with one or more embodiments. When a process such as process 1000 is implemented, a mutant peptide may be a neoantigen.

For a relatively long mutant peptide that is a neoantigen candidate 1200, it is possible that multiple epitopes (referred to as neoepitopes), all containing the same mutation or variant, may be presented by an MHC molecule. Thus, the immunogenicity of the neoantigen candidate may be predicted based on predictions generated for each of the neoepitope candidates 1202.

The immunogenicity can be predicted by, for example, generating a list of all possible neoepitopes that could emerge from a given neoantigen and producing predictions for each of some or all of the neoepitope candidates (with the flanks constituting the remaining amino acids upstream of the N-terminus and downstream of C-terminus of the epitope, up to 10 amino acids in length) in the list. From these presentation predictions the neoepitope candidate with the largest presentation likelihood with respect to the MHC candidates 1204 is chosen to represent the entire neoantigen. Alternatively, a summarized representation of multiple candidate neoepitope-MHC pairs may be used to obtain a summarized score representing the neoantigen. Such summarization may be conducted by either considering all candidate neoepitope-MHC pairs or by considering the best neoepitope per MHC and then summarizing across all MHC molecules. The summarization can be done by several mathematical functions including, for example, taking the arithmetic mean or harmonic mean of the presentation or binding affinity score of each candidate neoepitope-HLA pair.

Although Fig. 12 is described with respect to neoantigens and neoepitopes, a similar technique may be used for other types of relatively long mutant peptides containing a mutation or variant and having multiple possible epitope candidates. In some embodiments, this technique may be used in conjunction with antibody drug sequences.

II.C.2. <u>Exemplary Methodology: Peptides and TCRs</u>

Fig. 13 is a flowchart of a process for training a machine learning model and using the trained machine learning model to generate predictions relating to peptides and TCRs in accordance with one or more embodiments. Process 1300 may be performed using prediction system 130 in Fig. 1. For example, process 1300 may be implemented using machine learning model 132 in Figs. 1 and 3 or machine learning model 400 in Figs. 4A-4C. In some instances, part or all of process 1300 may be performed at a remote computing system that is remote relative to a user device and/or laboratory. The

remote computing system may be a cloud computing system. Steps 1302-1312 may be implemented in a manner similar steps 1002-1012 in Fig. 10, but with respect to TCRs.

Step 1302 includes accessing a training data set with training elements identifying training peptide sequence data, training TCR sequence data, and training immunological activity data. The training TCR sequence data may include one or more TCR sequences for training. A TCR sequence may, for example, identify amino acids within part or all of a TCR molecule.

The training immunological activity data may include, for example, one or more interaction indications for one or more peptide-TCR combinations and/or one or more immunogenicity predictions. The immunogenicity prediction may predict immunogenicity of a peptide with respect to TCR. For example, the training data set may include an interaction label that indicates whether a mutant peptide with amino acids as identified by a variant-coding sequence triggered an immunological response (e.g., whether the mutant peptide is immunogenic). Immunogenicity may indicate that the mutant peptide activated a T-cell receptor (e.g., a receptor of a CD8+ cytotoxic T lymphocyte or CD4+ helper T cell) and/or triggered an immunological response.

The training data set may have been generated by, for example, expressing various mutant peptides in a sample (e.g., one or more dendritic cells) and/or introducing various mutant peptides (e.g., to a sample or to a subject from which a sample was subsequently collected) via immunization and/or by a vaccine. The mutant peptides may have been expressed or introduced individually (e.g., thereby focusing each experiment on a single mutant peptide) or in groups.

Immunogenicity may have been tested by, for example, analyzing tumor infiltrating cells. It may have been determined that a mutant peptide triggered an immunological response (and is therefore immunogenic) if, for example, epitopes of the mutant peptide are detected (e.g., at a quantity above a threshold), a measured level of interferon gamma (IFN-y) or T cell immunoglobulin mucin-3 (TIM-3) exceeded a corresponding threshold, a detected quantity of cytotoxic T cells (e.g., in general or cytotoxic T cells displaying an epitope corresponding to the mutant peptide) exceeded a corresponding threshold, and/or at least a threshold degree of apoptosis is observed. As another example, the mutant peptide may have been expressed in a sample (e.g., one or more dendritic cells). It may have been determined that the mutant peptide triggered an immunological response (and is therefore immunogenic) if, for example, it is determined that the presented peptide is subsequently recognized by a T cell. It will be appreciated that some embodiments include collecting and/or determining at least part of the training data set (e.g., by performing one or more experiments and/or analyses disclosed herein).

Accessing the training data set may include, for example, retrieving the training data set from a local or remote storage, loading the training data set, and/or requesting (and receiving) part or all of the training data set from one or more data stores (e.g., a cloud data storage, a server system, or some other data source).

Step 1304 includes training a machine learning model using the training data set. The machine learning model may be, for example, machine learning model 132 in Figs. 1 and 3 or the machine learning model may be, for example, machine learning model 400 in Figs. 4A-4C.

Step 1306 includes accessing a subject-specific set of variant-coding sequences corresponding to a set of mutant peptides.

Step 1308 includes accessing a TCR sequence corresponding to a TCR. In some instances, the TCR sequence and the subject-specific set of variant-coding sequences are identified from a same sample from the subject or from multiple samples from the subject (e.g., a disease sample and a healthy sample). In some instances, the TCR sequence and the subject-specific set of variant-coding sequences are identified from samples from the subject and one or more other subjects. Thus, in some cases, the TCR sequence may be subject-specific. The TCR sequence may be or may have been determined using, for example, a sequencing and/or mass-spectrometry technique.

Accessing the TCR sequence may include, for example, retrieving the TCR sequence from a local or remote storage and/or requesting the subject-specific TCR sequence from another device. Accessing the TCR sequence can include and/or performed in combination with determining the TCR sequence.

Step 1310 includes, for example, processing the set of subject-specific variant-coding sequences and the TCR sequence using the trained machine learning model to generate an output. Step 1310 may include processing each unique combination (e.g., variant-coding-TCR combination or peptide-TCR combination) of a subject-specific variant-coding sequence of the set of subject-specific variant-coding sequences and the TCR sequence to generate the output.

The output generated by the machine learning model may be include a same or similar type of data as included in the training immunological activity data used to train the machine-learning model. For each unique combination, the machine-learning model generates an output that includes a set of immunogenicity predictions. An immunogenicity prediction in the set of immunogenicity predictions may indicate whether the mutant peptide triggered an immunological response (and is therefore immunogenic). In some cases, the immunogenicity prediction indicates a degree of immunogenicity (e.g., low, medium, high, very high, etc.).

Step 1312 includes generating a report based on the output of the machine-learning model. The report may be implemented as, for example, report 144 in Figs. 1 and 3. Step 1312 may be implemented in a manner similar to step 1012 in Fig. 10.

II.C.3. Exemplary Methodologies: Additional Considerations for Training and Prediction Using the Machine Learning Model

Thus, the embodiments described herein provide a machine learning model that can be used to generate predictions for the immunological activity associated with a peptide, which may be a mutant peptide. A peptide sequence that characterizes a mutant peptide-e.g., a variant-coding sequence-may be analyzed by the machine learning model with an IPC sequence characterizing an IPC in order to generate one or more predictions about one or more target interactions (interactions of interest) between the peptide and IPC and/or about the ability of the peptide to provoke an immune response. An output generated by the machine learning model may thus comprise one or more results that provide information about the one or more target interactions and/or the peptide's immunogenicity.

In some embodiments, one or more variant-coding sequences can be selected from a set of subject-specific set of variant coding sequences based on results from one or more machine-learning models described herein. Input data can include representations of an MHC sequence and a variant-coding sequence that corresponds to a mutant peptide. The machine-learning model may be trained using binding-affinity data and mass-spectrometry elution data that indicates which peptides are presented by MHC molecules. The binding-affinity data may include qualitative data (*e.g.,* as determined using ELISAs, pull-down assays and/or gel-shift assays, fluorescence resonance energy transfer assays and mass spectrometry assays) or quantitative data (*e.g.,* using a biosensor-based methodology, such as Surface Plasmon Resonance, Isothermal Titration Colorimetry, BioLayer Interferometry or MicroScale Thermophoresis). In some instances, binding affinity data can include data from a competitive binding assay, data from the Immune Epitope Database and/or data of a type that is in the Immune Epitope Database. Elution data can be collected using peptide-MHC immunoprecipitation, followed by elution and detection of presented MHC ligands by mass spectrometry. Training data included "positive" instances (for which mass-spectrometry results indicate that a peptide was presented by an MHC molecule) and "negative" instances (corresponding to, for example, simulated length-matched n-mers (nmers)) from the same proteins as positive instances but that were not detected in mass-spectrometry assessments).

In some instances, a quantity of positive instances in the training data is equal to a quantity of negative instances in the training data. In some instances, a quantity of positive instances is less than or greater than a quantity of negative instances. Each of one, more or all of the negative instances in the training data may be length-matched to a positive instance in the training data. In some instances, all of the sequences in the training data have a same length.

Part or all of a sequence may be represented, for example, using a data encoding. An encoding may be performed in accordance with a known and/or static rule or technique and/or using a trained network. For example, an encoding may include a one-hot encoding, such that each encoded sequence indicates, for each position of a sequence and for each of a set of (e.g., 21) amino acids, whether the particular amino acid is present at the position. Alternatively, evolution-motivated encodings such as BLOSUM, or learned encodings may be used for representing amino acids in a sequence. An encoding may include a positional encoding (e.g., a learned or fixed encoding).

In some instances, the machine-learning model includes one or more neural networks that are used for sequence processing. The neural network(s) can further or alternatively include, for example, an encoder neural network and/or part or all of a transformer network.

The machine-learning model can include an attention-based machine-learning model that includes one or more neural networks that are attention-based, lack any convolutional layer and/or lack any recurrent layer. The attention-based machine-learning model may (but need not) further include one or more other neural networks that are not attention-based, include one or more convolutional layers and/or include one or more recurrent layers.

An attention-based network may use a set of query weights, a set of key weights and a set of value weights to determine, for a given amino-acid representation, an extent to which each of one or more other amino acid representations are to be "attended to" when processing the given amino-acid representation. A self-attention layer can use keys, values and queries from a same layer, such that, for example, an encoder or decoder can attend to all positions in a previous layer of the encoder or decoder.

When predicting whether a given mutant peptide will bind to and/or be presented by a particular MHC molecule, one or more transformer encoders may separately process representations of different parts or all of the variant-coding sequence and/or MHC sequence. Each transformer encoder can include a self-attention layer and a feed-forward layer. Each attention layer can further include one or more embedding components configured to, for example, perform positional and/or non-positional embedding. In some instances, sequences of each of the N-flank region of a mutant peptide, epitope region of the mutant peptide, C-flank region of a mutant peptide, and the MHC molecule are separately processed different iterations of a transformer encoder. An encoded representation of a sequence may include, for each amino acid in the sequence, a feature vector representing the amino acid. Encoded representations of the sequences can then be concatenated and fed to yet another iteration of the transformer encoder. The concatenation may thus include a feature vector for each amino acid in part or all of the variant-coding sequence and for all or part the MHC sequence.

One or more additional feature vectors may be included in the concatenation. Each of the additional features may be, for example, assigned random or pseudorandom values for the feature vector. The concatenated representation (e.g.,

that includes the additional feature vector(s)) may be processed by an additional transformer encoder to generate an encoded concatenated representation. This encoded representation of the sequence combination may be processed by a feedforward network (e.g., a fully connected neural network) where dropout and/or batch normalization can be applied. In some instances, the encoded representation(s) of the additional feature vector(s) are selectively passed to the feedforward network (e.g., while feature vectors corresponding to individual amino acids of the MHC molecule and/or mutant peptide are not). For example, suppose that a subsequence of an MHC molecule includes $x_1$ amino acids, that a subsequence of a mutant peptide (e.g., and one or more flanks) includes $x_2$ amino acids, and that a feature transformation identifies y feature values to represent each amino acid. A concatenated representation that includes 1 additional feature vector could thus have a size of $[(x_1+x_2+1), y]$. Input fed to a feedforward network may have a size of $[1, y]$, in a case where one feature vector is selected for processing by the feedforward network. An advantage of using the additional-element approach is that the model can then process sequences of variable length.

Results produced by the feedforward network can correspond to predictions as to binding affinities between the mutant peptide and MHC molecule (e.g., an MHC molecule of the subject) and/or whether the mutant peptide will be presented by the MHC molecule. A binding-affinity prediction may be, for example, numeric (e.g., corresponding to a predicted probability that the mutant peptide will bind to the MHC molecule, a predicted binding strength and/or a predicted binding stability), categorical (e.g., predicting no, low or high binding stability between the mutant peptide and the MHC molecule) or binary (e.g., predicting whether the mutant peptide binds to the MHC molecule).

A presentation prediction generated in association with a mutant peptide may be, for example, numeric (e.g., corresponding to a predicted probability that an MHC molecule of the subject presents the mutant peptide at a cell surface or a predicted fraction of tumor cells in the subject that present the mutant peptide), categorical (e.g., predicting no, infrequent or frequent presentation of the mutant peptide by MHC molecules of the subject) or binary (e.g., predicting whether the mutant peptide is expressed by MHC molecules in the subject). A presentation prediction may (but need not) be normalized and/or represent a conditioned prediction. For example, a presentation prediction may correspond to a prediction as to whether an MHC molecule of the subject presents the mutant peptide if the mutant peptide has stably bound to the MHC molecule.

In some instances, a machine-learning model generates predictions corresponding to one or more potential interactions between a mutant peptide and an MHC-I molecule. For example, the machine-learning model may predict binding affinity of the MHC-I molecule and a mutant peptide and/or whether the MHC-I molecule will present the mutant peptide. The machine-learning model may receive, as input, and may process (e.g., using one or more self-attention layers) a sequence or subsequence of the MHC-I molecule and the variant-coding sequence associated with the mutant peptide.

In some instances, a machine-learning model generates predictions corresponding to one or more potential interactions between a mutant peptide and an MHC-II molecule. For example, the machine-learning model may predict a binding affinity for the MHC-II molecule and a mutant peptide and/or whether the MHC-II molecule will present the mutant peptide. The machine-learning model may receive, as input, and may process (e.g., using one or more self-attention layers) a sequence or subsequence of the MHC-II molecule and the variant-coding sequence of the mutant peptide.

In some instances, a machine-learning model generates predictions corresponding to one or more potential interactions between a mutant peptide, an MHC sequence or subsequence, and a T-cell receptor (e.g., instead of or in addition to generating predictions corresponding to one or more potential interactions between a mutant peptide and an MHC molecule). The machine-learning model may then predict, for example, a binding affinity between the mutant peptide and T-cell receptor and/or whether the mutant peptide activates and/or triggers an immunological response in the T-cell. The machine-learning model may receive, as input, and may process (e.g., using one or more self-attention layers) a sequence or subsequence of the T-cell receptor, a sequence or subsequence of MHC, and the variant-coding sequence of the mutant peptide

The immunogenicity of a mutant peptide (e.g., in relation to a particular subject) can be predicted based on one or more results generated by a machine-learning model disclosed herein (e.g., an attention-based machine-learning model). For example, it may be predicted that a neoantigen detected from a subject's disease sample will not trigger immunogenicity or will have low immunogenicity when a machine-learning-model result predicts that the mutant peptide will have low binding affinity with an MHC molecule; that an MHC molecule will not or is not likely to present the mutant peptide; and/or that a mutant peptide will not trigger an immunological response by a T-cell receptor. An immunogenicity prediction generated in association with a mutant peptide may be, for example, numeric (e.g., corresponding to a predicted probability that an immunogenicity response would be triggered in response to the mutant peptide and/or corresponding to a predicted intensity of any immunogenicity response to the mutant peptide), categorical (e.g., predicting no, low or high immunological response) or binary (e.g., predicting whether a given mutant peptide triggers an immunological response in the subject).

A predicted immunogenicity may further be based on predictions and/or experimental indications of one or more immunogenicity factors. Factors that dictate immunogenicity can include: i) a protein level of a mutant-peptide precursor; ii) an expression level of a transcript encoding the mutant-peptide precursor; iii) a processing efficiency of the mutant-peptide precursor by the immunoproteasome; iv) a timing of the expression of the transcript encoding the mutant-peptide precursor; v) a binding affinity of the mutant peptide to a T-cell receptor; vi) a position of a variant amino acid within the mutant peptide; vii) solvent exposure of the mutant peptide when bound to a MHC molecule; vii) a solvent exposure of the variant amino acid when bound to a MHC molecule; x) the content of aromatic residues in the peptide; xi) properties of the variant amino acid when compared to a wild type residue; and/or xii) a nature of the mutant-peptide precursor; xiii) microbial similarity of the mutant peptide to know microbial peptides; xiv) self-similarity or dissimilarity of the mutant peptide to the wild type proteome, xv) thymic expression of the wild type peptide. Immunogenicity factors can further or additionally include: a protein sequence and/or length of a mutant peptide (e.g., as indicating by a number of amino acids identified within the variant-coding sequence) and/or an expression level of an MHC allele in the subject (e.g. as measured by RNA-Seq or mass spectrometry).

Binding affinity predictions and/or predictions as to whether (or a probability that) mutant-peptide presentation will occur (e.g., by one or more tumor cells and/or one or more MHC molecules in the subject) may be generated in accordance with a technique disclosed herein (e.g., using an attention-based machine-learning model) for each of a set of mutant peptides (e.g., that were detected within a disease sample from a subject). These predictions can be used to select an incomplete subset of the set (e.g., less than 50% of the set, less than 25% of the set, less than 10% of the set, less than 5% of the set and/or less than 1% of the set). The incomplete subset may be selected using one or more relative thresholds (e.g., to identify mutant peptides within the set that have the most stable bounds with MHC molecules and/or the highest likelihoods of being presented relative to others in the group) or one or more absolute thresholds. For example, each selected mutant peptide can have a binding affinity with MHC with a relatively strong affinity value (e.g., within a best 50%, best 25%, best 10% or best 5% affinity values within the set) and/or absolutely strong affinity value (e.g., having an affinity value of better than a predefined threshold/cutoff, such as 5000 nM, 1000 nM or 500 nM, in case of IC50 values). The incomplete subset of the set may include 1, 2, 3, 4, 5, 6, 7, 8, 9, 10 or more mutant peptides irrespective of a predefined affinity value threshold/cutoff. The incomplete subset of the set may include 20 or more neoantigens or 30 or more mutant peptides.

Each selected mutant peptide may be manufactured, experimentally tested (e.g., to determine a binding affinity, presentation prevalence and/or other immunological factor), included in a composition (e.g., a pharmaceutical composition, such as a vaccine and/or treatment), and/or administered to a subject.

Each of the set of mutant peptides for which binding-affinity and presentation predictions are generated may include a mutant peptide associated with a particular subject (e.g., a particular human subject). Each of the set of mutant peptides can be a disease-specific, immunogenic mutant peptide identified using a disease-specific sample from an individual. The individual variant-coding sequence can be identified by sequencing genetic and/or nucleic-acid sequences (e.g., DNA, RNA and/or mRNA sequences) in a disease sample and comparing each identified genetic and/or nucleic-acid sequence to a reference-sample sequence. Codons within a genetic and/or nucleic-acid sequence are indicative of existence of a corresponding amino acid in a peptide. Notably, each of multiple codons may encode a given amino acid, so while a nucleic-acid sequence can indicate (e.g., deterministically) an amino-acid sequence, the same amino-acid sequence may be encoded by other nucleic-acid sequences.

Some of the sequences identified in a disease sample may be non-disease sequences that correspond to non-disease peptides. To identify disease-specific nucleic-acid sequences and/or disease-specific amino-acid sequences, for each sequence that is detected as a result of sequencing the disease-specific sample, it may be determined whether the sequence is also identified in a reference sequence data set. The reference sequence data set can include a set of reference sequences for which it is known, inferred or assumed that the sequence is not indicative or characteristic of a disease (e.g., any disease or a given disease). The reference sequence data set may, for example, include sequences identified by sequencing one or more reference sample sequences collected from a same subject from which the disease-specific sample was collected, sequencing one or more reference sample sequences collected from one or more other subjects not diagnosed with any disease or a disease corresponding to the disease-specific sample and/or sequencing one or more cell lines not associated with the specific disease. In some instances, the reference sequence data set may include sequences collected from one or more reference data repositories. A sequence that is detected in association with the disease-specific sample but that is not detected (or detected at a frequency below a predefined threshold) in a reference sequence data set can be classified as a variant-coding sequence (e.g., generally or for a subject from which the disease-specific sample was collected).

In some instances, multiple variant-coding sequences may be identified (e.g., each having been detected in the disease sample but not being represented in the reference-sample sequences), and a representation of each of the multiple variant-coding sequences can be processed (e.g., individually, sequentially and/or in parallel) using a machine-learning model disclosed herein (e.g., an attention-based machine-learning model) disclosed herein to predict a binding affinity and/or presentation prediction.

The disease sample can include, for example, tissue (e.g., a solid tumor), blood and/or a collection of cells (e.g., cancer cells, which may have been collected using fine need aspiration or laparoscopy). The disease sample may include cancerous cells collected from a subject that has been diagnosed with and/or that has, for example, lung cancer,

melanoma, breast cancer, ovarian cancer, prostate cancer, kidney cancer, gastric cancer, colon cancer, testicular cancer, head and neck cancer, pancreatic cancer, brain cancer, B-cell lymphoma, acute myelogenous leukemia, chronic myelogenous leukemia, chronic lymphocytic leukemia, and T cell lymphocytic leukemia, non-small cell lung cancer, or small cell lung cancer.

In some instances, an initial sample is separated into a disease sample and another remainder sample (e.g., which may be discarded or used as a reference sample). The reference sample can include a matched disease-free sample. Each of the disease sample and the reference sample may be collected from a same subject and/or may include or may be of a same or similar sample type (e.g., tissue type). In some instances, the disease sample is collected from a first subject (e.g., who has been diagnosed with a medical condition or disease), and the reference sample is collected from a different second subject (e.g., who has not been diagnosed with the medical condition or disease). In some instances, the reference-sample sequences are retrieved from a database of known genes associated with an organism.

Training data may further include sequences of one or more peptides, along with indications as to whether each of the peptides bound to an MHC molecule, was presented by an MHC molecule and/or triggered an immunological response. To collect training data that associates sequence data with observed presentation and/or binding data, the disease sample (and potentially the reference sample) may be (separately) processed to isolate MHC/peptide complexes (e.g., by performing immunoprecipitation using an antibody specific for MHC) and/or eluting (and thereby sequencing) the peptides from the MHC molecules (e.g., using chromatography and/or mass spectrometry). In some instances, reference-sample sequences are identified for use in generating presentation data by sequencing one or more cell lines engineered to express one or more MHC alleles (e.g., that were detected in the disease sample), which can include MHC class-I alleles and/or MHC class-II alleles. The one or more cell lines can include one or more human cell lines obtained or derived from one or more subjects. For purposes of this description, peptide sequences that are identified using a disease sample but that are not represented in a set of reference-sample sequences can be identified as variant-coding sequences.

In some embodiments, collecting immunogenicity-indicative metrics to use for training may be based on HLA-typing analysis, which can identify a subject-specific MHC molecule profile. When the subject is a human, this profile may be referred to as a Human Leukocyte Antigen (HLA) profile, as the HLA complex is a gene complex encoding MHC proteins in humans. An HLA-typing analysis can be performed using a sample (e.g., normal-tissue and/or non-disease sample) from the subject. The profile may be determined using a sequencing technique, such as PCR-based sequencing, direct sequencing and/or next-generation sequencing. The HLA-typing analysis may include, for example, high-resolution typing (e.g., which excludes indicating null alleles that are not expressed on the cell surface) or allele-level typing (e.g., which refers to exact nucleotide sequence HLA-gene determination). The HLA-typing analysis may include low-resolution typing and/or HLA supertyping that identifies broader families of alleles.

With respect to any type of sequencing (e.g., to identify sequences in a sample, peptides bond to an MHC molecule, HLA typing), a result may identify one or more nucleic-acid sequences or one or more amino-acid sequences. When nucleic-acid sequences are identified and an attention-based model (or other processing) is configured to process amino-acid sequences, a technique (e.g., lookup table) may be used to convert individual codons within the nucleic-acid sequences into individual amino acids.

Some embodiments including synthesizing a peptide (e.g., using a nucleic-acid sequence encoding a peptide, such as a selected peptide) or a precursor to a selected peptide. The synthesized peptide or precursor may then be used in an experiment to identify corresponding presentation and/or binding data (e.g., to verify predicted presentation and/or binding or to generate results to use for training). For example, an experiment may include assessing binding affinity of a selected peptide with a particular MHC molecule using an ELISA pull-down assay, gel-shift assays, or a biosensor-based methodology. As another example, an experiment may include collecting elution data indicative of whether a selected peptide was presented by an MHC molecule by using peptide-MHC immunoprecipitation, followed by elution and detection of presented MHC ligands by mass spectrometry.

In addition to or instead of training or verification data indicating whether individual peptides bound to and/or were presented by individual MHCs, training or verification data may indicate whether individual peptides triggered immunogenicity. Immunogenicity results may be determined using in vivo or in vitro testing. Testing the one or more selected peptides can be configured to investigate one or more immunogenicity factors (e.g., to determine whether and/or an extent to which a given event occurs) and/or immunogenicity (e.g., to determine whether and/or an extent to which the peptide triggers an immunological response). Testing can be configured to investigate whether administration of a composition (e.g., a vaccine) that includes one or more peptides to a given subject (e.g., for which an MHC sequence that was used during mutant-peptide selection has been identified) is effective in preventing or treating a medical condition (e.g., tumor) or disease (e.g., cancer). The subject may be a human subject.

Some embodiments include manufacturing a composition based on one or more selected mutant peptides (or a plurality of nucleic acids encoding the one or more selected mutant peptides). For example, each of the one or more selected mutant peptides may have been predicted to bind to and be presented by an MHC molecule of the subject (e.g., at least to a threshold degree). The composition may include each of the one or more selected mutant peptides, one or more precursors to the one or more selected mutant peptides, one or more polypeptide sequences corresponding to the one or

more selected mutant peptides, RNA (e.g., mRNA) corresponding to the one or more selected mutant peptides, DNA corresponding to the one or more selected mutant peptides, cells (e.g., antigen-presenting cells) including the one or more selected mutant peptides and/or nucleic acid(s) encoding such peptides, plasmids corresponding to the one or more selected mutant peptides and/or vectors corresponding to the one or more selected mutant peptides.

The composition may further include an adjuvant, an excipient, an immunomodulator, a checkpoint protein, an antagonist of PD-1 (e.g., an anti-PD-1 antibody) and/or an antagonist of PD-L1 (e.g., an anti-PD-L1 antibody). The composition may be a vaccine, such as a tumor vaccine. The composition may be an individualized vaccine manufactured or selected for a particular subject.

The composition may include a polynucleotide construct (e.g., a DNA construct or an RNA construct). The polynucleotide construct is an artificially constructed segment of nucleic acid which may be 'transplanted' into a target tissue or cell. The polynucleotide construct comprises a DNA or RNA (e.g., mRNA) insert, which contains the nucleotide sequence encoding the one or more selected mutant peptides. In order to increase antigen presentation (e.g., presentation of the one or more selected mutant peptides by a MHC molecule), the polynucleotide construct may further comprise a modification developed for improved antigen presentation, and thus improved immunogenicity to the one or more selected mutant peptides. In some instances, the modification is incorporation of a transmembrane region and a cytoplasmic region of a chain of the MHC molecule into the polynucleotide construct as described in International Publication WO2005038030A1, which is incorporated herein by reference in its entirety for all purposes.

To provide an RNA insert with increased stability and translation efficiency, the polynucleotide construct may further comprise a modification developed for improved stability and translation, and thus improved immunogenicity to the one or more selected mutant peptides. In some instances, the modification is incorporation of a nucleic acid sequence with at least two copies of a 3' - untranslated region of a human beta-globin gene into the polynucleotide construct as described in International Publication WO2007036366A2, which is incorporated herein by reference in its entirety for all purposes. In other instances, the modification is incorporation of a nucleic acid sequence that codes for a 3'-untranslated region such as F1 3' UTR described in International Publication WO2017060314A3, which is incorporated herein by reference in its entirety for all purposes.

To provide an RNA insert with increased stability and expression, the polynucleotide construct may further comprise a modification developed for improved stability and expression, and thus improved immunogenicity to the one or more selected mutant peptides. In some instances, the modification is incorporation of a cap on an end of the RNA such as a 5' - cap structure. The cap structure may be the D1 diastereomer of beta-S-ARCA as described in International Publication WO2011015347A1, which is incorporated herein by reference in its entirety for all purposes.

In order to deliver the polynucleotide construct with high selectivity to antigen presenting cells, the composition may further include cationic liposomes or a lipoplex for improved uptake of the polynucleotide construct, and thus improved immunogenicity to the one or more selected mutant peptides. In some instances, the composition includes nanoparticles comprising the polynucleotide construct. The nanoparticles may be lipoplexes comprising one or more lipids such as DOTMA and DOPE as described in International Publication WO2013143683A1, which is incorporated herein by reference in its entirety for all purposes.

Some embodiments include treating a medical condition (e.g., tumor) or disease (e.g., cancer) in an individual by administering, to the individual, an effective amount of a composition (e.g., a vaccine) including one or more selected mutant peptides. The individual may be the same individual from whom a disease sample was collected. In some instances, the vaccine is administered to a different individual as compared to the individual from whom the disease sample was collected. The different individual may, for example, be related to the individual from whom the disease sample was collected, have a genetic risk of developing a particular type of cancer, and/or have MHC molecules that have one, more or all alleles corresponding to a sequence that are the same (or similar) to one or more MHC alleles of the subject from who the disease sample was collected.

In some embodiments, for each of a set of mutant peptides (e.g., detected in a sample of a subject), one or more techniques disclosed herein are used to predict whether a the mutant peptide will bind to a subject's MHC molecule (or a strength, stability and/or prevalence of such binding) and/or to predict whether a subject's MHC molecule will present the mutant peptide (and/or a prevalence of such presentation). The predictions can be used to select an incomplete subset of the mutant peptides (e.g., for which it is predicted that MHC presentation of the mutant peptide is likely). The selection may include comparing, for each mutant peptide, a metric corresponding to the prediction metric to an absolute threshold and/or to prediction metrics of other mutant peptides' metrics (e.g., thereby performing a relative comparison. Each selected mutant peptide can be identified as having a: high likelihood of being presented on the tumor cell surface; high likelihood of being capable of inducing a tumor-specific immune response; high likelihood of being capable of being presented to naive T cells by professional antigen presenting cells (e.g., dendritic cells); low likelihood of being subject to inhibition via central or peripheral tolerance; and/or low likelihood of being capable of inducing an autoimmune response to normal tissue in the subject.

Some embodiments include generating and/or using a model to identify one or more peptides (e.g., mutant peptides) that are likely to bind to MHC molecules and to be presented by MHC molecules at surfaces of tumor cells. More

specifically, a training data set can include a set of data elements, each data element including: a sequence of an epitope (or peptide) (e.g., and potentially sequences of an N-flank of the peptide and a C-flank of the peptide), subsequence of an MHC molecule, and one or more experimental results pertaining to the peptide and MHC molecule (e.g., binding affinity and/or eluted-ligand presentation data).

An attention-based machine-learning model can be trained using at least part of the training data set. The training data set can include multiple training data elements. Each training data element can include a representation of a sequence and a result (e.g., indicating whether at least part of a peptide corresponding to the sequence is presented by an MHC molecule and/or triggers immunogenicity). Training data elements for which presentation was not detected may be generated computationally. For example, for each protein of origin in the positive set (corresponding to positive eluted-ligand presentation data), one, more or all possible peptide fragments (e.g., within a predefined length range, such as from 8 to 11) can be generated, potentially with uniform probability, for each length. N-terminal and C-terminal flanking sequences may be retained (e.g., potentially with a maximum length, such as 10 amino acids). In some instances, for each allele represented in positive instances in the training data, peptide fragments (e.g., of one, more or all lengths of 8:11) may be generated. The generation and/or subsequent selection can be performed such that a probability of occurrence of a sequence having a given length is uniform across lengths. N-terminal and C-terminal flanking sequences may be or may have been retained with a particular maximum length (e.g., a maximum length of 10 amino acids).

The attention-based machine-learning model can include 1, 2, 3, 4, 5, 6, 7, 8 or more transformer encoder networks (e.g., each including one-head attention and a feedforward network). For example, the attention-based machine-learning model can include multiple first-level transformer encoders, including a transformer encoder configured to process a representation of a peptide, a transformer encoder configured to process a representation of an MHC molecule, potentially a transformer encoder configured to process a representation of a peptide N-flank, and potentially a transformer encoder configured to process a representation of a peptide C-flank. The attention-based machine-learning model can further include a second-level transformer encoder configured to process aggregated (e.g., concatenated) results of generated by the first-level transformer encoders.

The attention-based machine-learning model can further include a feedforward network (e.g., a fully connected feedforward network with one, two or more hidden layers) configured to process a result from the fifth transformer encode (e.g., after dropout is applied) to generate a predicted (e.g., real-number) binding affinity and/or predicted presentation (e.g., as a binary prediction. The attention-based machine-learning model be one or multiple models (e.g., having a same configuration) within an ensemble of models. The training data set can be randomly parsed, shuffled and/or divided to train various models within the ensemble. A loss function can use an error term (e.g., mean squared error or median squared error) and/or an entropy term (e.g., cross entropy or binary cross entropy). Multitask learning can be used, such that the model is simultaneously trained to predict each of two different types of results (e.g., binding affinity and presentation occurrence). A static or non-static learning rate can be used. For example, learning rate annealing (e.g., using stepwise annealing or cosine annealing) can be used to reduce a learning rate over iterations. Validation-data assessment can be used to potentially terminate training early (e.g., upon determining that a performance target has been met).

The MHC includes multiple alleles in vivo (e.g., 6 alleles per human). Thus, for this single MHC molecule, multiple sequence inputs can be generated (e.g., each representing a single allele of the multiple alleles). Each of the multiple sequence inputs can be separately processed using the one or more neural networks (e.g., one or more transformer encoders) so as to generate a predicted binding or presentation value of a neoantigen in association with each of the alleles. A function (e.g., softmax function) can identify which allele from among the multiple alleles is associated with a highest presentation prediction. During training, this maximum presentation prediction for this particular sequence input can then be compared to a true presentation value using a binary loss function to generate error for tuning parameters.

In some instances, it is not known how many amino acids from a flank (e.g., N-flank) are used by peptidases to determine when to trim long peptides into a peptide core that is presented. To address this unknown in generating the training data, flanks may then be trimmed to a length selected based on a technique (e.g., pseudo-random selection technique), such as a length within a predefined range (e.g., 1 to 10 amino acids). The selection technique may select a length using a distribution (e.g., uniform or Gaussian distribution). In some instances, a flank that is below a threshold length (e.g., 10 amino acids) is not trimmed. In some instances, a flank trimming is defined in a manner so as to preserve the C side on an N-flank.

The trained model can then receive an input data set that include representation(s) of one or more mutant-peptide sequences (e.g., of an N-flank region, candidate epitope region and/or C-flank region) and a subsequence of an MHC molecule (associated with a subject) and generate a predicted binding affinity and/or presentation prediction. If it is predicted that the mutant-peptide will stably bind to and be presented by an MHC molecule, the mutant-peptide may be selected to be included in a composition (e.g., a vaccine) to be used to treat the subject.

**II.D. Exemplary Identification of** Input **Data for Machine Learning Model**

The exemplary methods and systems for identifying input data described herein may be used to identify input

data for, for example, machine learning model 132 in Figs. 1 and 3 and/or machine learning model 132 described in Figs. 4A-4C.

Each of a set of mutant peptides associated with a given subject can be analyzed using an attention-based machine-learning model to generate one or more predictions as to a binding affinity, presentation probability and/or immunogenicity of a mutant peptide. To generate these predictions, the machine-learning model can receive and process a peptide (e.g., coding) sequence corresponding to the mutant peptide and one or more other sequences or subsequences (e.g., corresponding to an MHC-I molecule, an MHC-II molecule or a T-cell receptor). In some instances, predictions are generated for each of a set of peptide sequences (e.g., a set of variant-coding sequences corresponding to a set of mutant peptides). The set of mutant peptides can correspond to peptides present in a disease sample collected from the subject but that are not observed in one or more non-disease samples (e.g., from the subject or another subject).

A variety of methods are available for identifying a set of mutant peptides associated with a given subject. Mutations can be present in the genome, transcription, proteome or exome of diseased cells of a subject but not in a non-diseased sample, for example, a non-diseased sample from the subject or from another subject. Mutations include, but are not limited to, (1) non-synonymous mutations leading to different amino acids in the protein; (2) read-through mutations in which a stop codon is modified or deleted, leading to translation of a longer protein with a novel tumor-specific sequence at the C-terminus; (3) splice site mutations that lead to the inclusion of an intron in the mature mRNA and thus a unique tumor-specific protein sequence; (4) chromosomal rearrangements that give rise to a chimeric protein with tumor-specific sequences at the junction of 2 proteins (i.e., gene fusion); (5) frameshift insertions or deletions that lead to a new open reading frame with a novel tumor-specific protein sequence. Mutations can also include one or more of nonframeshift indel, missense or nonsense substitution, splice site alteration, genomic rearrangement or gene fusion, or any genomic or expression alteration giving rise to a neoORF.

Peptides with mutations or mutated polypeptides arising from, for example, splice-site, frameshift, readthrough, or gene fusion mutations in diseased cells can be identified by sequencing DNA, RNA or protein in the diseased sample and comparing the obtained sequences with sequences from a non-diseased sample.

In some embodiments, whole genome sequencing (WGS) or whole exome sequencing (WES) data from a disease sample and a non-diseased sample can be obtained and compared. Following the alignment of non-diseased sample and diseased sample reads to the human reference genome, somatic variants, which include single nucleotide variants (SNV), gene fusions and insertion or deletion variants (indels), can be detected using variant-calling algorithms. One or more variant callers can be used to detect different somatic variant types (i.e., SNV, gene fusions, or indels) (See. Xu et al. "A review of somatic single nucleotide variant calling algorithms for next-generation sequencing data." Comput. Struct. Biotechnol. J. 16: 15-24 (2018), which is hereby incorporated by reference in its entirety for all purposes).

In some examples, the mutant peptides are identified based on the transcriptome sequences in the disease sample from the individual. For example, whole or partial transcriptome sequences (for example by methods such as RNA-Seq) can be obtained from a diseased tissue of the individual and subjected to sequencing analysis. The sequences obtained from the diseased tissue sample can then be compared to those obtained from a reference sample. Optionally, the diseased tissue sample is subjected to whole-transcriptome RNA-Seq. Optionally, the transcriptome sequences are "enriched" for specific sequences prior to the comparison to a reference sample. For example, specific probes can be designed to enrich certain desired sequences (for example disease-specific sequences) before being subjected to sequencing analysis. Methods of whole-transcriptome sequencing and targeted sequencing are known in the art and reported, for example, in Tang, F. et al., "mRNA-Seq whole-transcriptome analysis of a single cell," Nature Methods, 2009, v. 6, 377-382; Ozsolak, F., "RNA sequencing: advances, challenges and opportunities," Nature Reviews, 2011, v. 12, 87-98; German, M. A et al., "Global identification of microRNA-target RNA pairs by parallel analysis of RNA ends," Nature Biotechnology, 2008, v. 26, 941-946; and Wang, Z. et al., "RNA-Seq: a revolutionary tool for transcriptomics," Nature Reviews, 2009, v. 10, p. 57-63. Each of these references is hereby incorporated by reference in its entirety for all purposes.

In some embodiments, transcriptomic sequencing techniques include, but are not limited to, RNA poly(A) libraries, microarray analysis, parallel sequencing, massively parallel sequencing, PCR, and RNA-Seq. RNA-Seq is a high-throughput technique for sequencing part of, or substantially all of, the transcriptome. In short, an isolated population of transcriptomic sequences is converted to a library of cDNA fragments with adaptors attached to one or both ends. With or without amplification, each cDNA molecule is then analyzed to obtain short stretches of sequence information, typically 30-400 base pairs. These fragments of sequence information are then aligned to a reference genome, reference transcripts, or assembled *de novo* to reveal the structure of transcripts (i.e., transcription boundaries) and/or the level of expression.

Once obtained, the sequences in the diseased sample can be compared to the corresponding sequences in a reference sample. The sequence comparison can be conducted at the nucleic acid level, by aligning the nucleic acid sequences in the disease tissue with the corresponding sequences in a reference sample. Genetic sequence variations that lead to one or more changes in the encoded amino acids are then identified. Alternatively, the sequence comparison can be conducted at the amino acid level, that is, the nucleic acid sequences are first converted into amino acid sequences *in silico* before the comparison is carried out. Either the amino-acid-based approach or the nucleic-acid-based approach

can be used to identify one or more mutations (e.g., one or more point mutations) in the peptide. With regard to nucleic-acid-based approaches, the discovered variants can be used to identify one or more nucleic-acid sequences (e.g., DNA sequences, RNA sequences or mRNA sequences) that would give rise to a given observable mutant protein (e.g., via a look-up table that associated individual peptide mutations with multiple codon variants).

In some embodiments, comparison of a sequence from the disease sample to those of a reference sample can be completed by techniques known in the art, such as manual alignment, FAST-All (FASTA), and Basic Local Alignment Search Tool (BLAST). In some embodiments, comparison of a sequence from a disease sample to those of a reference sample can be completed using a short read aligner, for example GSNAP, BWA, and STAR.

In some embodiments, the reference sample is a matched, disease-free sample. As used herein, a "matched," disease-free tissue sample is one that is selected from the same or similar sample, for example, a sample from the same or similar tissue type as the disease sample. In some embodiments, a matched, disease-free tissue and a disease tissue may originate from the same individual. The reference sample described herein in some embodiments is a disease-free sample from the same individual. In some embodiments, the reference sample is a disease-free sample from a different individual (for example an individual not having the disease). In some embodiments, the reference sample is obtained from a population of different individuals. In some embodiments, the reference sample is a database of known genes associated with an organism. In some embodiments, a reference sample may be from a cell line. In some embodiments, a reference sample may be a combination of known genes associated with an organism and genomic information from a matched disease-free sample. In some embodiments, a variant-coding sequence may comprise a point mutation in the amino acid sequence. In some embodiments, the variant-coding sequence may comprise an amino acid deletion or insertion.

In some embodiments, the set of variant-coding sequences are first identified based on genomic and/or nucleic-acid sequences. This initial set is then further filtered to obtain a narrower set of expression variant-coding sequences based on the presence of the variant-coding sequences in a transcriptome sequencing database (and is thus deemed "expressed"). In some embodiments, the set of variant-coding sequences are reduced by at least about 10, 20, 30, 40, 50, or more times by filtering through a transcriptome sequencing database.

Alternatively, protein mass spectrometry can be used to identify or validate the presence of mutant peptides, for example, mutant bound to MHC proteins on tumor cells. Peptides can be acid-eluted from diseased cell, for example, tumor cells or from HLA molecules that are immunoprecipitated from the tumor, and then identified using mass spectrometry.

A mutant peptide can have, for example, 5 or more, 8 or more, 11 or more, 15 or more, 20 or more, 40 or more, 80 or more, 100 or more, 120 or fewer, 100 or fewer, 80 or fewer, 60 or fewer, 50 or fewer, 40 or fewer, 30 or fewer, 25 or fewer, 20 or fewer, 18 or fewer, 15 or fewer or 13 or fewer amino acids.

Tumor-specific T-cell receptor sequences can also be identified, for example, by single cell T-cell receptor sequencing. See, for example, De Simone et al. "Single Cell T Cell Receptor Sequencing: Techniques and Future Challenges," Front. Immunol. 9: 1638 (2018); Zong et al. "Very rapid cloning, expression and identifying specificity of T-cell receptors for T-cell engineering," PloS ONE 15(2):e0228112 (2020) (which is hereby incorporated by reference in its entirety for all purposes). High-throughput sequencing of T cell repertoires can also or alternatively be performed to identify tumor-specific signatures for a particular disease. See, for example, Wang et al. "High-throughput sequence of CD4+T cell repertoire reveals disease-specific signatures in IgG4-related disease," Arthritis Research & Therapy 21: 295 (2019) (which is hereby incorporated by reference in its entirety for all purposes).

MHC-I sequences and/or MHC-II sequences can be determined, for example, via HLA genotyping or mass spectroscopy (Caron et al., "Analysis of Major Histocompatibility Complex (MHC) Immunopeptides Using Mass Spectroscopy," Molecular and Cellular Proteomics 14(12): 3105-3117 (2015) (which is hereby incorporated by reference in its entirety for all purposes).

## II.E. <u>Exemplary Identification of Training Data for Machine Learning Model</u>

The exemplary methods and systems for identifying training data described herein may be used to identify training data for, for example, machine learning model 132 in Figs. 1 and 3 and/or machine learning model 132 described in Figs. 4A-4C. For example, these methods and systems may be used to identify training data 131 in Fig. 1.

A training set can be generated using data collected from multiple other samples (e.g., potentially being associated with one or more other subjects). Each of the multiple other samples can include, for example, tissue (e.g., a biopsy), single cell, multiple cells, fragments of cells or an aliquot of body fluid. In some instances, the multiple other samples are collected from a different type of subject as compared to a subject associated with input data to be processed by the trained model. For example, a machine-learning model may be trained using training data collected by processing samples from one or more cell lines, and the trained machine-learning model may be used to process input data determined by processing one or more samples from a human subject.

The training data set can include multiple training elements. Each of the multiple training elements can include input data that includes a set of peptide sequences (which includes a set of either wild-type or variant-coding sequences),

each of which code for and/or represent any variant in a corresponding peptide, and a subsequence or pseudosequence of an MHC molecule. The input data can be collected in accordance with one or more techniques disclosed herein (e.g., in Section II.D).

Each training element can also include one or more experiment-based results. An experiment-based result can indicate whether and/or an extent to which each of one or more particular types of interaction between a wild-type peptide or mutant peptide (associated with a variant-coding sequence in the training element) and an MHC molecule (associated with an MHC molecule subsequence in the training element) occurs. A particular type of interaction can include for example binding of a peptide to an MHC molecule and/or presentation of a peptide by the MHC molecule on a surface of a cell (e.g., a tumor cell).

A result can include a binding affinity between the peptide and the MHC molecule. The result can include or can be based on qualitative data and/or quantitative data characterizing whether a given peptide binds with a given MHC molecule, a strength of such a bond, a stability of such a bond, and/or a tendency of such a bond to occur. For example, a binary binding-affinity indicator or a qualitative binary-affinity result can be generated using an ELISA, pull-down assay, gel-shift assay, biosensor-based methodology, such as Surface Plasmon Resonance, Isothermal Titration Colorimetry, BioLayer Interferometry or MicroScale Thermophoresis.

The result can, for example, further or alternatively characterize whether and/or probability that a given MHC molecule presents a given peptide. MHC ligands may be immunoprecipitated out of a sample. Subsequent elution and mass spectrometry can be used to determine whether the MHC molecule presented the ligand.

## III. Pharmaceutically Acceptable Composition and Manufacture

One or more variant-coding sequences can be selected from a set of subject-specific set of variant coding sequences based on results from one or more machine-learning models described herein. For example, a selection can include identifying each of the set of subject-specific set of variant-coding sequences for which a predicted binding affinity is less than 500 nM, for which it is predicted that an MHC molecule will present a mutant peptide identified by the variant-coding sequence and/or for which it is predicted that the mutant peptide will trigger an immune response. It will be appreciated that outputs of the model may be on a different scale, such that 500 nM may correspond to, for example, another value (e.g., 0.42) on a [0,1] scale.

A pharmaceutically acceptable composition may be developed and/or manufactured using one, more or all of the selected variant-coding sequences. The composition may include mutant peptides corresponding to a single selected variant-coding sequence. The composition may include mutant peptides and/or mutant-peptide precursors corresponding to multiple selected variant-coding sequences. A subset of peptide candidates (e.g., associated with the 5, 10, 15, 20, 30 or any number in between, highest presentation predictions) may be used for further precursor development.

Each of one, more or all of the mutant peptides in the composition can have, for example, a length of about 7 to about 40 amino acids (e.g., about any of 7, 8, 9, 10, 11, 12, 13, 14, 15, 17, 20, 22, 25, 30, 35, 40, 45, 50, 60 or 70 amino acids in length). In some embodiments, a length of each of one, more or all of the mutant peptides in the composition are within a predefined range (e.g., 8 to 11 amino acids, 8 to 12 amino acids or 8 to 15 amino acids). In some embodiments, each of one, more or all of the mutant peptides in the composition is about 8 to 10 amino acids in length. Each of one, more or all of the mutant peptides in the compositions may be in its isolated form. Each of one, more of all of the mutant peptides in the composition may be a "long peptide" produced by adding one or more peptides to an end (or to each end) of the mutant peptide. Each of one, more or all of the mutant peptides in the composition may be tagged, may be a fusion protein, and/or may be a hybrid molecule.

A pharmaceutically acceptable composition may be developed and/or manufactured to include or by using one or more nucleic acids that encode - for each of one, more or all of the selected variant-coding sequences - the peptide that includes or is composed by amino acids as identified in the variant-coding sequence. The nucleic acid(s) can include DNA, RNA and/or mRNA. Given that any of multiple codons can encode a given amino acids, the codons may be selected to, for example, optimize or promote expression in a given type of organism. Such selection may be based on a frequency that each of multiple potential codons are used by the given type of organism, the translational efficiency of each of multiple potential codons in the given type of organism, and/or the given type of organism's degree of bias towards each of the multiple potential codons.

In some instances, the composition may include nucleic acids encoding the mutant peptide(s) or precursor of the mutant peptide(s) described above. The nucleic acid may include sequences flanking the sequence coding the mutant peptide (or precursor thereof). In some instances, the nucleic acid includes epitopes corresponding to more than one selected variant-coding sequence. In some instances, the nucleic acid is DNA having a polynucleotide sequence encoding the mutant peptides or precursors described above.

In some instances, the nucleic acid is RNA. In some instances, the RNA is reverse transcribed from a DNA template having a polynucleotide sequence encoding the mutant peptides or precursors described above. In some instances, the RNA is mRNA. In some instances, the RNA is naked mRNA. In some instances, the RNA is modified mRNA

(e.g., mRNA protected from degradation using protamine. mRNA containing modified 5'CAP structure, or mRNA containing modified nucleotides). In some embodiments, the RNA is single-stranded mRNA.

The composition may include cells comprising the mutant peptide and/or nucleic acid(s) encoding the mutant peptide described above. The composition may further comprise one or more suitable vectors and/or one or more delivery systems for the mutant peptide and/or nucleic acid(s) encoding the mutant peptide. In some instances, the cells comprising the mutant peptide and/or nucleic acids encoding the mutant peptide are non-human cells, for example, bacterial cells, protozoan cells, fungal cells, or non-human animal cells. In some instances, the cells comprising the mutant peptide and/or nucleic acids encoding the mutant peptide are human cells. In some instances, the human cells are immune cells. In some instances, the immune cells are antigen-presenting cells (APCs). In some instances, the APCs are professional APCs, such as macrophages, monocyte, dendritic cells, B cells, and microglia. In other instances, the professional APCs are macrophages or dendritic cells. In some instances, the APCs comprising the mutant peptide and/or nucleic acid sequence(s) encoding the mutant peptide are used as a cellular vaccine, thereby inducing a CD4+ or a CD8+ immune response. In other instances, the composition used as a cellular vaccine includes mutant peptide-specific T cells primed by APCs comprising the mutant peptide and/or nucleic acid sequence(s) encoding the mutant peptide.

The composition may include a pharmaceutically acceptable adjuvant and/or pharmaceutically acceptable excipient. Adjuvants refer to any substance for which admixture into a composition modifies an immune response to a mutant peptide. Adjuvants may be conjugated using, for example, an immune stimulation agent. Excipients can increase the molecular weight of a particular mutant peptide to increase activity or immunogenicity, confer stability, increase biological activity, and/or increase serum half-life.

The pharmaceutically acceptable composition may be a vaccine, which can include an individualized vaccine that is specific to (e.g., and potentially developed for) a particular subject. For example, an MHC sequence may have been identified using a sample from the particular subject, and the composition may be developed for and/or used to treat the particular subj ect.

The vaccine may be a nucleic acid vaccine. The nucleic acid can encode a mutant peptide or precursor of the mutant peptide. The nucleic acid vaccine may include sequences flanking the sequence coding the mutant peptide (or precursor thereof). In some instances, the nucleic acid vaccine includes epitopes corresponding to more than one selected variant-coding sequence. In some instances, the nucleic acid vaccine is a DNA-based vaccine. In some instances, the nucleic acid vaccine is a RNA-based vaccine. In some instances, the RNA-based vaccine comprises mRNA. In some instances, the RNA-based vaccine comprises naked mRNA. In some instances, the RNA-based vaccine comprises modified mRNA (e.g., mRNA protected from degradation using protamine. mRNA containing modified 5'CAP structure, or mRNA containing modified nucleotides). In some embodiments, the RNA-based vaccine comprises single-stranded mRNA.

A nucleic-acid vaccine may include an individualized neoantigen specific therapy manufactured for a particular subject to be used as part of next-generation immunotherapy. The individualized vaccine may have been designed by first detecting mutant peptides in a sample of the particular subject and subsequently predicting, for each detected mutant peptide, whether and/or a degree to which the peptide will bind to an MHC of the particular subject, be presented by the MHC, bind to a T-cell receptor of the particular subject and/or trigger an immunological response. Based on these predictions, a subset of the detected mutant peptides can be selected (e.g., a subset having at least 1, at least 2, at least 3, at least 5, at least 8, at least 10, at least 12, at least 15, at least 18, up to 40, up to 30, up to 25, up to 20, up to 18, up to 15 and/or up to 10 mutant peptides). For each selected mutant peptide, a synthetic mRNA sequence can be identified that codes for the mutant peptide. An mRNA vaccine may include mRNA (that encodes part or all of a mutant peptide) complexed with lipids to form an mRNA-lipoplex. Administration of a vaccine that includes the mRNA-lipoplex can result in the mRNA stimulating TLR7 and TLR8, triggering T-cell activation by dendritic cells. Further, the administration can result in translation of mRNA into a mutant peptide, which can then bind to and be presented by MHC molecules and induce T-cell response.

The composition may include substantially pure mutant peptides, substantially pure precursors thereof, and/or substantially pure nucleic acids encoding the mutant peptides or precursors thereof. The composition may include on more suitable vectors and/or one or more delivery systems to contain the mutant peptides, precursors thereof, and/or nucleic acids encoding the mutant peptides or precursors thereof. Suitable vectors and delivery systems include viral, such as systems based on adenovirus, vaccinia virus, retroviruses, herpes virus, adeno-associated virus or hybrids containing elements of more than one virus. Non-viral delivery systems include cationic lipids and cationic polymers (e.g., cationic liposomes). In some embodiments, physical delivery, such as with a 'gene-gun' may be used.

In certain embodiments, the RNA-based vaccine includes an RNA molecule including, in the 5'→3' direction: (1) a 5' cap; (2) a 5' untranslated region (UTR); (3) a polynucleotide sequence encoding a secretory signal peptide; (4) a polynucleotide sequence encoding the one or more mutant peptides resulting from cancer-specific somatic mutations present in the tumor specimen; (5) a polynucleotide sequence encoding at least a portion of a transmembrane and cytoplasmic domain of a major histocompatibility complex (MHC) molecule; (6) a 3' UTR including: (a) a 3' untranslated region of an Amino-Terminal Enhancer of Split (AES) mRNA or a fragment thereof; and (b) non-coding RNA of a

mitochondrially encoded 12S RNA or a fragment thereof; and (7) a poly(A) sequence. This example RNA molecule was also used in evaluating an example implementation of an attention-based prediction model, as discussed with respect to Section V, below.

In certain embodiments, the RNA molecule further includes a polynucleotide sequence encoding an amino acid linker; wherein the polynucleotide sequences encoding the amino acid linker and a first of the one or more mutant peptides form a first linker-neoepitope module; and wherein the polynucleotide sequences forming the first linker-neoepitope module are between the polynucleotide sequence encoding the secretory signal peptide and the polynucleotide sequence encoding the at least portion of the transmembrane and cytoplasmic domain of the MHC molecule in the 5'→3' direction. In certain embodiments, the amino acid linker includes the sequence GGSGGGGSGG. In certain embodiments, the polynucleotide sequence encoding the amino acid linker includes the sequence GGCGGCUCUGGAGGAGGCGGCUCCGGAGGC.

In certain embodiments, the RNA molecule further includes, in the 5' →3' direction: at least a second linker-epitope module, wherein the at least second linker-epitope module includes a polynucleotide sequence encoding an amino acid linker and a polynucleotide sequence encoding a neoepitope; wherein the polynucleotide sequences forming the second linker-neoepitope module are between the polynucleotide sequence encoding the neoepitope of the first linker-neoepitope module and the polynucleotide sequence encoding the at least portion of the transmembrane and cytoplasmic domain of the MHC molecule in the 5'→3' direction; and wherein the neoepitope of the first linker-epitope module is different from the neoepitope of the second linker-epitope module. In certain embodiments, the RNA molecule includes 5 linker-epitope modules, wherein the 5 linker-epitope modules each encode a different neoepitope. In certain embodiments, the RNA molecule includes 10 linker-epitope modules, wherein the 10 linker-epitope modules each encode a different neoepitope. In certain embodiments, the RNA molecule includes 20 linker-epitope modules, wherein the 20 linker-epitope modules each encode a different neoepitope.

In certain embodiments, the RNA molecule further includes a second polynucleotide sequence encoding an amino acid linker, wherein the second polynucleotide sequence encoding the amino acid linker is between the polynucleotide sequence encoding the neoepitope that is most distal in the 3' direction and the polynucleotide sequence encoding the at least portion of the transmembrane and cytoplasmic domain of the MHC molecule.

In certain embodiments, the 5' cap includes a D1 diastereoisomer of the structure:

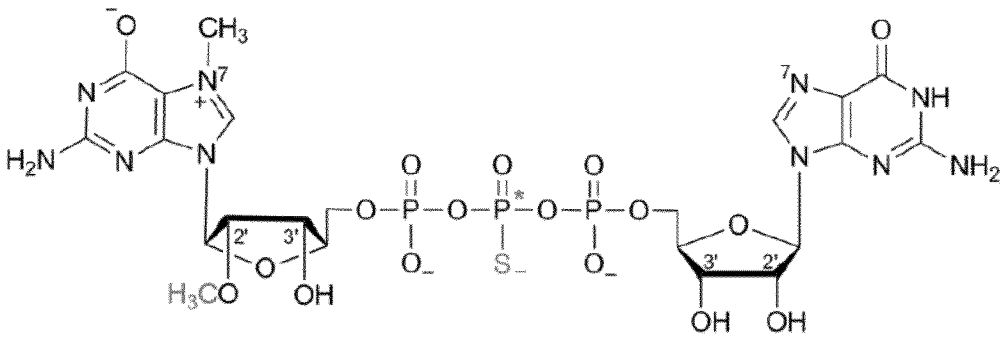

In certain embodiments, the 5' UTR includes the sequence UUCUUCUGGUCCCCACAGACUCAGAGAGAACCCGCCACC. In certain embodiments, the 5' UTR includes the sequence GGCGAACUAGUAUUCUUCUGGU CCCCACAGACUCAGAGAGAACCCGCCACC.

In certain embodiments, the secretory signal peptide includes the amino acid sequence MRVMAPRTLILLLSGALALTETWAGS. In certain embodiments, the polynucleotide sequence encoding the secretory signal peptide includes the sequence AUGAGAGUGAUGGCCCCCAGAACCCUGAUCCUGCUGCUGUCUGGCGCCCUGGC CCUGACAGAGACAUGGGCCGGAAGC.

In certain embodiments, the at least portion of the transmembrane and cytoplasmic domain of the MHC molecule includes the amino acid sequence IVGIVAGLAVLAVVVIGAVVATVMCRRKSSGGKGGSYSQAASSDSAQGSDVSLTA. In certain embodiments, the polynucleotide sequence encoding the at least portion of the transmembrane and cytoplasmic domain of the MHC molecule includes the sequence AUCGUGGGAAUUGUGGCAGGACUGGCAGUGCUGGCCGU GGUGGUGAUCGGAG CCGUGGUGGCUACCGUGAUGUGCAGACGGAAGUCCAGCGGAGGCAAGGGCGGC AG CUACAGCCAGGCCGCCAGCUCUGAUAGCGCCCAGGGCAGCGACGUGUCACU GACAGCC.

In certain embodiments, the 3' untranslated region of the AES mRNA includes the sequence CUGGUACUG CAUGCACGCAAUGCUAGCUGCCCCUUUCCCGUCCUGGGUACCCC GAGUCUCCCCCGACCUCGGGUCCCAG GUAUGCUCCCACCUCCACCUGCCCCACU CACCACCUCUGCUAGUUCCAGACACCUCC. In certain embodiments, the non-coding RNA of the mitochondrially encoded 12S RNA includes the sequence CAAGCACGCAGCAAUGCAG CUCAAAACGCUUAGCCUAGCCACACCCCCACGGG AAACAGCAGUGAUUAACCUUUAGCAAUAAACGAAAGUU

UAACUAAGCUAUAC UAACCCCAGGGUUGGUCAAUUUCGUGCCAGCCACACCG. In certain embodiments, the 3' UTR includes the sequence CUCGAGCUGGUACUGCAUGCACGCAAUGCUAGCUGCCCCUUUCCCGUCCUGGG UACCCCGAGUCUCCCCCGACCUCGGGUCCCAGGUAUGCUCCCACCUCCACCUGC CCCACUCACCACCUCUG CUAGUUCCAGACACCUCCCAAGCACGCAGCAAUGCA GCUCAAAACGCUUAGCCUAGCCACACCCCCACGGGA AACAGCAGUGAUUAACC UUUAGCAAUAAACGAAAGUUUAACUAAGCUAUACUAACCCCAGGGUUGGUCA AUU UCGUGCCAGCCACACCGAGACCUGGUCCAGAGUCGCUAGCCGCGUCGCU.

In certain embodiments, the poly(A) sequence includes 120 adenine nucleotides.

In certain embodiments, the RNA-based vaccine includes an RNA molecule including, in the 5'→3' direction: the polynucleotide sequence GGCGAACUAGUAUUCUUCUGGUCCCCACAGACUCAGAGAGAACCCGCCACCAU GAG AGUGAUGGCCCCCAGAACCCUGAUCCUGCUGCUGUCUGGCGCCCUGGCCC UGACAGAGACAUGGGCCG-GAAGC; a polynucleotide sequence encoding the one or more mutant peptides resulting from cancer-specific somatic mutations present in the tumor specimen; and the polynucleotide sequence AUCGUGGGAAUUGUGGCAGGACUGG CAGUGCUGGCCGUGGUGGUGAUCGGAG CCGUGGUGGCUACCGUGAUGUGCAGACGGAAGUCCAGCGGAGG CAAGGGCGGC AGCUACAGCCAGGCCGCCAGCUCUGAUAGCGCCCAGGGCAGCGACGUGUCACU GACAGCC UAGUAACUCGAGCUGGUACUGCAUGCACGCAAUGCUAGCUGCCCCU UUCCCGUCCUGGGUACCCCGAGUCU CCCCCGACCUCGGGUCCCAGGUAUGCUC CCACCUCCACCUGCCCCACUCACCACCUCUGCUAGUUCCAGAC ACCUCCCAAGC ACGCAGCAAUGCAGCUCAAAACGCUUAGCCUAGCCACACCCCCACGGAAACA GCAGUGA UUAACCUUUAGCAAUAAACGAAAGUUUAACUAAGCUAUACUAACC CCAGGGUUGGUCAAUUUCGUGCCAGCC ACACCGAGACCUGGUCCAGAGUCGCU AGCCGCGUCGCU.

In some embodiments, mutant peptides described herein (e.g., including or consisting of an ordered set of amino acids as identified by variant-coding sequences selected based on results from a machine-learning technique described herein) can be used for making mutant peptide specific therapeutics, such as antibody therapeutics. For example, the mutant peptides can be used to raise and/or identify antibodies specifically recognizing the mutant peptides. These antibodies can be used as therapeutics. Synthetic short peptides have been used to generate protein-reactive antibodies. An advantage of immunizing with synthetic peptides is that unlimited quantity of pure stable antigen can be used. This approach involves synthesizing the short peptide sequences, coupling them to a large carrier molecule, and immunizing a subject with the peptide-carrier molecule. The properties of antibodies are dependent on the primary sequence information. A good response to the desired peptide usually can be generated with careful selection of the sequence and coupling method. Most peptides can elicit a good response. An advantage of anti-peptide antibodies is that they can be prepared immediately after determining the amino acid sequence of a mutant peptide and the particular regions of a protein can be targeted specifically for antibody production. Selecting mutant peptides for which a machine-learning model predicted immunogenicity and/or screening for the same can lead to a high chance that the resulting antibody will recognize the native protein in the tumor setting. A mutant peptide may be, for example, 15 or fewer, 18 or fewer or 20 or fewer, 25 or fewer, 30 or fewer, 35 or fewer, 40 or fewer, 50 or fewer, 60 or fewer, 70 or fewer, 85 or fewer, 100 or fewer, 110 or fewer residues. A mutant peptide may be, for example, 9 or more, 10 or more, 15 or more, 20 or more, 25 or more, 30 or more, 50 or more, or 70 or more residues. Shorter peptides can improve antibody production.

Peptide-carrier protein coupling can be used to facilitate production of high titer antibodies. A coupling method can include, for example, site-directed coupling and/or a technique that relies on the reactive functional groups in amino acids, such as -NH2, - COOH, -SH, and phenolic -OH. Any suitable method used in anti-peptide antibody production can be utilized with the mutant peptides identified by the methods of the present invention. Two such known methods are the Multiple Antigenic Peptide system (MAPs) and the Lipid Core Peptides (LCP method). An advantage of MAPs is that the conjugation method is not necessary. No carrier protein or linkage bond is introduced into the immunized host. One disadvantage is that the purity of the peptide is more difficult to control. In addition, MAPs can bypass the immune response system in some hosts. The LCP method is known to provide higher titers than other anti-peptide vaccine systems and thus can be advantageous.

Also provided herein are isolated MHC/peptide complexes comprising one or more mutant peptides identified using a technique disclosed herein. Such MHC/peptide complexes can be used, for example, for identifying antibodies, soluble TCRs, or TCR analogs. One type of these antibodies has been termed TCR mimics, as they are antibodies that bind peptides from tumor associated antigens in the context of specific HLA environments. This type of antibody has been shown to mediate the lysis of cells expressing the complex on their surface as well as protect mice from implanted cancer cells lines that express the complex (see, e.g., Wittman et al., J. of Immunol. 177:4187-4195 (2006)). One advantage of TCR mimics as IgGmAbs is that affinity maturation can be performed, and the molecules are coupled with immune effector functions through the present Fc domain. These antibodies can also be used to target therapeutic molecules to tumors, such as toxins, cytokines, or drug products.

Other types of molecules that have been developed using mutant peptides such as those selected using the methods of the present invention using non-hybridoma based antibody production or production of binding competent antibody fragments such as anti-peptide Fab molecules on bacteriophage. These fragments can also be conjugated to other therapeutic molecules for tumor delivery such as anti-peptide MHC Fab-immunotoxin conjugates, anti-peptide MHC

Fab-cytokine conjugates and anti-peptide MHC Fab-drug conjugates.

IV. Methods of Treatment Comprising **Immunogenic** Vaccines or T Cells

Some embodiments provide methods of treatment including a vaccine, which can be an immunogenic vaccine. In some embodiments, a method of treatment for disease (such as cancer) is provided, which may include administering to an individual an effective amount of a composition described herein, a mutant peptide identified using a technique disclosed herein, a precursor thereof, or nucleic acids encoding a mutant peptide (or precursor) identified using a technique described herein.

In some embodiments, a method of treatment for a disease (such as cancer) is provided. The method may include collecting a sample (e.g., a blood sample) from a subject. T cells can be isolated and stimulated. The isolation can be performed using, for example, density gradient sedimentation (e.g., and centrifugation), immunomagnetic selection, and/or antibody-complex filtering. The stimulation may include, for example, antigen-independent stimulation, which may use a mitogen (e.g., PHA or Con A) or anti-CD3 antibodies (e.g., to bind to CD3 and activate the T-cell receptor complex) and anti-CD28 antibodies (e.g., to bind to CD28 and stimulate T cells). One or more mutant peptides can be (or may have been) selected to use in the treatment of the subject (e.g., based on results produced by a machine-learning model corresponding to predictions as to whether and/or an extent to which each of set of mutant peptides would bind to an MHC molecule of the individual, be presented by an MHC molecule of the individual and/or trigger an immune response in the individual, in accordance with one or more techniques disclosed herein). The one or more mutant peptides may have been selected based on a technique disclosed herein that includes identifying and processing one or more sequence representations associated with the subject (e.g., a representation of: an MHC sequence, a set of variant-coding sequences and/or a T-cell receptor sequence). The one or more sequences may have been detected using the sample from which the T cells were isolated or a different sample.

In some instances, the one or more mutant peptides (or precursors thereof) can be used to produce mutant peptide (for example, neoantigen) specific T cells. For example, peripheral blood T cells can be isolated from a subject and contacted with one or more mutant peptides to induce mutant peptide-specific T-cells populations that can be administered to a subject. In some examples, the T cell receptor sequence of the mutant peptide-reactive T cells can be sequenced. If the sequencing identifies an ordered set of nucleic acids, each codon of nucleic acids may be translated to an amino acid (e.g., via a look-up technique). Once a T-cell receptor sequence (e.g., amino-acid T-cell receptor sequence) is obtained, T cells can be engineered to include the T cell receptor that specifically recognizes the mutant peptide. These engineered T cells can then be administered to a subject. See, for example, Matsuda et al. "Induction of Neoantigen-Specific Cytotoxic T Cells and Construction of T-cell Receptor Engineered T Cells for Ovarian Cancer," Clin. Cancer Res. 1-11 (2018), which is hereby incorporated by reference in its entirety for all purposes. In any of the methods provided herein, The T cells can be expanded *in vitro* and/or *ex vivo* prior to administration to a subject. The subject may then be administered (e.g., infused with) a composition that includes the expanded population of T cells.

In some instances, a method of treatment for a disease (such as cancer) is provided, which may include administering to an individual a composition that includes one or more mutant peptides (or one or more precursors thereof) in an amount effective to, for example, prime, activate and expand T cells *in vivo.*

In some embodiments, a method of treatment for a disease (such as cancer) is provided, which may include administering to an individual an effective amount of a composition including a precursor of a mutant peptide selected using a technique described herein. In some embodiments, an immunogenic vaccine may include a pharmaceutically acceptable mutant peptide selected using a technique described herein. In some embodiments, an immunogenic vaccine may include a pharmaceutically acceptable precursor to a mutant peptide selected using a technique described herein (such as a protein, peptide, DNA and/or RNA). In some embodiments, a method of treatment for a disease (such as cancer) is provided, which may include administering to an individual an effective amount of an antibody specifically recognizing a mutant peptide selected using a technique described herein. In some embodiments, a method of treatment for a disease (such as cancer) is provided, which may include administering to an individual an effective amount of a soluble TCR or TCR analog specifically recognizing a mutant peptide selected using a technique described herein.

In some embodiments, the cancer is any one of: carcinoma, lymphoma, blastema, sarcoma, leukemia, squamous cell cancer, lung cancer (including small cell lung cancer, non-small cell lung cancer, adenocarcinoma of the lung, and squamous carcinoma of the lung), cancer of the peritoneum, hepatocellular cancer, gastric or stomach cancer (including gastrointestinal cancer), pancreatic cancer, glioblastoma, cervical cancer, ovarian cancer, liver cancer, bladder cancer, hepatoma, breast cancer, colon cancer, melanoma, endometrial or uterine carcinoma, salivary gland carcinoma, kidney or renal cancer, liver cancer, prostate cancer, vulval cancer, thyroid cancer, hepatic carcinoma, head and neck cancer, colorectal cancer, rectal cancer, soft-tissue sarcoma, Kaposi's sarcoma, B-cell lymphoma (including low grade/follicular non-Hodgkin's lymphoma (NHL), small lymphocytic (SL) NHL, intermediate grade/follicular NHL, intermediate grade diffuse NHL, high grade immunoblastic NHL, high grade lymphoblastic NHL, high grade small non-cleaved cell NHL, bulky disease NHL, mantle cell lymphoma, AIDS-related lymphoma, and Waldenstrom's macroglobulinemia),

chronic lymphocytic leukemia (CLL), acute lymphoblastic leukemia (ALL), myeloma, Hairy cell leukemia, chronic myeloblasts leukemia, and post-transplant lymphoproliferative disorder (PTLD), as well as abnormal vascular proliferation associated with phakomatoses, edema (such as that associated with brain tumors), and Meigs' syndrome.

Embodiments disclosed herein can including identifying part or all of and/or implementing part or all of an individualized-medicine strategy. For example, one or more mutant peptides may be selected for use in a vaccine by: determining an MHC sequence and/or a set of variant-coding sequences using a sample from an individual; and processing representations of the MHC sequence and the variant-coding sequences using a machine-learning model disclosed herein (e.g., an attention-based machine learning model). The one or more mutant peptides (and/or precursors thereof) may then be administered to the same individual.

In some embodiments, a method of treating a disease (such as cancer) in an individual is provided that includes: a) identifying a one or more mutant peptides in the individual (e.g., based on results produced by a machine-learning model corresponding to predictions as to whether and/or an extent to which each of set of mutant peptides would bind to an MHC molecule of the individual, be presented by an MHC molecule of the individual and/or trigger an immune response in the individual, in accordance with one or more techniques disclosed herein); b) synthesizing the identified mutant peptide(s) or one or more precursors of the mutant peptide(s) or nucleic acid(s) (e.g., polynucleotides such as DNA or RNA) encoding the identified peptide(s) or peptide precursor(s); and c) administering the mutant peptide(s), mutant-peptide precursor(s) or nucleic acid(s) to the individual.

In some embodiments, a method of treating a disease (such as cancer) in an individual is provided that includes: a) identifying a one or more mutant peptides in the individual (e.g., based on results produced by a machine-learning model corresponding to predictions as to whether and/or an extent to which each of set of mutant peptides would bind to an MHC molecule of the individual, be presented by an MHC molecule of the individual and/or trigger an immune response in the individual, in accordance with one or more techniques disclosed herein); b) identifying a set of nucleic acids (e.g., polynucleotides such as DNA or RNA) that encode the identified mutant peptide(s) or one or more precursors of the mutant peptide(s); c) synthesizing the set of nucleic acids; and d) administering the set of nucleic acids to the individual.

In some embodiments, a method of treating a disease (such as cancer) in an individual is provided that includes: a) identifying a one or more mutant peptides in the individual (e.g., based on results produced by a machine-learning model corresponding to predictions as to whether and/or an extent to which each of set of mutant peptides would bind to an MHC molecule of the individual, be presented by an MHC molecule of the individual and/or trigger an immune response in the individual, in accordance with one or more techniques disclosed herein); b) producing an antibody specifically recognizing the mutant peptide; and c) administering the peptide to the individual.

The methods provided herein can be used to treat an individual (e.g., human) who has been diagnosed with or is suspected of having cancer. In some embodiments, an individual may be a human. In some embodiments, an individual may be at least about any of 18, 20, 25, 30, 35, 40, 45, 50, 55, 60, 65, 70, 75, 80, or 85 years old. In some embodiments, an individual may be a male. In some embodiments, an individual may be a female. In some embodiments, an individual may have refused surgery. In some embodiments, an individual may be medically inoperable. In some embodiments, an individual may be at a clinical stage of Ta, Tis, T1, T2, T3a, T3b, or T4. In some embodiments, a cancer may be recurrent. In some embodiments, an individual may be a human who exhibits one or more symptoms associated with cancer. In some of embodiments, an individual may be genetically or otherwise predisposed (e.g., having a risk factor) to developing cancer.

The methods provided herein may be practiced in an adjuvant setting. In some embodiments, the method is practiced in a neoadjuvant setting, i.e., the method may be carried out before the primary/definitive therapy. In some embodiments, the method is used to treat an individual who has previously been treated. Any of the methods of treatment provided herein may be used to treat an individual who has not previously been treated. In some embodiments, the method is used as a first-line therapy. In some embodiments, the method is used as a second-line therapy.

In some embodiments, there is provided a method of reducing incidence or burden of preexisting cancer tumor metastasis (such as pulmonary metastasis or metastasis to the lymph node) in an individual, comprising administering to the individual an effective amount of a composition disclosed herein. In some embodiments, there is provided a method of prolonging time to disease progression of cancer in an individual, comprising administering to the individual an effective amount of a composition disclosed herein. In some embodiments, there is provided a method of prolonging survival of an individual having cancer, comprising administering to the individual an effective amount of a composition disclosed herein.

In some embodiments, at least one or more chemotherapeutic agents may be administered in addition to the composition disclosed herein. In some embodiments, the one or more chemotherapeutic agents may (but not necessarily) belong to different classes of chemotherapeutic agents.

In some embodiments, there is provided a method of treating a disease (such as cancer) in an individual, comprising administering: a) a vaccine disclosed herein (e.g., that includes a mutant peptide selected based on a machine-learning technique disclosed herein or a precursor thereof), and b) an immunomodulator. In some embodiments, there is provided a method of treating a disease (such as cancer) in an individual, comprising administering: a) a vaccine disclosed herein (e.g., that includes a mutant peptide selected based on a machine-learning technique disclosed herein or a precursor thereof), and b) an antagonist of a checkpoint protein. In some embodiments, there is provided a method of

treating a disease (such as cancer) in an individual, comprising administering: a) a vaccine disclosed herein (e.g., that includes a mutant peptide selected based on a machine-learning technique disclosed herein or a precursor thereof), and b) an antagonist of programmed cell death 1 (PD-1), such as anti-PD-1. In some embodiments, there is provided a method of treating a disease (such as cancer) in an individual, comprising administering: a) a vaccine disclosed herein (e.g., that includes a mutant peptide selected based on a machine-learning technique disclosed herein or a precursor thereof), and b) an antagonist of programmed death-ligand 1 (PD-L1), such as anti-PD-L1. In some embodiments, there is provided a method of treating a disease (such as cancer) in an individual, comprising administering: a) a vaccine disclosed herein (e.g., that includes a mutant peptide selected based on a machine-learning technique disclosed herein or a precursor thereof), and b) an antagonist of cytotoxic T-lymphocyte-associated protein 4 (CTLA-4), such as anti-CTLA-4.

It will be appreciated that various disclosures refer to use of amino-acid sequences. Nucleic-acid sequences may additionally or alternatively be used. For example, a disease-specific sample may be sequenced to identify a set of nucleic-acid sequence that are not present in a corresponding non-disease-specific sample (e.g., from a same subject or different subject). Similarly, a nucleic-acid sequence of an MHC molecule and/or T-cell receptor may further be identified. Representations of each of a nucleic-acid disease-specific nucleic-acid sequence and of an MHC molecule (or of a T-cell receptor) may be processed by an attention-based model as described herein (e.g., and potentially having been trained using representations of nucleic-acid sequences).

## V. Examples

### V.A. Overview

An exemplary peptide-MHC (MHC Class I) attention-based machine learning model (herein "P-MHC-I Model") and an exemplary peptide-MHC (MHC Class II) attention-based machine learning model (herein "P-MHC-II Model") (collectively and individually referred to herein as P-MHC Model) were developed. These models are examples of implementations for machine learning model 132 in Fig. 1. Both the P-MHC-I Model architecture and the P-MHC-II Model architecture were implemented in correspondence with the architecture depicted in Fig. 3 and in Fig. 4A.

The P-MHC Model is an exemplary attention-based deep learning model for predicting neoantigen presentation in individualized cancer vaccine development. The P-MHC Model receives N-flank sequence, peptide sequence, and MHC sequence (MHC pseudosequence) as inputs and outputs a presentation or eluted ligand (EL) score. A vocabulary was built that spans the space of naturally occurring amino acids, tokenizing them to represent amino acid sequences. The input amino acid sequences were tokenized to be characters, each of which is represented by a unique character. The model pairs the input N-flank sequence and peptide sequence with one of 6 MHC alleles and the 6 paired interactions were feedforwarded into the P-MHC-I Model, and one of 12 MHC allotypes and the 12 paired interactions were feedforwarded into the P-MHC-II model, for selecting the specific binding MHC allele.

Thus, the P-MHC Model internally performs deconvolution of multi-allelic data. The most likely to elute peptide-MHC interaction output is normalized as a value between 0 and 1 and is compared to the true presentation value using a binary cross-entropy loss function to generate the error for tuning the model parameters. To prevent overfitting and increase the model robustness, the P-MHC Model uses ensemble methods in model training.

Exemplary results and statistics corresponding to the training and performance of the P-MHC-I Model and the P-MHC-II Model as compared to other previously available models (e.g., NetMHCpan-4.0 (herein "Model A"), Immune Epitope Database and Analysis Resource (IEDB) v2.13 (herein "Model B") for P-MHC-I Model and NetMHCIIpan-4.1 (herein "Model C") for P-MHC-II Model. The P-MHC-I and P-MHC-II Models consistently performed better than the other models for peptide presentation and the P-MHC-I Model performed better than the other models for CD8 T cell response prediction. The P-MHC Model performs better at least because it performs deconvolution of peptide-MHC pairs from multiallelic data, can readily be trained on augmented training data in both monoallelic and multiallelic formats.

### V.B. Materials and Methods

#### V.B.1. Training P-MHC Model - Immunopeptidomics data

Peptide elution data from mass spectrometry experiments was used to build the immunopeptidomics data set for training P-MHC Model. This data includes a mixture of private and public data sets, which include multi-allelic data and monoallelic peptide elution data from cell lines, tissue samples, and PBMC donors.

##### V.B.1.a. Presentation-Labeled Data

**Positive Set (EL=1).** For each batch, the positive peptide-MHC (e.g., peptide-HLA) pairs were processed in the following manner:

1) Peptides were aligned to the human proteome.
2) For each peptide, flanking sequences, of length up to 10 amino acids, were retained on the N-terminal and C-terminal position.
3) Peptides that mapped to multiple genes were removed from downstream analysis. Such peptides did not feature in EL=1 sets. (No such restriction was imposed on EL=0, since the EL=0 peptides were only generated from proteins that had evidence of EL=1 peptides.). 48,329 Class I peptides were filtered out by this criteria. Although this is a large number, it increases confidence in the negative set.
4) Peptides that map to the same gene, but with different flanking sequences were also removed from downstream analysis. This further removed 11,443 Class I peptides.
5) Peptides that contained post translational modifications (PTM) were also removed from downstream analysis. 7,080 Class I such peptides were removed.

**Negative set (EL=0).** The negative peptide-MHC (e.g., peptide-HLA) pairs were generated computationally. For each allele, for each protein of origin in the positive set (EL=1), all possible peptide fragments of length 8:11 were generated for MHC Class I and 8-30 for MHC Class II, with uniform probability for each length. N-terminal and C-terminal flanking sequences were also retained with max length of 10 amino acids. All peptide-genotype pairs featured in EL=1 data were removed from the EL=0 data. Additionally, for datasets constructed for MHC Class II, peptide-genotype pairs with any length 9 subsequence that can be found in an EL=1 peptide (paired with the same genotype) is removed.

V.B.1.b. Benchmark Data Set

A benchmark data set was created by splitting the above EL data discussed Section VI.B.1.a into training, validation, and test sets. The training and validation sets were used for training the P-MHC Model, while the test set was explicitly not used for training and used only to quantify performance of the model. For MHC Class I data, Mono-allelic data was used to generate the test data set, by holding out 10% of peptides from monoallelic data for each allele. For MHC Class II data, all data, multiallelic and monoallelic are used to generate the test/validation datasets.

Features of the dataset include: All peptide lengths were restricted to be in the range of [8, 14] amino acids for Class I, and [8,30] amino acids for Class II. All peptides were restricted to contain canonical amino acids in the main sequence (i.e., epitope) and flanking sequences. All allele names were replaced by 34 amino acid subsequences defined by the following amino acid positions within the MHCI protein: (7, 9, 24, 45, 59, 62, 63, 66, 67, 69, 70, 73, 74, 76, 77, 80, 81, 84, 95, 97, 99, 114, 116, 118, 143, 147, 150, 152, 156, 158, 159, 163, 167, 171), or positions within the alpha and beta MHCII proteins:

alpha: 9,11,22,24,31,52,53,58,59,61,65,66,68,72,73; and
beta: 9,11,13,26,28,30,47,57,67,70,71,74,77,78,81,85,86,89,90.

These positions have been previously described as the positions in the binding pocket where the MHC-I/II protein contacts the peptide. The set of unique subsequences for a data point may henceforth be referred to as 'pseudoGenotype'. In some cases, multiple allele names may feature the same 34 amino acid subsequence. These alleles were considered identical for training the attention-based P-MHC Model. All empty flanking sequences (peptide maps to the end of protein) were assigned a special amino acid character, "$". Six data points where the flanking sequences read as 'NA' in the amino acid alphabet were removed from consideration due to certain programming languages interpreting NA as "Not Applicable.

The train/validation/test splits were conducted in the following manner:

**For EL = 1:** For each processing batch (each batch was based on the original source of the data set), monoallelic data was randomly split across train/validation/test groups at a ratio of 70/20/10. For MHC Class II it is insured that no length 9 subsequence from the peptide sequence overlaps between the train/validation/test datasets for peptides with exact genotype matches. The monoallelic data is composed of 105 (41) unique subsequences representing 111 (39) unique MHC Class I (MHC Class II), respectively, alleles across the whole dataset. All multi allelic data was entirely used for training for Class I datasets. The multiallelic data is composed of 126 (76) unique MHC Class I (MHC Class II) genotypes across the whole data set. Data across processing batches was combined and duplicate {peptide, nFlank, cFlank, mhc0, mhc1, mhc2, mhc3, mhc4, mhc5} (MHC Class I), and {peptide, nFlank, cFlank, mhc_dq1_1, mhc_dq1_2, mhc_dq1_3, mhc_dq1_4, mhc_dp1_1, mhc_dp1_2, mhc_dp1_3, mhc_dp1_4, mhc_dr1_1, mhc_drl_2, mhc_dr3_1, mhc_dr3_2, mhc_dr4_1, mhc_dr4_2, mhc - dr5 _1, mhc_dr5_2} (MHC Class II) tuples were removed.

**For EL = 0:** For EL = 0: For each processing batch, for each {peptide, pseudoGenotype} pair, negative peptide data was sampled at a 1:1 ratio with the EL=1 data in the train and validation groups. In the test group, it was sampled at a 1:99 ratio for MHC Class I, and 1:9 ratio for MHC Class II. Data across processing batches were combined and duplicate observations were removed. This finally resulted in 1.71 % of the test data as positives (instead of 1%) for MHC Class I, and 11.15% for MHC Class II (instead of 10%).

For observations with multiple subsequences in the 'pseudoGenotype,' i.e. multi-allelic data, negative peptides were generated by eliminating positive peptides for each of the alleles, and then random peptides were chosen from the source proteins.

V.B.1.c. Benchmark QC

The following downstream QC procedure was followed to ensure no redundancy in the data: 1) only canonical amino acids are allowed in peptide, Nflank, and Cflank sequences; 2) each set of {Nflank, peptide, Cflank, pseudoGenotype} tuple is unique; and 3) there is no overlap of {Nflank, peptide, Cflank, pseudoGenotype} tuples in the EL=1 and EL=0 set. For MHC Class II it is further insured that there is no overlap between length 9 subsequences within the peptide sequence between EL=1 and EL=0, for peptides with identical pseudoGenotypes.

The number of MHC (HLA) pseudogenotypes, may be different from the number of alleles, since some alleles with different allele names (at 2-field resolution, i.e. 4-digit resolution) may have the same pseudogenotype.

V.B.2. Immunogenicity Datasets to Evaluate P-MHC-1 Model Performance

To evaluate the performance of the P-MHC-I Model, two different datasets were used. For a first test immunogenicity dataset, oncology subjects had their DNA sequenced, and from that standard P-MHC binding, predictions were conducted using IEDPv.2.13BA to predict neoantigens that were presented by and/or bound in MHC. Neoantigens thus predicted were further prioritized using their expression, variant allelic frequency, and clonality in the tumor tissue. The subjects were subsequently dosed with an RNA vaccine as introduced above. T cell responses to the neoantigens introduced in the RNA vaccine were monitored in the dosed subjects using multimer and ELISPOT assays. T cell responses believed to be technical artifacts, using several controls in these assays, were removed. In a second test immunogenicity dataset, sequencing data was obtained from oncology subjects receiving checkpoint blockade therapy (but not RNA vaccine therapy) identified by the Tumor Neoantigen Selection Alliance (TESLA) consortium. P-MHC binding prediction was conducted using NetMHCcons 1.0 to predict neoantigens that were presented by and/or bound in MHC. Immunogenicity assays were run on the neoantigens predicted by P-MHC-I Model and used to evaluate P-MHC-I Model's performance.

V.B.2.a. Dosed subject multimer assay

For the first test immunogenicity dataset, multimer assay data were assessed for a positive or negative outcome for detection of a CD8 T cell by peptide-MHC multimers. Conservative criteria was used to declare positive outcome: specifically, whether dual tetramer positive CD8 T cell count was greater than 0.05%. Some of the neoepitopes were called positive despite having lower than 0.05% neoepitope-specific CD8 T cells, if closer T cell phenotype examination strongly suggested a T cell response. From the multimer assay data, 1318 neoepitopes were declared negative, and based on the conservative criteria, a small fraction of these are expected to be false negatives. 27 neoepitope-HLA pairs were declared as positive only post-vaccination (referred to as de novo responses) and 20 pairs were declared as pre-existing CD8 T cell responses.

V.B.2.b. Dosed subject ELISpot assay

Further for the first test immunogenicity dataset, ELISpot data was collected. A statistical assessment was conducted, of spot counts of negative controls without peptide restimulation, and test cases with peptide restimulations, to declare positive calls (using a permutations approach), and further verified manually, to assign a positive or negative outcome for immunogenicity of a neoantigen for a given subject visit. A neoantigen was declared as positive in the ELISpot assay if it showed a positive outcome in any of the subject visits, whether pre-treatment or post-treatment. Neoantigens were further filtered based on the following criteria: (a) adjudicator-decided assay outcome value was not 'NA'; (b) none of the evaluated P-MHC-1 scoring methods (P-MHC-I, Model A, Model B) assigned an 'NA' value to the neoantigen; and (c) pooled neoantigens were used for restimulation removed from consideration.

After all the filtering steps, distribution of positive (immunogenic) and negative (non-immunogenic) neoantigens for each cell type evaluated in the ELISpot assays is shown below. Assay.value_binary=TRUE implies an immunogenic neoantigen, and non-immunogenic outcomes were labeled as Assay.value_binary=FALSE.

| | Assay.value_binary | |
|---|---|---|
| Assay.t_cell_type | FALSE | TRUE |
| CD4 | 144 | 17 |

(continued)

| | Assay.value_binary | |
|---|---|---|
| Assay.t_cell_type | FALSE | TRUE |
| CD8 | 207 | 59 |
| PBMC | 522 | 62 |

The positive assays were further classified into two sets, based on spot counts from the ELISpot assay. Each ELISpot assay had replicate experiments, and a mean spot count was specified across the replicates. For a positive neoantigen, the maximum value of the mean spot count across all visits was considered, and the positive neoantigens were split into two sets, one with this spot count value < 50, and the other with this spot count value >=50. The latter set represents neoantigens that induced more extensive T cell responses, and is less likely to contain false positive interpretations of the ELISpot results compared to the set with fewer spot counts. The choice of 50 spots was an arbitrary decision, as it was reasonably higher than the original threshold used for calling ELISpot positives (spot count > 15).

V.B.2.c. TESLA multimer assay

For the second test immunogenicity dataset, the TESLA consortium had validated neoantigen predictions. Assay data was available for subjects 1, 2, 3, 4, 10, 12 and 16 from TESLA's subject identifiers. Assay results were provided by TESLA based on four different assays: TCR_FLOW_I, TCR_FLOW_II, a nanoparticle assay and a TCR reactivity assay. The TCR_FLOW_I assay results were used in this Example. The other assays were disregarded because of the following reasons: (a) the nanoparticle assay is expected to have higher false positive rate as it is a single cell assay designed to be very sensitive; (b) TCR_FLOW_II is largely redundant with TCR_FLOW_I, with both being performed at different labs and TCR_FLOW_II having fewer data points. The TCR reactivity assay is an intracellular IFNg / TNFa staining assay following prestimulation of T cells with IL-2 and short peptides for 7 days, followed by restimulation with a short peptide. The TESLA team did not endorse using this assay for evaluating peptide-MHC presentation prediction. The selected assay had 16 positive outcomes and 196 negative outcomes.

V.B.3. Comparison models - NetMHCpan and IEDB scores

For performance comparison against P-MHC-I Model, Model A and Model B were used to assign BA and EL values to peptide-HLA pairs. For performance comparison against P-MHC-II Model, Model C was used to assign EL values to peptide-MHC (HLA) pairs. The BA and EL values, output as percentile scores by these methods, are referred to (in this Example) as BA or EL. These percentile values behave such that a lower value implies higher affinity or likelihood of presentation. A transformed scoring scheme was used by taking inverse of these values to obtain scores (e.g., for MHC-I, binding affinity score for Model A, an elution score for Model A, and a binding affinity score for Model B; for MHC-II, binding affinity score for Model C)that behave such that a higher value indicates stronger affinity or presentation likelihood. For neoepitopes-HLA pairs, a single such score is obtained. For neoantigens, all neoepitope-HLA pairs were considered for 8-14 mer long neoepitope candidates containing the mutation, and the pair with the highest score was chosen to represent the neoantigen score.

**V.C. Results**

V.C. 1. P-MHC-I Model Performance on Presentation data

Figs. 14A-C are plots with exemplary precision-recall (PR) curves in accordance with one or more embodiments. Figs. 14A-C illustrate the performance of the P-MHC-I Model as compared to previously used approaches. An Eluted ligand (EL) test dataset was used to evaluate the presentation prediction performance between the EL output of the P-MHC-I Model, the EL output of Model A, and the binding affinity (BA) output of Model C.

Fig. 14A includes plot 1400 indicating the performance of the P-MHC-I Model. Fig. 14B includes plot 1402 indicating the performance of Model A with respect to its elution output. Fig. 14C includes plot 1404 indicating the performance of Model B with respect to its binding affinity output. The dot on the curve of each of plots 1400, 1402, and 1404 corresponds to a score threshold for the top 1.71% quantile of the score (selected due to 1.71% of the gold standard test data being positive). Average precision (AP) is representative of threshold-independent performance. The F1 score, precision, and recall values are based on the 1.71% threshold.

Model A and Model B values were percentile rank outputs from these methods. The P-MHC-I Model values were taken from the output (of the final node) of the P-MHC-I Model. Based on these PR curves, the results in Figs. 14A-C indicate that P-MHC-I Model showed improved performance over both Model A and Model C (AP value of 0.85 vs 0.78 for

Model A and 0.57 for Model B). AP values of the methods were compared on a per-allele basis.

Fig. 15 is a plot 1500 comparing exemplary average precision values of elution-ligand outputs of Model A and the P-MHC-I Model for each allele in a test data set in accordance with one or more embodiments. The test data set, which was monoallelic, included at least 1000 data points, with 67 alleles satisfied the criteria. As shown in plot 1500, the P-MHC-I Model over Model A showed higher performance. Patterns of the markers in plot 1500 indicate whether the allele was from HLA-A, B, or C gene. Sizes of the markers represent the amount of monoallelic data used in training the P-MHC-I Model for that allele, which also correlates with the amount of test data for each allele.

Figs. 16A and 16B are of plots 1600 and plot 1602, respectively, that compare the performance of the P-MHC-I Model on a human dataset with the performance of the P-MHC-I Model on a mouse dataset in accordance with one or more embodiments. As shown by these plots, the P-MHC-I Model performed well for both datasets with the average precision of the P-MHC-I Model being similar for both the human and mouse datasets. These results demonstrate that the P-MHC-I Model may be a pan-species model that can be used with desirable performance across various species.

V.C.2. P-MHC-II Model Performance on Presentation data

Figs. 17A and 17B are plot 1700 and plot 1702, respectively, that compare the performance of the P-MHC-II Model with Model C on the presentation data in accordance with one or more embodiments. Model C values were percentile rank outputs. The P-MHC-II Model values were taken from the output (of the final node) of the P-MHC-II Model. Using average precision from PR curves, the results in Figs. 17A and 17B indicate that the P-MHC-II Model, having an AP of .69, showed improved performance over Model C, having an AP of .31. AP values of these two methods were compared on a per-allele basis.

Figs. 18A and 18B are plot 1800 and plot 1802, respectively, that compare the performance of the P-MHC-II Model with Model C, respectively, on a holdout dataset in accordance with one or more embodiments. Again, the P-MHC-II Model, having an AP of .84, shows improved performance over Model C, having an AP of .46.

Fig. 19 is plot 1900 showing a per genotype comparison of average precision for the P-MHC-II Model with Model C on a test dataset in accordance with one or more embodiments. On a per genotype basis, the P-MHC-II Model had improved performance over Model C.

V.C.3. Performance on the First and Second Test Immunogenicity Datasets

The first and the second test immunogenicity datasets were used to evaluate the performance of the P-MHC presentation predictions on T cell response data. In these evaluations, no training was done on the immunogenicity data, and only the amino acid sequence of the neoantigens and the MHC proteins were used to calculate the P-MHC presentation scores. Other features, for example, expression of the gene or of the mutant allele, were not used to allow evaluation of the contribution of the P-MHC presentation prediction on predicting CD8 T cell response in a reductionist manner.

V.C.3.a. Dosed subject multimer assay

Fig. 20 is a plot 2000 of receiver operating characteristic (ROC) curves that illustrates performance of the P-MHC-I Model (EL output), Model A (EL output), and Model B (BA output) with respect to CD 8 multimer assay data (first test immunogenicity dataset) in accordance with one or more embodiments. Performance was evaluated with respect to the ability to predict positive neoepitopes from the multimer assay. For Model A and Model B, values were inverse-transformed to obtain EL and BA scores, respectively, such that a higher value indicated stronger binding affinity or presentation likelihood. The area-under-curve (AUC) was calculated based on the step function. The step function for plotting the ROC curve connected the points representing true positive rates (tpr) and false positive rates (fpr), in a horizontal then vertical direction. The true positive rate(tpr) and false positive rate (fpr) values were calculated using the R package ROCR.

V.C.3.b. Dosed subject ELISpot assay

Figs. 21A-D are plots 2102, 2104, 2106, and 2108, respectively, that illustrate the performance of the P-MHC-I Model (El output), Model A (EL output), and Model B (BA output) with respect to ELISpot assays (first test immunogenicity dataset) in accordance with one or more embodiments. As illustrated, the P-MHC-I Model performed well with strong predictive power. The plots show exemplary ROC curves with separate subplots shown for PBMC ELISpots (Fig. 21A, PBMC panel), and CD8 ELISpot (Fig. 21B, CD8 panel). Positive CD8 ELISpot data were further split into two sets, and ROC curves were made, for stronger T cell responses (Fig. 21C, CD8, spots >= 50), and relatively weaker T cell responses (Fig. D, CD8, spots < 50). To make the ROC curves for these two sets, the same negative set of neoantigens was used.

V.C.3.c. TESLA multimer assay

Figs. 22A-D are plots 2202, 2204, 2206, and 2208, respectively, that illustrate the performance of Model A (BA output), Model A (EL output), Model C (BA output), and the P-MHC-I Model (EL output), respectively in accordance with one or more embodiments. Performance was evaluated on the TESLA immunogenicity data (second test immunogenicity dataset), with results from multimer assays being used. These plots are scatter plots corresponding to exemplary neoepitope-HLA pairs evaluated by multimer assays from the TESLA study. A response is TRUE for positive hits from the assay as specified by TESLA, and FALSE for non-immunogenic neoepitopes. The Wilcoxon rank sum test was used to calculate p-values for a two-sided alternative hypothesis. Y-axes show transformed scores such that a higher value corresponds to stronger peptide-MHC binding or presentation.

Fig. 23 is an illustration of a plot 2300 comparing ROC curves for the Model A (EL output), Model B (BA output), and P-MHC-I Model (EL output) using TESLA multimer assay data in accordance with one or more embodiments. The multimer assay was the TCR_FLOW_I assay. The area under the curve was highest for the P-MHC-I Model.

## V.D. Conclusion

Thus, P-MHC presentation prediction methods were evaluated on two types of evaluation data sets: P-MHC presentation data from immunopeptidomics experiments and T cell response data from various immunogenicity assays. The presentation predictors trained on immunopeptidomics data perform better compared to the current production method (IEDBv2.13BA output) on many of these data sets. P-MHC Model showed improved performance values across many of the data sets. Accordingly, using attention-based techniques trained on immunopeptidomics data may be superior to models based on in vitro binding affinity data.

## VI. Computer Implemented System

Figure 26 is a block diagram of a computer system in accordance with various embodiments. Computer system 2600 may be an example of one implementation for computing platform 102 described above in Figure 1.

In one or more examples, computer system 2600 can include a bus 2602 or other communication mechanism for communicating information, and a processor 2604 coupled with bus 2602 for processing information. In various embodiments, computer system 2600 can also include a memory, which can be a random-access memory (RAM) 2606 or other dynamic storage device, coupled to bus 2602 for determining instructions to be executed by processor 2604. Memory also can be used for storing temporary variables or other intermediate information during execution of instructions to be executed by processor 2604. In various embodiments, computer system 2600 can further include a read only memory (ROM) 2608 or other static storage device coupled to bus 2602 for storing static information and instructions for processor 2604. A storage device 2610, such as a magnetic disk or optical disk, can be provided and coupled to bus 2602 for storing information and instructions.

In various embodiments, computer system 2600 can be coupled via bus 2602 to a display 2612, such as a cathode ray tube (CRT) or liquid crystal display (LCD), for displaying information to a computer user. An input device 2614, including alphanumeric and other keys, can be coupled to bus 2602 for communicating information and command selections to processor 2604. Another type of user input device is a cursor control 2616, such as a mouse, a joystick, a trackball, a gesture input device, a gaze-based input device, or cursor direction keys for communicating direction information and command selections to processor 2604 and for controlling cursor movement on display 2612. This input device 2614 typically has two degrees of freedom in two axes, a first axis (e.g., x) and a second axis (e.g., y), that allows the device to specify positions in a plane. However, it should be understood that input devices 2614 allowing for three-dimensional (e.g., x, y, and z) cursor movement are also contemplated herein.

Consistent with certain implementations of the present teachings, results can be provided by computer system 2600 in response to processor 2604 executing one or more sequences of one or more instructions contained in RAM 2606. Such instructions can be read into RAM 2606 from another computer-readable medium or computer-readable storage medium, such as storage device 2610. Execution of the sequences of instructions contained in RAM 2606 can cause processor 2604 to perform the processes described herein. Alternatively, hard-wired circuitry can be used in place of or in combination with software instructions to implement the present teachings. Thus, implementations of the present teachings are not limited to any specific combination of hardware circuitry and software.

The term "computer-readable medium" (e.g., data store, data storage, storage device, data storage device, etc.) or "computer-readable storage medium" as used herein refers to any media that participates in providing instructions to processor 2604 for execution. Such a medium can take many forms, including but not limited to, non-volatile media, volatile media, and transmission media. Examples of non-volatile media can include, but are not limited to, optical, solid state, magnetic disks, such as storage device 2610. Examples of volatile media can include, but are not limited to, dynamic memory, such as RAM 2606. Examples of transmission media can include, but are not limited to, coaxial cables, copper

wire, and fiber optics, including the wires that comprise bus 2602.

Common forms of computer-readable media include, for example, a floppy disk, a flexible disk, hard disk, magnetic tape, or any other magnetic medium, a CD-ROM, any other optical medium, punch cards, paper tape, any other physical medium with patterns of holes, a RAM, PROM, and EPROM, a FLASH-EPROM, any other memory chip or cartridge, or any other tangible medium from which a computer can read.

In addition to computer readable medium, instructions or data can be provided as signals on transmission media included in a communications apparatus or system to provide sequences of one or more instructions to processor 2604 of computer system 2600 for execution. For example, a communication apparatus may include a transceiver having signals indicative of instructions and data. The instructions and data are configured to cause one or more processors to implement the functions outlined in the disclosure herein. Representative examples of data communications transmission connections can include, but are not limited to, telephone modem connections, wide area networks (WAN), local area networks (LAN), infrared data connections, NFC connections, optical communications connections, etc.

It should be appreciated that the methodologies described herein, flow charts, diagrams, and accompanying disclosure can be implemented using computer system 2600 as a standalone device or on a distributed network of shared computer processing resources such as a cloud computing network.

The methodologies described herein may be implemented by various means depending upon the application. For example, these methodologies may be implemented in hardware, firmware, software, or any combination thereof. For a hardware implementation, the processing unit may be implemented within one or more application specific integrated circuits (ASICs), digital signal processors (DSPs), digital signal processing devices (DSPDs), programmable logic devices (PLDs), field programmable gate arrays (FPGAs), processors, controllers, micro-controllers, microprocessors, electronic devices, other electronic units designed to perform the functions described herein, or a combination thereof.

In various embodiments, the methods of the present teachings may be implemented as firmware and/or a software program and applications written in conventional programming languages such as C, C++, Python, etc. If implemented as firmware and/or software, the embodiments described herein can be implemented on a non-transitory computer-readable medium in which a program is stored for causing a computer to perform the methods described above. It should be understood that the various engines described herein can be provided on a computer system, such as computer system 2600, whereby processor 2604 would execute the analyses and determinations provided by these engines, subject to instructions provided by any one of, or a combination of, the memory components RAM 2606, ROM, 2608, or storage device 2610 and user input provided via input device 2614.

## VII. Exemplary Descriptions of Terms

As used herein, the terms "peptide," "polypeptide," and "protein" are used interchangeably to refer to a polymer of amino acid residues. The terms encompass amino acid chains of any length, including full-length proteins with amino acid residues linked by covalent peptide bonds.

As used herein, a "mutant peptide" may refer to a peptide that is not present in the normal tissue (e.g., in the wild type amino acid sequences of normal tissue) of an individual subject. A mutant peptide comprises at least one mutant amino acid and may be present in a diseased tissue (e.g., collected from a particular subject) but not in a normal tissue (e.g., collected from the particular subject, collected from a different subject, and/or as identified in a database as corresponding to normal tissue). A mutant peptide may include an epitope. An epitope is the portion of a mutant peptide to which an MHC molecule or a T cell receptor (TCR) binds. Thus, this binding between the epitope of the mutant peptide and the MHC molecule or TCR can induce an immune response (as a result of the mutant peptide not being associated with a subject's "self'). A mutant peptide can include or can be a neoantigen. A mutant peptide can arise from, for example: a non-synonymous mutation leading to different amino acids in the protein (e.g., point mutation); a read-through mutation in which a stop codon is modified or deleted, leading to translation of a longer protein with a novel tumor-specific sequence at the C-terminus; a splice site mutation that leads to a unique tumor-specific protein sequence; a chromosomal rearrangement that gives rise to a chimeric protein with a tumor-specific sequence at a junction of two proteins (i.e., gene fusion) and/or a frameshift insertion or deletion that leads to a new open reading frame with a tumor-specific protein sequence. A mutant peptide can include a polypeptide (as characterized by a polypeptide sequence) and/or may be encoded by a nucleotide sequence.

As used herein, a "C-flank" of a peptide refers to one or more amino acids upstream of the C-terminus of the peptide, from the parent protein. Optionally, a C-flank of a peptide includes one, two, three, four, five, or more amino acid residues upstream of the C-terminus of the peptide.

As used herein, an "N-flank" of a peptide refers to one or more amino acids downstream of the N-terminus of the peptide, from the parent protein. Optionally, an N-flank of a peptide includes one, two, three, four, five, or more amino acid residues downstream of the N-terminus of the peptide.

As used herein, an "epitope" of a peptide may refer to a region of the peptide between the C-flank and N-flank and can be recognized by a TCR. The epitope of the peptide is a part of the peptide that is recognized by TCR on a T cell and

MHC I on an antigen presenting cell. For example, the epitope can be a peptide to which a TCR binds, for example, a peptide to which the TCR binds when the peptide is bound to MHC I on an antigen presenting cell.

As used herein, a "ligand" is a peptide that is found to be presented by an MHC molecule at the cell surface from elution experiments or found to be bound to MHC in an in vitro assay.

As used herein, a "sequence" refers to an amino-acid sequence that includes an ordered set of amino-acid identifiers.

As used herein, a "peptide sequence" refers to a sequence that identifies amino acids of at least a portion of a peptide. In some cases, the peptide sequence includes a variant-coding sequence that includes a variant that is not observed in a corresponding reference sequence.

When the peptide includes a mutant peptide, the variant-coding sequence, identifies amino acids of the mutation or variant. However, when the peptide does not include a mutation or variant, the variant-coding sequence does not identify amino acids of a mutation or variant (and in that instance is the same as the reference sequence). A variant-coding sequence can be determined by collecting a disease and/or tumor sample (e.g., that includes tumor cells) and performing a sequencing analysis to identify one or more sequences corresponding to disease and/or tumor cells in the sample. In some instances, a sequencing analysis outputs an amino-acid sequence. In some instances, a sequencing analysis outputs a nucleic-acid sequence, which may be subsequently processed to transform codons into amino-acid identifiers and thus to produce an amino-acid sequence. A variant-coding sequence can include a sequence of a neoantigen. A variant-coding sequence may, but need not, include one or more termini (e.g., the C-terminus and/or the N-terminus) of the peptide. A variant-coding sequence may include an epitope of the peptide. A variant-coding sequence can identify amino acids within a peptide having one or more variants (e.g., one or more amino-acid distinctions) relative to a corresponding reference sequence. In some instances, a variant-coding sequence includes an ordered set of amino acids. In some instances, a variant-coding sequence identifies a reference peptide (e.g., by identifying a genetic reference sequence, such as by gene, start position and/or end position; or by gene, start position and/or length) and one or more point mutations relative to the reference peptide.

As used herein, a "reference sequence" may refer to a sequence that identifies amino acids within at least part of a non-mutant peptide or wild-type peptide (e.g., wild-type, parental sequence). The non-mutant or wild-type peptide may include no variants or fewer variants than are included in a mutant peptide. The reference sequence may include an amino-acid sequence encoded by a genetic sequence within a same gene relative to a gene that includes a corresponding variant-coding sequence. The reference sequence may include an amino-acid sequence encoded by a genetic sequence spanning a same start and stop within a gene relative to intra-gene positions associated with a genetic sequence associated with a corresponding variant-coding sequence. The reference sequence may be identified by collecting a non-disease and/or non-tumor sample from one or more subjects (who may, but need not, include a subject from which a disease sample was collected to determine a variant-coding sequence) and performing a sequencing analysis using the sample.

As used herein, a "pseudosequence" of an MHC molecule may refer to an ordered set of amino acids of the MHC molecule that contacts a peptide.

As used herein, a "representation" of a sequence can include a set of values that represent or identify amino acids in the sequence and/or a set of values that represent or identify nucleic acids that encode the sequence. For example, each amino acid may be represented by a binary string and/or vector of values that is distinct from each other binary string and/or vector representing each other amino acid. The representation may be generated using, for example, one-hot encoding or using a BLOcks SUbstitution Matrix (BLOSUM) matrix. For example, a multi-dimensional (e.g., 20- or 21-dimensional) array be initialized (e.g., randomly or pseudorandomly initialized). The initialized array may include, for each amino acid, a unique vector corresponding to that amino acid. The values may be fixed such that use of such a unique vector can be assumed to represent the corresponding amino acid. There may be multiple possible nucleic-acid representations of a given sequence, given that any of multiple codons can encode a single amino acid.

As used herein, "presentation" of a peptide refers to at least part of the peptide being presented on a surface of a cell by virtue of being bound to an MHC molecule in a particular manner. The presented peptide can then be accessible to other cells, such as nearby T cells.

As used herein, a "sample" can include tissue (e.g., a biopsy), single cell, multiple cells, fragments of cells, or an aliquot of body fluid. The sample may be obtained from a subject by means such as, for example, without limitation, venipuncture, excretion, ejaculation, massage, biopsy, needle aspirate, lavage sample, scraping, surgical incision, intervention, another type of sample collection means, or a combination thereof.

As used herein, a "subject" encompasses one or more cells, tissue, or an organism. The subject may be a human or non-human, whether in vivo, ex vivo, or in vitro, male or female. A subject can be a mammal, such as a human.

As used herein, "binding affinity" refers to affinity of binding between a peptide (e.g., of a specific antigen) and an MHC (e.g., an MHC molecule and/or MHC allele). The binding affinity may characterize a stability, tendency, and/or strength of the binding between the peptide and MHC molecule.

As used herein, "immunogenicity" may refer to the ability to elicit an immune response (e.g., via T cells and/or B

cells). A peptide that is "immunogenic" may be one that is capable of eliciting an immune response.

As used herein, "MHC" refers to the major histocompatibility complex. The human MHC is also called the human leukocyte antigen (HLA) complex.

## VIII. Exemplary Embodiments

Embodiment 1. A method is provided. The method includes accessing a set of peptide sequences characterizing a set of peptides, each peptide sequence of the set of peptide sequences having been identified by processing a disease sample from a subject. The method includes accessing an immunoprotein complex (IPC) sequence identified for an immunoprotein complex (IPC) of the subject. The method includes processing a set of peptide representations that represents the set of peptide sequences using a first attention block in an initial attention subsystem of an attention-based machine-learning model and an immunoprotein complex (IPC) representation that represents the IPC sequence using a second attention block in the initial attention subsystem to generate an output, wherein the output includes at least one of an interaction prediction, an interaction affinity prediction, or an immunogenicity prediction for a corresponding peptide-IPC combination. The method includes generating a report based on the output.

Embodiment 2. The method of embodiment 1, includes wherein at least one peptide sequence of the set of peptide sequences comprises a variant-coding sequence that includes a variant with respect to a corresponding reference sequence.

Embodiment 3. The method of embodiment 1 or embodiment 2, includes wherein the processing comprises: receiving a peptide representation of the set of peptide representations for a corresponding peptide sequence of the set of peptide sequences; and transforming the peptide representation via the first attention block into a transformed peptide representation, wherein the first attention block includes a set of attention sub-blocks in which each attention sub-block of the set of attention sub-blocks includes a self-attention layer.

Embodiment 4. The method of any one of embodiments 1-3, includes wherein the processing comprises: receiving the IPC representation; and transforming the IPC representation via the second attention block into a transformed IPC representation, wherein the second attention block includes a set of attention sub-blocks in which each attention sub-block of the set of attention sub-blocks includes a self- attention layer.

Embodiment 5. The method of any one of embodiments 1-4, includes wherein at least a portion of the peptide representation corresponds to a monomer in the peptide sequence and at least a portion of the IPC representation corresponds to a monomer in the IPC sequence; and wherein the processing comprises: generating a transformed peptide representation based on the peptide representation using the first attention block and a first set of weights; generating a transformed IPC representation based on the IPC representation using the second attention block and a second set of weights; and generating a composite representation using the transformed peptide representation and the transformed MHC representation.

Embodiment 6. The method of any one of embodiments 1-5, further includes embedding a peptide sequence of the set of peptide sequences to generate an embedded peptide representation for the peptide sequence; and encoding, positionally, the embedded peptide representation for the peptide sequence to generate a peptide representation of the set of peptide representations that represents the peptide sequence.

Embodiment 7. The method of any one of embodiments 1-6, includes wherein: the first attention block comprises a set of attention sub-blocks; and each attention sub-block of the set of attention sub-blocks includes a neural network that comprises at least one self-attention layer.

Embodiment 8. The method of any one of embodiments 1-7, includes wherein: the second attention block comprises a set of attention sub-blocks; and each attention sub-block of the set of attention sub-blocks includes a neural network that comprises at least one self-attention layer.

Embodiment 9. The method of any one of embodiments 1-8, includes wherein: the first attention block comprises a first plurality of attention sub-blocks; the second attention block comprises a first plurality of attention sub-blocks; and each attention sub-block of the first set of attention sub-blocks and the second set of attention sub-blocks includes a neural network that comprises at least one self-attention layer.

Embodiment 10. The method of any one of embodiments 1-9, includes wherein: a peptide representation of the set of peptide representations forms a first portion of an aggregate representation processed using the first attention block; and a second portion of the aggregate representation represents at least one of an N-flank sequence or a C-flank sequence.

Embodiment 11. The method of any one of embodiments 1-10, includes wherein: a peptide sequence of the set of peptide sequences forms a first portion of an aggregate sequence; and a second portion of the aggregate sequence includes at least one of an N-flank sequence or a C-flank sequence; and the attention-based machine learning model includes a representation block that receives and processes the aggregate sequence to form an aggregate representation that includes a peptide representation of the set of peptide representations corresponding to the peptide sequence, wherein the aggregate representation is processed by the first attention block.

Embodiment 12. The method of any one of embodiments 1-11, further includes embedding the IPC sequence to generate an embedded IPC representation of the IPC sequence; and encoding, positionally, the embedded IPC representation of the IPC sequence to generate the IPC representation.

Embodiment 13. The method of any one of embodiments 1-12, includes wherein the attention-based machine-learning model includes a plurality of self-attention layers and for each of the plurality of self-attention layers, a corresponding downstream feedforward neural network.

Embodiment 14. The method of any one of embodiments 1-13, includes wherein: the first attention block includes a first neural network configured to receive and process a peptide representation of the set of peptide representations to generate a transformed peptide representation; and the second attention block includes a second neural network configured to receive and process the IPC representation to generate a transformed IPC representation; and wherein each of the first neural network and the second neural network includes at least one self-attention layer; and wherein the attention-based machine-learning model is configured to generate a composite representation using the transformed peptide representation and the transformed IPC representation.

Embodiment 15. The method of any one of embodiments 1-14, includes wherein the attention-based machine-learning model further includes: a composite attention block that includes a neural network configured to receive and process the composite representation, wherein the neural network includes a self-attention layer.

Embodiment 16. The method of any one of embodiments 1-15, includes wherein the attention-based machine-learning model further includes: a composite attention block that includes a set of attention sub-blocks, wherein each attention sub-block of the set of attention sub-blocks includes a neural network that comprises at least one self-attention layer. Embodiment 17. The method of any one of embodiments 1-16, includes wherein the IPC comprises a major histocompatibility complex (MHC) and the corresponding peptide-IPC combination includes a peptide of the set of peptides and the MHC, and wherein: the interaction affinity prediction for the corresponding peptide-IPC combination predicts a binding affinity between the peptide and the MHC; the interaction prediction for the corresponding peptide-IPC combination predicts whether the MHC will present the peptide at a cell surface.

Embodiment 18. The method of any one of embodiments 1-17, includes wherein the attention-based machine-learning model is trained using a training data set that includes at least one of experimental interaction affinity data or experimental interaction data for a plurality of training peptide sequences and a set of training MHC sequences.

Embodiment 19. The method of any one of embodiments 1-18, includes wherein the IPC is a T cell receptor (TCR) and the corresponding peptide-IPC pair includes a peptide of the set of peptides and either the TCR or the TCR and a major histocompatibility complex (MHC), and wherein: the immunogenicity prediction for a corresponding peptide-IPC combination predicts an immunogenicity of the peptide with respect to the TCR; and the attention-based machine-learning model is trained using a training data set that includes experimental immunogenicity data for a plurality of training peptide sequences and a set of training TCR sequences.

Embodiment 20. The method of any one of embodiments 1-19, includes wherein the training data set includes a plurality of training data elements, at least one training data element of the plurality of training data elements comprises at least one of: a training peptide sequence characterizing a training peptide not included in the set of peptides; a training IPC sequence characterizing a training IPC that is different from the IPC; and an experiment-based result identifying an interaction affinity indication between the training peptide and the training IPC, wherein the interaction affinity indication was detected using an assay or biosensor-based methodology.

Embodiment 21. The method of any one of embodiments 1-20, includes wherein the training data set includes a plurality of training data elements, at least one training data element of the plurality of training data elements comprises at least one of: a training peptide sequence characterizing a training peptide not included in the set of peptides; a training MHC sequence characterizing a training MHC that is different from the IPC; and an experiment-based result including an interaction indication that identifies whether the training peptide was presented by the training MHC at a cell surface, wherein at least one of immunoprecipitation or mass spectrometry was used to determine the interaction indication.

Embodiment 22. The method of any one of embodiments 1-21, further includes training the attention-based machine-learning model, prior to the processing step, using a training data set that includes at least one of binding affinities, interaction indications, or immunogenicity indications for a plurality of peptide-IPC combinations, wherein the training data set includes a plurality of training peptide sequences and at least one of a plurality of training major histocompatibility complex (MHC) sequences or a plurality of training T cell receptor (TCR) sequences.

Embodiment 23. The method of any one of embodiments 1-22, includes wherein the processing comprises: processing the set of peptide representations using the first attention block and the IPC representation using the second attention block to generate a set of composite representations for a set of peptide-IPC combinations; processing the set of composite representations to generate a set of results; selecting a subset of the set of peptide-IPC combinations, wherein a set of selected interactions is more likely to occur with each peptide-IPC combination of the subset as compared to a remaining subset of the set of peptide-IPC combinations, wherein the report identifies each peptide within the subset.

Embodiment 24. The method of any one of embodiments 1-23, includes wherein: each peptide of the set of peptides is used to form a set of peptide-IPC combinations; and the attention-based machine-learning model is configured

to generate the immunogenicity prediction for each peptide-IPC combination of the set of peptide-IPC combinations, the immunogenicity prediction for a peptide-IPC combination of the set of peptide-IPC combinations being a prediction of tumor-specific immunogenicity of a peptide in the peptide-IPC combination.

Embodiment 25. The method of any one of embodiments 1-24, includes wherein the report identifies a subset of peptides from the set of peptides having increased tumor-specific immunogenicity relative to a remaining portion of the set of peptides.

Embodiment 26. The method of any one of embodiments 1-25, includes wherein: the IPC is a major histocompatibility complex (MHC); each peptide of the set of peptides is used to form a set of peptide-MHC combinations; and the attention-based machine-learning model is configured to generate the interaction prediction for each peptide-MHC combination of the set of peptide-MHC combinations, the interaction prediction for a peptide-MHC combination of the set of peptide-MHC combinations being a prediction of whether a peptide in the peptide-MHC combination is presented by the MHC at a cell surface.

Embodiment 27. The method of embodiment 26, includes wherein the report identifies a subset of peptides from the set of peptides having an increased likelihood of presentation by the MHC relative to a remaining portion of the set of peptides.

Embodiment 28. The method of any one of embodiments 1-27, includes wherein: a peptide sequence of the set of peptide sequences is a variant-coding sequence characterizing a mutant peptide, the variant-coding sequence comprising: a first part identifying a sequence at an N-terminus of the mutant peptide; and a second part identifying a sequence of an epitope of the mutant peptide; and the processing comprises: processing a first representation of the first part of the variant-coding sequence using a first self-attention layer of the initial attention subsystem; and processing a second representation of the second part of the variant-coding sequence using a second self-attention layer of the initial attention subsystem.

Embodiment 29. The method of embodiment 28, includes wherein the first representation and the second representation are processed within the first attention block.

Embodiment 30. The method of any one of embodiments 1-29, includes wherein the attention-based machine-learning model includes one or more transformer encoders, wherein each of the one or more transformer encoders includes a self-attention layer.

Embodiment 31. The method of any one of embodiments 1-30, includes wherein the IPC sequence and each of the set of peptide sequences includes an ordered set of amino-acid identifiers.

Embodiment 32. The method of any one of embodiments 1-31, includes wherein the IPC sequence is identified using the disease sample.

Embodiment 33. The method of any one of embodiments 1-32, includes wherein the IPC sequence is identified using a biological sample from the subject.

Embodiment 34. The method of any one of embodiments 1-33, includes wherein the disease sample includes cancer cells.

Embodiment 35. The method of any one of embodiments 1-34, includes wherein: the IPC of the subject includes a major histocompatibility complex (MHC); the IPC sequence includes an MHC sequence; and the IPC representation includes an MHC representation.

Embodiment 36. The method of embodiment 35, includes wherein the MHC includes an MHC class-I molecule.

Embodiment 37. The method of embodiment 35, includes wherein the MHC includes an MHC class-II molecule.

Embodiment 38. The method of any one of embodiments 1-35, includes wherein: the IPC of the subject includes a T cell receptor (TCR); the IPC sequence includes a TCR sequence; and the IPC representation includes a TCR representation.

Embodiment 39. The method of any one of embodiments 1-38, includes wherein the disease sample includes tissue.

Embodiment 40. The method of any one of embodiments 1-39, includes wherein at least one peptide of the set of peptides is a neoantigen.

Embodiment 41. The method of any one of embodiments 1-40, includes wherein at least one peptide sequence of the set of peptide sequences is a genomic sequence derived from the disease sample.

Embodiment 42. The method of any one of embodiments 1-41, includes wherein each of at least one of the set of variant-coding sequences is based on RNA sequences of the disease sample.

Embodiment 43. The method of any one of embodiments 1-42, includes wherein: the corresponding peptide-IPC combination includes a peptide from the set of peptides and the IPC; the IPC is a major histocompatibility complex (MHC); the interaction affinity prediction is a prediction of a binding affinity for a binding between the peptide and the MHC; and the interaction prediction is a prediction of presentation of the peptide by the MHC at a cell surface.

Embodiment 44. The method of any one of embodiments 1-43, further includes receiving input data entered by a user, the input data corresponding to the subject; wherein the set of peptide sequences and the IPC sequence are accessed, in response to receiving the input data, via retrieval from a data store; and wherein the report identifies a subset

of peptides from the set of peptides to include in an individualized vaccine to treat a medical condition of the subj ect.

Embodiment 45. The method of embodiment 44, further includes generating a treatment recommendation to the subject that includes the individualized vaccine.

Embodiment 46. The method of any one of embodiments 1-45, further includes receiving input data entered by a user, the input data corresponding to the subject; wherein the set of peptide sequences and the IPC sequence are accessed, in response to receiving the input data, via retrieval from a data store; and determining a set of treatment peptides for inclusion in an individualized vaccine based on the report; and initiating an action that facilitates manufacture of the individualized vaccine that includes the set of treatment peptides.

Embodiment 47. The method of embodiment 46, includes wherein the initiating the action comprises: generating an alert that triggers a computerized process involved in the manufacture of the individualized vaccine.

Embodiment 48. The method of any one of embodiments 1-47, includes wherein the processing comprises: receiving, from an embedding block in the attention-based machine-learning model, a representation that comprises a plurality of elements, wherein the representation is either a peptide representation of the set of peptide representations that represents a peptide sequence in the set of peptide sequences or the IPC representation representing the IPC sequence; and wherein each element in the multi-element data set corresponds to a monomer in either the peptide sequence or the IPC sequence; determining, for each element of the plurality of elements, a key vector, a value vector, and a query vector based on a set of key weights, a set of value weights, and a set of query weights, respectively, associated with a self-attention layer of the attention-based machine learning model; performing a transformation of the plurality of elements to form a plurality of modified elements, wherein the transformation is performed using attention scores generated for the plurality of elements and the value vector determined for each of the plurality of elements; and generating the output based on the plurality of modified elements.

Embodiment 49. The method of embodiment 48, includes wherein performing the transformation for a selected element of the plurality of elements comprises determining an attention score of the selected element using the key vector and the query vector of the element, wherein a remaining portion of the plurality of elements other than the selected element forms a set of remaining elements; determining an additional attention score for each remaining element of the set of remaining elements using a key vector of the remaining element and the query vector of the selected element to form a set of additional attention scores; and generating a modified element using the attention score, the set of additional attention scores, and the value vector of each element of the plurality of elements.

Embodiment 50. The method of any one of embodiments 1-49, further includes displaying the report on a graphical user interface on a display system.

Embodiment 51. The method of any one of embodiments 1-50, includes wherein the processing is performed on a first computing platform and further includes sending the report to a second computing platform over a set of communications links that includes at least one of a wired communications link or a wireless communications link.

Embodiment 52. The method of any one of embodiments 1-51, further includes determining to include at least one peptide of the set of peptides as a target for an immunotherapy based on the report.

Embodiment 53. The method of embodiment 52, includes wherein the immunotherapy is selected from a group consisting of a T cell therapy, a personalized cancer therapy, an antigen-specific immunotherapy, an antigen-dependent immunotherapy, a vaccine, and a natural killer (NK) cell therapy.

Embodiment 54. The method of any one of embodiments 1-53, further includes determining to exclude at least one peptide of the set of peptides as a target for an immunotherapy based on the report.

Embodiment 55. The method of embodiment 54, includes wherein the immunotherapy is selected from a group consisting of a T cell therapy, a personalized cancer therapy, an antigen-specific immunotherapy, an antigen-dependent immunotherapy, a vaccine, and a natural killer (NK) cell therapy.

Embodiment 56. The method of any one of embodiments 1-55, includes wherein the IPC is a human leukocyte antigen (HLA) molecule.

Embodiment 57. The method of any one of any one of embodiments 1-56, further includes sequencing the disease sample from the subject; defining the set of peptide sequences based on the sequencing of the disease sample from the subject; identifying, based on the report, a subset of the set of peptide sequences; synthesizing mRNA that codes for at least one peptide included in the subset of the set of peptides; complexing the mRNA with lipids to produce a mRNA-lipoplex treatment; and administering the mRNA-lipoplex treatment to the subj ect.

Embodiment 58. A vaccine includes one or more peptides; a plurality of nucleic acids that encode the one or more peptides; or a plurality of cells expressing the one or more peptides, wherein the one or more peptides are selected from among the set of peptides based on the report generated by the method of any of embodiments 1-49, wherein the one or more peptides are an incomplete subset of the set of peptides.

Embodiment 59. The vaccine of embodiment 58, includes wherein the vaccine includes either DNA that includes the plurality of nucleic acids or RNA that includes the plurality of nucleic acids.

Embodiment 60. The vaccine of embodiment 58 or embodiment 59, includes wherein the vaccine includes mRNA that includes the plurality of nucleic acids.

Embodiment 61. The vaccine of any one of embodiments 58-60, includes wherein the vaccine is a tumor vaccine.
Embodiment 62. A method of manufacturing a vaccine includes producing a vaccine comprising: one or more peptides; a plurality of nucleic acids that encode the one or more peptides; or a plurality of cells expressing the one or more peptides, wherein the one or more peptides are selected from among the set of peptides based on the report generated by the method of any of embodiments 1-49, wherein the one or more peptides are an incomplete subset of the set of peptides.
Embodiment 63. The method of embodiment 62, includes wherein the vaccine includes DNA that includes the plurality of nucleic acids, RNA that includes the plurality of nucleic acids, or mRNA that includes the plurality of nucleic acids.
Embodiment 64. The method of embodiment 62 or embodiment 63, further includes identifying, based on amino acids within the one or more peptides, the plurality of nucleic acids that the encode the one or more peptides, wherein the vaccine includes the plurality of nucleic acids.
Embodiment 65. The method of any one of embodiments 62-64, includes wherein the vaccine is a tumor vaccine.
Embodiment 66. The method of embodiment 65, includes wherein, for each peptide of the one or more peptides, the tumor vaccine comprises at least one of: a nucleotide sequence encoding each peptide, an amino acid sequence corresponding to each peptide, RNA corresponding to each peptide, DNA corresponding to each peptide, a cell corresponding to each peptide, a plasmid corresponding to each peptide, or a vector corresponding to each peptide.
Embodiment 67. The method of any one of embodiments 62-66, includes wherein the vaccine further includes at least one of an excipient or an adjuvant.
Embodiment 68. The method of any one of embodiments 62-67, includes wherein the vaccine includes an RNA molecule including, in the 5'→3' direction:

a 5' cap;
a 5' untranslated region (UTR);
a polynucleotide sequence encoding a secretory signal peptide;
a polynucleotide sequence encoding the one or more peptides;
a polynucleotide sequence encoding at least a portion of a transmembrane and cytoplasmic domain of a major histocompatibility complex (MHC) molecule;
a 3' UTR including:

a 3' untranslated region of an Amino-Terminal Enhancer of Split (AES) mRNA or a fragment thereof; and
non-coding RNA of a mitochondrially encoded 12S RNA or a fragment thereof; and a poly(A) sequence.

Embodiment 69. A pharmaceutical composition includes one or more peptides selected from among the set of peptides based on the report generated by the method of any of embodiments 1-49, wherein the one or more peptides are an incomplete subset of the set of peptides.
Embodiment 70. A pharmaceutical composition includes a nucleic acid sequence that encodes one or more peptides having been selected from among the set of peptides based on the report generated by the method of any of embodiments 1-49, wherein the one or more peptides are an incomplete subset of the set of peptides.
Embodiment 71. An immunogenic peptide is identified based on the report generated by the method of any of embodiments 1-49.
Embodiment 72. A nucleic acid sequence is identified based on the report generated by the method of any of embodiments 1-49.
Embodiment 73. The nucleic acid sequence of embodiment 72, includes wherein the nucleic acid sequence includes a DNA sequence.
Embodiment 74. The nucleic acid sequence of embodiment 72 or embodiment 73, includes wherein the nucleic acid sequence includes an RNA sequence.
Embodiment 75. The nucleic acid sequence of any one of embodiments 72-74, includes wherein the nucleic acid sequence includes an mRNA sequence.
Embodiment 76. A method of treating a subject includes administering at least one of one or more peptides, one or more pharmaceutical compositions, or one or more nucleic acid sequences identified based on the report generated by the method of any of embodiments 1-49.
Embodiment 77. A method includes processing a set of biological samples obtained from a subject to generate a set of peptide sequences characterizing a set of peptides; processing the set of biological samples obtained from the subject to generate an immunoprotein complex (IPC) sequence identified for an immunoprotein complex (IPC) of the subject; generating a set of peptide representations that represents the set of peptide sequences using a first attention block in an initial attention subsystem of an attention-based machine-learning model; generating an immunoprotein complex (IPC) representation that represents the IPC sequence using a second attention block in the initial attention subsystem; processing the set of peptide representations and the IPC representation to generate an output, wherein the

output includes at least one of an interaction prediction, an interaction affinity prediction, or an immunogenicity prediction for a corresponding peptide-IPC combination, the corresponding peptide-IPC combination including a peptide of the set of peptides.

Embodiment 78. The method of embodiment 77, includes wherein processing a set of biological samples obtained from the subject to generate a set of peptide sequences includes processing a disease sample in the set of biological sampled obtained from the subject to generate the set of peptide sequences.

Embodiment 79. The method of embodiment 77 or embodiment 78, further includes obtaining the set of biological samples from the subject, wherein the set of biological samples includes a disease sample.

Embodiment 80. The method of any one of embodiments 77-79, further includes generating a report based on the output.

Embodiment 81. A method includes receiving, at a user device, a request to design an individualized vaccine for a subject; transmitting, from the user device, a communication to a remote system, the communication including an identifier of the subject, wherein the remote system is configured to: access a set of peptide sequences characterizing a set of peptides, each peptide sequence of the set of peptide sequences having been identified by processing a disease sample from a subject, and access an immunoprotein complex (IPC) sequence identified for an immunoprotein complex (IPC) of the subject; process a set of peptide representations that represents the set of peptide sequences using a first attention block in an initial attention subsystem of an attention-based machine-learning model and an immunoprotein complex (IPC) representation that represents the IPC sequence using a second attention block in the initial attention subsystem to generate an output, wherein the output includes at least one of an interaction prediction, an interaction affinity prediction, or an immunogenicity prediction for a corresponding peptide-IPC combination; and generate a report based on the output; and transmit the report to the user device; and receiving, at the user device, the report.

Embodiment 82. The method of embodiment 81, further includes collecting a disease sample from the subject; eluting multiple peptides that include the set of peptides from MHC molecules in the disease sample using at least one of chromatography or mass spectrometry; sequencing the set of peptides to generate a set of initial sequences; comparing each initial sequence of the set of initial sequences to a reference sequence; and defining the set of peptide sequences based on the comparisons, wherein each peptide sequence in the set of peptide sequences is a variant-coding sequence that includes a variant with respect to the reference sequence.

Embodiment 83. A method for manufacturing a treatment for a subject is provided. The method includes receiving a report from a computing device that is configured to: access a set of peptide sequences characterizing a set of peptides, each peptide sequence of the set of peptide sequences having been identified by processing a disease sample from a subject, and access an immunoprotein complex (IPC) sequence identified for an immunoprotein complex (IPC) of the subject; process a set of peptide representations that represents the set of peptide sequences using a first attention block in an initial attention subsystem of an attention-based machine-learning model and an immunoprotein complex (IPC) representation that represents the IPC sequence using a second attention block in the initial attention subsystem to generate an output, wherein the output includes at least one of an interaction prediction, an interaction affinity prediction, or an immunogenicity prediction for a corresponding peptide-IPC combination; and generate the report based on the output; and generating a treatment manufacturing plan for manufacturing the treatment based on the report.

Embodiment 84. The method of embodiment 83, further includes manufacturing the treatment based on the treatment manufacturing plan.

Embodiment 85. A method includes inputting a plurality of variant-coding sequences characterizing a plurality of mutant peptides into an attention-based machine-learning model, each variant-coding sequence of the plurality of variant-coding sequences having been identified by processing a disease sample from a subject; inputting an immunoprotein complex (IPC) sequence identified for an immunoprotein complex (IPC) of the subject into the attention-based machine-learning model, wherein the attention-based machine-learning model is configured to process a plurality of variant representations that represents the plurality of variant-coding sequences using a first attention block in an initial attention subsystem of an attention-based machine-learning model and an immunoprotein complex (IPC) representation that represents the IPC sequence using a second attention block in the initial attention subsystem to generate an output, wherein the output includes at least one of an interaction prediction, an interaction affinity prediction, or an immunogenicity prediction for a corresponding mutant peptide-IPC combination; and receiving a report generated based on the output; and selecting, based on the report, a subset of the plurality of mutant peptides to use in a treatment for the subject.

Embodiment 86. A method includes receiving a peptide sequence that characterizes a mutant peptide, the peptide sequence including a variant with respect to a corresponding reference sequence; receiving an MHC sequence identified for a major histocompatibility complex (MHC); processing the peptide sequence and the MHC sequence using different processing paths within an attention-based machine-learning model to generate an output, wherein the output provides information about an immunological activity relating to both the mutant peptide and the MHC; generating a report based on the output.

Embodiment 87. The method of embodiment 86, includes wherein the processing includes processing the peptide sequence via a peptide processing path within the attention-based machine-learning model, the peptide

processing path including a first embedding block and a first attention block that includes at least one self-attention layer; and

processing the MHC sequence via an MHC processing path within the attention-based machine-learning model, the MHC processing path including a second embedding block and a second attention block that includes at least one self-attention layer.

Embodiment 88. The method of embodiment 87, further includes receiving a TCR sequence identified for a T cell receptor (TCR); and wherein the processing further includes processing the TCR sequence via a TCR processing path within the attention-based machine-learning model, the TCR processing path including a third embedding block and a third attention block that includes at least one self-attention layer.

Embodiment 89. The method of any one of embodiments 86-88, includes wherein the immunological activity includes an immune response and the information includes a prediction about an ability of the mutant peptide to provoke the immune response.

Embodiment 90. The method of any one of embodiments 86-89, includes wherein the processing includes generating a transformed peptide representation of the peptide sequence via the peptide processing path; generating a transformed MHC representation of the MHC sequence via the MHC processing path; \generating a composite representation using the transformed peptide representation and the transformed MHC representation; processing the composite representation to generate the output.

Embodiment 91. The method of any one of embodiments 86-90, includes wherein the immunological activity includes a binding of the mutant peptide to the MHC and wherein the output includes at least one of a first prediction corresponding to whether the mutant peptide binds to the MHC or a second prediction corresponding to an affinity associated with the binding.

Embodiment 92. The method of any one of embodiments 86-91, further includes determining to include the mutant peptide as a target for an immunotherapy based on the report.

Embodiment 93. The method of embodiment 92, includes wherein the immunotherapy is selected from a group consisting of a T cell therapy, a personalized cancer therapy, an antigen-specific immunotherapy, an antigen-dependent immunotherapy, a vaccine, and a natural killer (NK) cell therapy.

Embodiment 94. The method of any one of embodiments 86-93, further includes at least one of: determining to exclude the mutant peptide as a target for an immunotherapy based on the report.

Embodiment 95. The method of embodiment 94, includes wherein the immunotherapy is selected from a group consisting of a T cell therapy, a personalized cancer therapy, an antigen-specific immunotherapy, an antigen-dependent immunotherapy, a vaccine, and a natural killer (NK) cell therapy.

Embodiment 96. The method of any one of embodiments 86-95, further includes determining, based on the report, to include at least one of the mutant peptide, a precursor of the mutant peptide, nucleic acids that encode the mutant peptide, or a plurality of cells that express the mutant peptide in a treatment; and manufacturing the treatment.

Embodiment 97. The method of embodiment 96, further includes treating a subject with the treatment.

Embodiment 98. The method of any one of embodiments 86-97, includes wherein the peptide sequence characterizing the mutant peptide was identified by sequencing a disease sample from a subject, wherein the peptide sequence has at least one sequence variation relative to a corresponding reference sequence, and wherein a treatment is designed for the subject based on the report.

Embodiment 99. A method includes receiving a peptide sequence that characterizes a mutant peptide, the peptide sequence including a variant with respect to a corresponding reference sequence; receiving a TCR sequence identified for a T cell receptor (TCR); processing the peptide sequence and the TCR sequence using different processing paths within an attention-based machine-learning model to generate an output, wherein the output provides information about an immunological activity relating to both the mutant peptide and the TCR; generating a report based on the output.

Embodiment 100. The method of embodiment 99, includes wherein the processing includes processing the peptide sequence via a peptide processing path within the attention-based machine-learning model, the peptide processing path including a first embedding block and a first attention block; and processing the TCR sequence via a TCR processing path within the attention-based machine-learning model, the TCR processing path including a second embedding block and a second attention block.

Embodiment 101. The method of embodiment 100, further includes receiving an MHC sequence identified for a major histocompatibility complex (MHC); and wherein the processing further includes processing the MHC sequence via an MHC processing path within the attention-based machine-learning model, the MHC processing path including a third embedding block and an MHC third block.

Embodiment 102. The method of any one of embodiments 99-101, includes wherein the immunological activity includes an immune response and the information includes a prediction about an ability of the mutant peptide to provoke the immune response.

Embodiment 103. The method of any one of embodiments 99-102, includes wherein the processing includes generating a transformed peptide representation of the peptide sequence via the peptide processing path; generating a

transformed TCR representation of the TCR sequence via the TCR processing path; generating a composite representation using the transformed peptide representation and the transformed TCR representation; processing the composite representation to generate the output.

Embodiment 104. The method of any one of embodiments 99-103, includes wherein the immunological activity includes a binding of the mutant peptide to the MHC and wherein the output includes at least one of a first prediction corresponding to whether the mutant peptide binds to the MHC or a second prediction corresponding to an affinity associated with the binding.

Embodiment 105. The method of embodiment any one of embodiments 99-104, further includes determining to include the mutant peptide as a target for an immunotherapy based on the report.

Embodiment 106. The method of embodiment 105, includes wherein the immunotherapy is selected from a group consisting of a T cell therapy, a personalized cancer therapy, an antigen-specific immunotherapy, an antigen-dependent immunotherapy, a vaccine, and a natural killer (NK) cell therapy.

Embodiment 107. The method of any one of embodiments 99-106, further includes at least one of: determining to exclude the mutant peptide as a target for an immunotherapy based on the report.

Embodiment 108. The method of embodiment 107, includes wherein the immunotherapy is selected from a group consisting of a T cell therapy, a personalized cancer therapy, an antigen-specific immunotherapy, an antigen-dependent immunotherapy, a vaccine, and a natural killer (NK) cell therapy.

Embodiment 109. The method of any one of embodiments 99-108, further includes determining, based on the report, to include at least one of the mutant peptide, a precursor of the mutant peptide, nucleic acids that encode the mutant peptide, or a plurality of cells that express the mutant peptide in a treatment; and manufacturing the treatment.

Embodiment 110. The method of embodiment 109, further includes treating a subject with the treatment.

Embodiment 111. The method of any one of embodiments 99-110, includes wherein the peptide sequence characterizing the mutant peptide was identified by sequencing a disease sample from a subject, wherein the peptide sequence has at least one sequence variation relative to a corresponding reference sequence, and wherein a treatment is designed for the subject based on the report.

Embodiment 112. A system comprising: one or more data processors; and a non-transitory computer readable storage medium containing instructions is provided which, when executed on the one or more data processors, cause the one or more data processors to perform any one of embodiments 1-49, 77-81, 83, 85-95, and 99-108.

Embodiment 113. A computer-program product tangibly embodied in a non-transitory machine-readable storage medium, including instructions configured to cause one or more data processors is provided to perform any one of embodiments 1-49, 77-81, 83, 85-95, and 99-108.

## IX. Additional Considerations

Some embodiments of the present disclosure include a system including one or more data processors. In some embodiments, the system includes a non-transitory computer readable storage medium containing instructions which, when executed on the one or more data processors, cause the one or more data processors to perform part or all of one or more methods and/or part or all of one or more processes disclosed herein. Some embodiments of the present disclosure include a computer-program product tangibly embodied in a non-transitory machine-readable storage medium, including instructions configured to cause one or more data processors to perform part or all of one or more methods and/or part or all of one or more processes disclosed herein.

The terms and expressions which have been employed are used as terms of description and not of limitation, and there is no intention in the use of such terms and expressions of excluding any equivalents of the features shown and described or portions thereof, but it is recognized that various modifications are possible within the scope of the invention claimed. Thus, it should be understood that although the present invention as claimed has been specifically disclosed by embodiments and optional features, modification and variation of the concepts herein disclosed may be resorted to by those skilled in the art, and that such modifications and variations are considered to be within the scope of this invention as defined by the appended claims.

The description provides preferred exemplary embodiments only, and is not intended to limit the scope, applicability or configuration of the disclosure. Rather, the description of the preferred exemplary embodiments will provide those skilled in the art with an enabling description for implementing various embodiments. It is understood that various changes may be made in the function and arrangement of elements without departing from the spirit and scope as set forth in the appended claims.

Specific details are given in the following description to provide a thorough understanding of the embodiments. However, it will be understood that the embodiments may be practiced without these specific details. For example, circuits, systems, networks, processes, and other components may be shown as components in block diagram form in order not to obscure the embodiments in unnecessary detail. In other instances, well-known circuits, processes, algorithms, structures, and techniques may be shown without unnecessary detail in order to avoid obscuring the embodiments.

The following items are disclosed herein:

1. A method comprising:

accessing a set of peptide sequences characterizing a set of peptides, each peptide sequence of the set of peptide sequences having been identified by processing a disease sample from a subject;

accessing an immunoprotein complex (IPC) sequence identified for an immunoprotein complex (IPC) of the subject;

processing a set of peptide representations that represents the set of peptide sequences using a first attention block in an initial attention subsystem of an attention-based machine-learning model and an immunoprotein complex (IPC) representation that represents the IPC sequence using a second attention block in the initial attention subsystem to generate an output, wherein the output includes at least one of an interaction prediction, an interaction affinity prediction, or an immunogenicity prediction for a corresponding peptide-IPC combination; and

generating a report based on the output.

2. The method of item 1, wherein at least one peptide sequence of the set of peptide sequences comprises a variant-coding sequence that includes a variant with respect to a corresponding reference sequence.

3. The method of item 1, wherein the processing comprises:

receiving a peptide representation of the set of peptide representations for a corresponding peptide sequence of the set of peptide sequences; and

transforming the peptide representation via the first attention block into a transformed peptide representation, wherein the first attention block includes a set of attention sub-blocks in which each attention sub-block of the set of attention sub-blocks includes a self-attention layer.

4. The method of item 1, wherein the processing comprises:

receiving the IPC representation; and

transforming the IPC representation via the second attention block into a transformed IPC representation, wherein the second attention block includes a set of attention sub-blocks in which each attention sub-block of the set of attention sub-blocks includes a self- attention layer.

5. The method of item 1, wherein at least a portion of the peptide representation corresponds to a monomer in the peptide sequence and at least a portion of the IPC representation corresponds to a monomer in the IPC sequence; and wherein the processing comprises:

generating a transformed peptide representation based on the peptide representation using the first attention block and a first set of weights;

generating a transformed IPC representation based on the IPC representation using the second attention block and a second set of weights; and

generating a composite representation using the transformed peptide representation and the transformed MHC representation.

6. The method of item 1, further comprising:

embedding a peptide sequence of the set of peptide sequences to generate an embedded peptide representation for the peptide sequence; and

encoding, positionally, the embedded peptide representation for the peptide sequence to generate a peptide representation of the set of peptide representations that represents the peptide sequence.

7. The method of item 1, wherein:

the first attention block comprises a set of attention sub-blocks; and

each attention sub-block of the set of attention sub-blocks includes a neural network that comprises at least one self-attention layer.

8. The method of item 1, wherein:

the second attention block comprises a set of attention sub-blocks; and
each attention sub-block of the set of attention sub-blocks includes a neural network that comprises at least one self-attention layer.

9. The method of item 1, wherein:

the first attention block comprises a first plurality of attention sub-blocks;
the second attention block comprises a first plurality of attention sub-blocks; and
each attention sub-block of the first set of attention sub-blocks and the second set of attention sub-blocks includes a neural network that comprises at least one self-attention layer.

10. The method of item 1, wherein:

a peptide representation of the set of peptide representations forms a first portion of an aggregate representation processed using the first attention block; and
a second portion of the aggregate representation represents at least one of an N-flank sequence or a C-flank sequence.

11. The method of item 1, wherein:

a peptide sequence of the set of peptide sequences forms a first portion of an aggregate sequence; and
a second portion of the aggregate sequence includes at least one of an N-flank sequence or a C-flank sequence; and
the attention-based machine learning model includes a representation block that receives and processes the aggregate sequence to form an aggregate representation that includes a peptide representation of the set of peptide representations corresponding to the peptide sequence, wherein the aggregate representation is processed by the first attention block.

12. The method of item 1, further comprising:

embedding the IPC sequence to generate an embedded IPC representation of the IPC sequence; and
encoding, positionally, the embedded IPC representation of the IPC sequence to generate the IPC representation.

13. The method of item 1, wherein the attention-based machine-learning model includes a plurality of self-attention layers and for each of the plurality of self-attention layers, a corresponding downstream feedforward neural network.

14. The method of item 1, wherein:

the first attention block includes a first neural network configured to receive and process a peptide representation of the set of peptide representations to generate a transformed peptide representation; and
the second attention block includes a second neural network configured to receive and process the IPC representation to generate a transformed IPC representation; and
wherein each of the first neural network and the second neural network includes at least one self-attention layer; and
wherein the attention-based machine-learning model is configured to generate a composite representation using the transformed peptide representation and the transformed IPC representation.

15. The method of item 1, wherein the attention-based machine-learning model further includes:
a composite attention block that includes a neural network configured to receive and process the composite representation, wherein the neural network includes a self-attention layer.

16. The method of item 1, wherein the attention-based machine-learning model further includes:
a composite attention block that includes a set of attention sub-blocks, wherein each attention sub-block of the set of attention sub-blocks includes a neural network that comprises at least one self-attention layer.

17. The method of item 1, wherein the IPC comprises a major histocompatibility complex (MHC) and the corresponding peptide-IPC combination includes a peptide of the set of peptides and the MHC, and wherein:

the interaction affinity prediction for the corresponding peptide-IPC combination predicts a binding affinity between the peptide and the MHC; and
the interaction prediction for the corresponding peptide-IPC combination predicts whether the MHC will present the peptide at a cell surface.

18. The method of item 1, wherein the attention-based machine-learning model is trained using a training data set that includes at least one of experimental interaction affinity data or experimental interaction data for a plurality of training peptide sequences and a set of training MHC sequences.

19. The method of item 1, wherein the IPC is a T cell receptor (TCR) and the corresponding peptide-IPC pair includes a peptide of the set of peptides and either the TCR or the TCR and a major histocompatibility complex (MHC), and wherein:

the immunogenicity prediction for a corresponding peptide-IPC combination predicts an immunogenicity of the peptide with respect to the TCR; and
the attention-based machine-learning model is trained using a training data set that includes experimental immunogenicity data for a plurality of training peptide sequences and a set of training TCR sequences.

20. The method of item 1, wherein the training data set includes a plurality of training data elements, at least one training data element of the plurality of training data elements comprises at least one of:

a training peptide sequence characterizing a training peptide not included in the set of peptides;
a training IPC sequence characterizing a training IPC that is different from the IPC; and
an experiment-based result identifying an interaction affinity indication between the training peptide and the training IPC, wherein the interaction affinity indication was detected using an assay or biosensor-based methodology.

21. The method of item 1, wherein the training data set includes a plurality of training data elements, at least one training data element of the plurality of training data elements comprises at least one of:

a training peptide sequence characterizing a training peptide not included in the set of peptides;
a training MHC sequence characterizing a training MHC that is different from the IPC; and
an experiment-based result including an interaction indication that identifies whether the training peptide was presented by the training MHC at a cell surface, wherein at least one of immunoprecipitation or mass spectrometry was used to determine the interaction indication.

22. The method of item 1, further comprising:

training the attention-based machine-learning model, prior to the processing step, using a training data set that includes at least one of binding affinities, interaction indications, or immunogenicity indications for a plurality of peptide-IPC combinations,
wherein the training data set includes a plurality of training peptide sequences and at least one of a plurality of training major histocompatibility complex (MHC) sequences or a plurality of training T cell receptor (TCR) sequences.

23. The method of item 1, wherein the processing comprises:

processing the set of peptide representations using the first attention block and the IPC representation using the second attention block to generate a set of composite representations for a set of peptide-IPC combinations;
processing the set of composite representations to generate a set of results;
selecting a subset of the set of peptide-IPC combinations, wherein a set of selected interactions is more likely to occur with each peptide-IPC combination of the subset as compared to a remaining subset of the set of peptide-IPC combinations,
wherein the report identifies each peptide within the subset.

24. The method of item 1, wherein:

each peptide of the set of peptides is used to form a set of peptide-IPC combinations; and
the attention-based machine-learning model is configured to generate the immunogenicity prediction for each peptide-IPC combination of the set of peptide-IPC combinations, the immunogenicity prediction for a peptide-IPC combination of the set of peptide-IPC combinations being a prediction of tumor-specific immunogenicity of a peptide in the peptide-IPC combination.

25. The method of item 24, wherein the report identifies a subset of peptides from the set of peptides having increased tumor-specific immunogenicity relative to a remaining portion of the set of peptides.

26. The method of item 1, wherein:

the IPC is a major histocompatibility complex (MHC);
each peptide of the set of peptides is used to form a set of peptide-MHC combinations; and
the attention-based machine-learning model is configured to generate the interaction prediction for each peptide-MHC combination of the set of peptide-MHC combinations, the interaction prediction for a peptide-MHC combination of the set of peptide-MHC combinations being a prediction of whether a peptide in the peptide-MHC combination is presented by the MHC at a cell surface.

27. The method of item 26, wherein the report identifies a subset of peptides from the set of peptides having an increased likelihood of presentation by the MHC relative to a remaining portion of the set of peptides.

28. The method of item 1, wherein:

a peptide sequence of the set of peptide sequences is a variant-coding sequence characterizing a mutant peptide, the variant-coding sequence comprising:

a first part identifying a sequence at an N-terminus of the mutant peptide; and
a second part identifying a sequence of an epitope of the mutant peptide; and

the processing comprises:

processing a first representation of the first part of the variant-coding sequence using a first self-attention layer of the initial attention subsystem; and
processing a second representation of the second part of the variant-coding sequence using a second self-attention layer of the initial attention subsystem.

29. The method of item 28, wherein the first representation and the second representation are processed within the first attention block.

30. The method of item 1, wherein the attention-based machine-learning model includes one or more transformer encoders, wherein each of the one or more transformer encoders includes a self-attention layer.

31. The method of item 1, wherein the IPC sequence and each of the set of peptide sequences includes an ordered set of amino-acid identifiers.

32. The method of item 1, wherein the IPC sequence is identified using the disease sample.

33. The method of item 1, wherein the IPC sequence is identified using a biological sample from the subject.

34. The method of item 1, wherein the disease sample includes cancer cells.

35. The method of item 1, wherein:

the IPC of the subject includes a major histocompatibility complex (MHC);
the IPC sequence includes an MHC sequence; and the IPC representation includes an MHC representation.

36. The method of item 35, wherein the MHC includes an MHC class-I molecule.

37. The method of item 35, wherein the MHC includes an MHC class-II molecule.

38. The method of item 1, wherein:

the IPC of the subject includes a T cell receptor (TCR);
the IPC sequence includes a TCR sequence; and
the IPC representation includes a TCR representation.

39. The method of item 1, wherein the disease sample includes tissue.

40. The method of item 1, wherein at least one peptide of the set of peptides is a neoantigen.

41. The method of item 1, wherein at least one peptide sequence of the set of peptide sequences is a genomic sequence derived from the disease sample.

42. The method of item 1, wherein each of at least one of the set of variant-coding sequences is based on RNA sequences of the disease sample.

43. The method of item 1, wherein:

the corresponding peptide-IPC combination includes a peptide from the set of peptides and the IPC;
the IPC is a major histocompatibility complex (MHC);
the interaction affinity prediction is a prediction of a binding affinity for a binding between the peptide and the MHC; and
the interaction prediction is a prediction of presentation of the peptide by the MHC at a cell surface.

44. The method of item 1, further comprising:

receiving input data entered by a user, the input data corresponding to the subject;
wherein the set of peptide sequences and the IPC sequence are accessed, in response to receiving the input data, via retrieval from a data store; and
wherein the report identifies a subset of peptides from the set of peptides to include in an individualized vaccine to treat a medical condition of the subject.

45. The method of item 44, further comprising:

generating a treatment recommendation to the subject that includes the individualized vaccine.

46. The method of item 1, further comprising:

receiving input data entered by a user, the input data corresponding to the subject;
wherein the set of peptide sequences and the IPC sequence are accessed, in response to receiving the input data, via retrieval from a data store; and
determining a set of treatment peptides for inclusion in an individualized vaccine based on the report; and
initiating an action that facilitates manufacture of the individualized vaccine that includes the set of treatment peptides.

47. The method of item 46, wherein the initiating the action comprises:
generating an alert that triggers a computerized process involved in the manufacture of the individualized vaccine.

48. The method of item 1, wherein the processing comprises:

receiving, from an embedding block in the attention-based machine-learning model, a representation that comprises a plurality of elements,

wherein the representation is either a peptide representation of the set of peptide representations that

represents a peptide sequence in the set of peptide sequences or the IPC representation representing the IPC sequence; and

wherein each element in the multi-element data set corresponds to a monomer in either the peptide sequence or the IPC sequence;

determining, for each element of the plurality of elements, a key vector, a value vector, and a query vector based on a set of key weights, a set of value weights, and a set of query weights, respectively, associated with a self-attention layer of the attention-based machine learning model;

performing a transformation of the plurality of elements to form a plurality of modified elements, wherein the transformation is performed using attention scores generated for the plurality of elements and the value vector determined for each of the plurality of elements; and

generating the output based on the plurality of modified elements.

49. The method of item 48, wherein performing the transformation for a selected element of the plurality of elements comprises:

determining an attention score of the selected element using the key vector and the query vector of the element, wherein a remaining portion of the plurality of elements other than the selected element forms a set of remaining elements;

determining an additional attention score for each remaining element of the set of remaining elements using a key vector of the remaining element and the query vector of the selected element to form a set of additional attention scores; and

generating a modified element using the attention score, the set of additional attention scores, and the value vector of each element of the plurality of elements.

50. The method of item 1, further comprising:
displaying the report on a graphical user interface on a display system.

51. The method of item 1, wherein the processing is performed on a first computing platform and further comprising: sending the report to a second computing platform over a set of communications links that includes at least one of a wired communications link or a wireless communications link.

52. The method of item 1, further comprising:
determining to include at least one peptide of the set of peptides as a target for an immunotherapy based on the report.

53. The method of item 52, wherein the immunotherapy is selected from a group consisting of a T cell therapy, a personalized cancer therapy, an antigen-specific immunotherapy, an antigen-dependent immunotherapy, a vaccine, and a natural killer (NK) cell therapy.

54. The method of item 1, further comprising:
determining to exclude at least one peptide of the set of peptides as a target for an immunotherapy based on the report.

55. The method of item 54, wherein the immunotherapy is selected from a group consisting of a T cell therapy, a personalized cancer therapy, an antigen-specific immunotherapy, an antigen-dependent immunotherapy, a vaccine, and a natural killer (NK) cell therapy.

56. The method of item 1, wherein the IPC is a human leukocyte antigen (HLA) molecule.

57. The method of any one of item 1, further comprising:

sequencing the disease sample from the subject;

defining the set of peptide sequences based on the sequencing of the disease sample from the subject;

identifying, based on the report, a subset of the set of peptide sequences;

synthesizing mRNA that codes for at least one peptide included in the subset of the set of peptides;

complexing the mRNA with lipids to produce a mRNA-lipoplex treatment; and

administering the mRNA-lipoplex treatment to the subject.

58. A vaccine comprising:

one or more peptides;
a plurality of nucleic acids that encode the one or more peptides; or
a plurality of cells expressing the one or more peptides,
wherein the one or more peptides are selected from among the set of peptides based on a report generated by a method comprising:

accessing a set of peptide sequences characterizing a set of peptides, each peptide sequence of the set of peptide sequences having been identified by processing a disease sample from a subject;
accessing an immunoprotein complex (IPC) sequence identified for an immunoprotein complex (IPC) of the subject;
processing a set of peptide representations that represents the set of peptide sequences using a first attention block in an initial attention subsystem of an attention-based machine-learning model and an immunoprotein complex (IPC) representation that represents the IPC sequence using a second attention block in the initial attention subsystem to generate an output, wherein the output includes at least one of an interaction prediction, an interaction affinity prediction, or an immunogenicity prediction for a corresponding peptide-IPC combination; and
generating the report based on the output; and

wherein the one or more peptides are an incomplete subset of the set of peptides.

59. The vaccine of item 58, wherein the vaccine includes either DNA that includes the plurality of nucleic acids or RNA that includes the plurality of nucleic acids.

60. The vaccine of item 58, wherein the vaccine includes mRNA that includes the plurality of nucleic acids.

61. The vaccine of item 58, wherein the vaccine is a tumor vaccine.

62. A method of manufacturing a vaccine comprising:

producing a vaccine comprising:

one or more peptides;
a plurality of nucleic acids that encode the one or more peptides; or
a plurality of cells expressing the one or more peptides,
wherein the one or more peptides are selected from among the set of peptides based on a report generated by a method comprising:

accessing a set of peptide sequences characterizing a set of peptides, each peptide sequence of the set of peptide sequences having been identified by processing a disease sample from a subject;
accessing an immunoprotein complex (IPC) sequence identified for an immunoprotein complex (IPC) of the subject;
processing a set of peptide representations that represents the set of peptide sequences using a first attention block in an initial attention subsystem of an attention-based machine-learning model and an immunoprotein complex (IPC) representation that represents the IPC sequence using a second attention block in the initial attention subsystem to generate an output, wherein the output includes at least one of an interaction prediction, an interaction affinity prediction, or an immunogenicity prediction for a corresponding peptide-IPC combination; and
generating the report based on the output; and

wherein the one or more peptides are an incomplete subset of the set of peptides.

63. The method of item 62, wherein the vaccine includes DNA that includes the plurality of nucleic acids, RNA that includes the plurality of nucleic acids, or mRNA that includes the plurality of nucleic acids.

64. The method of item 62, further comprising:
identifying, based on amino acids within the one or more peptides, the plurality of nucleic acids that the encode the one or more peptides, wherein the vaccine includes the plurality of nucleic acids.

65. The method of item 62, wherein the vaccine is a tumor vaccine.

66. The method of item 65, wherein, for each peptide of the one or more peptides, the tumor vaccine comprises at least one of: a nucleotide sequence encoding each peptide, an amino acid sequence corresponding to each peptide, RNA corresponding to each peptide, DNA corresponding to each peptide, a cell corresponding to each peptide, a plasmid corresponding to each peptide, or a vector corresponding to each peptide.

67. The method of item 62, wherein the vaccine further includes at least one of an excipient or an adjuvant.

68. The method of item 62, wherein the vaccine includes an RNA molecule including, in the 5'→3' direction:

a 5' cap;
a 5' untranslated region (UTR);
a polynucleotide sequence encoding a secretory signal peptide;
a polynucleotide sequence encoding the one or more peptides;
a polynucleotide sequence encoding at least a portion of a transmembrane and cytoplasmic domain of a major histocompatibility complex (MHC) molecule;
a 3' UTR including:

a 3' untranslated region of an Amino-Terminal Enhancer of Split (AES) mRNA or a fragment thereof; and
non-coding RNA of a mitochondrially encoded 12S RNA or a fragment thereof; and

a poly(A) sequence.

69. A pharmaceutical composition comprising one or more peptides selected from among the set of peptides based on a report generated by a method comprising:

accessing a set of peptide sequences characterizing a set of peptides, each peptide sequence of the set of peptide sequences having been identified by processing a disease sample from a subject;
accessing an immunoprotein complex (IPC) sequence identified for an immunoprotein complex (IPC) of the subject;
processing a set of peptide representations that represents the set of peptide sequences using a first attention block in an initial attention subsystem of an attention-based machine-learning model and an immunoprotein complex (IPC) representation that represents the IPC sequence using a second attention block in the initial attention subsystem to generate an output, wherein the output includes at least one of an interaction prediction, an interaction affinity prediction, or an immunogenicity prediction for a corresponding peptide-IPC combination; and
generating the report based on the output; and,
wherein the one or more peptides are an incomplete subset of the set of peptides.

70. A pharmaceutical composition comprising a nucleic acid sequence corresponding to one or more peptides having been selected from among the set of peptides based on a report generated by a method comprising:

accessing a set of peptide sequences characterizing a set of peptides, each peptide sequence of the set of peptide sequences having been identified by processing a disease sample from a subject;
accessing an immunoprotein complex (IPC) sequence identified for an immunoprotein complex (IPC) of the subject;
processing a set of peptide representations that represents the set of peptide sequences using a first attention block in an initial attention subsystem of an attention-based machine-learning model and an immunoprotein complex (IPC) representation that represents the IPC sequence using a second attention block in the initial attention subsystem to generate an output, wherein the output includes at least one of an interaction prediction, an interaction affinity prediction, or an immunogenicity prediction for a corresponding peptide-IPC combination; and
generating the report based on the output; and,
wherein the one or more peptides are an incomplete subset of the set of peptides.

71. The pharmaceutical composition of item 70, wherein the one or more peptides includes a mutant peptide.

72. The pharmaceutical composition of item 70, wherein the nucleic acid sequence includes a DNA sequence.

73. The pharmaceutical composition of item 70, wherein the nucleic acid sequence includes an RNA sequence.

74. The pharmaceutical composition of item 70, wherein the nucleic acid sequence includes an mRNA sequence.

75. An immunogenic peptide identified based on a report generated by a method comprising:

accessing a set of peptide sequences characterizing a set of peptides, each peptide sequence of the set of peptide sequences having been identified by processing a disease sample from a subject;
accessing an immunoprotein complex (IPC) sequence identified for an immunoprotein complex (IPC) of the subject;
processing a set of peptide representations that represents the set of peptide sequences using a first attention block in an initial attention subsystem of an attention-based machine-learning model and an immunoprotein complex (IPC) representation that represents the IPC sequence using a second attention block in the initial attention subsystem to generate an output, wherein the output includes at least one of an interaction prediction, an interaction affinity prediction, or an immunogenicity prediction for a corresponding peptide-IPC combination; and
generating the report based on the output.

76. A method of treating a subject comprising administering at least one of one or more peptides, one or more pharmaceutical compositions, or one or more nucleic acid sequences identified based on a report generated by a method comprising:

accessing a set of peptide sequences characterizing a set of peptides, each peptide sequence of the set of peptide sequences having been identified by processing a disease sample from a subject;
accessing an immunoprotein complex (IPC) sequence identified for an immunoprotein complex (IPC) of the subject;
processing a set of peptide representations that represents the set of peptide sequences using a first attention block in an initial attention subsystem of an attention-based machine-learning model and an immunoprotein complex (IPC) representation that represents the IPC sequence using a second attention block in the initial attention subsystem to generate an output, wherein the output includes at least one of an interaction prediction, an interaction affinity prediction, or an immunogenicity prediction for a corresponding peptide-IPC combination; and
generating the report based on the output.

77. A method comprising:

processing a set of biological samples obtained from a subject to generate a set of peptide sequences characterizing a set of peptides;
processing the set of biological samples obtained from the subject to generate an immunoprotein complex (IPC) sequence identified for an immunoprotein complex (IPC) of the subject;
generating a set of peptide representations that represents the set of peptide sequences using a first attention block in an initial attention subsystem of an attention-based machine-learning model;
generating an immunoprotein complex (IPC) representation that represents the IPC sequence using a second attention block in the initial attention subsystem;
processing the set of peptide representations and the IPC representation to generate an output, wherein the output includes at least one of an interaction prediction, an interaction affinity prediction, or an immunogenicity prediction for a corresponding peptide-IPC combination, the corresponding peptide-IPC combination including a peptide of the set of peptides.

78. The method of item 77, wherein processing a set of biological samples obtained from the subject to generate a set of peptide sequences comprises:
processing a disease sample in the set of biological sampled obtained from the subject to generate the set of peptide sequences.

79. The method of item 77, further comprising:
obtaining the set of biological samples from the subject, wherein the set of biological samples includes a disease

sample.

80. The method of item 77, further comprising:
generating a report based on the output.

81. A method comprising:

receiving, at a user device, a request to design an individualized vaccine for a subject;
transmitting, from the user device, a communication to a remote system, the communication including an identifier of the subject, wherein the remote system is configured to:

access a set of peptide sequences characterizing a set of peptides, each peptide sequence of the set of peptide sequences having been identified by processing a disease sample from a subject, and
access an immunoprotein complex (IPC) sequence identified for an immunoprotein complex (IPC) of the subject;
process a set of peptide representations that represents the set of peptide sequences using a first attention block in an initial attention subsystem of an attention-based machine-learning model and an immunoprotein complex (IPC) representation that represents the IPC sequence using a second attention block in the initial attention subsystem to generate an output, wherein the output includes at least one of an interaction prediction, an interaction affinity prediction, or an immunogenicity prediction for a corresponding peptide-IPC combination; and
generate a report based on the output; and
transmit the report to the user device; and

receiving, at the user device, the report.

82. The method of item 81, further comprising:

collecting a disease sample from the subject;
eluting multiple peptides that include the set of peptides from MHC molecules in the disease sample using at least one of chromatography or mass spectrometry;
sequencing the set of peptides to generate a set of initial sequences;
comparing each initial sequence of the set of initial sequences to a reference sequence; and
defining the set of peptide sequences based on the comparisons, wherein each peptide sequence in the set of peptide sequences is a variant-coding sequence that includes a variant with respect to the reference sequence.

83. A method for manufacturing a treatment for a subject, the method comprising:

receiving a report from a computing device that is configured to:

access a set of peptide sequences characterizing a set of peptides, each peptide sequence of the set of peptide sequences having been identified by processing a disease sample from a subject, and
access an immunoprotein complex (IPC) sequence identified for an immunoprotein complex (IPC) of the subject;
process a set of peptide representations that represents the set of peptide sequences using a first attention block in an initial attention subsystem of an attention-based machine-learning model and an immunoprotein complex (IPC) representation that represents the IPC sequence using a second attention block in the initial attention subsystem to generate an output, wherein the output includes at least one of an interaction prediction, an interaction affinity prediction, or an immunogenicity prediction for a corresponding peptide-IPC combination; and

generate the report based on the output; and
generating a treatment manufacturing plan for manufacturing the treatment based on the report.

84. The method of item 83, further comprising:
manufacturing the treatment based on the treatment manufacturing plan.

85. A method comprising:

inputting a plurality of variant-coding sequences characterizing a plurality of mutant peptides into an attention-based machine-learning model, each variant-coding sequence of the plurality of variant-coding sequences having been identified by processing a disease sample from a subject;
inputting an immunoprotein complex (IPC) sequence identified for an immunoprotein complex (IPC) of the subject into the attention-based machine-learning model,

wherein the attention-based machine-learning model is configured to process a plurality of variant representations that represents the plurality of variant-coding sequences using a first attention block in an initial attention subsystem of an attention-based machine-learning model and an immunoprotein complex (IPC) representation that represents the IPC sequence using a second attention block in the initial attention subsystem to generate an output,
wherein the output includes at least one of an interaction prediction, an interaction affinity prediction, or an immunogenicity prediction for a corresponding mutant peptide-IPC combination; and

receiving a report generated based on the output; and
selecting, based on the report, a subset of the plurality of mutant peptides to use in a treatment for the subject.

86. A method comprising:

receiving a peptide sequence that characterizes a mutant peptide, the peptide sequence including a variant with respect to a corresponding reference sequence;
receiving an MHC sequence identified for a major histocompatibility complex (MHC);
processing the peptide sequence and the MHC sequence using different processing paths within an attention-based machine-learning model to generate an output,
wherein the output provides information about an immunological activity relating to both the mutant peptide and the MHC; and
generating a report based on the output.

87. The method of item 86, wherein the processing comprises:

processing the peptide sequence via a peptide processing path within the attention-based machine-learning model, the peptide processing path including a first embedding block and a first attention block that includes at least one self-attention layer; and
processing the MHC sequence via an MHC processing path within the attention-based machine-learning model, the MHC processing path including a second embedding block and a second attention block that includes at least one self-attention layer.

88. The method of item 87, further comprising:

receiving a TCR sequence identified for a T cell receptor (TCR); and
wherein the processing further comprises:
processing the TCR sequence via a TCR processing path within the attention-based machine-learning model, the TCR processing path including a third embedding block and a third attention block that includes at least one self-attention layer.

89. The method of item 86, wherein the immunological activity includes an immune response and the information includes a prediction about an ability of the mutant peptide to provoke the immune response.

90. The method of item 86, wherein the processing comprises:

generating a transformed peptide representation of the peptide sequence via the peptide processing path;
generating a transformed MHC representation of the MHC sequence via the MHC processing path;
generating a composite representation using the transformed peptide representation and the transformed MHC representation;
processing the composite representation to generate the output.

91. The method of item 86, wherein the immunological activity includes a binding of the mutant peptide to the MHC and wherein the output includes at least one of a first prediction corresponding to whether the mutant peptide binds to the

MHC or a second prediction corresponding to an affinity associated with the binding.

92. The method of item 86, further comprising:
determining to include the mutant peptide as a target for an immunotherapy based on the report.

93. The method of item 92, wherein the immunotherapy is selected from a group consisting of a T cell therapy, a personalized cancer therapy, an antigen-specific immunotherapy, an antigen-dependent immunotherapy, a vaccine, and a natural killer (NK) cell therapy.

94. The method of item 86, further comprising, at least one of:
determining to exclude the mutant peptide as a target for an immunotherapy based on the report.

95. The method of item 94, wherein the immunotherapy is selected from a group consisting of a T cell therapy, a personalized cancer therapy, an antigen-specific immunotherapy, an antigen-dependent immunotherapy, a vaccine, and a natural killer (NK) cell therapy.

96. The method of item 86, further comprising:

> determining, based on the report, to include at least one of the mutant peptide, a precursor of the mutant peptide, nucleic acids that encode the mutant peptide, or a plurality of cells that express the mutant peptide in a treatment; and
> manufacturing the treatment.

97. The method of item 96, further comprising:
treating a subject with the treatment.

98. The method of item 86, wherein the peptide sequence characterizing the mutant peptide was identified by sequencing a disease sample from a subject, wherein the peptide sequence has at least one sequence variation relative to a corresponding reference sequence, and wherein a treatment is designed for the subject based on the report.

99. A method comprising:

> receiving a peptide sequence that characterizes a mutant peptide, the peptide sequence including a variant with respect to a corresponding reference sequence;
> receiving a TCR sequence identified for a T cell receptor (TCR);
> processing the peptide sequence and the TCR sequence using different processing paths within an attention-based machine-learning model to generate an output,
> wherein the output provides information about an immunological activity relating to both the mutant peptide and the TCR; and
> generating a report based on the output.

100. The method of item 99, wherein the processing comprises:

> processing the peptide sequence via a peptide processing path within the attention-based machine-learning model, the peptide processing path including a first embedding block and a first attention block; and
> processing the TCR sequence via a TCR processing path within the attention-based machine-learning model, the TCR processing path including a second embedding block and a second attention block.

101. The method of item 100, further comprising:

> receiving an MHC sequence identified for a major histocompatibility complex (MHC); and
>
> wherein the processing further comprises:
> processing the MHC sequence via an MHC processing path within the attention-based machine-learning model, the MHC processing path including a third embedding block and an MHC third block.

102. The method of item 99, wherein the immunological activity includes an immune response and the information

includes a prediction about an ability of the mutant peptide to provoke the immune response.

103. The method of item 99, wherein the processing comprises:

generating a transformed peptide representation of the peptide sequence via the peptide processing path;
generating a transformed TCR representation of the TCR sequence via the TCR processing path;
generating a composite representation using the transformed peptide representation and the transformed TCR representation;
processing the composite representation to generate the output.

104. The method of item 99, wherein the immunological activity includes a binding of the mutant peptide to the MHC and wherein the output includes at least one of a first prediction corresponding to whether the mutant peptide binds to the MHC or a second prediction corresponding to an affinity associated with the binding.

105. The method of item 99, further comprising:
determining to include the mutant peptide as a target for an immunotherapy based on the report.

106. The method of item 105, wherein the immunotherapy is selected from a group consisting of a T cell therapy, a personalized cancer therapy, an antigen-specific immunotherapy, an antigen-dependent immunotherapy, a vaccine, and a natural killer (NK) cell therapy.

107. The method of item 99, further comprising, at least one of:
determining to exclude the mutant peptide as a target for an immunotherapy based on the report.

108. The method of item 107, wherein the immunotherapy is selected from a group consisting of a T cell therapy, a personalized cancer therapy, an antigen-specific immunotherapy, an antigen-dependent immunotherapy, a vaccine, and a natural killer (NK) cell therapy.

109. The method of item 99, further comprising:

determining, based on the report, to include at least one of the mutant peptide, a precursor of the mutant peptide, nucleic acids that encode the mutant peptide, or a plurality of cells that express the mutant peptide in a treatment; and
manufacturing the treatment.

110. The method of item 109, further comprising:
treating a subject with the treatment.

111. The method of item 99, wherein the peptide sequence characterizing the mutant peptide was identified by sequencing a disease sample from a subject, wherein the peptide sequence has at least one sequence variation relative to a corresponding reference sequence, and wherein a treatment is designed for the subject based on the report.

112. A system comprising:

one or more data processors; and
a non-transitory computer readable storage medium containing instructions which, when executed on the one or more data processors, cause the one or more data processors configured to:

access a set of peptide sequences characterizing a set of peptides, each peptide sequence of the set of peptide sequences having been identified by processing a disease sample from a subject;
access an immunoprotein complex (IPC) sequence identified for an immunoprotein complex (IPC) of the subject;
process a set of peptide representations that represents the set of peptide sequences using a first attention block in an initial attention subsystem of an attention-based machine-learning model and an immunoprotein complex (IPC) representation that represents the IPC sequence using a second attention block in the initial attention subsystem to generate an output, wherein the output includes at least one of an interaction prediction, an interaction affinity prediction, or an immunogenicity prediction for a corresponding peptide-IPC

combination; and
generate a report based on the output.

## Claims

**1.** A computer-implemented method comprising:

accessing a set of peptide sequences characterizing a set of peptides, each peptide sequence of the set of peptide sequences having been identified by processing a disease sample from a subject;
accessing an immunoprotein complex (IPC) sequence identified for an immunoprotein complex (IPC) of the subject, wherein the IPC comprises a major histocompatibility complex (MHC);
processing a set of peptide representations that represents the set of peptide sequences using a first attention block in an initial attention subsystem of an attention-based machine-learning model and an immunoprotein complex (IPC) representation that represents the IPC sequence using a second attention block in the initial attention subsystem to generate an output, wherein the output includes at least one of an interaction prediction or an interaction affinity prediction for a corresponding peptide-IPC combination;
wherein:

the corresponding peptide-IPC combination includes a peptide of the set of peptides and the MHC;
the interaction affinity prediction for the corresponding peptide-IPC class-combination predicts a binding affinity between the peptide and the MHC; and
the interaction prediction for the corresponding peptide-IPC combination predicts whether the MHC will present the peptide at a cell surface; and

the method further comprises generating a report based on the output.

**2.** The method of claim 1, wherein at least one peptide sequence of the set of peptide sequences comprises a variant-coding sequence that includes a variant with respect to a corresponding reference sequence.

**3.** The method of any preceding claim, wherein the processing comprises:

receiving a peptide representation of the set of peptide representations for a corresponding peptide sequence of the set of peptide sequences; and
transforming the peptide representation via the first attention block into a transformed peptide representation, wherein the first attention block includes a set of attention sub-blocks in which each attention sub-block of the set of attention sub-blocks includes a self-attention layer.

**4.** The method of any preceding claim, wherein the processing comprises:

receiving the IPC representation; and
transforming the IPC representation via the second attention block into a transformed IPC representation, wherein the second attention block includes a set of attention sub-blocks in which each attention sub-block of the set of attention sub-blocks includes a self- attention layer.

**5.** The method of any preceding claim, wherein at least a portion of the peptide representation corresponds to a monomer in the peptide sequence and at least a portion of the IPC representation corresponds to a monomer in the IPC sequence; and wherein the processing comprises:

generating a transformed peptide representation based on the peptide representation using the first attention block and a first set of weights;
generating a transformed IPC representation based on the IPC representation using the second attention block and a second set of weights; and
generating a composite representation using the transformed peptide representation and the transformed MHC representation.

**6.** The method of any preceding claim, further comprising:

either:

embedding a peptide sequence of the set of peptide sequences to generate an embedded peptide representation for the peptide sequence; and
encoding, positionally, the embedded peptide representation for the peptide sequence to generate a peptide representation of the set of peptide representations that represents the peptide sequence;
or:

embedding the IPC sequence to generate an embedded IPC representation of the IPC sequence; and
encoding, positionally, the embedded IPC representation of the IPC sequence to generate the IPC representation.

**7.** The method of any preceding claim, wherein:

the first attention block comprises a set of attention sub-blocks; and
each attention sub-block of the set of attention sub-blocks includes a neural network that comprises at least one self-attention layer; and/or wherein:
the second attention block comprises a set of attention sub-blocks; and
each attention sub-block of the set of attention sub-blocks includes a neural network that comprises at least one self-attention layer.

**8.** The method of any preceding claim, wherein:

a peptide representation of the set of peptide representations forms a first portion of an aggregate representation processed using the first attention block; and
a second portion of the aggregate representation represents at least one of an N-flank sequence or a C-flank sequence.

**9.** The method of any preceding claim, wherein:

a peptide sequence of the set of peptide sequences forms a first portion of an aggregate sequence; and
a second portion of the aggregate sequence includes at least one of an N-flank sequence or a C-flank sequence; and
the attention-based machine learning model includes a representation block that receives and processes the aggregate sequence to form an aggregate representation that includes a peptide representation of the set of peptide representations corresponding to the peptide sequence, wherein the aggregate representation is processed by the first attention block.

**10.** The method of any preceding claim, wherein the attention-based machine-learning model includes a plurality of self-attention layers and for each of the plurality of self-attention layers, a corresponding downstream feedforward neural network.

**11.** The method of any preceding claim, wherein:

the first attention block includes a first neural network configured to receive and process a peptide representation of the set of peptide representations to generate a transformed peptide representation; and
the second attention block includes a second neural network configured to receive and process the IPC representation to generate a transformed IPC representation; and
wherein each of the first neural network and the second neural network includes at least one self-attention layer; and
wherein the attention-based machine-learning model is configured to generate a composite representation using the transformed peptide representation and the transformed IPC representation.

**12.** The method of any preceding claim, wherein the attention-based machine-learning model further includes:
a composite attention block that includes a neural network configured to receive and process the composite representation, wherein the neural network includes a self-attention layer.

**13.** The method of any preceding claim, wherein the attention-based machine-learning model further includes:

a composite attention block that includes a set of attention sub-blocks, wherein each attention sub-block of the set of attention sub-blocks includes a neural network that comprises at least one self-attention layer.

**14.** The method of any preceding claim, wherein the attention-based machine-learning model is trained using a training data set that includes at least one of experimental interaction affinity data or experimental interaction data for a plurality of training peptide sequences and a set of training MHC sequences.

**15.** The method of any preceding claim, wherein the processing comprises:

processing the set of peptide representations using the first attention block and the IPC representation using the second attention block to generate a set of composite representations for a set of peptide-IPC combinations;
processing the set of composite representations to generate a set of results;
selecting a subset of the set of peptide-IPC combinations, wherein a set of selected interactions is more likely to occur with each peptide-IPC combination of the subset as compared to a remaining subset of the set of peptide-IPC combinations,
wherein the report identifies each peptide within the subset.

**16.** The method of any preceding claim, wherein:

each peptide of the set of peptides is used to form a set of peptide-MHC combinations; and
the attention-based machine-learning model is configured to generate the interaction prediction for each peptide-MHC combination of the set of peptide-MHC combinations, the interaction prediction for a peptide-MHC combination of the set of peptide-MHC combinations being a prediction of whether a peptide in the peptide-MHC combination is presented by the MHC at a cell surface.

**17.** The method of claim 16, wherein the report identifies a subset of peptides from the set of peptides having an increased likelihood of presentation by the MHC relative to a remaining portion of the set of peptides.

**18.** The method of any preceding claim, wherein:

a peptide sequence of the set of peptide sequences is a variant-coding sequence characterizing a mutant peptide, the variant-coding sequence comprising:

a first part identifying a sequence at an N-terminus of the mutant peptide; and
a second part identifying a sequence of an epitope of the mutant peptide; and

the processing comprises:

processing a first representation of the first part of the variant-coding sequence using a first self-attention layer of the initial attention subsystem; and
processing a second representation of the second part of the variant-coding sequence using a second self-attention layer of the initial attention subsystem.

**19.** The method of any preceding claim, wherein the attention-based machine-learning model includes one or more transformer encoders, wherein each of the one or more transformer encoders includes a self-attention layer.

**20.** The method of any preceding claim, wherein the IPC sequence is identified using the disease sample and/or a biological sample from the subject.

**21.** The method of any preceding claim, wherein:

the IPC sequence includes an MHC sequence; and
the IPC representation includes an MHC representation.

**22.** The method of claim 21, wherein the MHC includes an MHC class-I molecule.

**23.** The method of claim 21, wherein the MHC includes an MHC class-II molecule.

**24.** The method of any preceding claim, wherein:

the IPC of the subject includes a T cell receptor (TCR);
the IPC sequence includes a TCR sequence; and
the IPC representation includes a TCR representation.

**25.** The method of any one of claims 2-24 wherein each of at least one of the set of variant-coding sequences is based on RNA sequences of the disease sample.

**26.** The method of any preceding claim, further comprising:

receiving input data entered by a user, the input data corresponding to the subject;
wherein the set of peptide sequences and the IPC sequence are accessed, in response to receiving the input data, via retrieval from a data store; and
wherein the report identifies a subset of peptides from the set of peptides to include in an individualized vaccine to treat a medical condition of the subject.

**27.** The method of claim 26, further comprising:
generating a treatment recommendation to the subject that includes the individualized vaccine.

**28.** The method of any preceding claim, further comprising:

receiving input data entered by a user, the input data corresponding to the subject;
wherein the set of peptide sequences and the IPC sequence are accessed, in response to receiving the input data, via retrieval from a data store; and
determining a set of treatment peptides for inclusion in an individualized vaccine based on the report; and
initiating an action that facilitates manufacture of the individualized vaccine that includes the set of treatment peptides.

**29.** The method of any preceding claim, wherein the processing comprises:

receiving, from an embedding block in the attention-based machine-learning model, a

representation that comprises a plurality of elements,
wherein the representation is either a peptide representation of the set of peptide representations that represents a peptide sequence in the set of peptide sequences or the IPC representation representing the IPC sequence; and
wherein each element in the multi-element data set corresponds to a monomer in either the peptide sequence or the IPC sequence;

determining, for each element of the plurality of elements, a key vector, a value vector, and a query vector based on a set of key weights, a set of value weights, and a set of query weights, respectively, associated with a self-attention layer of the attention-based machine learning model;
performing a transformation of the plurality of elements to form a plurality of modified elements, wherein the transformation is performed using attention scores generated for the plurality of elements and the value vector determined for each of the plurality of elements; and
generating the output based on the plurality of modified elements.

**30.** The method of claim 29, wherein performing the transformation for a selected element of the plurality of elements comprises:

determining an attention score of the selected element using the key vector and the query vector of the element, wherein a remaining portion of the plurality of elements other than the selected element forms a set of remaining elements;
determining an additional attention score for each remaining element of the set of remaining elements using a key vector of the remaining element and the query vector of the selected element to form a set of additional attention scores; and
generating a modified element using the attention score, the set of additional attention scores, and the value

vector of each element of the plurality of elements.

**31.** The method of any preceding claim, wherein the IPC is a human leukocyte antigen (HLA) molecule.

**32.** A system comprising:

one or more data processors; and
a computer readable storage medium containing instructions which, when executed on the one or more data processors, cause the one or more data processors to perform any one of claims 1-31.

**33.** A computer-program product tangibly embodied in a machine-readable storage medium, including instructions configured to cause one or more data processors to perform any one of claims 1-31.

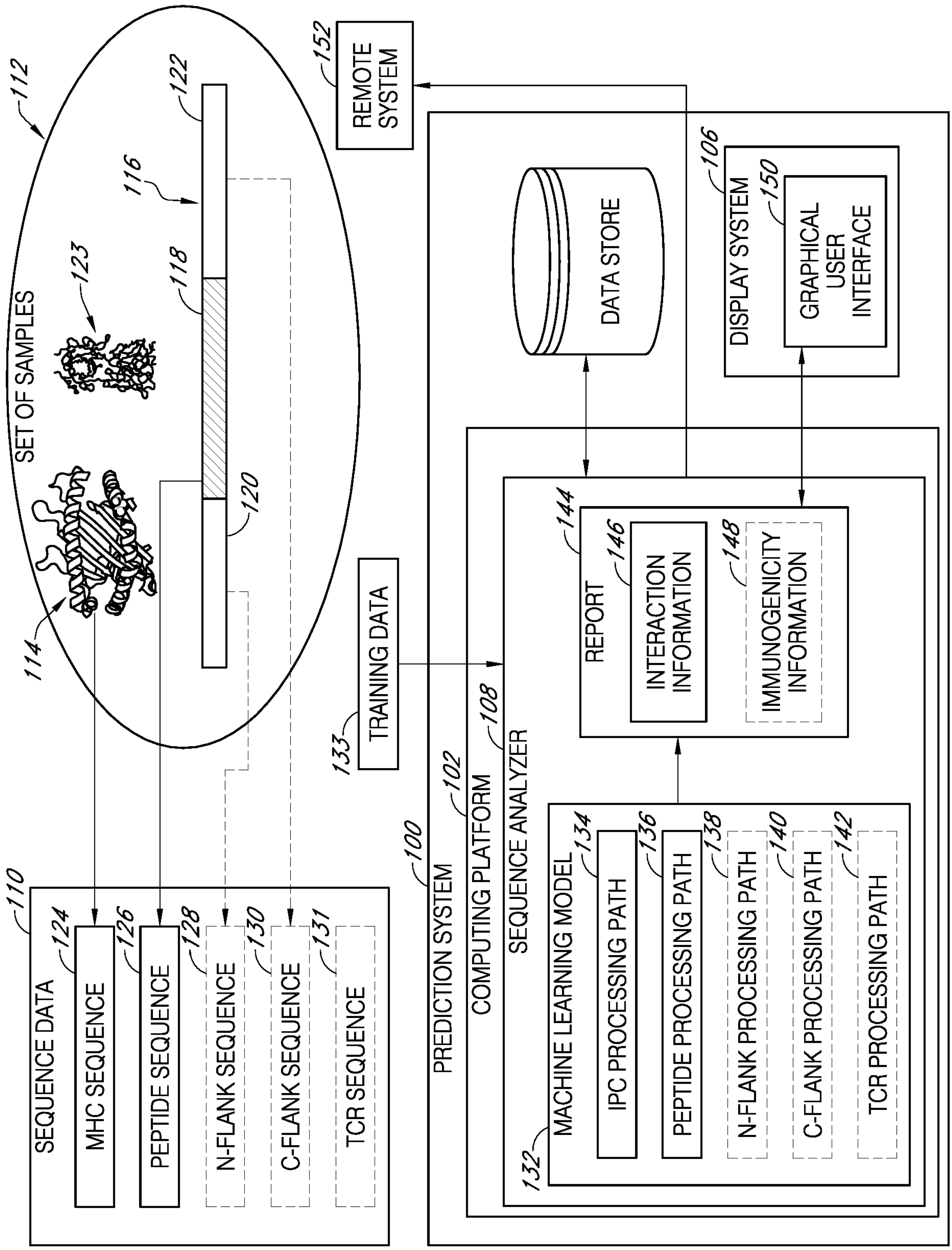
SET OF SAMPLES
112
114
116
118
120
122
123
SEQUENCE DATA
110
MHC SEQUENCE
124
PEPTIDE SEQUENCE
126
N-FLANK SEQUENCE
128
C-FLANK SEQUENCE
130
TCR SEQUENCE
131
TRAINING DATA
133
PREDICTION SYSTEM
100
COMPUTING PLATFORM
102
SEQUENCE ANALYZER
108
MACHINE LEARNING MODEL
132
IPC PROCESSING PATH
134
PEPTIDE PROCESSING PATH
136
N-FLANK PROCESSING PATH
138
C-FLANK PROCESSING PATH
140
TCR PROCESSING PATH
142
REPORT
144
INTERACTION INFORMATION
146
IMMUNOGENICITY INFORMATION
148
DATA STORE
DISPLAY SYSTEM
106
GRAPHICAL USER INTERFACE
150
REMOTE SYSTEM
152

FIG. 1

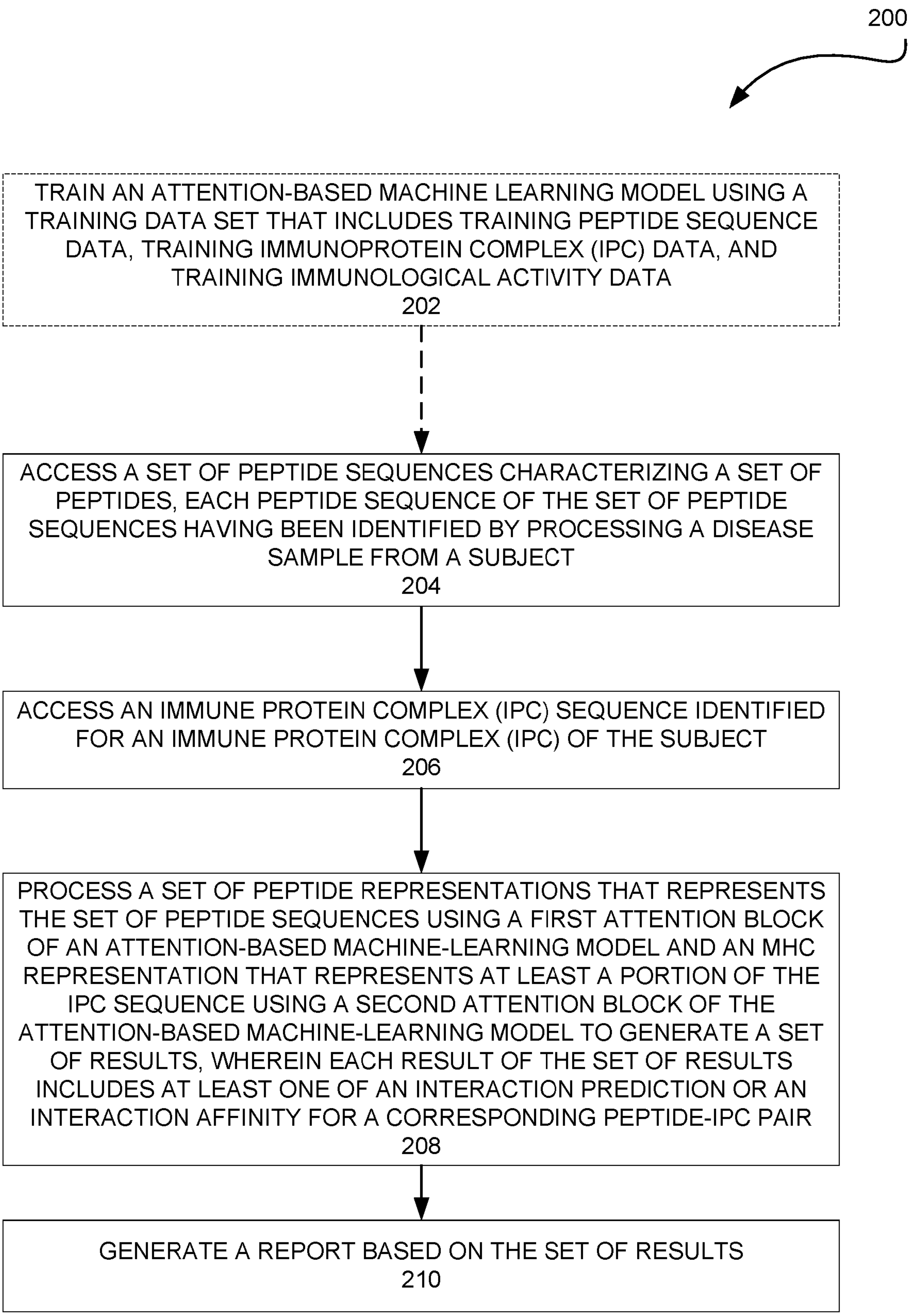
200
TRAIN AN ATTENTION-BASED MACHINE LEARNING MODEL USING A TRAINING DATA SET THAT INCLUDES TRAINING PEPTIDE SEQUENCE DATA, TRAINING IMMUNOPROTEIN COMPLEX (IPC) DATA, AND TRAINING IMMUNOLOGICAL ACTIVITY DATA
202
ACCESS A SET OF PEPTIDE SEQUENCES CHARACTERIZING A SET OF PEPTIDES, EACH PEPTIDE SEQUENCE OF THE SET OF PEPTIDE SEQUENCES HAVING BEEN IDENTIFIED BY PROCESSING A DISEASE SAMPLE FROM A SUBJECT
204
ACCESS AN IMMUNE PROTEIN COMPLEX (IPC) SEQUENCE IDENTIFIED FOR AN IMMUNE PROTEIN COMPLEX (IPC) OF THE SUBJECT
206
PROCESS A SET OF PEPTIDE REPRESENTATIONS THAT REPRESENTS THE SET OF PEPTIDE SEQUENCES USING A FIRST ATTENTION BLOCK OF AN ATTENTION-BASED MACHINE-LEARNING MODEL AND AN MHC REPRESENTATION THAT REPRESENTS AT LEAST A PORTION OF THE IPC SEQUENCE USING A SECOND ATTENTION BLOCK OF THE ATTENTION-BASED MACHINE-LEARNING MODEL TO GENERATE A SET OF RESULTS, WHEREIN EACH RESULT OF THE SET OF RESULTS INCLUDES AT LEAST ONE OF AN INTERACTION PREDICTION OR AN INTERACTION AFFINITY FOR A CORRESPONDING PEPTIDE-IPC PAIR
208
GENERATE A REPORT BASED ON THE SET OF RESULTS
210

**Figure 2**

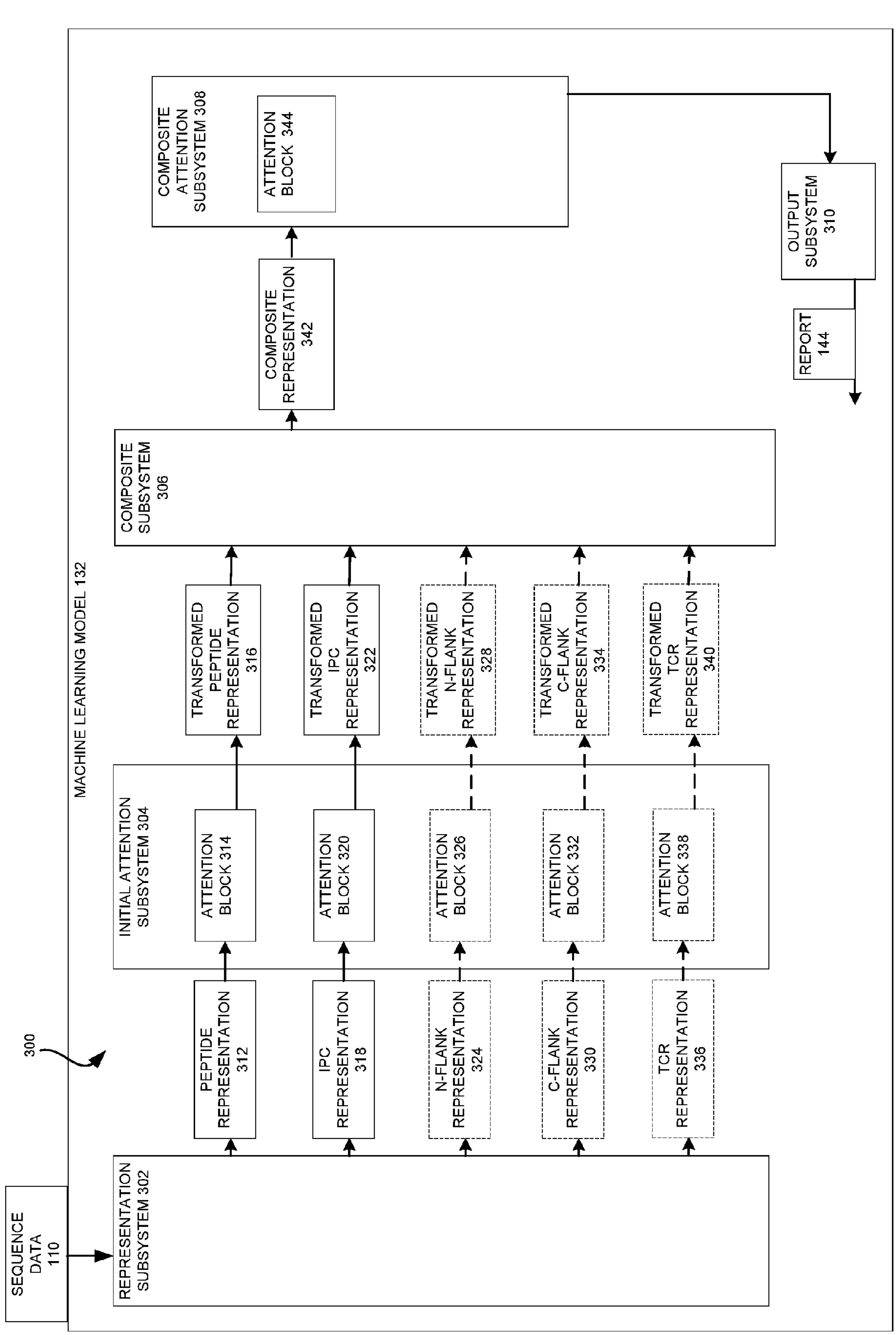
300
MACHINE LEARNING MODEL 132
SEQUENCE DATA 110
REPRESENTATION SUBSYSTEM 302
PEPTIDE REPRESENTATION 312
IPC REPRESENTATION 318
N-FLANK REPRESENTATION 324
C-FLANK REPRESENTATION 330
TCR REPRESENTATION 336
INITIAL ATTENTION SUBSYSTEM 304
ATTENTION BLOCK 314
ATTENTION BLOCK 320
ATTENTION BLOCK 326
ATTENTION BLOCK 332
ATTENTION BLOCK 338
TRANSFORMED PEPTIDE REPRESENTATION 316
TRANSFORMED IPC REPRESENTATION 322
TRANSFORMED N-FLANK REPRESENTATION 328
TRANSFORMED C-FLANK REPRESENTATION 334
TRANSFORMED TCR REPRESENTATION 340
COMPOSITE SUBSYSTEM 306
COMPOSITE REPRESENTATION 342
COMPOSITE ATTENTION SUBSYSTEM 308
ATTENTION BLOCK 344
OUTPUT SUBSYSTEM 310
REPORT 144

Figure 3

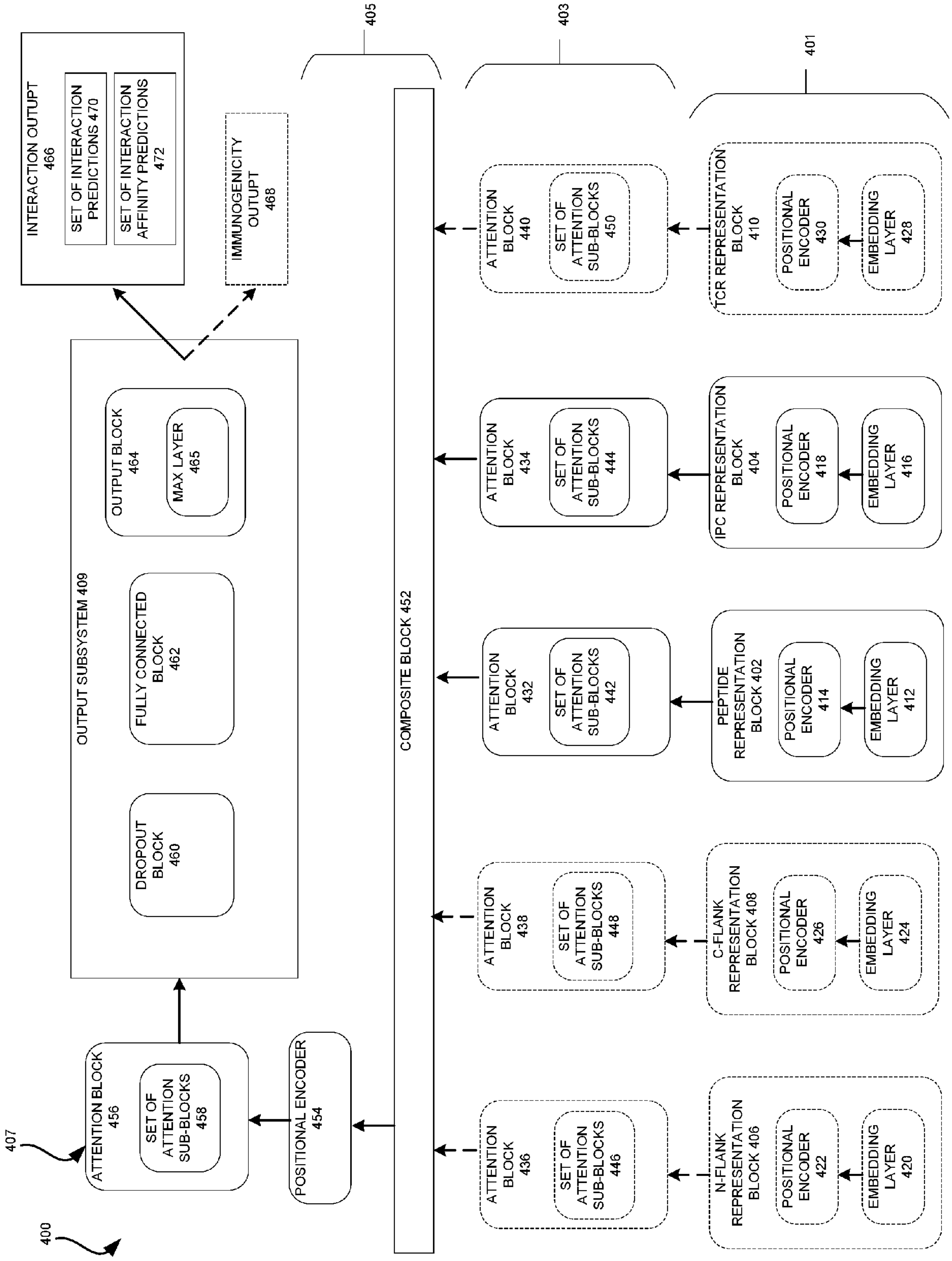
400
407
ATTENTION BLOCK 456
SET OF ATTENTION SUB-BLOCKS 458
POSITIONAL ENCODER 454
OUTPUT SUBSYSTEM 409
DROPOUT BLOCK 460
FULLY CONNECTED BLOCK 462
OUTPUT BLOCK 464
MAX LAYER 465
INTERACTION OUTPUT 466
SET OF INTERACTION PREDICTIONS 470
SET OF INTERACTION AFFINITY PREDICTIONS 472
IMMUNOGENICITY OUTPUT 468
COMPOSITE BLOCK 452
405
403
401
ATTENTION BLOCK 436
SET OF ATTENTION SUB-BLOCKS 446
ATTENTION BLOCK 438
SET OF ATTENTION SUB-BLOCKS 448
ATTENTION BLOCK 432
SET OF ATTENTION SUB-BLOCKS 442
ATTENTION BLOCK 434
SET OF ATTENTION SUB-BLOCKS 444
ATTENTION BLOCK 440
SET OF ATTENTION SUB-BLOCKS 450
N-FLANK REPRESENTATION BLOCK 406
POSITIONAL ENCODER 422
EMBEDDING LAYER 420
C-FLANK REPRESENTATION BLOCK 408
POSITIONAL ENCODER 426
EMBEDDING LAYER 424
PEPTIDE REPRESENTATION BLOCK 402
POSITIONAL ENCODER 414
EMBEDDING LAYER 412
IPC REPRESENTATION BLOCK 404
POSITIONAL ENCODER 418
EMBEDDING LAYER 416
TCR REPRESENTATION BLOCK 410
POSITIONAL ENCODER 430
EMBEDDING LAYER 428

Figure 4A

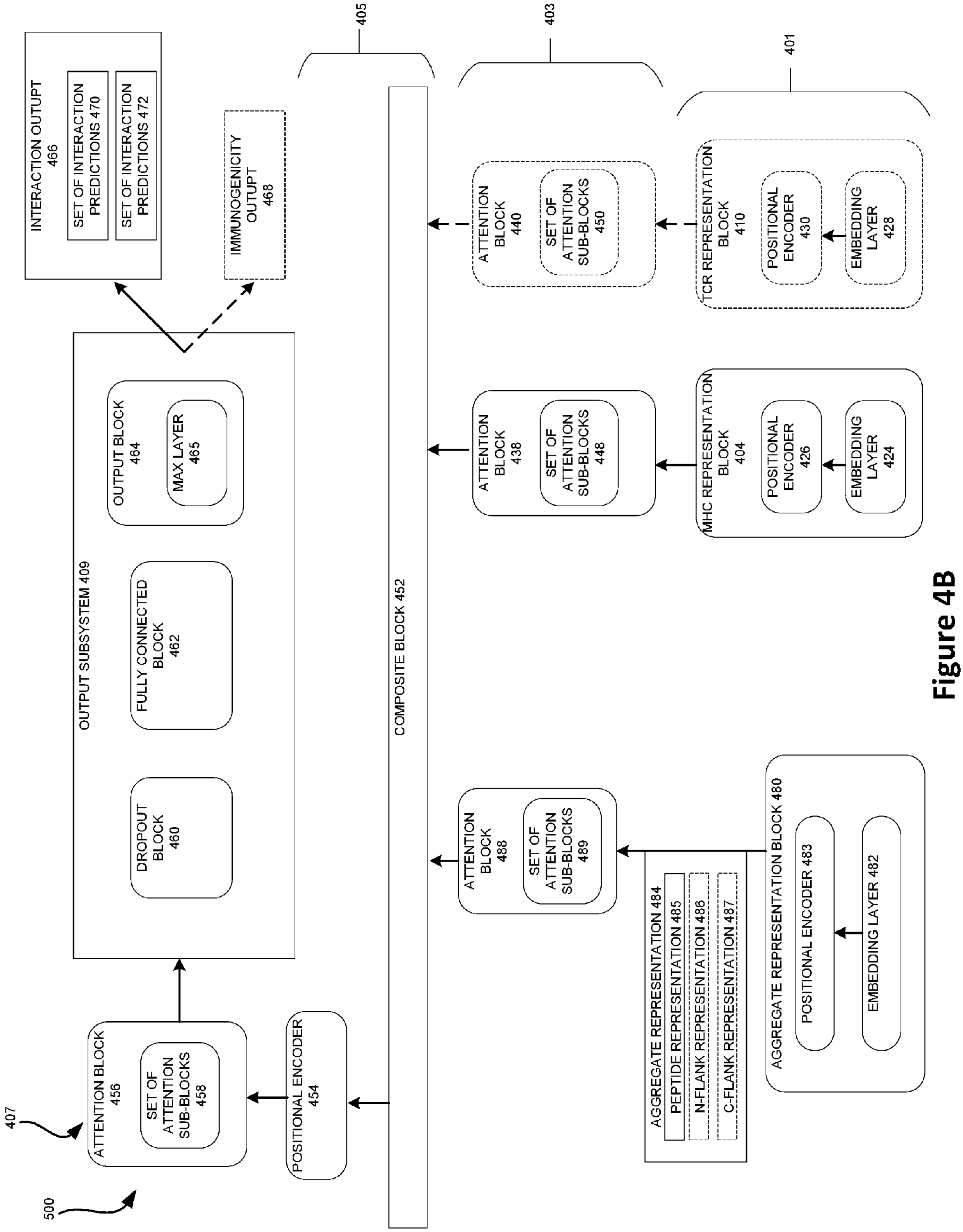
500
407
ATTENTION BLOCK 456
SET OF ATTENTION SUB-BLOCKS 458
POSITIONAL ENCODER 454
OUTPUT SUBSYSTEM 409
DROPOUT BLOCK 460
FULLY CONNECTED BLOCK 462
OUTPUT BLOCK 464
MAX LAYER 465
INTERACTION OUTPUT 466
SET OF INTERACTION PREDICTIONS 470
SET OF INTERACTION PREDICTIONS 472
IMMUNOGENICITY OUTPUT 468
COMPOSITE BLOCK 452
405
403
401
ATTENTION BLOCK 488
SET OF ATTENTION SUB-BLOCKS 489
ATTENTION BLOCK 438
SET OF ATTENTION SUB-BLOCKS 448
ATTENTION BLOCK 440
SET OF ATTENTION SUB-BLOCKS 450
AGGREGATE REPRESENTATION 484
PEPTIDE REPRESENTATION 485
N-FLANK REPRESENTATION 486
C-FLANK REPRESENTATION 487
AGGREGATE REPRESENTATION BLOCK 480
POSITIONAL ENCODER 483
EMBEDDING LAYER 482
MHC REPRESENTATION BLOCK 404
POSITIONAL ENCODER 426
EMBEDDING LAYER 424
TCR REPRESENTATION BLOCK 410
POSITIONAL ENCODER 430
EMBEDDING LAYER 428

Figure 4B

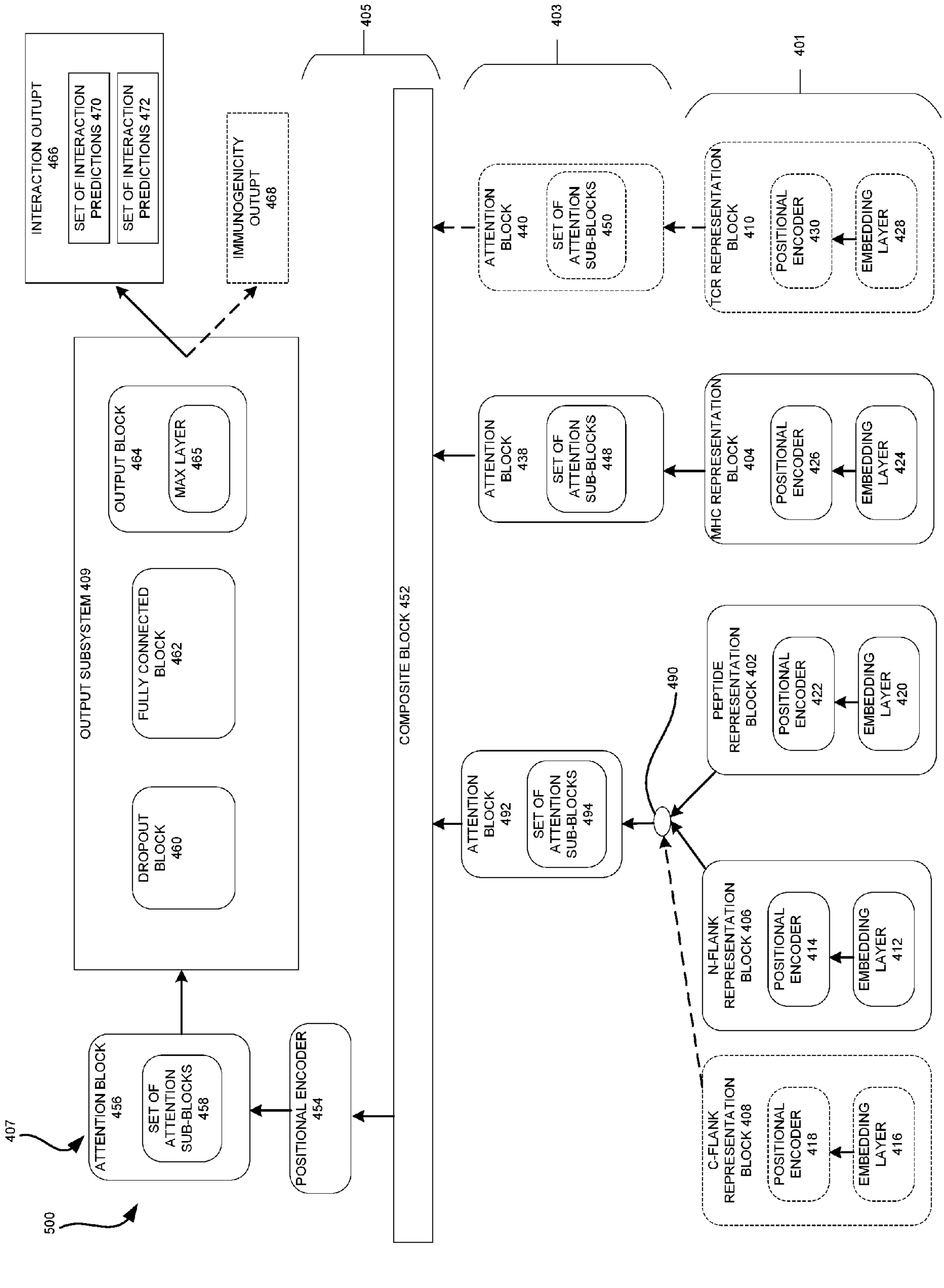
INTERACTION OUTUPT 466
SET OF INTERACTION PREDICTIONS 470
SET OF INTERACTION PREDICTIONS 472
IMMUNOGENICITY OUTUPT 468
405
403
401
OUTPUT SUBSYSTEM 409
OUTPUT BLOCK 464
MAX LAYER 465
FULLY CONNECTED BLOCK 462
DROPOUT BLOCK 460
COMPOSITE BLOCK 452
ATTENTION BLOCK 440
SET OF ATTENTION SUB-BLOCKS 450
TCR REPRESENTATION BLOCK 410
POSITIONAL ENCODER 430
EMBEDDING LAYER 428
ATTENTION BLOCK 438
SET OF ATTENTION SUB-BLOCKS 448
MHC REPRESENTATION BLOCK 404
POSITIONAL ENCODER 426
EMBEDDING LAYER 424
PEPTIDE REPRESENTATION BLOCK 402
POSITIONAL ENCODER 422
EMBEDDING LAYER 420
490
ATTENTION BLOCK 492
SET OF ATTENTION SUB-BLOCKS 494
N-FLANK REPRESENTATION BLOCK 406
POSITIONAL ENCODER 414
EMBEDDING LAYER 412
C-FLANK REPRESENTATION BLOCK 408
POSITIONAL ENCODER 418
EMBEDDING LAYER 416
407
ATTENTION BLOCK 456
SET OF ATTENTION SUB-BLOCKS 458
POSITIONAL ENCODER 454
500

Figure 4C

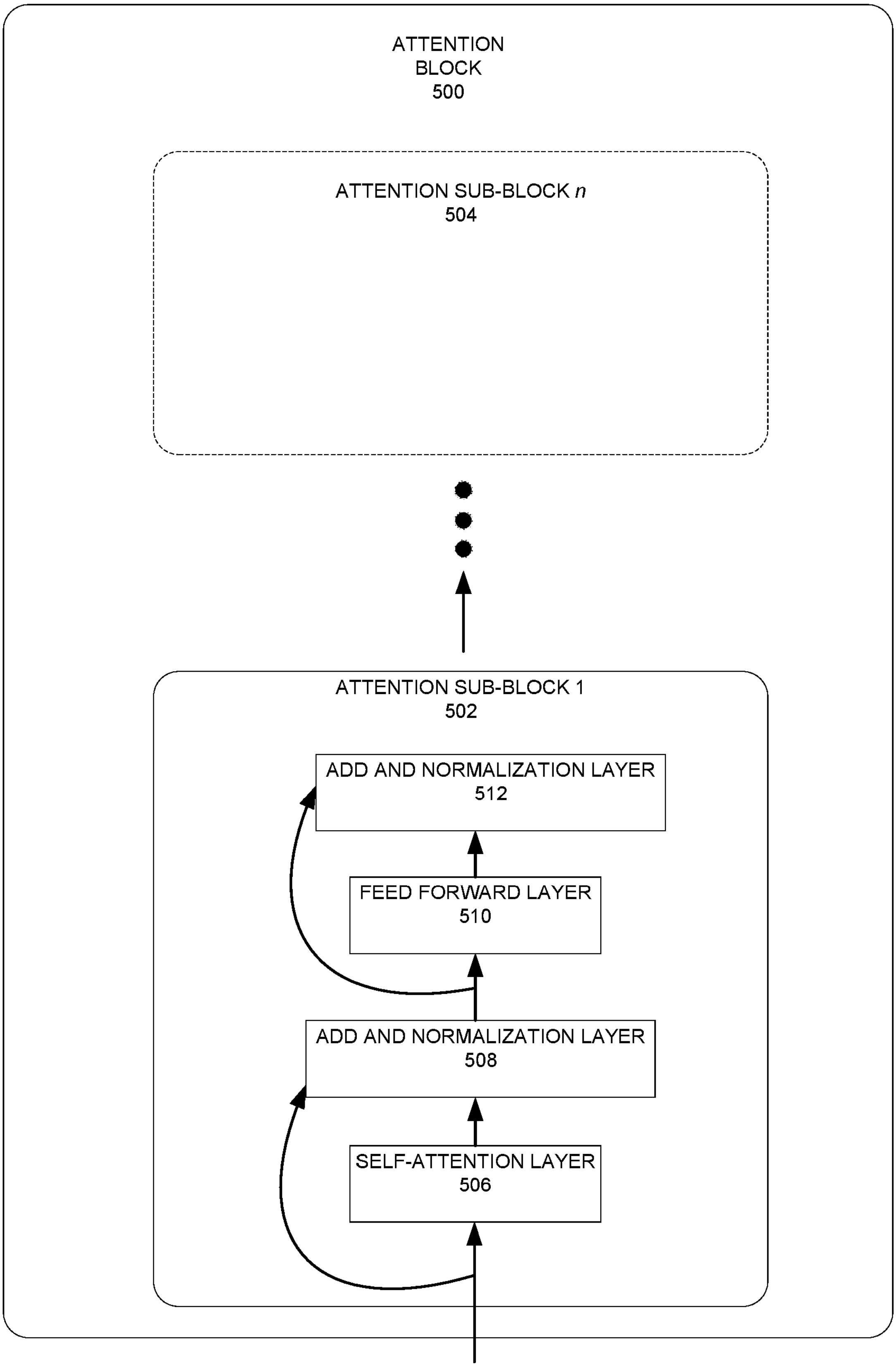
ATTENTION
BLOCK
500
ATTENTION SUB-BLOCK *n*
504
ATTENTION SUB-BLOCK 1
502
ADD AND NORMALIZATION LAYER
512
FEED FORWARD LAYER
510
ADD AND NORMALIZATION LAYER
508
SELF-ATTENTION LAYER
506

Figure 5

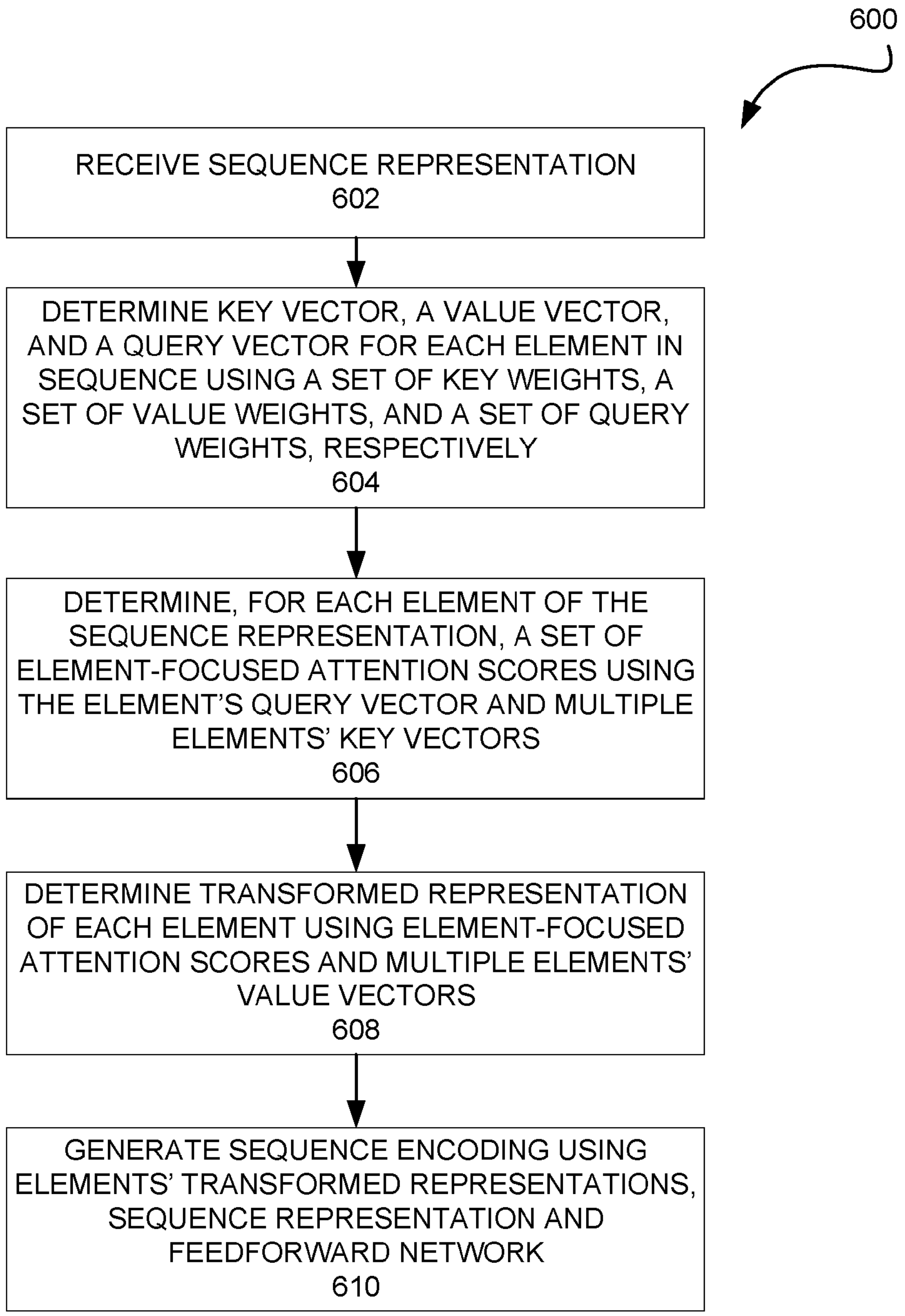
600
RECEIVE SEQUENCE REPRESENTATION
602
DETERMINE KEY VECTOR, A VALUE VECTOR, AND A QUERY VECTOR FOR EACH ELEMENT IN SEQUENCE USING A SET OF KEY WEIGHTS, A SET OF VALUE WEIGHTS, AND A SET OF QUERY WEIGHTS, RESPECTIVELY
604
DETERMINE, FOR EACH ELEMENT OF THE SEQUENCE REPRESENTATION, A SET OF ELEMENT-FOCUSED ATTENTION SCORES USING THE ELEMENT'S QUERY VECTOR AND MULTIPLE ELEMENTS' KEY VECTORS
606
DETERMINE TRANSFORMED REPRESENTATION OF EACH ELEMENT USING ELEMENT-FOCUSED ATTENTION SCORES AND MULTIPLE ELEMENTS' VALUE VECTORS
608
GENERATE SEQUENCE ENCODING USING ELEMENTS' TRANSFORMED REPRESENTATIONS, SEQUENCE REPRESENTATION AND FEEDFORWARD NETWORK
610

**Figure 6**

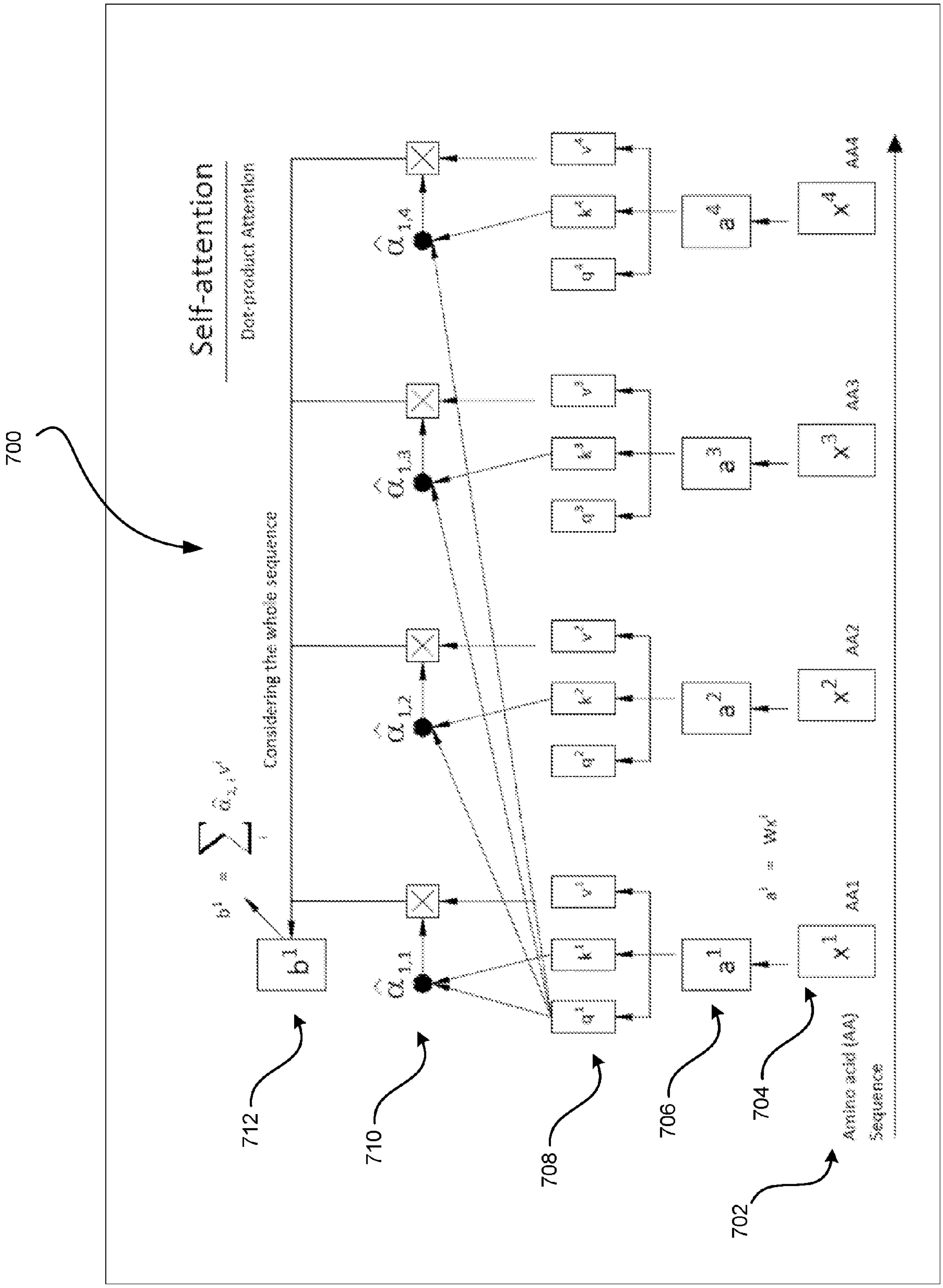
700
Self-attention
Dot-product Attention
Considering the whole sequence
Amino acid (AA) Sequence
AA1
AA2
AA3
AA4
702
704
706
708
710
712

Figure 7

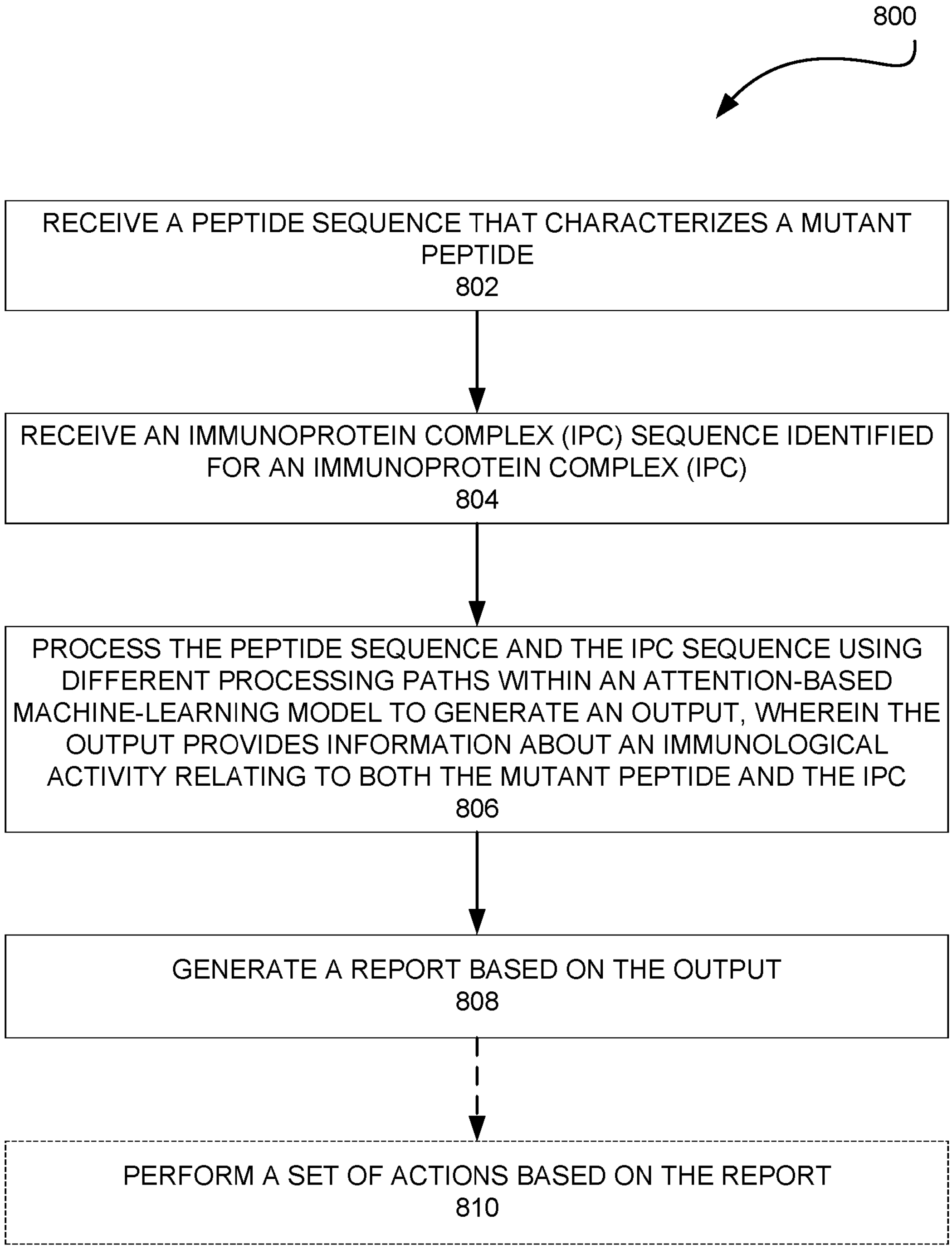
800
RECEIVE A PEPTIDE SEQUENCE THAT CHARACTERIZES A MUTANT PEPTIDE
802
RECEIVE AN IMMUNOPROTEIN COMPLEX (IPC) SEQUENCE IDENTIFIED FOR AN IMMUNOPROTEIN COMPLEX (IPC)
804
PROCESS THE PEPTIDE SEQUENCE AND THE IPC SEQUENCE USING DIFFERENT PROCESSING PATHS WITHIN AN ATTENTION-BASED MACHINE-LEARNING MODEL TO GENERATE AN OUTPUT, WHEREIN THE OUTPUT PROVIDES INFORMATION ABOUT AN IMMUNOLOGICAL ACTIVITY RELATING TO BOTH THE MUTANT PEPTIDE AND THE IPC
806
GENERATE A REPORT BASED ON THE OUTPUT
808
PERFORM A SET OF ACTIONS BASED ON THE REPORT
810

Figure 8

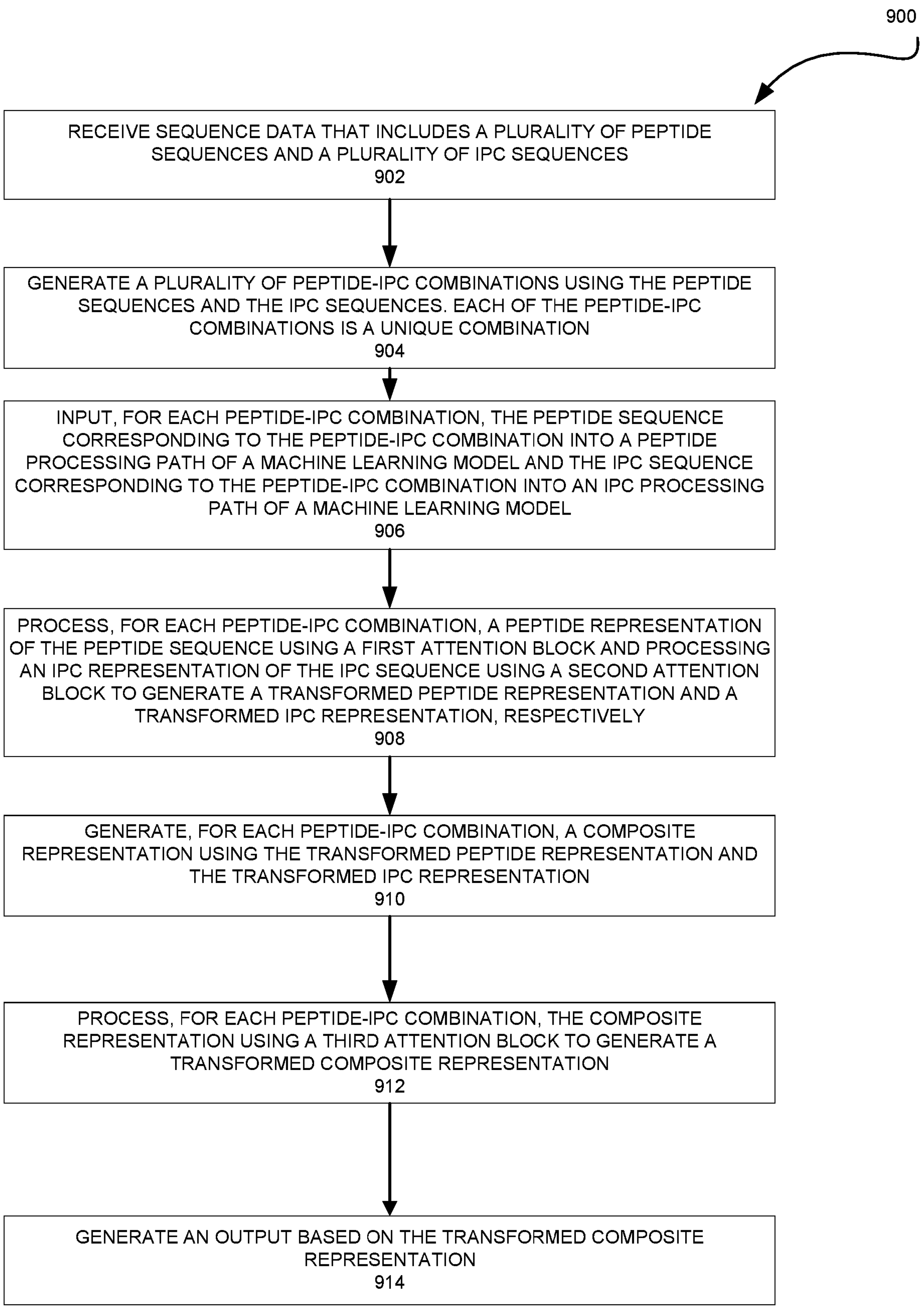
900
RECEIVE SEQUENCE DATA THAT INCLUDES A PLURALITY OF PEPTIDE SEQUENCES AND A PLURALITY OF IPC SEQUENCES
902
GENERATE A PLURALITY OF PEPTIDE-IPC COMBINATIONS USING THE PEPTIDE SEQUENCES AND THE IPC SEQUENCES. EACH OF THE PEPTIDE-IPC COMBINATIONS IS A UNIQUE COMBINATION
904
INPUT, FOR EACH PEPTIDE-IPC COMBINATION, THE PEPTIDE SEQUENCE CORRESPONDING TO THE PEPTIDE-IPC COMBINATION INTO A PEPTIDE PROCESSING PATH OF A MACHINE LEARNING MODEL AND THE IPC SEQUENCE CORRESPONDING TO THE PEPTIDE-IPC COMBINATION INTO AN IPC PROCESSING PATH OF A MACHINE LEARNING MODEL
906
PROCESS, FOR EACH PEPTIDE-IPC COMBINATION, A PEPTIDE REPRESENTATION OF THE PEPTIDE SEQUENCE USING A FIRST ATTENTION BLOCK AND PROCESSING AN IPC REPRESENTATION OF THE IPC SEQUENCE USING A SECOND ATTENTION BLOCK TO GENERATE A TRANSFORMED PEPTIDE REPRESENTATION AND A TRANSFORMED IPC REPRESENTATION, RESPECTIVELY
908
GENERATE, FOR EACH PEPTIDE-IPC COMBINATION, A COMPOSITE REPRESENTATION USING THE TRANSFORMED PEPTIDE REPRESENTATION AND THE TRANSFORMED IPC REPRESENTATION
910
PROCESS, FOR EACH PEPTIDE-IPC COMBINATION, THE COMPOSITE REPRESENTATION USING A THIRD ATTENTION BLOCK TO GENERATE A TRANSFORMED COMPOSITE REPRESENTATION
912
GENERATE AN OUTPUT BASED ON THE TRANSFORMED COMPOSITE REPRESENTATION
914

**Figure 9**

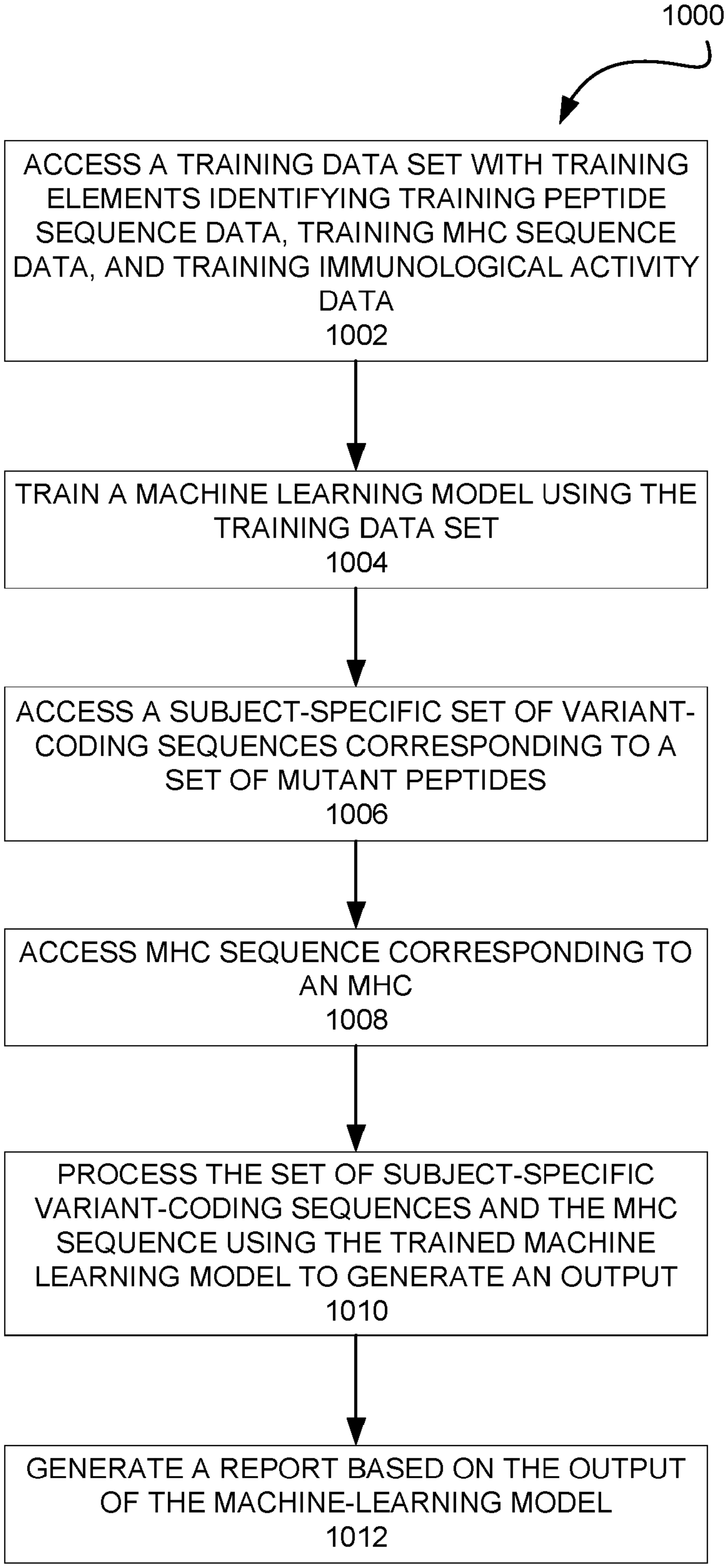
1000
ACCESS A TRAINING DATA SET WITH TRAINING ELEMENTS IDENTIFYING TRAINING PEPTIDE SEQUENCE DATA, TRAINING MHC SEQUENCE DATA, AND TRAINING IMMUNOLOGICAL ACTIVITY DATA
1002
TRAIN A MACHINE LEARNING MODEL USING THE TRAINING DATA SET
1004
ACCESS A SUBJECT-SPECIFIC SET OF VARIANT-CODING SEQUENCES CORRESPONDING TO A SET OF MUTANT PEPTIDES
1006
ACCESS MHC SEQUENCE CORRESPONDING TO AN MHC
1008
PROCESS THE SET OF SUBJECT-SPECIFIC VARIANT-CODING SEQUENCES AND THE MHC SEQUENCE USING THE TRAINED MACHINE LEARNING MODEL TO GENERATE AN OUTPUT
1010
GENERATE A REPORT BASED ON THE OUTPUT OF THE MACHINE-LEARNING MODEL
1012

**Figure 10**

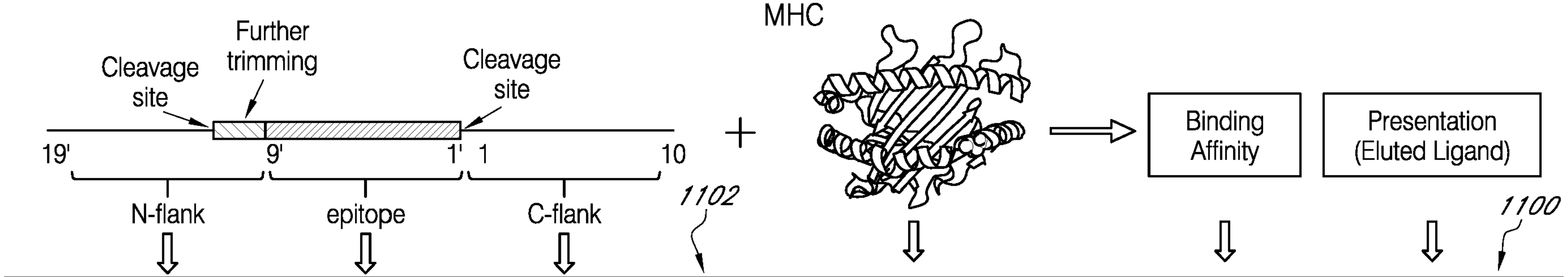


| allele | nFlank | peptide | cFlank | pseudosequence | BA | EL |
|---|---|---|---|---|---|---|
| A*02:01 | | PVILSKLML | | YFAMYGEKVAHTHVDTLYVRYHYYTWAVLAYTWY | 0.82329626 | |
| B*73:01 | EANLSEQQIV | SNTLVRALMT | AVCYSAIIFE | YHTEYRNICAKTDVGNLYWTYNFYTWAVLAYEWH | | 0 |
| B*57:01 | MVA | RVWSLMRFL | IKGSVAGGAV | YYAMYGENMASTYENIAYIVYDSYTWAVLAYLWY | | 1 |
| C*15:02 | GLSHQYPGPY | FLACLLFWP | ENQELDQDSK | YYAGYRENYRQTDVNKLYIRYDLYTWAELAYTWY | | 0 |
| B*15:01 | VALAGRLREL | LRDLPPENR | ASLQYLLRHL | YYAMYREISTNTYESNLYLRYDSYTWAEWAYLWY | | 0 |
| B*44:02 | RNDAVILPSC | VEAEAKIHET | QETTDKSADD | YYTKYREISTNTYENTAYIRYDDYTWAVDAYLSY | | 0 |

1106 1108 1110 1112 1114 1116 1118

FIG. 11

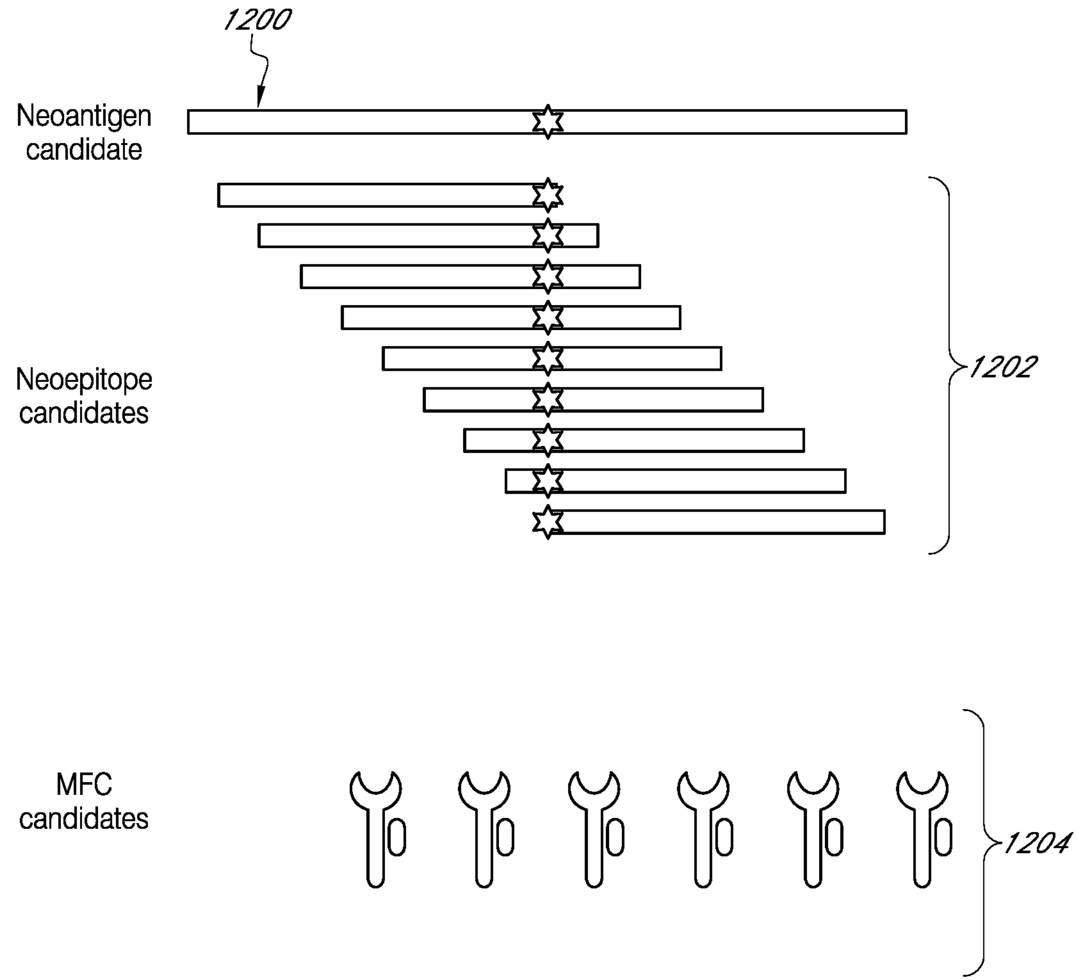
1200
Neoantigen candidate
Neoepitope candidates
1202
MFC candidates
1204

FIG. 12

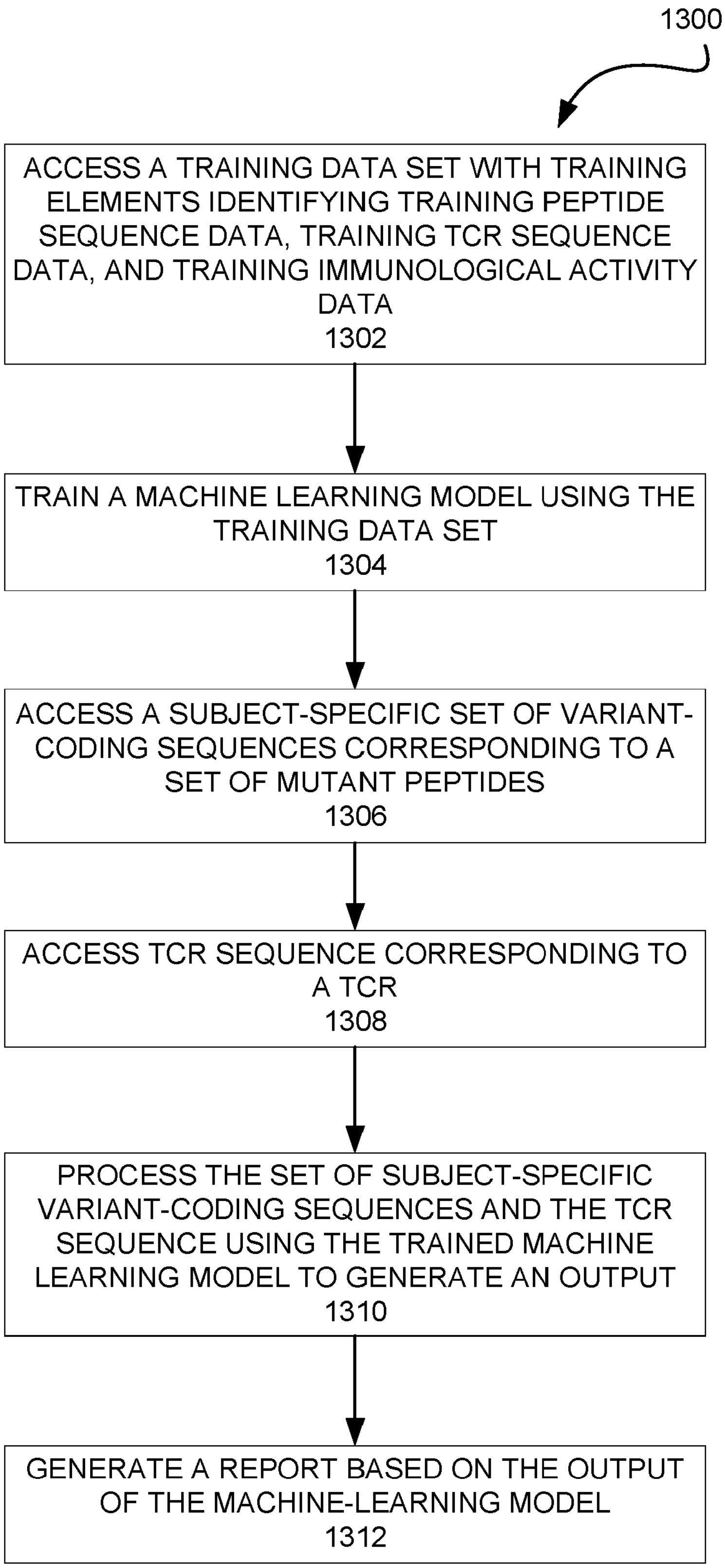
1300
ACCESS A TRAINING DATA SET WITH TRAINING ELEMENTS IDENTIFYING TRAINING PEPTIDE SEQUENCE DATA, TRAINING TCR SEQUENCE DATA, AND TRAINING IMMUNOLOGICAL ACTIVITY DATA
1302
TRAIN A MACHINE LEARNING MODEL USING THE TRAINING DATA SET
1304
ACCESS A SUBJECT-SPECIFIC SET OF VARIANT-CODING SEQUENCES CORRESPONDING TO A SET OF MUTANT PEPTIDES
1306
ACCESS TCR SEQUENCE CORRESPONDING TO A TCR
1308
PROCESS THE SET OF SUBJECT-SPECIFIC VARIANT-CODING SEQUENCES AND THE TCR SEQUENCE USING THE TRAINED MACHINE LEARNING MODEL TO GENERATE AN OUTPUT
1310
GENERATE A REPORT BASED ON THE OUTPUT OF THE MACHINE-LEARNING MODEL
1312

Figure 13

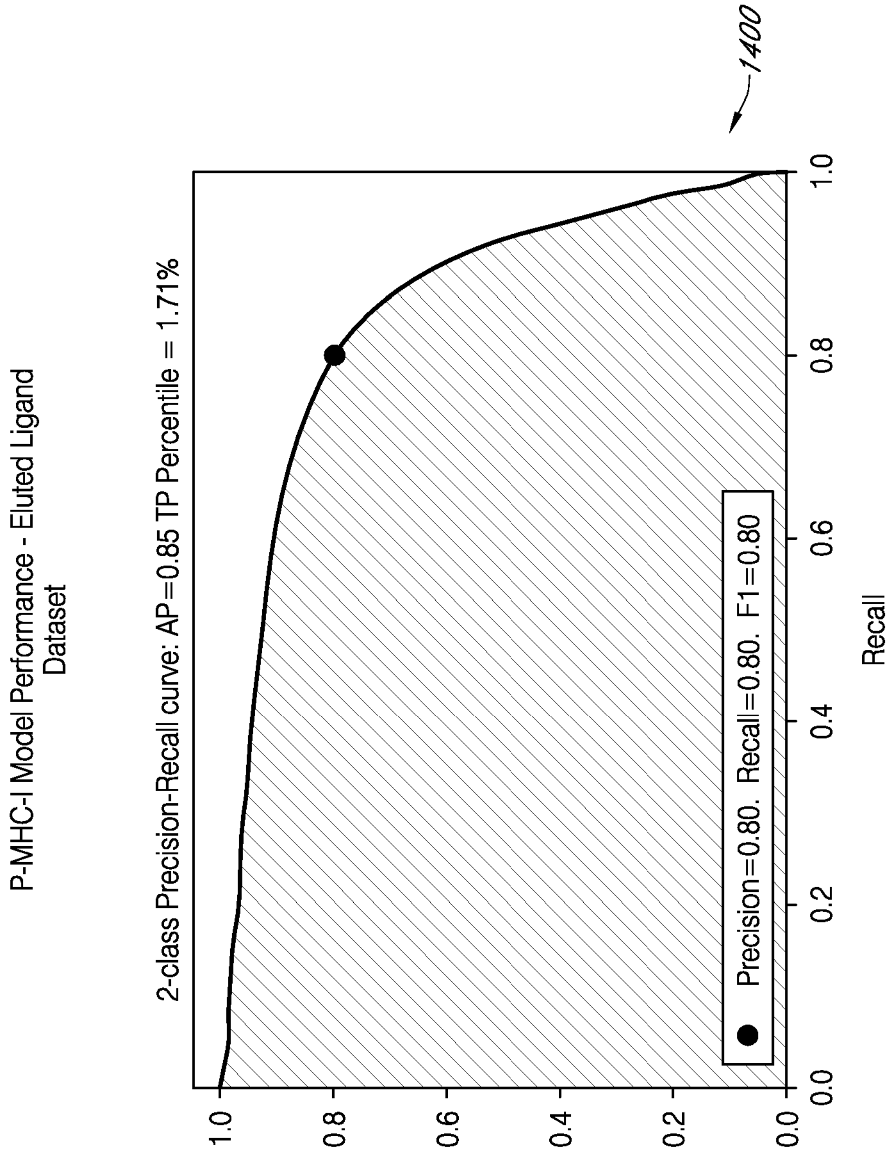
P-MHC-I Model Performance - Eluted Ligand Dataset
2-class Precision-Recall curve: AP=0.85 TP Percentile = 1.71%
Precision
1.0
0.8
0.6
0.4
0.2
0.0
Recall
0.0
0.2
0.4
0.6
0.8
1.0
Precision=0.80. Recall=0.80. F1=0.80
1400

FIG. 14A

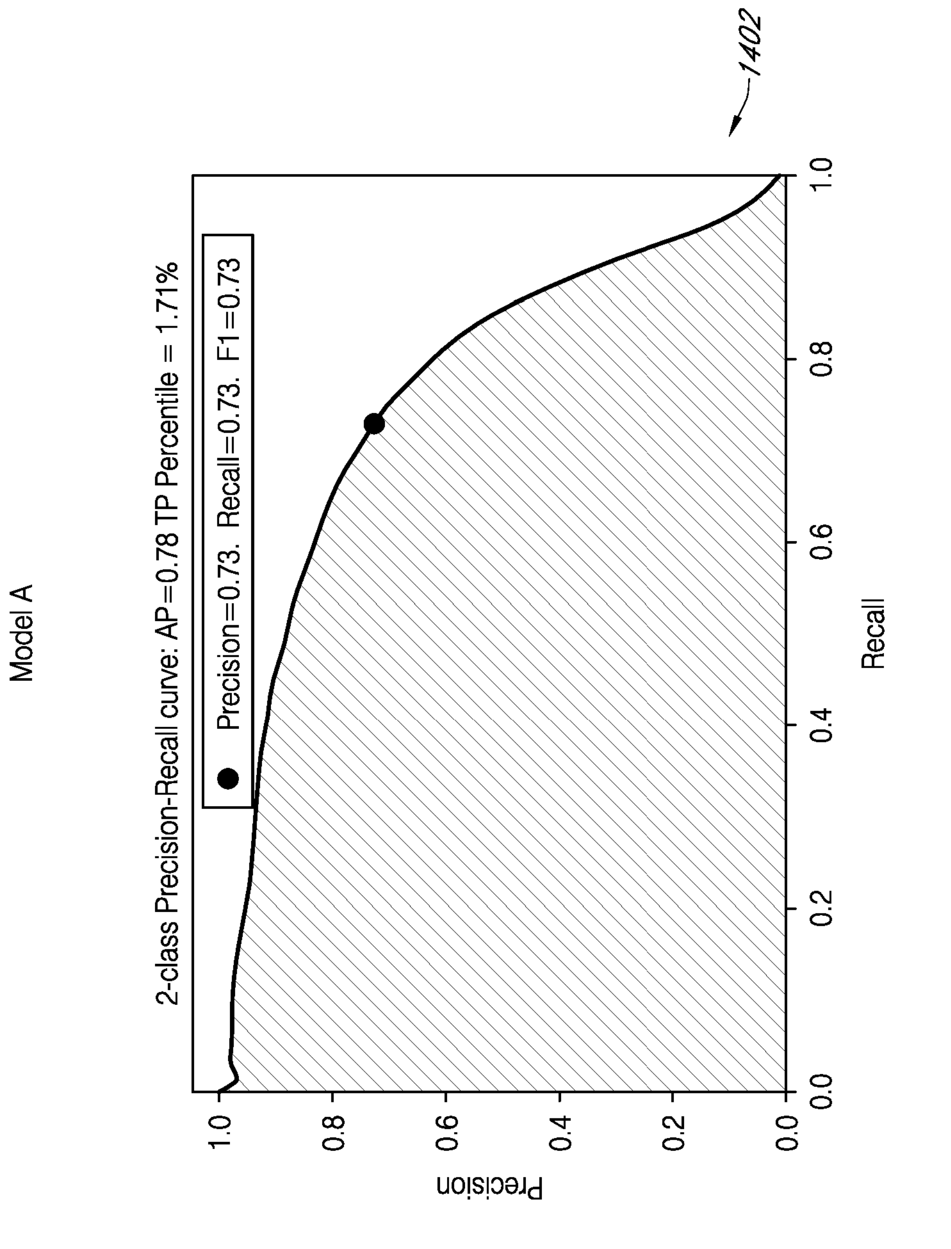
Model A
2-class Precision-Recall curve: AP=0.78 TP Percentile = 1.71%
Precision=0.73. Recall=0.73. F1=0.73
Precision
1.0
0.8
0.6
0.4
0.2
0.0
Recall
0.0
0.2
0.4
0.6
0.8
1.0
1402

FIG. 14B

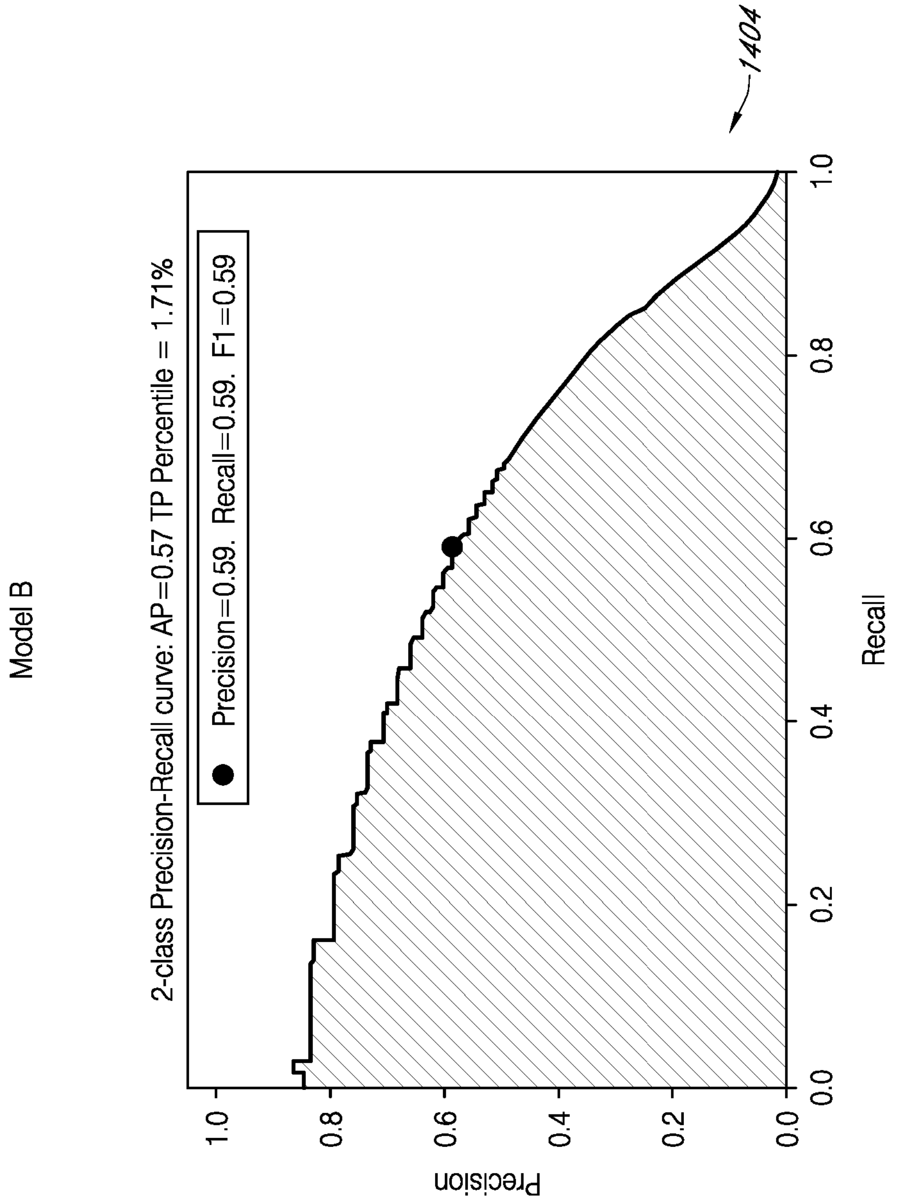
Model B
2-class Precision-Recall curve: AP=0.57 TP Percentile = 1.71%
Precision=0.59. Recall=0.59. F1=0.59
Precision
1.0
0.8
0.6
0.4
0.2
0.0
Recall
0.0
0.2
0.4
0.6
0.8
1.0
1404

FIG. 14C

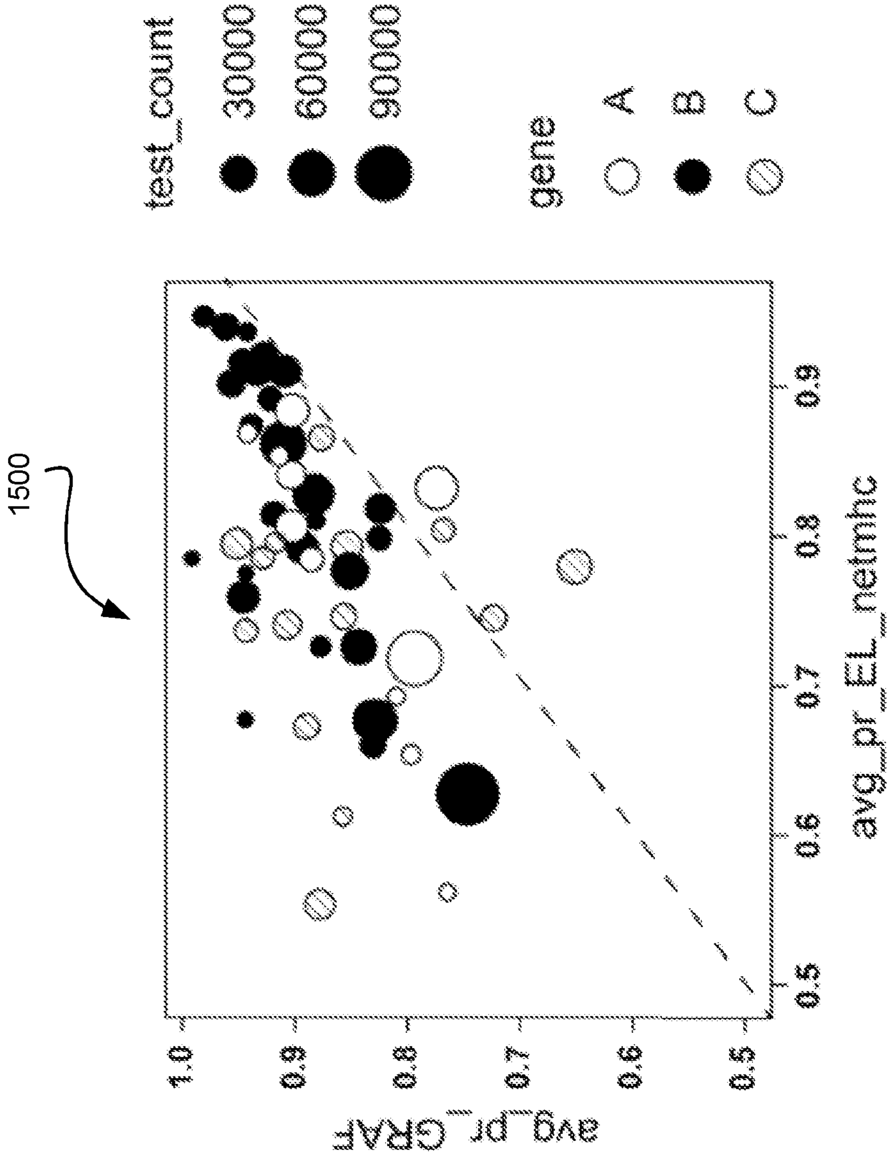
1500
avg_pr_GRAF
1.0
0.9
0.8
0.7
0.6
0.5
avg_pr_EL_netmhc
0.5
0.6
0.7
0.8
0.9
test_count
30000
60000
90000
gene
A
B
C

Figure 15

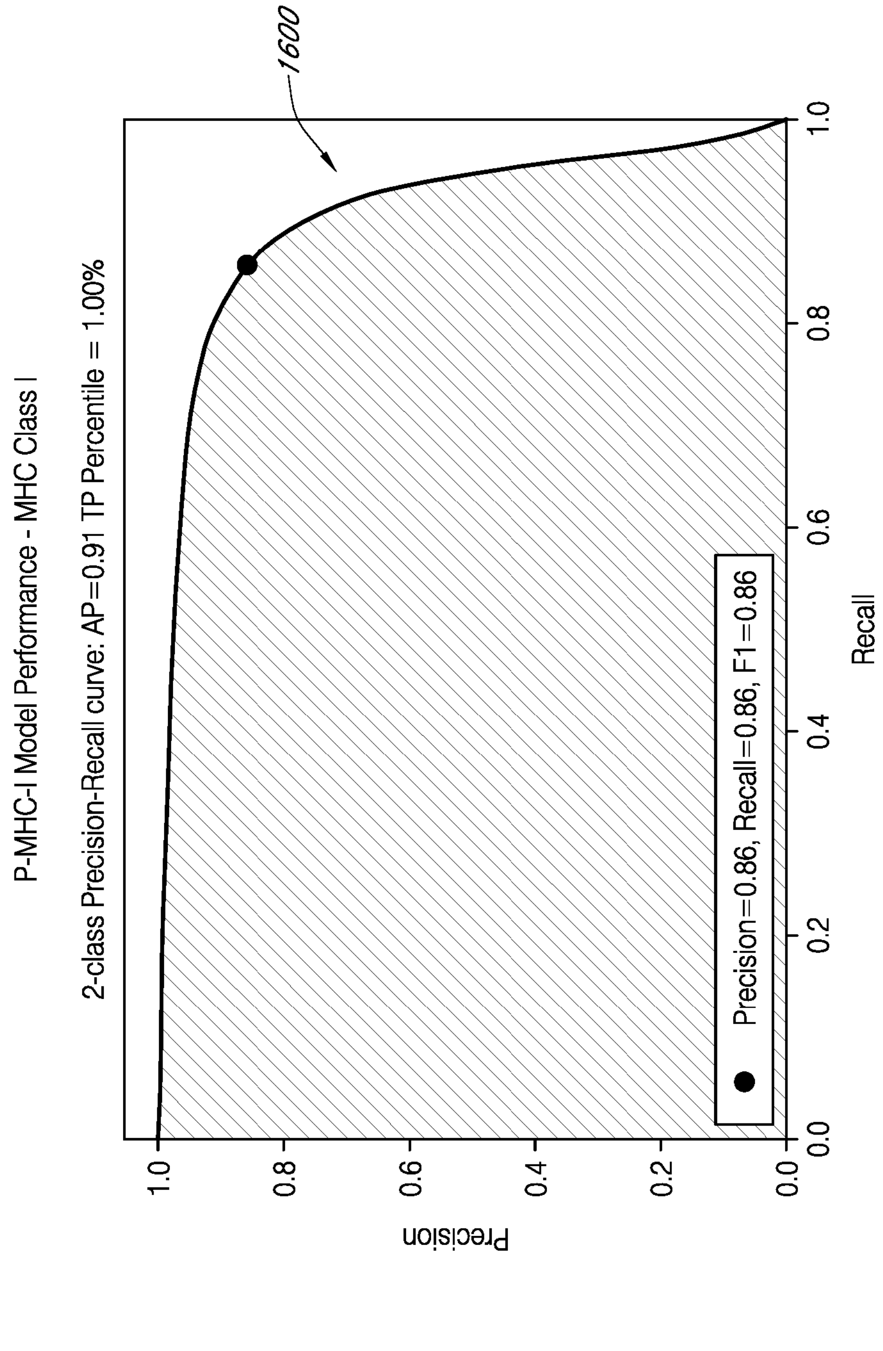
P-MHC-I Model Performance - MHC Class I
2-class Precision-Recall curve: AP=0.91 TP Percentile = 1.00%
1600
Precision=0.86, Recall=0.86, F1=0.86
Precision
1.0
0.8
0.6
0.4
0.2
0.0
Recall
0.0
0.2
0.4
0.6
0.8
1.0

FIG. 16A

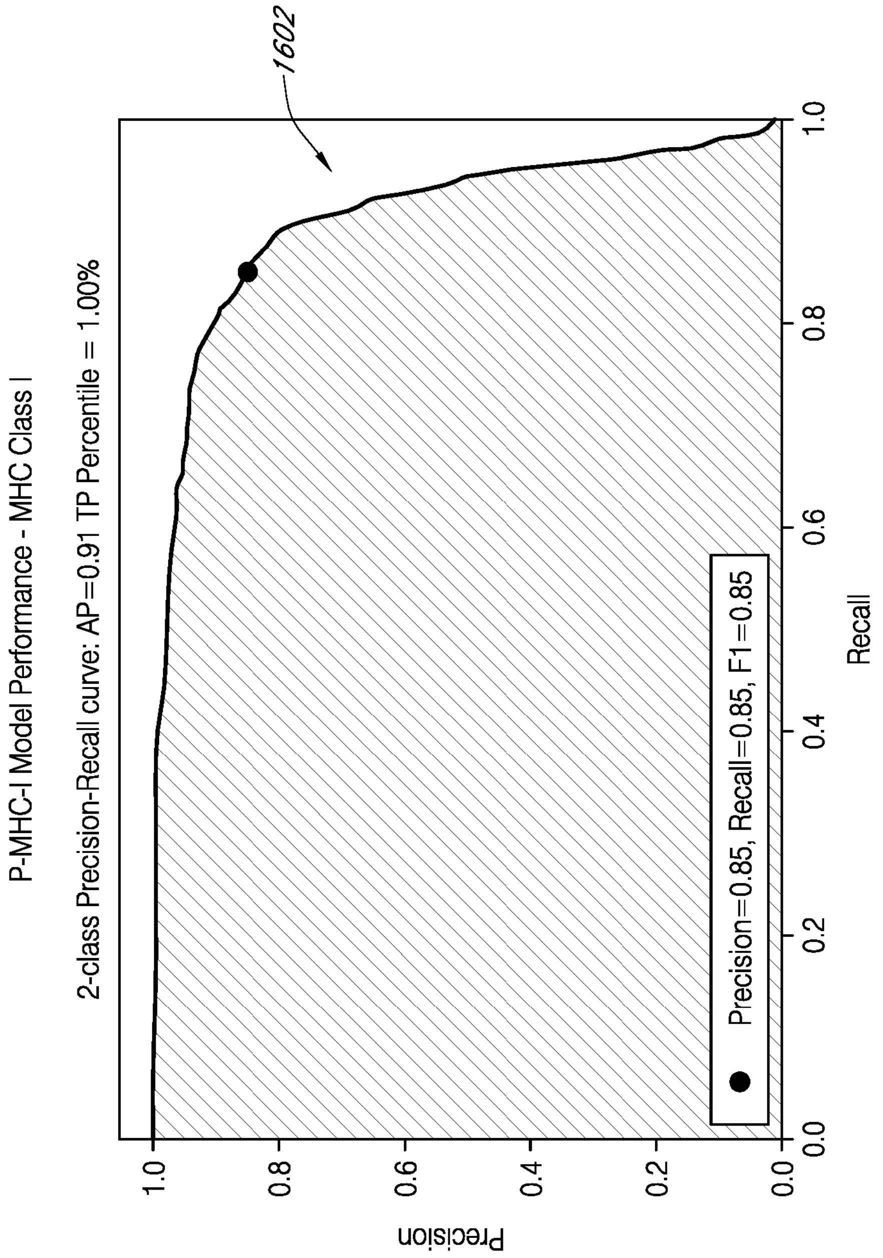
P-MHC-I Model Performance - MHC Class I
2-class Precision-Recall curve: AP=0.91 TP Percentile = 1.00%
1602
Precision
1.0
0.8
0.6
0.4
0.2
0.0
Recall
0.0
0.2
0.4
0.6
0.8
1.0
Precision=0.85, Recall=0.85, F1=0.85

FIG. 16B

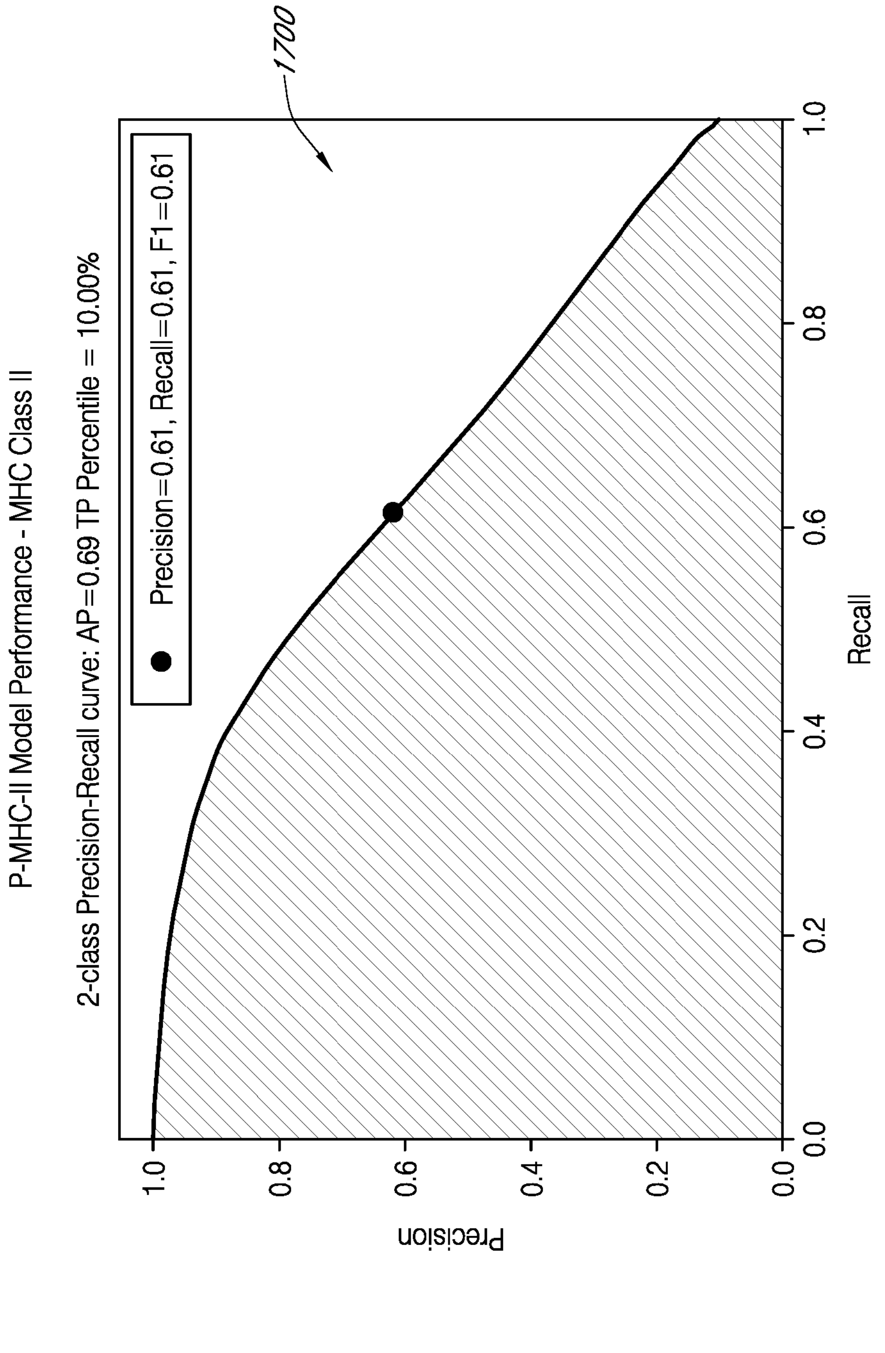
P-MHC-II Model Performance - MHC Class II
2-class Precision-Recall curve: AP=0.69 TP Percentile = 10.00%
Precision=0.61, Recall=0.61, F1=0.61
1700
Precision
1.0
0.8
0.6
0.4
0.2
0.0
Recall
0.0
0.2
0.4
0.6
0.8
1.0

FIG. 17A

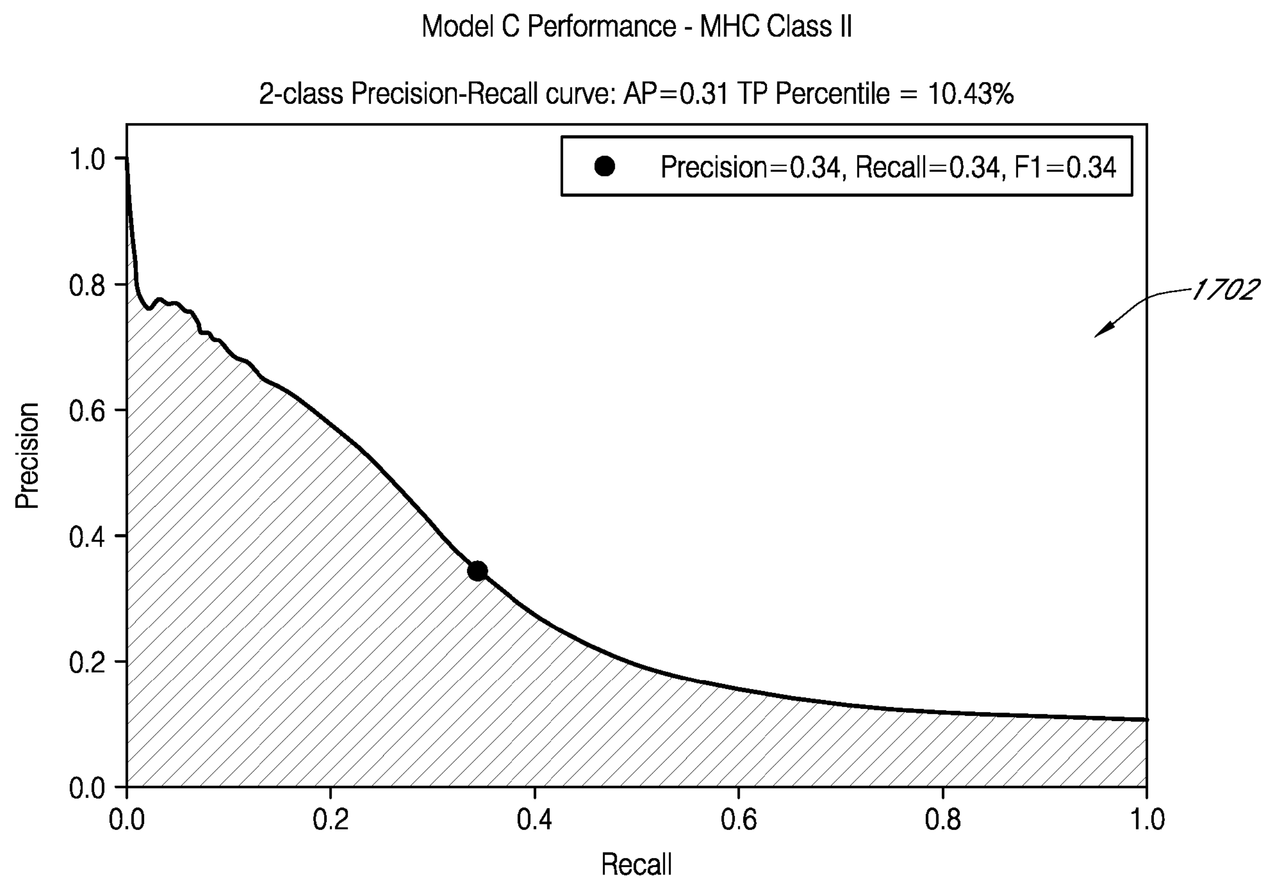
Model C Performance - MHC Class II
2-class Precision-Recall curve: AP=0.31 TP Percentile = 10.43%
Precision=0.34, Recall=0.34, F1=0.34
1702
Precision
1.0
0.8
0.6
0.4
0.2
0.0
0.0
0.2
0.4
0.6
0.8
1.0
Recall

FIG. 17B

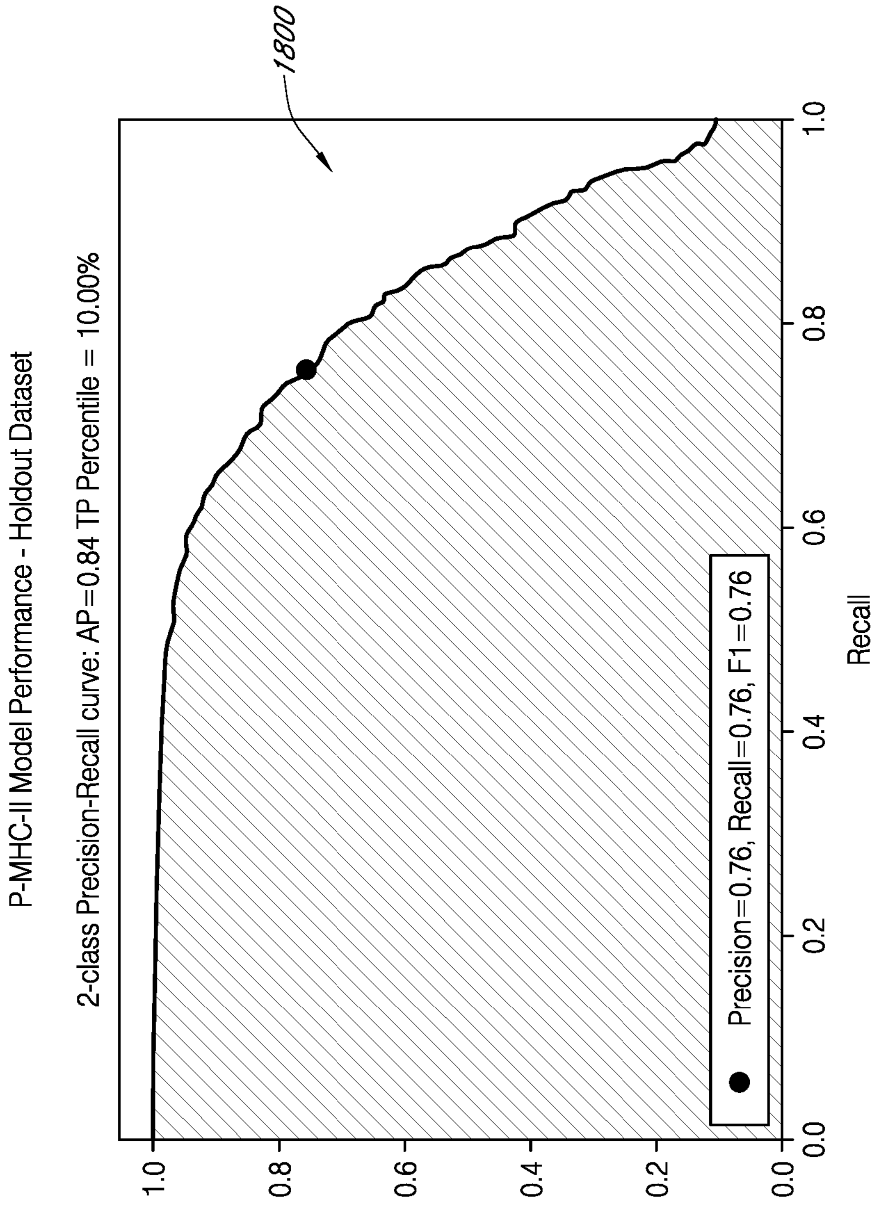
P-MHC-II Model Performance - Holdout Dataset
2-class Precision-Recall curve: AP=0.84 TP Percentile = 10.00%
1800
Precision
1.0
0.8
0.6
0.4
0.2
0.0
Recall
0.0
0.2
0.4
0.6
0.8
1.0
Precision=0.76, Recall=0.76, F1=0.76

FIG. 18A

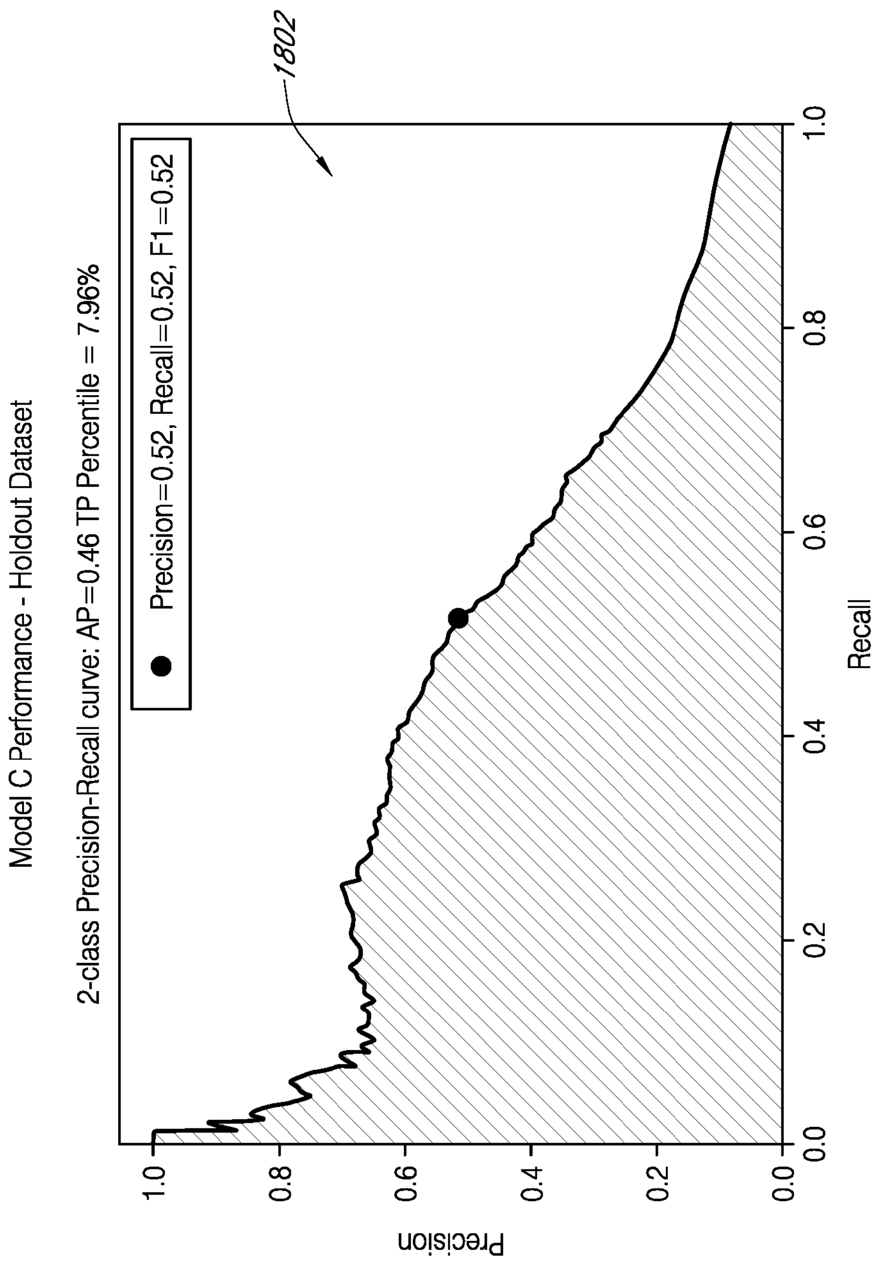
Model C Performance - Holdout Dataset
2-class Precision-Recall curve: AP=0.46 TP Percentile = 7.96%
Precision=0.52, Recall=0.52, F1=0.52
1802
Precision
1.0
0.8
0.6
0.4
0.2
0.0
Recall
0.0
0.2
0.4
0.6
0.8
1.0

FIG. 18B

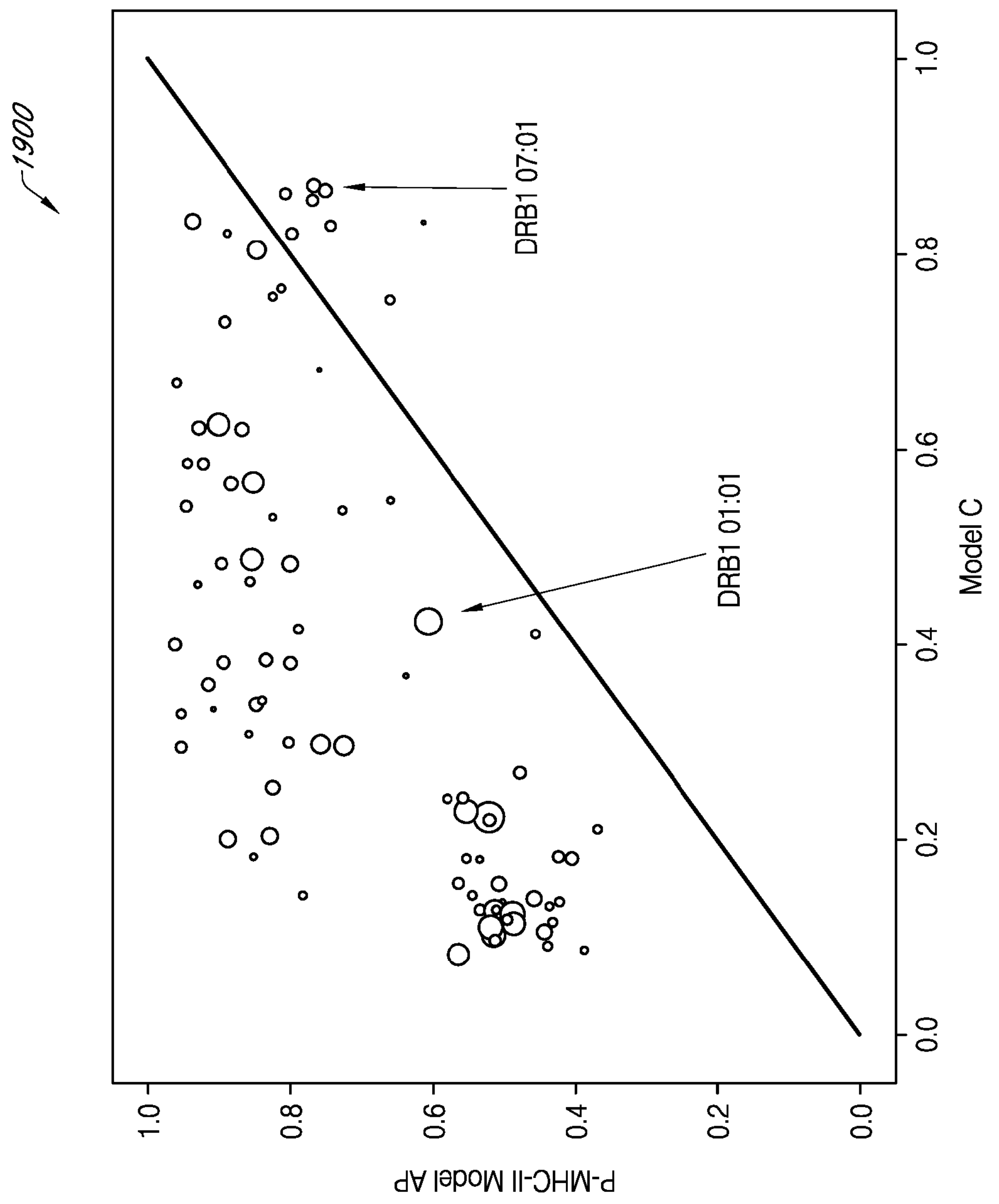
1900
DRB1 07:01
DRB1 01:01
P-MHC-II Model AP
Model C
0.0
0.2
0.4
0.6
0.8
1.0

FIG. 19

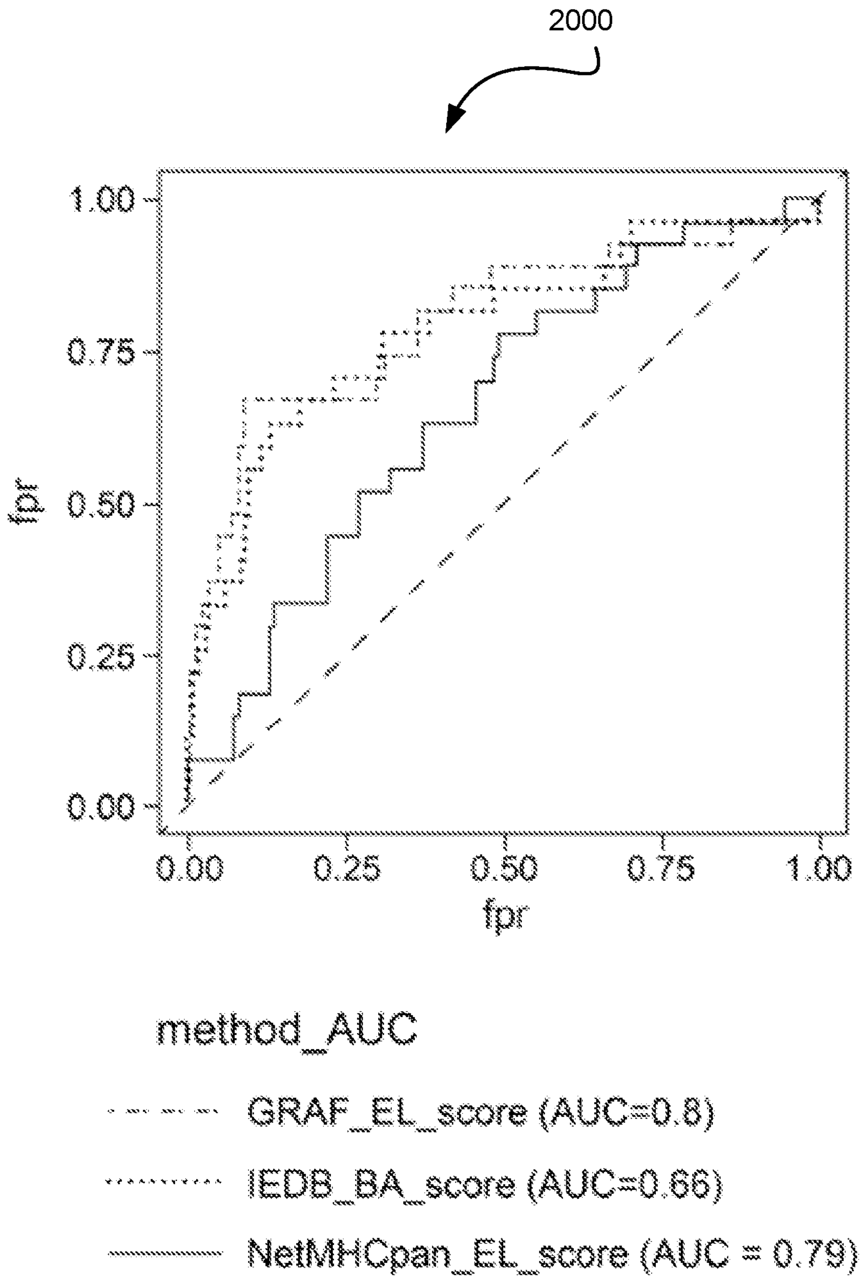
2000
1.00
0.75
0.50
0.25
0.00
fpr
0.00
0.25
0.50
0.75
1.00
fpr
method_AUC
GRAF_EL_score (AUC=0.8)
IEDB_BA_score (AUC=0.66)
NetMHCpan_EL_score (AUC = 0.79)

**Figure 20**

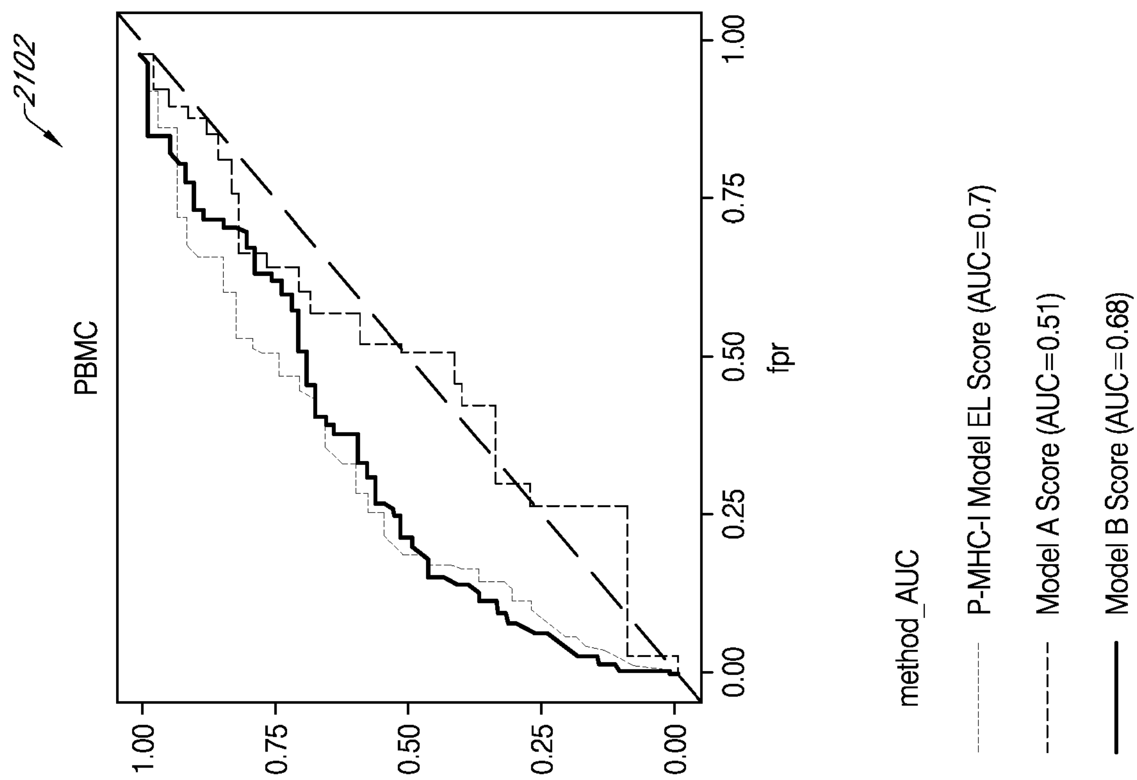
2102
PBMC
1.00
0.75
0.50
0.25
0.00
0.00
0.25
0.50
0.75
1.00
fpr
method_AUC
P-MHC-I Model EL Score (AUC=0.7)
Model A Score (AUC=0.51)
Model B Score (AUC=0.68)

FIG. 21A

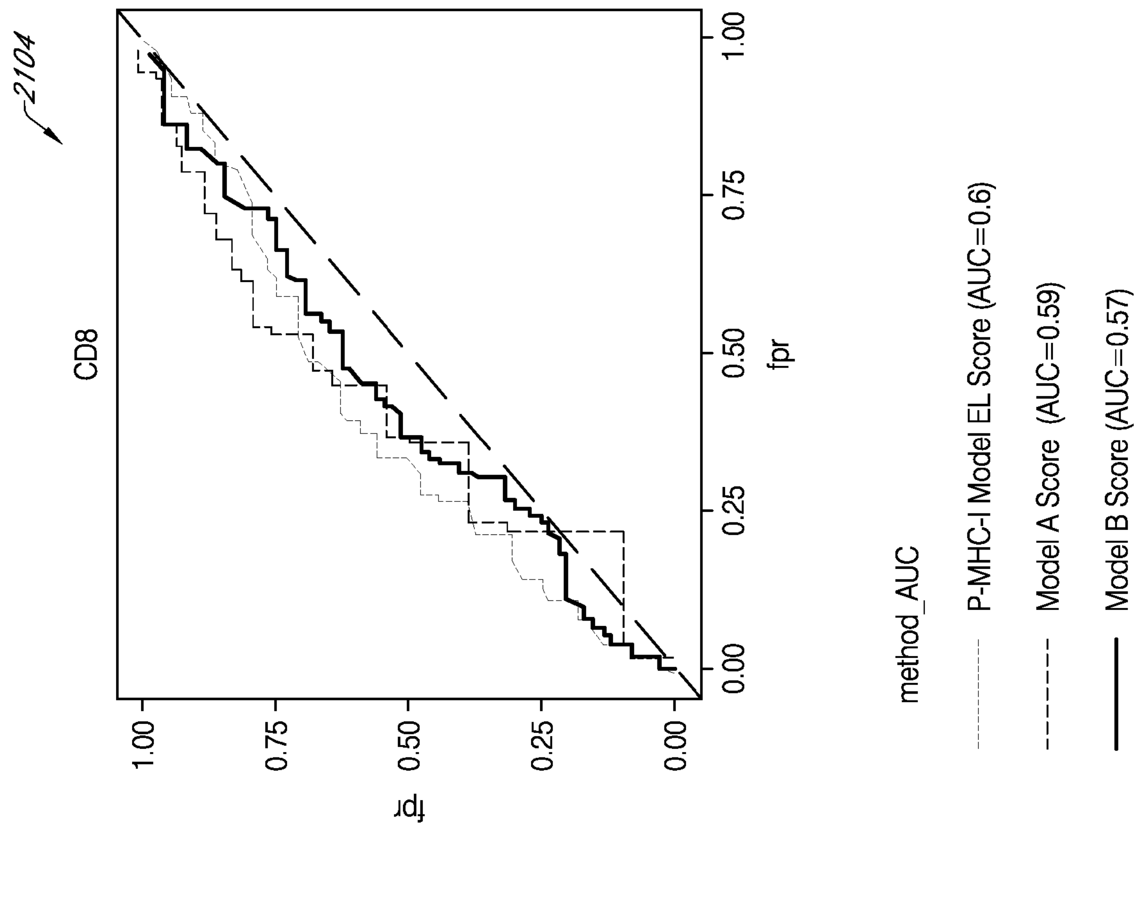
2104
CD8
fpr
fpr
0.00
0.25
0.50
0.75
1.00
method_AUC
P-MHC-I Model EL Score (AUC=0.6)
Model A Score (AUC=0.59)
Model B Score (AUC=0.57)

FIG. 21B

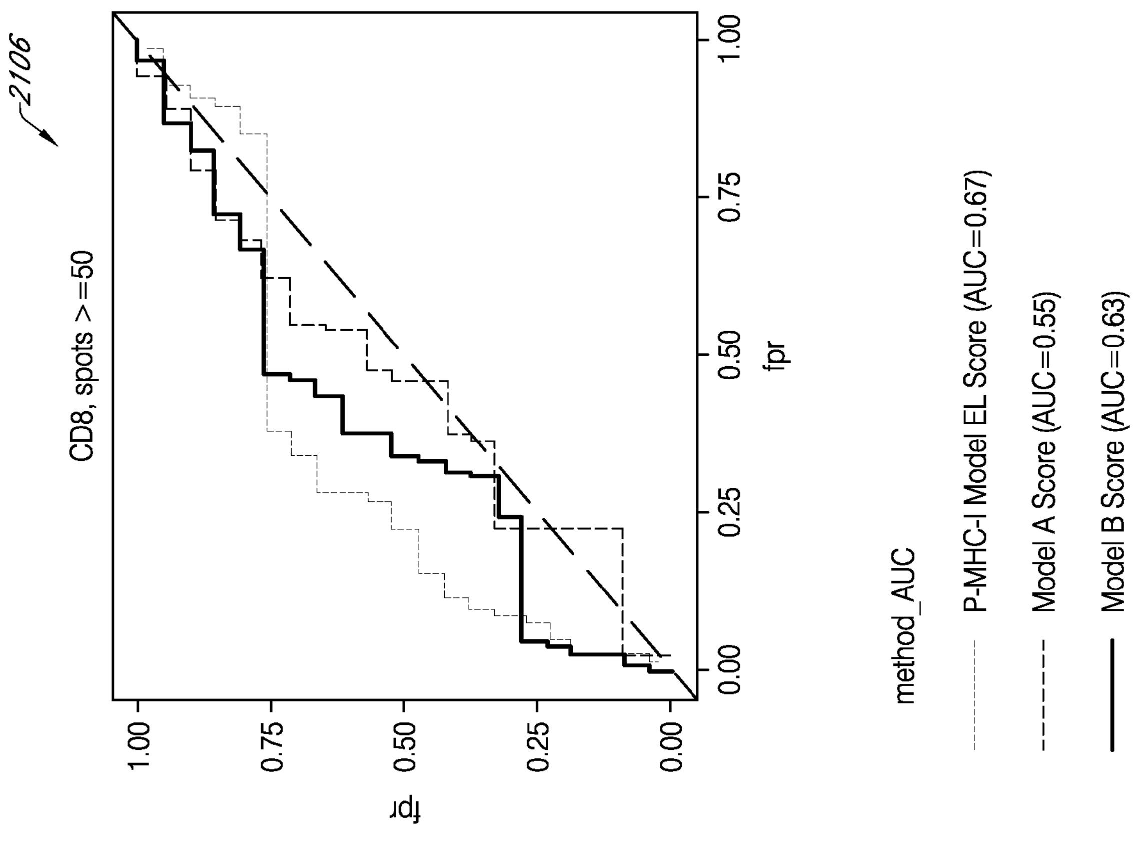
2106
CD8, spots >=50
tpr
1.00
0.75
0.50
0.25
0.00
fpr
0.00
0.25
0.50
0.75
1.00
method_AUC
P-MHC-I Model EL Score (AUC=0.67)
Model A Score (AUC=0.55)
Model B Score (AUC=0.63)

FIG. 21C

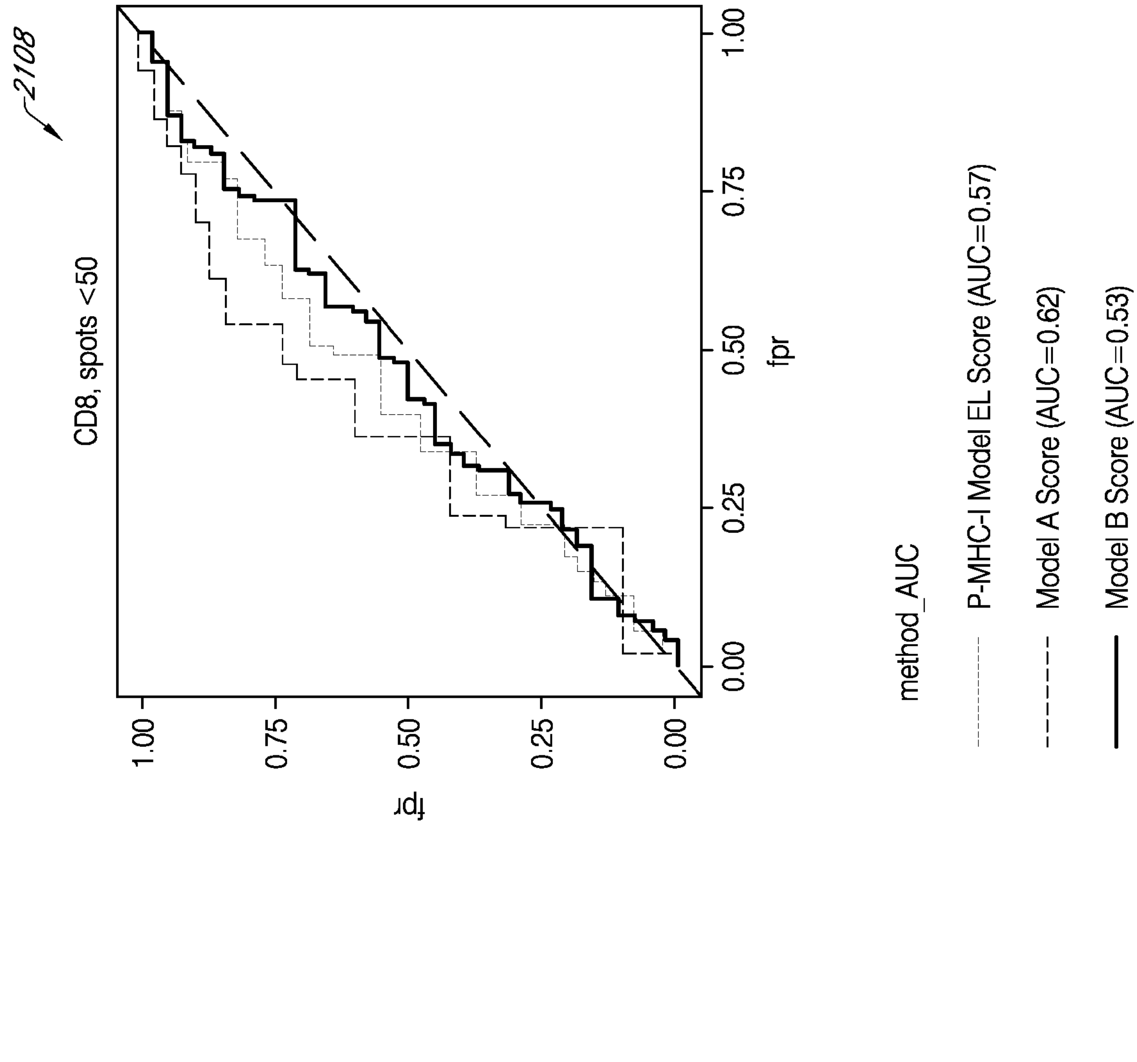
2108
CD8, spots <50
1.00
0.75
0.50
0.25
0.00
tpr
fpr
method_AUC
P-MHC-I Model EL Score (AUC=0.57)
Model A Score (AUC=0.62)
Model B Score (AUC=0.53)

FIG. 21D

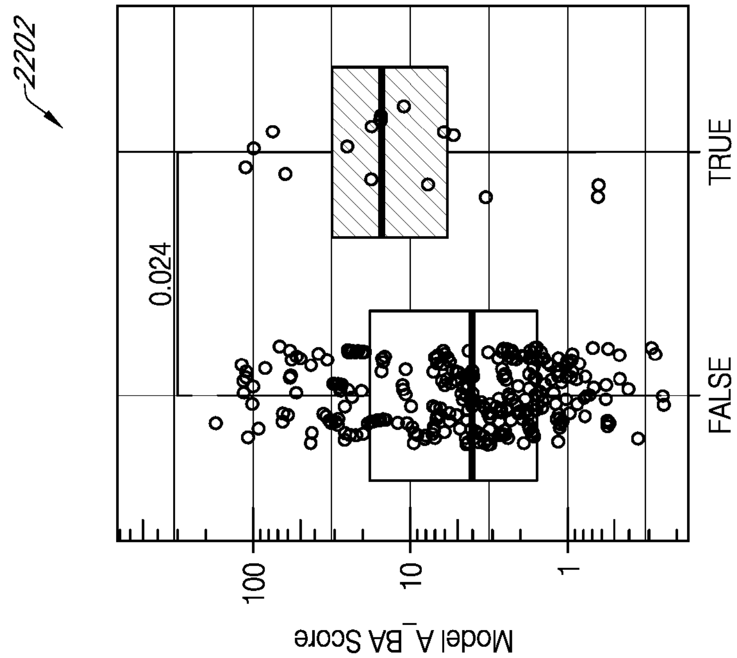


FIG. 22A

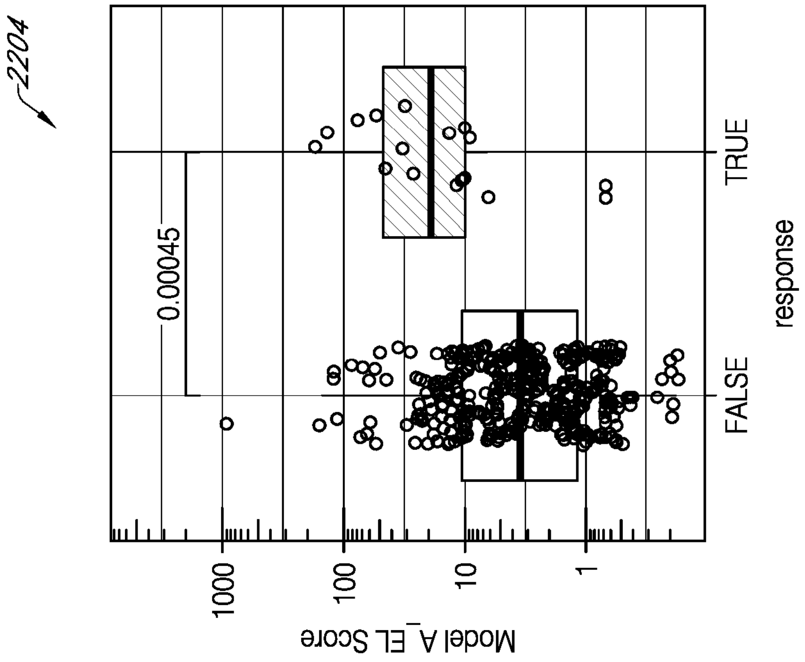
2204
Model A_EL Score
1000
100
10
1
0.00045
FALSE
TRUE
response

FIG. 22B

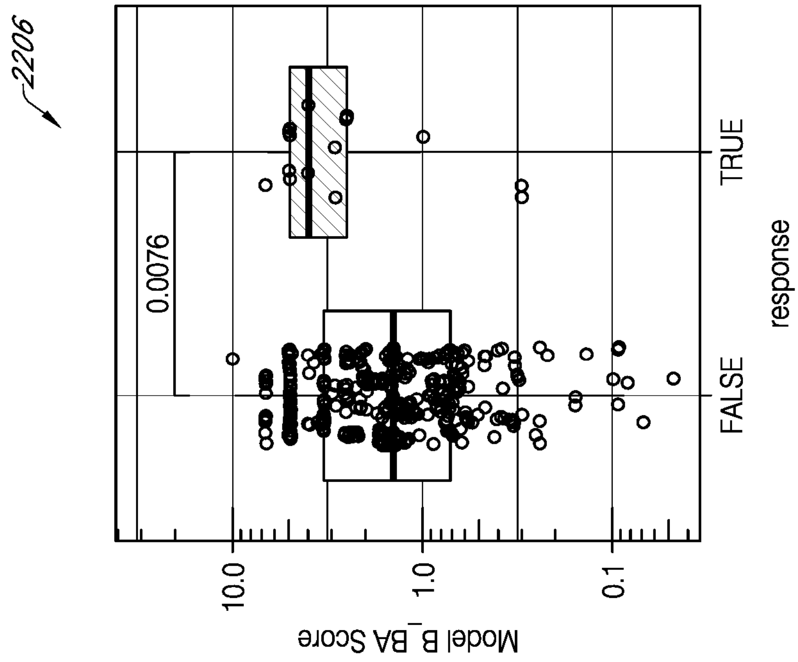


FIG. 22C

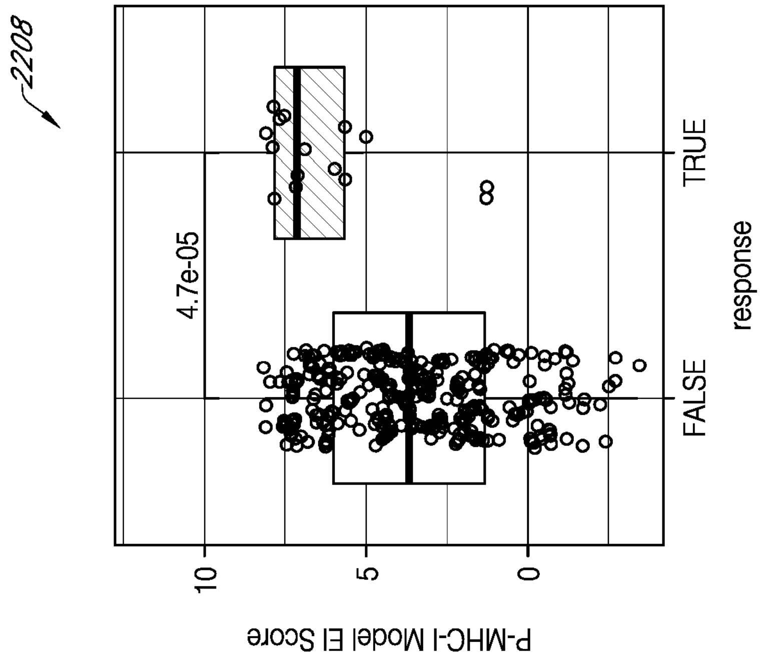


FIG. 22D

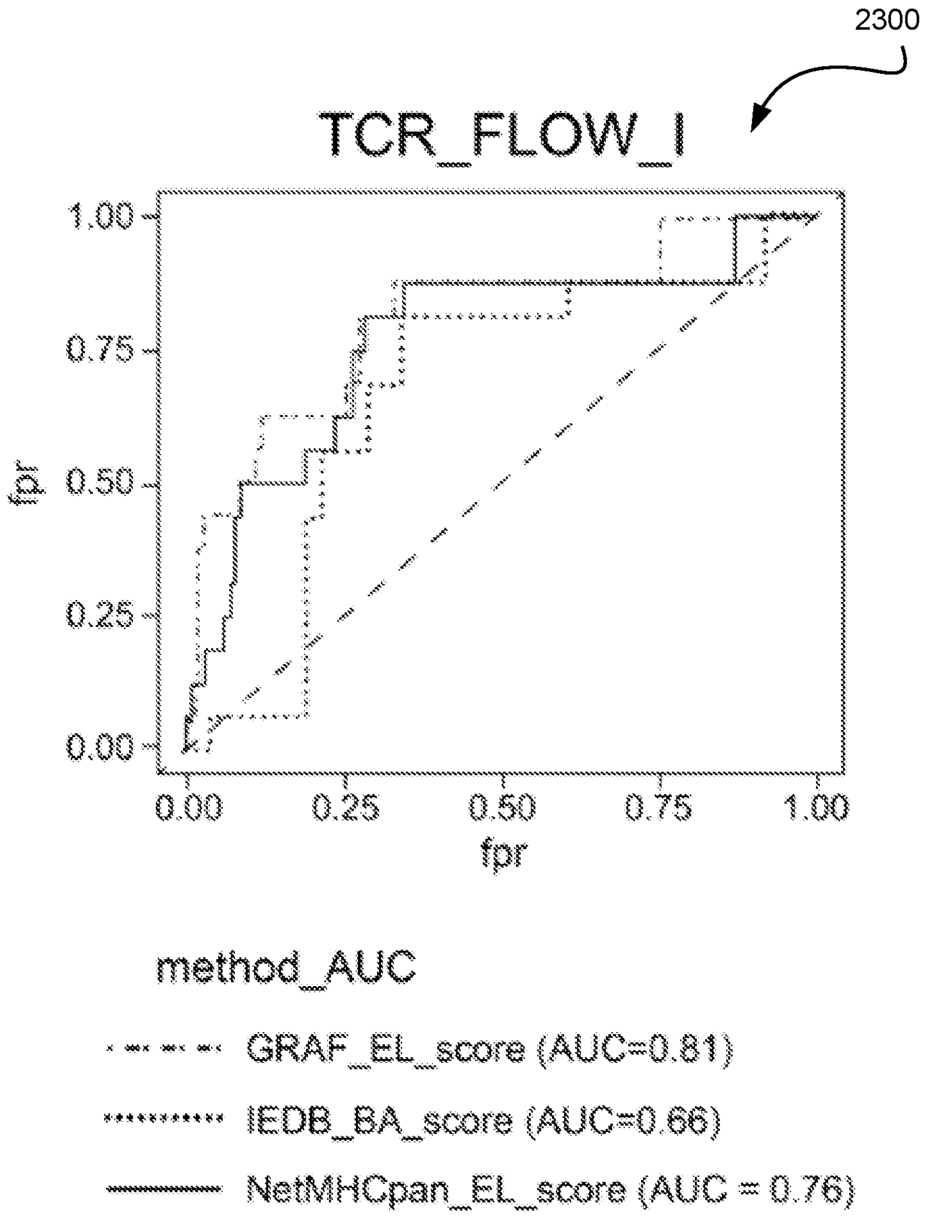
2300
TCR_FLOW_I
1.00
0.75
0.50
0.25
0.00
fpr
0.00
0.25
0.50
0.75
1.00
fpr
method_AUC
GRAF_EL_score (AUC=0.81)
IEDB_BA_score (AUC=0.66)
NetMHCpan_EL_score (AUC = 0.76)

**Figure 23**

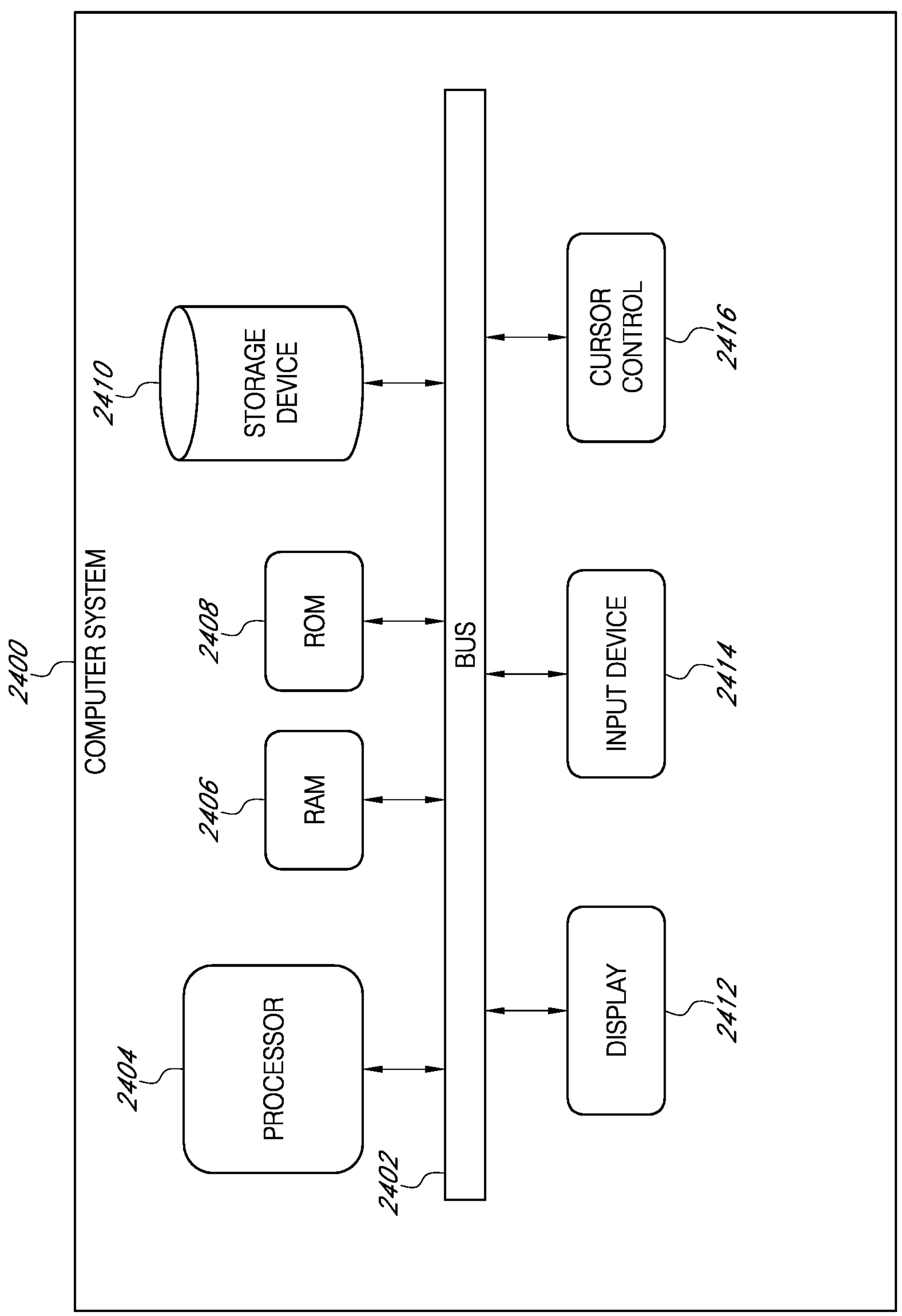
2400
COMPUTER SYSTEM
2404
PROCESSOR
2406
RAM
2408
ROM
2410
STORAGE DEVICE
BUS
2402
DISPLAY
2412
INPUT DEVICE
2414
CURSOR CONTROL
2416

FIG. 24

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 63053307
- WO 2005038030 A1
- WO 2007036366 A2
- WO 2017060314 A3
- WO 2011015347 A1
- WO 2013143683 A1

### Non-patent literature cited in the description


- **VASWANI, A et al.** Attention is All You Need. *31st Conference on Neural Information Systems*, 2017, http://papers.nips.cc/paper/7181-attention-is-all-you-need.pdf
- **XU et al.** A review of somatic single nucleotide variant calling algorithms for next-generation sequencing data. *Comput. Struct. Biotechnol. J.*, 2018, vol. 16, 15-24
- **TANG, F. et al.** mRNA-Seq whole-transcriptome analysis of a single cell. *Nature Methods*, 2009, vol. 6, 377-382
- **OZSOLAK, F.** RNA sequencing: advances, challenges and opportunities. *Nature Reviews*, 2011, vol. 12, 87-98
- **GERMAN, M. A et al.** Global identification of microRNA-target RNA pairs by parallel analysis of RNA ends. *Nature Biotechnology*, 2008, vol. 26, 941-946
- **WANG, Z. et al.** RNA-Seq: a revolutionary tool for transcriptomics. *Nature Reviews*, 2009, vol. 10, 57-63
- **DE SIMONE et al.** Single Cell T Cell Receptor Sequencing: Techniques and Future Challenges. *Front. Immunol.*, 2018, vol. 9, 1638
- **ZONG et al.** Very rapid cloning, expression and identifying specificity of T-cell receptors for T-cell engineering. *PloS ONE*, 2020, vol. 15 (2), e0228112
- **WANG et al.** High-throughput sequence of CD4+T cell repertoire reveals disease-specific signatures in IgG4-related disease. *Arthritis Research & Therapy*, 2019, vol. 21, 295
- **CARON et al.** Analysis of Major Histocompatibility Complex (MHC) Immunopeptides Using Mass Spectroscopy. *Molecular and Cellular Proteomics*, 2015, vol. 14 (12), 3105-3117
- **WITTMAN et al.** *J. of Immunol.*, 2006, vol. 177, 4187-4195
- **MATSUDA et al.** Induction of Neoantigen-Specific Cytotoxic T Cells and Construction of T-cell Receptor Engineered T Cells for Ovarian Cancer. *Clin. Cancer Res.*, 2018, 1-11